# EUROPEAN PATENT APPLICATION

(11) **EP 4 831 186 A1**
(43) Date of publication of application: **28.10.2026**
(21) Application number: 24906391.8
(22) Date of filing: 18.12.2024
(51) Int. Cl.: C07F 9/6561, C07D 498/16, A61K 31/553, A61K 31/554, A61P 35/00

(54) **FUSED TRICYCLIC COMPOUND AND MEDICAL USE THEREOF**

(30) Priority: 19.12.2023 CN 202311755340; 04.02.2024 CN 202410160703; 12.12.2024 CN 202411837405
(71) Applicant: Chia Tai Tianqing Pharmaceutical Group Co., Ltd., Lianyungang, Jiangsu 222062 (CN)
(72) Inventor: LIU, Fei, Lianyungang, Jiangsu 222062 (CN); ZHU, Yizhong, Lianyungang, Jiangsu 222062 (CN); WANG, Bin, Lianyungang, Jiangsu 222062 (CN); ZHANG, Xiquan, Lianyungang, Jiangsu 222062 (CN); XU, Hongjiang, Lianyungang, Jiangsu 222062 (CN); WANG, Jinan, Lianyungang, Jiangsu 222062 (CN)
(74) Representative: v. Bezold & Partner Patentanwälte - PartG mbB
(86) International application number: PCT/CN2024/140262
(87) International publication number: WO 2025/130912

(57) **Abstract**

The present application belongs to the field of medical chemistry, and relates to a fused tricyclic compound and the medical use thereof. The compound has a structure as represented by formula (I). Particularly, the present application further relates to a method for preparing the compound, a pharmaceutical composition of the compound and the use of the compound in the treatment of diseases.

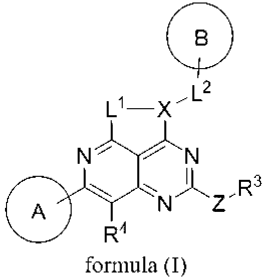

## Description

### CROSS-REFERENCE TO RELATED APPLICATIONS

The present application claims priority to and benefit of the Chinese Patent Application No. 202311755340.0 filed with China National Intellectual Property Administration on December 19, 2023, the Chinese Patent Application No. 202410160703.4 filed with China National Intellectual Property Administration on February 04, 2024, and the Chinese Patent Application No. 202411837405.0 filed with China National Intellectual Property Administration on December 12, 2024, the disclosure of each of which is incorporated herein by reference in its entirety.

### TECHNICAL FIELD

The present disclosure relates to a tricyclic compound, a preparation method therefor, a pharmaceutical composition comprising the compound, and use thereof for treating cancer.

### BACKGROUND

The *Ras* gene is an important proto-oncogene, named after its discovery in the rat sarcoma virus. The Ras protein it encodes is localized on the inner side of the cell membrane. This protein has the ability to bind to GTP/GDP and, with the assistance of GTPase activating protein (GAP), can hydrolyze GTP. By interconverting between its active (GTP-bound) and inactive (GDP-bound) conformations, the Ras protein controls the "on" and "off" states in signal transduction processes involving growth factors and cytokines, playing an important role in cellular processes such as proliferation, differentiation, senescence, and apoptosis (Bos J. L. et al., Cell, 2007, 129(5): 865-877). The human *Ras* gene family has three members: Harvey rat sarcoma viral oncogene homolog (*HRas*), neuroblastoma rat sarcoma viral oncogene homolog (*NRas*), and Kirsten rat sarcoma viral oncogene homolog (*Kras*), with *Kras* being primarily expressed in the intestine, lung, and thymus (Rajalingam K. et al., Biochim Biophys Acta, 2007, 1773(8): 1177-1195).

Studies have shown that *Ras* gene mutations are present in over 30% of human tumors, with *Kras* mutations accounting for about 86% of these cases (Riely G. J. et al., Proc Am Thorac Soc, 2009, 6(2): 201-205). For *Kras* mutations, mutations of glycine at position 12 (G12) account for about 80%, and the G12D mutation (where glycine at position 12 is mutated to aspartic acid) is the primary mutation form of G12 mutations (Prior I. A. et al., Cancer Res, 2012, 72(10): 2457-2467). G12 mutations reduce the catalytic activity of GAP, ultimately leading to the sustained activation of Ras, which makes Ras unable to effectively regulate cell signal transduction, thereby promoting the occurrence and development of tumors.

Currently, mutant Kras has become an attractive anti-cancer target.

### SUMMARY

The present disclosure relates to a compound of formula (I) or a pharmaceutically acceptable salt thereof, 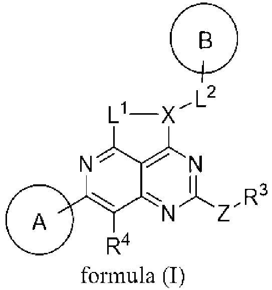 wherein,
X is selected from the group consisting of -N- and -CH- optionally substituted with R^{x}
R^{x} is selected from the group consisting of deuterium, halogen, -OH, -NH₂, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₁₋₄ heteroalkenylene;
each R¹ is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L² is selected from a single bond, and ring B is selected from the group consisting of the following groups optionally substituted with one or more R^{B} or R^{C}: 3- to 12-membered cycloalkenyl, 3- to 12-membered heterocycloalkenyl, 6-to 10-membered aryl, and 5- to 10-membered heteroaryl;
or L² is selected from -C(R^{2a}R^{2b})-, and ring B is selected from the group consisting of the following groups optionally substituted with one or more R^{B} or R^{C}: 3- to 12-membered cycloalkyl, 3- to 12-membered heterocyclyl, 6- to 10-membered aryl, and 5- to 10-membered heteroaryl;
R^{2a} is selected from the group consisting of H, deuterium, halogen, -OH, -NH₂, -CN, and the following groups optionally substituted with one or more R^{2a1}: C₁₋₆ alkyl, C₁₋₆ alkoxy, C₁₋₆ alkylthio, C₁₋₆ alkylamino, di-C₁₋₆ alkylamino, 3- to 12-membered cycloalkyl, and 3- to 12-membered heterocyclyl;
each R^{2a1} is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R^{2b} is selected from the group consisting of H, deuterium, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
or R^{2a} and R^{2b} form =O together;
each R^{B} is independently selected from the group consisting of deuterium, oxo, halogen, -CN, -OH, -NH₂, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
ring A is selected from the group consisting of the following groups optionally substituted with one or more R^{A} or R^{C}: 3- to 12-membered cycloalkyl, 3- to 12-membered cycloalkenyl, 3- to 12-membered heterocyclyl, 6- to 10-membered aryl, and 5- to 10-membered heteroaryl;
each R^{A} is independently selected from the group consisting of deuterium, oxo, halogen, -CN, -OH, -NH₂, and the following groups optionally substituted with one or more R^{A1}: C₁₋₆ alkyl, C₂₋₆ alkenyl, C₂₋₆ alkynyl, C₁₋₆ alkoxy, C₁₋₆ alkylthio, C₁₋₆ alkylamino, di-C₁₋₆ alkylamino, 3- to 12-membered cycloalkyl, 3- to 12-membered heterocyclyl, 6-to 10-membered aryl, and 5- to 10-membered heteroaryl;
each R^{A1} is independently selected from the group consisting of deuterium, oxo, halogen, -OH, -NH₂, -CN, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino;
Z is selected from the group consisting of a single bond, -S-, -O-, and the following groups optionally substituted with one or more R^{z}: -NH- and -N(C₁₋₆ alkyl)-;
each R^{z} is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R³ is selected from the group consisting of H, deuterium, and the following groups optionally substituted with one or more R^{3a} or R^{C}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3a} is independently selected from the group consisting of deuterium, oxo, halogen, -OH, -NH₂, -CN, and the following groups optionally substituted with one or more R^{3b}: =NH, =CH₂, =N(C₁₋₆ alkyl), =CH(C₁₋₆ alkyl), =C(C₁₋₆ alkyl)₂ C₁₋₆ alkyl, C₁₋₆ alkoxy, C₁₋₆ alkylthio, C₁₋₆ alkylamino, and di-C₁₋₆ alkylamino;
each R^{3b} is independently selected from the group consisting of deuterium, oxo, halogen, -OH, -NH₂, -CN, C₁₋₄ alkoxy, C₁₋₄ alkylthio, C₁₋₄ alkylamino, and di-C₁₋₄ alkylamino;
each R^{C} is independently selected from the group consisting of -NR^{C1}C(O)R^{C2}, -NR^{C1}C(O)OR^{C2}, -NR^{C1}C(O)OC₁₋₆ alkylene OC(O)R^{C2}, -NR^{C1}C(O)OC₁₋₆ alkylene NR^{C1}C(O)R^{C2}, -NR^{C1}C(O)NR^{C1}R^{C2}, -NR^{C1}C(O)NR^{C1}C₁₋₆ alkylene NR^{C1}C(O)R^{C2}, -NR^{C1}C(O)NR^{C1}C₁₋₆ alkylene OC(O)R^{C2}, -OC(O)R^{C2}, -OC(O)OR^{C2}, -OC(O)OC₁₋₆ alkylene OC(O)R^{C2}, -OC(O)OC₁₋₆ alkylene NR^{C1}C(O)R^{C2}, -OC(O)NR^{C1}R^{C2}, -OC(O)NR^{C1}C₁₋₆ alkylene OC(O)R^{C2}, - OC(O)NR^{C1}C₁₋₆ alkylene NR^{C1}C(O)R^{C2}, -P(O)(OR^{C2})₂, -C₁₋₆ alkylene P(O)(OR^{C2})₂, -C₁₋₆ alkylene NR^{C1}P(O)(OR^{C2})₂, -C₁₋₆ alkylene OP(O)(OR^{C2})₂, -NR^{C1}P(O)(OR^{C2})₂, -NR^{C1}C₁₋₆ alkylene P(O)(OR^{C2})₂, -NR^{C1}C₁₋₆ alkylene OP(O)(OR^{C2})₂, -NR^{C1}C₁₋₆ alkylene NR^{C1}P(O)(OR^{C2})₂, -OP(O)(OR^{C2})₂, -OC₁₋₆ alkylene P(O)(OR^{C2})₂, - OC₁₋₆ alkylene OP(O)(OR^{C2})₂, -OC₁₋₆ alkylene NR^{C1}P(O)(OR^{C2})₂, -P(O)HOR^{C2}, -C₁₋₆ alkylene P(O)HOR^{C2}, -C₁₋₆ alkylene NR^{C1}P(O)HOR^{C2}, -C₁₋₆ alkylene OP(O)HOR^{C2}, -NR^{C1}P(O)HOR^{C2}, -NR^{C1}C₁₋₆ alkylene P(O)HOR^{C2}, - NR^{C1}C₁₋₆ alkylene OP(O)HOR^{C2}, -NR^{C1}C₁₋₆ alkylene NR^{C1}P(O)HOR^{C2}, -OP(O)HOR^{C2}, -OC₁₋₆ alkylene P(O)HOR^{C2}, -OC₁₋₆ alkylene OP(O)HOR^{C2}, and -OC₁₋₆ alkylene NR^{C1}P(O)HOR^{C2}.
R^{C1} is independently selected from the group consisting of H and C₁₋₆ alkyl;
R^{C2} is independently selected from the group consisting of H and the following groups optionally substituted with one or more R^{C3}: C₁₋₁₂ alkyl, C₁₋₁₂ heteroalkyl, 3- to 12-membered cycloalkyl, 3- to 12-membered heterocyclyl, 6-to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{C3} is independently selected from the group consisting of deuterium, oxo, halogen, -OH, -CN, -NH₂, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, di-C₁₋₄ haloalkylamino, -P(O)(OH)₂, -C₁₋₄ alkylene P(O)(OH)₂, -C₁₋₄ alkylene OP(O)(OH)₂, -C₁₋₄ alkylene NR^{C1}P(O)(OH)₂, -NR^{C1}P(O)(OH)₂, -NR^{C1}C₁₋₄ alkylene P(O)(OH)₂, -NR^{C1}C₁₋₄ alkylene OP(O)(OH)₂, -NR^{C1}C₁₋₄ alkylene NR^{C1}P(O)(OH)₂, -OP(O)(OH)₂, -OC₁₋₄ alkylene P(O)(OH)₂, -OC₁₋₄ alkylene OP(O)(OH)₂, -OC₁₋₄ alkylene NR^{C1}P(O)(OH)₂, -P(O)HOH, -C₁₋₄ alkylene P(O)HOH, -C₁₋₄ alkylene OP(O)HOH, - C₁₋₄ alkylene NR^{C1}P(O)HOH, -NR^{C1}P(O)HOH, -NR^{C1}C₁₋₄ alkylene P(O)HOH, -NR^{C1}C₁₋₄ alkylene OP(O)HOH, - NR^{C1}C₁₋₄ alkylene NR^{C1}P(O)HOH, -OP(O)HOH, -OC₁₋₄ alkylene P(O)HOH, -OC₁₋₄ alkylene OP(O)HOH, -OC₁₋₄ alkylene NR^{C1}P(O)HOH, -C(O)NR^{C1}C₁₋₆ alkyl, -NR^{C1}C(O)C₁₋₆ alkyl, -NR^{C1}C(O)OC₁₋₆ alkyl, -NR^{C1}C(O)NR^{C1}C₁₋₆ alkyl, -C(O)OC₁₋₆ alkyl, -OC(O)C₁₋₆ alkyl, -OC(O)OC₁₋₆ alkyl, and -OC(O)NR^{C1}C₁₋₆ alkyl;
R⁴ is selected from the group consisting of H, deuterium, halogen, -CN, C₁₋₆ alkyl, C₁₋₆ alkoxy, C₁₋₆ haloalkyl, and C₁₋₆ haloalkoxy;
each R^{x}, L¹, R¹, R^{2a}, R^{2a1}, R^{2b}, R^{B}, R^{A}, R^{A1}, R², R³, R^{3a} R^{3b} R^{C}, R^{C1}, R^{C2}, R^{C3}, or R⁴ is independently optionally substituted with one or more substituents;
provided that the compound of formula (I) comprises at least one R^{C}.

In some embodiments, L² is selected from a single bond, and ring B is selected from the group consisting of the following groups optionally substituted with one or more R^{B} or R^{C}: 3- to 12-membered cycloalkenyl, 3- to 12-membered heterocycloalkenyl, 6- to 10-membered aryl, and 5- to 10-membered heteroaryl;
R³ is selected from the group consisting of H, deuterium, and the following groups optionally substituted with one or more R^{3a} or R^{C}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{C} is independently selected from the group consisting of -C₁₋₆ alkylene OP(O)(OH)₂, -NR^{C1}P(O)(OH)₂, and -NR^{C1}C₁₋₆ alkylene OP(O)(OH)₂.

In some embodiments, L² is selected from -C(R^{2a}R^{2b})-, and ring B is selected from the group consisting of the following groups optionally substituted with one or more R^{B} or R^{C}: 3- to 12-membered cycloalkyl, 3- to 12-membered heterocyclyl, 6- to 10-membered aryl, and 5- to 10-membered heteroaryl;
ring A is selected from the group consisting of the following groups optionally substituted with one or more R^{A} or R^{C}: 3- to 12-membered cycloalkyl, 3- to 12-membered cycloalkenyl, 3- to 12-membered heterocyclyl, 6- to 10-membered aryl, and 5- to 10-membered heteroaryl;
R³ is selected from the group consisting of H, deuterium, and the following groups optionally substituted with one or more R^{3a} or R^{C}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{C} is independently selected from the group consisting of -NR^{C1}C(O)OR^{C2}, -NR^{C1}P(O)(OR^{C2})₂, - NR^{C1}P(O)HOR^{C2}, -NR^{C1}C₁₋₆ alkylene OP(O)(OR^{C2})₂, and -C₁₋₆ alkylene OP(O)(OR^{C2})₂;
R^{C1} is independently selected from the group consisting of H and C₁₋₆ alkyl;
R^{C2} is independently selected from the group consisting of the following groups substituted with one or more R^{C3}: C₁₋₁₂ alkyl, 6- to 10-membered aryl C₁₋₆ alkylene, and 5- to 10-membered heteroaryl C₁₋₆ alkylene;
each R^{C3} is independently selected from the group consisting of -NR^{C1}P(O)(OH)₂, -NR^{C1}C₁₋₄ alkylene OP(O)(OH)₂, and -OC(O)C₁₋₆ alkyl.

In some embodiments, R^{x} is selected from the group consisting of deuterium, halogen, -OH, -NH₂, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino.

In some embodiments, R^{x} is selected from the group consisting of deuterium, halogen, -OH, -NH₂, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, R^{x} is selected from the group consisting of deuterium, -F, -Cl, -Br, -I, -OH, -NH₂, C₁₋₃ alkyl, C₁₋₃ alkoxy, C₁₋₃ haloalkyl, and C₁₋₃ haloalkoxy.

In some embodiments, R^{x} is selected from the group consisting of deuterium, -F, -Cl, -Br, -OH, -NH₂, methyl, ethyl, isopropyl, methoxy, ethoxy, isopropoxy, trifluoromethyl, and trifluoromethoxy.

In some embodiments, X is selected from the group consisting of -N-, -CH-, -C(C₁₋₆ alkyl)-, and -C(C₁₋₆ haloalkyl)-.

In some embodiments, X is selected from the group consisting of -N-, -CH-, -C(C₁₋₃ alkyl)-, and -C(C₁₋₃ haloalkyl)-.

In some embodiments, X is selected from -N-.

In some embodiments, L¹ is selected from the group consisting of -O-, -S-, and the following groups optionally substituted with one or more R¹: -NH-, C₂₋₄ alkylene, C₁₋₃ heteroalkylene, C₂₋₄ alkenylene, and C₁₋₃ heteroalkenylene.

In some embodiments, L¹ is selected from the group consisting of -O- and the following groups optionally substituted with one or more R¹: -NH-, C₂₋₄ alkylene, and C₁₋₃ heteroalkylene.

In some embodiments, L¹ is selected from the group consisting of -O- and the following groups optionally substituted with one or more R¹: -NH-, -CH₂CH₂-, -(CH₂)₃-, -(CH₂)₄-, -OCH₂-, -CH₂O-, -OCH₂CH₂-, -CH₂OCH₂-, -CH₂CH₂O-, -O(CH₂)₃-, -CH₂OCH₂CH₂-, -CH₂CH₂OCH₂-, -CH₂CH₂CH₂O-, -NHCH₂-, -NHCH₂CH₂-, - CH₂NHCH₂-, -CH₂CH₂NH-, -NH(CH₂)₃-, -CH₂NHCH₂CH₂-, -CH₂CH₂NHCH₂-, and -CH₂CH₂CH₂NH-.

In some embodiments, L¹ is selected from the group consisting of the following groups optionally substituted with one or more R¹: -CH₂CH₂-, -(CH₂)₃-, -(CH₂)₄-, -OCH₂-, -OCH₂CH₂-, -O(CH₂)₃-, -NHCH₂-, -NHCH₂CH₂-, and - NH(CH₂)₃-.

In some embodiments, L¹ is selected from the group consisting of the following groups optionally substituted with one or more R¹: -(CH₂)₃-, -OCH₂CH₂-, and -NHCH₂CH₂-.

In some embodiments, L¹ is selected from -OCH₂CH₂- optionally substituted with one or more R¹.

In some embodiments, when X is selected from -N-, L¹ is not selected from the group consisting of -O-, -S-, -NH-, and -N(C₁₋₆ alkyl)-.

In some embodiments, each R¹ is independently selected from the group consisting of deuterium, oxo, halogen, - OH, -NH₂, -CN, C₁₋₄ alkyl, C₁₋₄ alkoxy, C₁₋₄ alkylthio, C₁₋₄ alkylamino, di-C₁₋₄ alkylamino, C_{1-A} haloalkyl, C_{1-A} haloalkoxy, C_{1-A} haloalkylthio, C₁₋₄ haloalkylamino, and di-C₁₋₄ haloalkylamino.

In some embodiments, each R¹ is independently selected from the group consisting of deuterium, oxo, halogen, - OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, each R¹ is independently selected from the group consisting of deuterium, oxo, halogen, - OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy.

In some embodiments, each R¹ is independently selected from the group consisting of deuterium, oxo, -F, -Cl, -Br, -I, -OH, -NH₂, -CN, C₁₋₃ alkyl, C₁₋₃ alkoxy, C₁₋₃ haloalkyl, and C₁₋₃ haloalkoxy.

In some embodiments, each R¹ is independently selected from the group consisting of deuterium, oxo, -F, -Cl, -Br, -OH, -NH₂, -CN, methyl, ethyl, isopropyl, methoxy, ethoxy, isopropoxy, -CH₂F, trifluoromethyl, and trifluoromethoxy.

In some embodiments, each R¹ is independently selected from the group consisting of C₁₋₃ alkyl and C₁₋₃ fluoroalkyl.

In some embodiments, each R¹ is independently selected from the group consisting of methyl, ethyl, isopropyl, trifluoromethyl, and -CH₂F.

In some embodiments, L² is selected from a single bond, and ring B is selected from the group consisting of the following groups optionally substituted with one or more R^{B} or R^{C}: 3- to 10-membered cycloalkenyl, 3- to 10-membered heterocycloalkenyl, 6- to 10-membered aryl, and 5- to 10-membered heteroaryl.

In some embodiments, L² is selected from -C(R^{2a}R^{2b})-, and ring B is selected from the group consisting of the following groups optionally substituted with one or more R^{B} or R^{C}: 3- to 10-membered cycloalkyl, 3- to 10-membered heterocyclyl, 6- to 10-membered aryl, and 5- to 10-membered heteroaryl.

In some embodiments, L² is selected from a single bond, and ring B is selected from 5- to 10-membered heterocycloalkenyl optionally substituted with one or more R^{B} or R^{C}.

In some embodiments, L² is selected from -C(R^{2a}R^{2b})-, and ring B is selected from the group consisting of the following groups optionally substituted with one or more R^{B} or R^{C}: 3- to 6-membered cycloalkyl, 4- to 7-membered heterocycloalkyl, phenyl, and 5- to 6-membered heteroaryl.

In some embodiments, L² is selected from a single bond, and ring B is selected from the group consisting of the following groups optionally substituted with one or more R^{B} or R^{C}: indolinyl, cyclopentenopyridinyl, diliydropyrrolopyridinyl, tetrahydroquinolinyl, tetrabydroisoquinolinyl, and tetrahydronaphthyridinyl.

In some embodiments, L² is selected from -C(R^{2a}R^{2b})-, and ring B is selected from the group consisting of the following groups optionally substituted with one or more R^{B} or R^{C}: cyclopropanyl, cyclobutanyl, cyclopentanyl, cyclohexanyl, oxetanyl, azetidinyl, tetrahydrofuranyl, pyrrolidinyl, piperidinyl, piperazinyl, morpholinyl, phenyl, furanyl, pyrrolyl, pyrazolyl, imidazolyl, oxazolyl, isoxazolyl, thienyl, thiazolyl, isothiazolyl, pyranyl, pyridinyl, pyridazinyl, pyrimidinyl, and pyrazinyl.

In some embodiments, L² is selected from a single bond, and ring B is selected from the group consisting of the following groups optionally substituted with one or more R^{B} or R^{C}: cyclopentenylpyridinyl, tetrahydroquinolinyl, and tetrahydroisoquinolinyl.

In some embodiments, L² is selected from -C(R^{2a}R^{2b})-, and ring B is selected from the group consisting of the following groups optionally substituted with one or more R^{B} or R^{C}: cyclopropanyl, cyclobutanyl, cyclopentanyl, oxetanyl, azetidinyl, pyrrolidinyl, piperidinyl, pyrazolyl, phenyl, pyridinyl, pyridazinyl, pyrimidinyl, and pyrazinyl.

In some embodiments, L² is selected from a single bond, and ring B is selected from the group consisting of the following groups optionally substituted with one or more R^{B} or R^{C}:

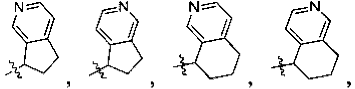

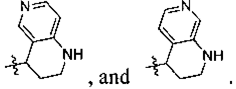

In some embodiments, L² is selected from -C(R^{2a}R^{2b})-, and ring B is selected from the group consisting of the following groups optionally substituted with one or more R^{B} or R^{C}: 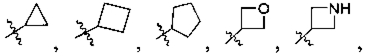 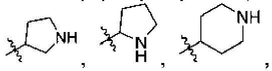 phenyl,

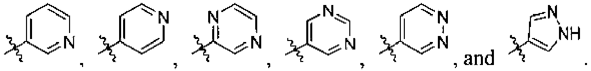

In some embodiments, L² is selected from a single bond, and ring B is selected from tetrahydroisoquinolinyl optionally substituted with one or more R^{B} or R^{C}.

In some embodiments, L² is selected from -C(R^{2a}R^{2b})-, and ring B is selected from the group consisting of the following groups optionally substituted with one or more R^{B} or R^{C}: phenyl and 5- to 6-membered heteroaryl.

In some embodiments, L² is selected from a single bond, and ring B is selected from 5,6,7,8-tetrahydroisoquinolinyl optionally substituted with one or more R^{B} or R^{C}.

In some embodiments, L² is selected from -C(R^{2a}R^{2b})-, and ring B is selected from the group consisting of the following groups optionally substituted with one or more R^{B} or R^{C}: phenyl,

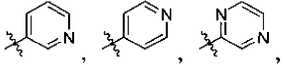

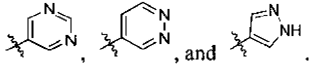

In some embodiments, L² is selected from -C(R^{2a}R^{2b})-, and ring B is selected from pyridinyl optionally substituted with one or more R^{B} or R^{C}.

In some embodiments, L² is selected from a single bond, and ring B is selected from the group consisting of the following groups optionally substituted with one or more R^{B} or R^{C}:

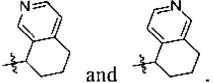

In some embodiments, L² is selected from -C(R^{2a}R^{2b})-, and ring B is selected from the group consisting of the following groups optionally substituted with one or more R^{B} or R^{C}:

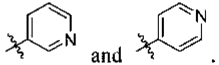

In some embodiments, ring B is optionally substituted with one or more R^{B}. In some embodiments, ring B is not substituted with R^{C}.

In some embodiments, L² is selected from a single bond, and ring B is selected from the group consisting of the following groups optionally substituted with one or more R^{B}: 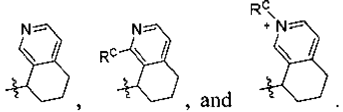 In some embodiments, L² is selected from a single bond, and ring B is selected from the group consisting of

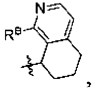

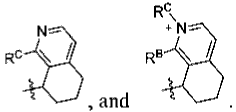

In some embodiments, L² is selected from -C(R^{2a}R^{2b})-, and ring B is selected from the group consisting of the following groups optionally substituted with one or more R^{B}: 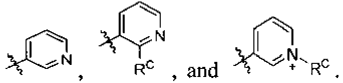 In some embodiments, L² is selected from -C(R^{2a}R^{2b})-, and ring B is selected from the group consisting of

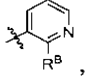

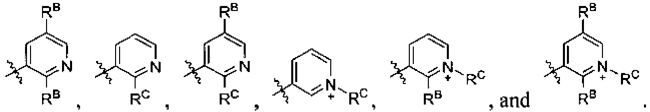

**In** some embodiments, L² is selected from a single bond, and ring B is selected from the group consisting of

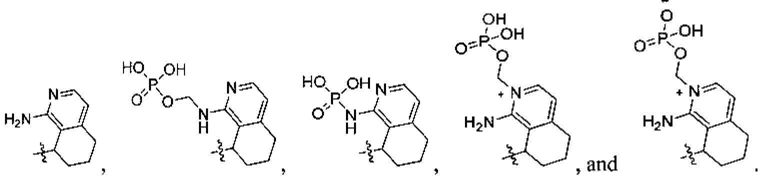

In some embodiments, L² is selected from -C(R^{2a}R^{2b})-, and ring B is selected from the group consisting of cyclopropanyl, 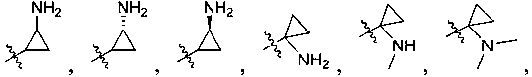 cyclobutanyl, 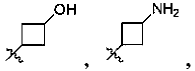 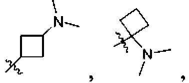 cyclopentanyl,

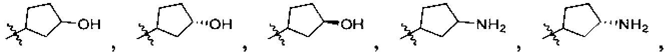

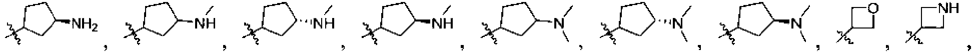

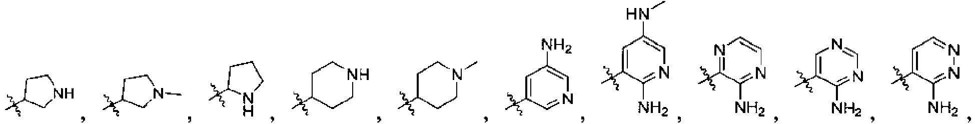

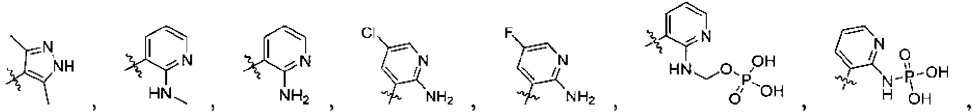

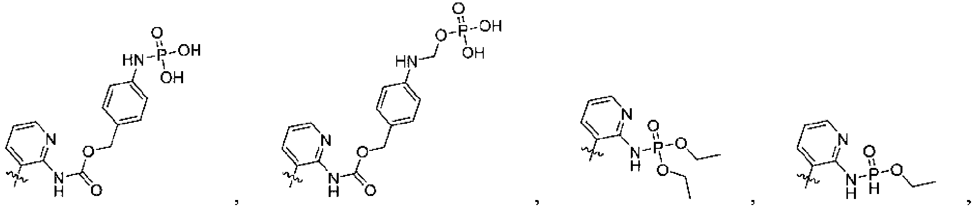

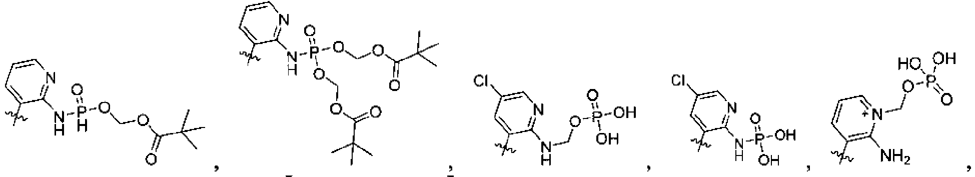

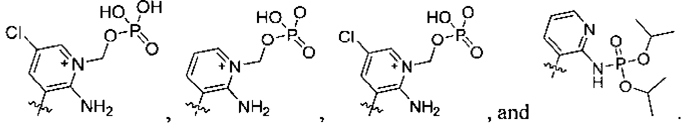

In some embodiments, L² is selected from -C(R^{2a}R^{2b})-, and ring B is selected from the group consisting of cyclopropanyl, 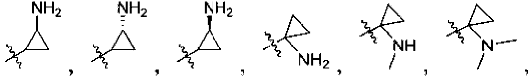 cyclobutanyl, 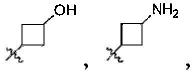 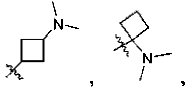 cyclopentanyl, 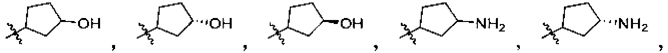 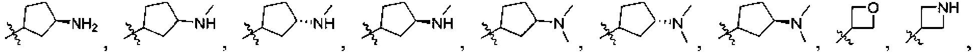 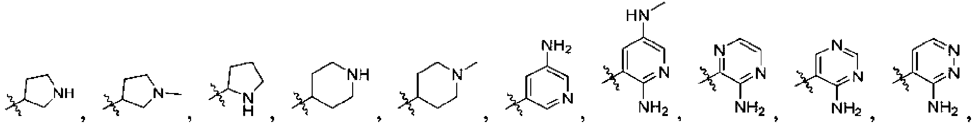 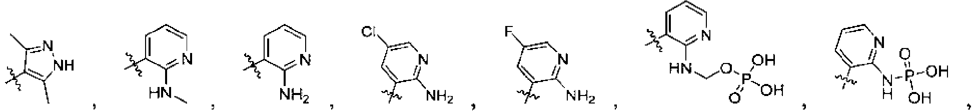 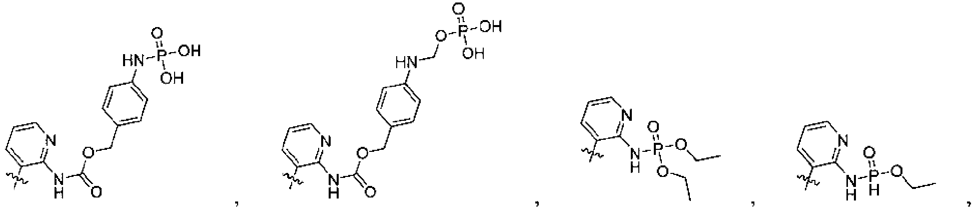 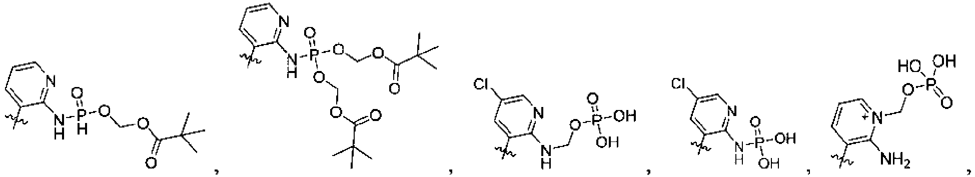 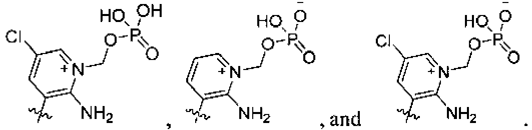 In some embodiments, L² is selected from -C(R^{2a}R^{2b})-, and ring B is selected from 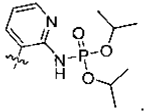 In some embodiments, L² is selected from -C(R^{2a}R^{2b})-, and ring B is selected from the group consisting of cyclopropanyl, 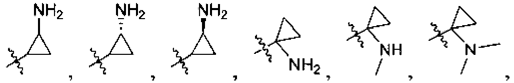 cyclobutanyl, 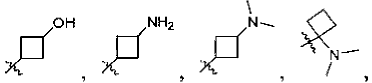 cyclopentanyl, 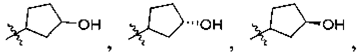 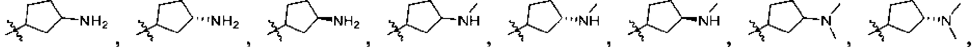 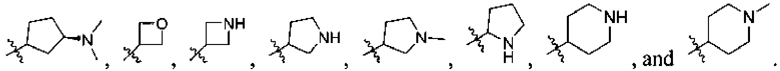 In some embodiments, L² is selected from -C(R^{2a}R^{2b})-, and ring B is selected from the group consisting of, 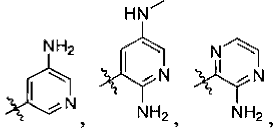 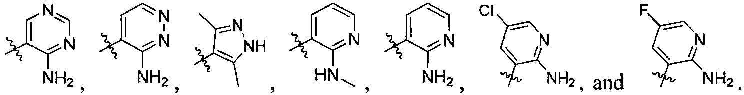 In some embodiments, L² is selected from -C(R^{2a}R^{2b})-, and ring B is selected from the group consisting of

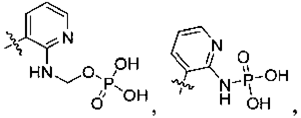

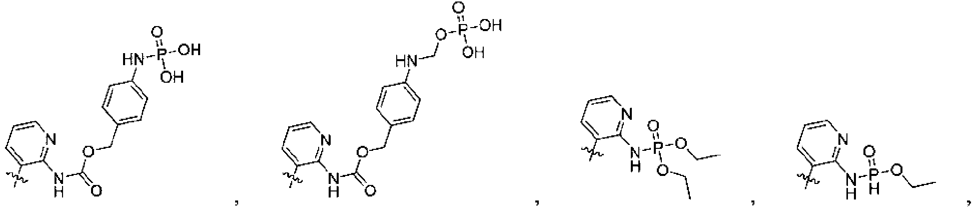

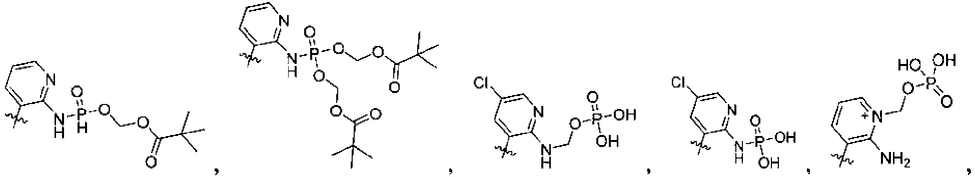

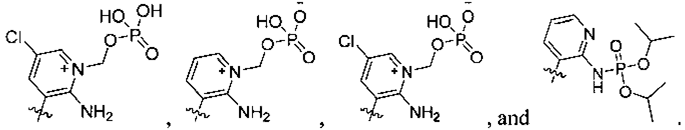

In some embodiments, R^{2a} is selected from the group consisting of H, deuterium, halogen, -OH, -NH₂, -CN, and the following groups optionally substituted with one or more R^{2a1}; C_{1-A} alkyl, C₁₋₄ alkoxy, C₁₋₄ alkylthio, C₁₋₄ alkylamino, di-C₁₋₄ alkylamino, 3- to 8-membered cycloalkyl, and 4- to 8-membered heterocyclyl.

In some embodiments, R^{2a} is selected from the group consisting of H, deuterium, and C₁₋₄ alkyl optionally substituted with one or more R^{2a1}

In some embodiments, R^{2a} is selected from the group consisting of H, deuterium, halogen, -OH, -NH₂, -CN, and the following groups optionally substituted with one or more R^{2a1}: C₁₋₃ alkyl, C₁₋₃ alkoxy, C₁₋₃ alkylthio, C₁₋₃ alkylamino, di-C₁₋₃ alkylamino, 3- to 8-membered cycloalkyl, and 4- to 8-membered heterocycloalkyl.

In some embodiments, R^{2a} is selected from the group consisting of H, deuterium, -F, -Cl, -Br, -I, -OH, -NH₂, -CN, and the following groups optionally substituted with one or more R^{2a1}: C₁₋₃ alkyl, C₁₋₃ alkoxy, C₁₋₃ alkylthio, C₁₋₃ alkylamino, di-C₁₋₃ alkylamino, 3- to 6-membered cycloalkyl, and 4- to 6-membered heterocycloalkyl.

In some embodiments, R^{2a} is selected from the group consisting of H, deuterium, -F, -Cl, -Br, -OH, -NH₂, -CN, and the following groups optionally substituted with one or more R^{2a1}: methyl, ethyl, n-propyl, isopropyl, methoxy, ethoxy, methylthio, methylamino, ethylamino, dimethylamino, cyclopropanyl, cyclobutanyl, cyclopentanyl, cyclohexanyl, oxetanyl, azetidinyl, pyrrolidinyl, piperidinyl, and piperazinyl.

In some embodiments, R^{2a} is selected from the group consisting of H, deuterium, -F, -Cl, -Br, -OH, -NH₂, -CN, and the following groups optionally substituted with one or more R^{2a1}: methyl, methoxy, methylthio, methylamino, dimethylamino, cyclopropanyl, and azetidinyl.

In some embodiments, R^{2a} is selected from the group consisting of H, deuterium, -F, -Cl, -Br, methyl, trifluoromethyl, methoxy, trifluoromethoxy, methylamino, dimethylamino, and cyclopropanyl.

In some embodiments, each R^{2a1} is independently selected from the group consisting of deuterium, oxo, halogen, - OH, -NH₂, -CN, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino.

In some embodiments, each R^{2a1} is independently selected from the group consisting of deuterium, oxo, halogen, - OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, each R^{2a1} is independently selected from the group consisting of deuterium, oxo, halogen, - OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy.

In some embodiments, each R^{2a1} is independently selected from the group consisting of deuterium, oxo, -F, -Cl, - Br, -I, -OH, -NH₂, -CN, methyl, methoxy, methylamino, dimethylamino, trifluoromethyl, and trifluoromethoxy.

In some embodiments, R^{2b} is independently selected from the group consisting of H, deuterium, halogen, -OH, - NH₂, -CN, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino.

In some embodiments, R^{2b} is independently selected from the group consisting of H, deuterium, halogen, -OH, - NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, R^{2b} is independently selected from the group consisting of H, deuterium, halogen, -OH, - NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy.

In some embodiments, R^{2b} is independently selected from the group consisting of H, deuterium, -F, -Cl, -Br, -I, - OH, -NH₂, -CN, C₁₋₃ alkyl, C₁₋₃ alkoxy, C₁₋₃ haloalkyl, and C₁₋₃ haloalkoxy.

In some embodiments, R^{2b} is independently selected from the group consisting of H, deuterium, -F, -Cl, -Br, -OH, -NH₂, -CN, methyl, ethyl, isopropyl, methoxy, ethoxy, isopropoxy, -CH₂F, trifluoromethyl, and trifluoromethoxy.

In some embodiments, R^{2b} is independently selected from the group consisting of H, deuterium, -F, -Cl, methyl, - CH₂F, and trifluoromethyl.

In some embodiments, R^{2a} is selected from methyl, and R^{2b} is selected from the group consisting of H and deuterium. In some embodiments, R^{2a} is selected from methyl, and R^{2b} is selected from H.

In some embodiments, each R^{B} is independently selected from the group consisting of deuterium, oxo, halogen, - CN, -OH, -NH₂, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino.

In some embodiments, each R^{B} is independently selected from the group consisting of deuterium, oxo, halogen, - CN, -OH, -NH₂, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, each R^{B} is independently selected from the group consisting of deuterium, oxo, -F, -Cl, -Br, -I, -CN, -OH, -NH₂, methyl, ethyl, isopropyl, methoxy, ethoxy, methylthio, methylamino, ethylamino, dimethylamino, fluoromethyl, fluoromethoxy, fluoromethylamino, and fluorodimethylamino.

In some embodiments, each R^{B} is independently selected from the group consisting of deuterium, oxo, -F, -Cl, -Br, -CN, -OH, -NH₂, methyl, methoxy, methylthio, methylamino, dimethylamino, trifluoromethyl, and trifluoromethoxy.

In some embodiments, each R^{B} is independently selected from the group consisting of -F, -Cl, -OH, -NH₂, methyl, methylamino, and dimethylamino.

In some embodiments, R^{B} is selected from the group consisting of -F, -Cl, and -NH₂.

In some embodiments, ring A is selected from the group consisting of the following groups optionally substituted with one or more R^{A} or R^{C}: 3- to 6-membered cycloalkyl, 5- to 10-membered cycloalkenyl, 5- to 10-membered heterocyclyl, 6- to 10-membered aryl, and 5- to 10-membered heteroaryl.

In some embodiments, ring A is selected from the group consisting of the following groups optionally substituted with one or more R^{A} or R^{C}: benzo 4- to 6-membered cycloalkenyl, benzo 4- to 6-membered heterocyclyl, 6- to 10-membered aryl, and 5- to 10-membered heteroaryl.

In some embodiments, ring A is selected from the group consisting of the following groups optionally substituted with one or more R^{A} or R^{C}: phenyl, naphthyl, benzocyclohexenyl, benzocyclopentenyl, pyrrolyl, pyrazolyl, imidazolyl, furanyl, oxazolyl, isoxazolyl, thienyl, thiazolyl, isothiazolyl, pyranyl, pyridinyl, pyridazinyl, pyrimidinyl, pyrazinyl, indolyl, benzopyrazolyl, benzimidazolyl, benzothiazolyl, quinolyl, isoquinolyl, benzopyrimidinyl, benzothienyl, pyridopyrazolyl, and pyridopyrrolyl.

In some embodiments, ring A is selected from the group consisting of the following groups optionally substituted with one or more R^{A} or R^{C}: phenyl, naphthyl, pyrazolyl, pyridinyl, indolyl, benzopyrazolyl, benzimidazolyl, benzothiazolyl, quinolyl, isoquinolyl, benzothienyl, pyridopyrazolyl, and pyridopyrrolyl.

In some embodiments, ring A is selected from the group consisting of the following groups optionally substituted with one or more R^{A} or R^{C}

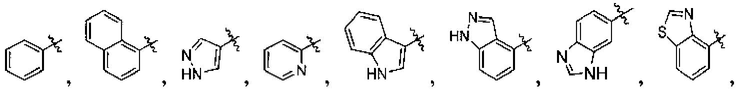

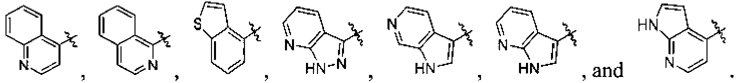

**In** some embodiments, ring A is selected from the group consisting of the following groups optionally substituted with one or more R^{A} or R^{C}: phenyl, naphthyl, and benzothienyl.

In some embodiments, ring A is selected from the group consisting of the following groups optionally substituted with one or more R^{A} or R^{C}: 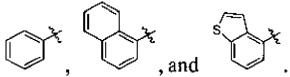 In some embodiments, ring A is selected from 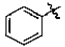 optionally substituted with one or more R^{A} or R^{C}. In some embodiments, ring A is selected from 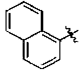 optionally substituted with one or more R^{A} or R^{C}. In some embodiments, ring A is selected from 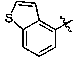 optionally substituted with one or more R^{A} or R^{C}.

In some embodiments, ring A is optionally substituted with one or more R^{A}. In some embodiments, ring A is not substituted with R^{C}.

In some embodiments, ring A is selected from the group consisting of the following groups optionally substituted with one or more R^{A}: 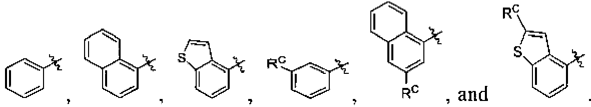 **In** some embodiments, ring A is selected from the group consisting of

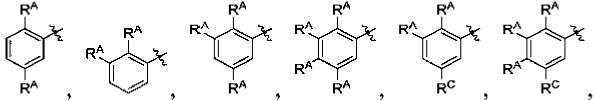

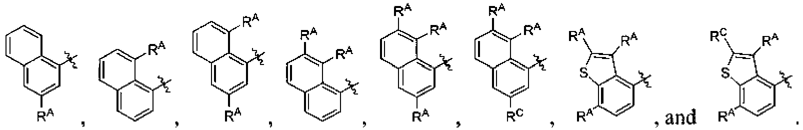

In some embodiments, ring A is selected from the group consisting of

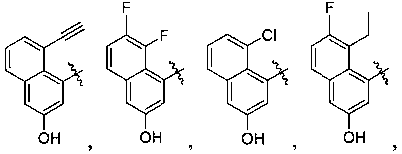

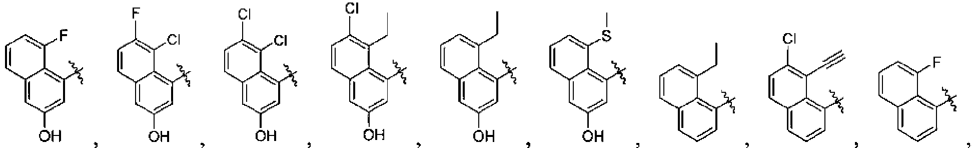

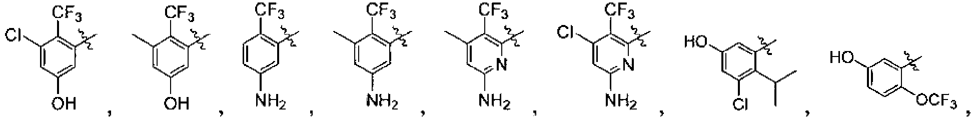

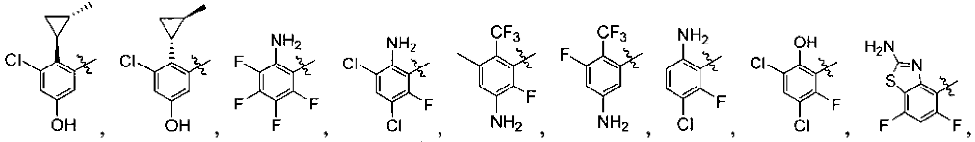

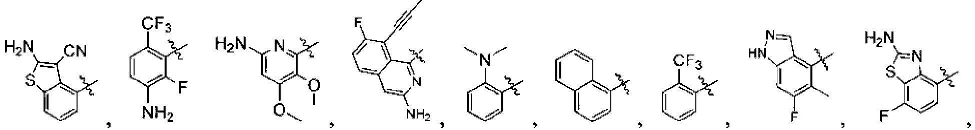

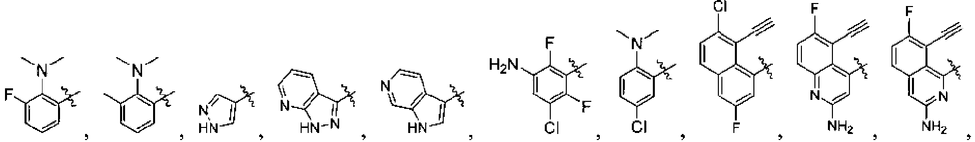

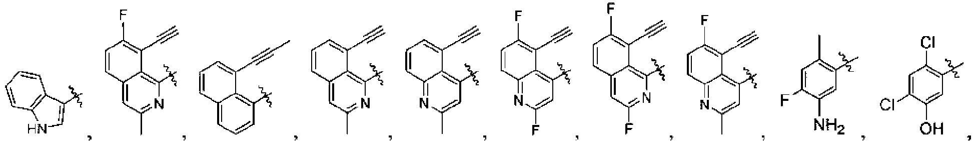

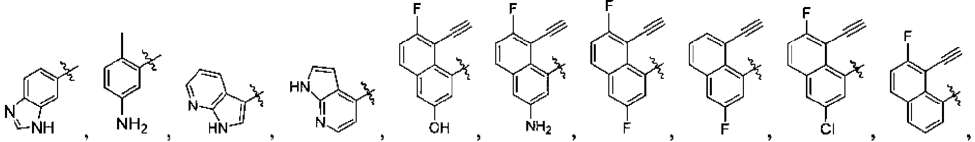

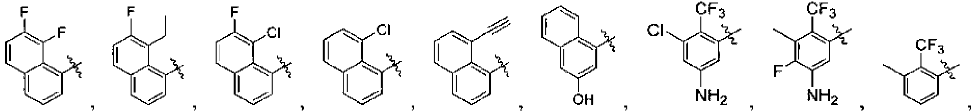

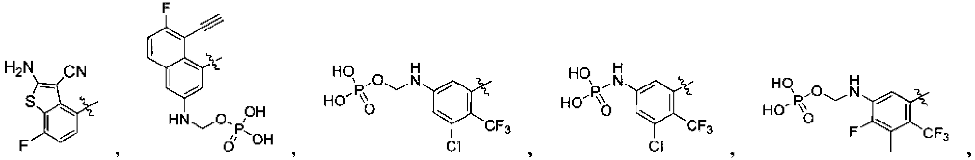

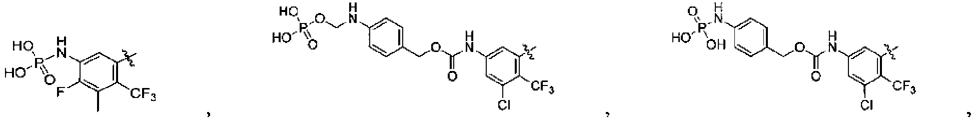

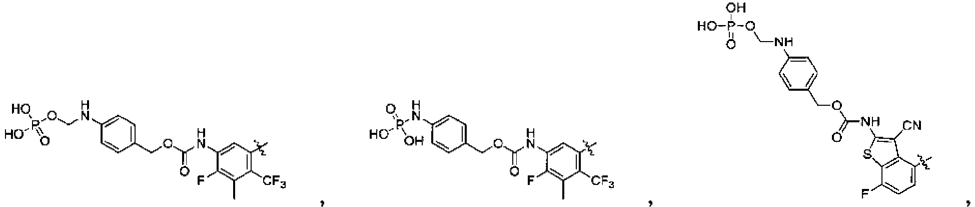

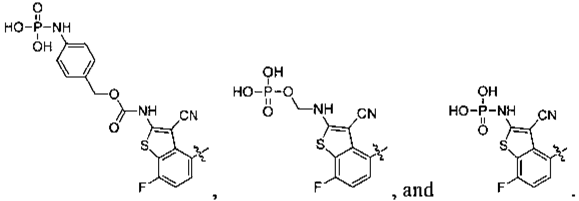

In some embodiments, ring A is selected from the group consisting of 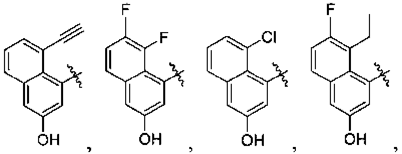 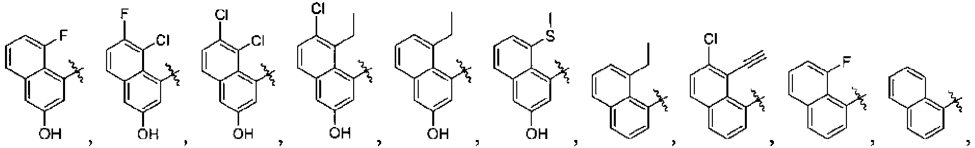 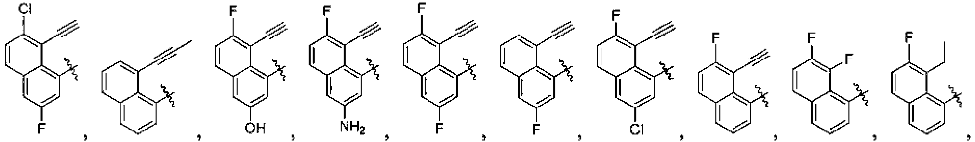 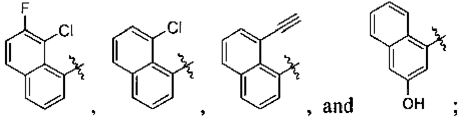 in some embodiments, ring A is selected from the group consisting of 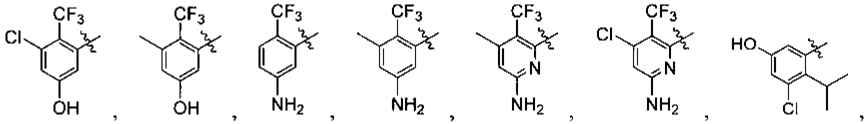 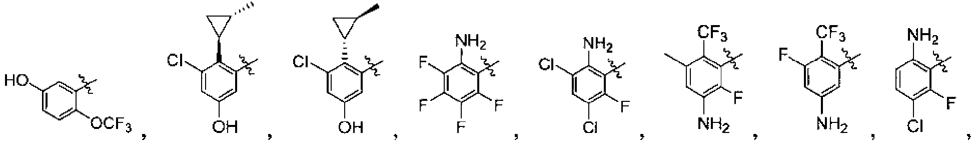 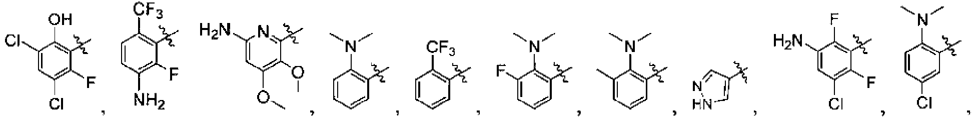 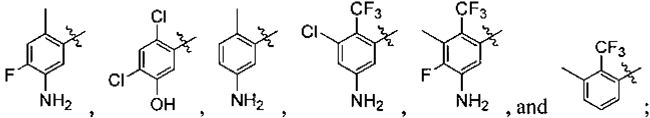 in some embodiments, ring A is selected from the group consisting of 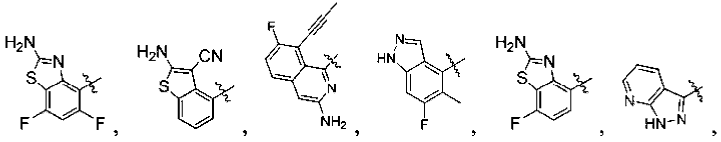 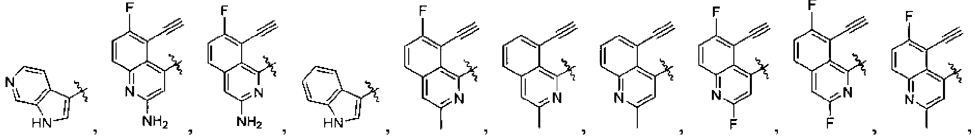 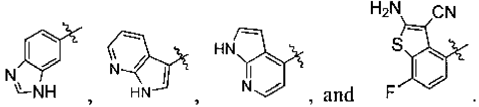 **In** some embodiments, ring A is selected from the group consisting of

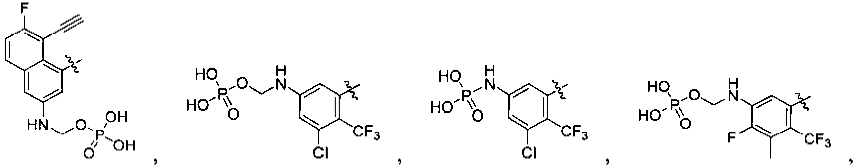

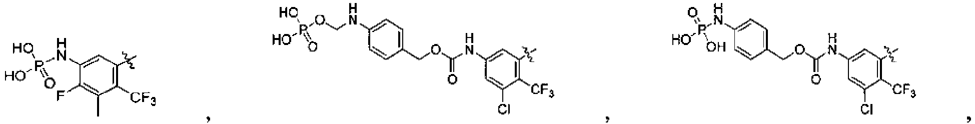

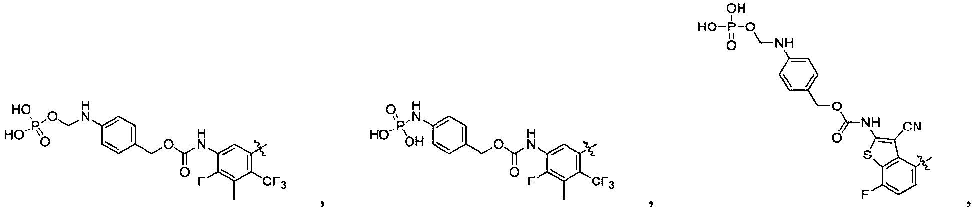

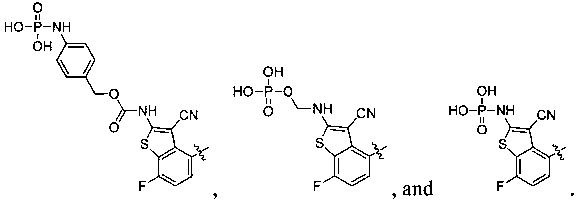

**In** some embodiments, each R^{A} is independently selected from the group consisting of deuterium, oxo, halogen, - CN, -OH, -NH₂, and the following groups optionally substituted with one or more R^{A1}: C₁₋₄ alkyl, C₂₋₄ alkenyl, C₂₋₄ alkynyl, C₁₋₄ alkoxy, C₁₋₄ alkylthio, C₁₋₄ alkylamino, di-C₁₋₄ alkylamino, 3- to 10-membered cycloalkyl, 3- to 10-membered heterocyclyl, 6- to 10-membered aryl, and 5- to 10-membered heteroaryl.

In some embodiments, each R^{A} is independently selected from the group consisting of deuterium, oxo, halogen, - CN, -OH, -NH₂, and the following groups optionally substituted with one or more R^{A1}: C₁₋₃ alkyl, C₂₋₃ alkenyl, C₂₋₃ alkynyl, C₁₋₃ alkoxy, C₁₋₃ alkylthio, C₁₋₃ alkylamino, di-C₁₋₃ alkylamino, 3- to 6-membered cycloalkyl, 4- to 7-membered heterocyclyl, phenyl, and 5- to 6-membered heteroaryl.

In some embodiments, each R^{A} is independently selected from the group consisting of deuterium, oxo, -F, -Cl, -Br, -I, -CN, -OH, -NH₂, and the following groups optionally substituted with one or more R^{A1}: methyl, ethyl, isopropyl, ethenyl, ethynyl, propynyl, methoxy, methylthio, methylamino, dimethylamino, 3- to 6-membered cycloalkyl, and 4- to 7-membered heterocycloalkyl.

In some embodiments, each R^{A} is independently selected from the group consisting of deuterium, -F, -Cl, -Br, -CN, -OH, -NH₂, and the following groups optionally substituted with one or more R^{A1}: methyl, ethyl, isopropyl, ethenyl, ethynyl, propynyl, methoxy, methylthio, methylamino, dimethylamino, cyclopropanyl, cyclobutanyl, oxetanyl, and azetidinyl.

In some embodiments, each R^{A} is independently selected from the group consisting of deuterium, -F, -Cl, -Br, -CN, -OH, -NH₂, and the following groups optionally substituted with one or more R^{A1}: methyl, ethyl, isopropyl, ethenyl, ethynyl, propynyl, methoxy, methylthio, methylamino, dimethylamino, and cyclopropanyl.

In some embodiments, each R^{A} is independently selected from the group consisting of deuterium, -F, -Cl, -Br, -CN, -OH, -NH₂, methyl, ethyl, isopropyl, ethenyl, ethynyl, propynyl, trifluoromethyl, hydroxymethylene, methoxy, trifluoromethoxy, methylamino, dimethylamino, methylthio, trifluoromethylthio, and cyclopropanyl optionally substituted with -F, -Cl, -Br, or methyl.

In some embodiments, each R^{A} is independently selected from the group consisting of deuterium, -F, -Cl, -CN, - OH, -NH₂, methyl, ethyl, isopropyl, ethynyl, propynyl, trifluoromethyl, methoxy, trifluoromethoxy, dimethylamino, methylthio, and cyclopropanyl substituted with methyl.

In some embodiments, each R^{A} is independently selected from the group consisting of deuterium, -F, -Cl, -CN, - OH, -NH₂, methyl, ethyl, ethynyl, and trifluoromethyl.

In some embodiments, each R^{A1} is independently selected from the group consisting of deuterium, oxo, halogen, - OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, each R^{A1} is independently selected from the group consisting of deuterium, oxo, -F, -Cl, -Br, -I, -OH, -NH₂, -CN, methyl, ethyl, isopropyl, methoxy, methylthio, methylamino, dimethylamino, diethylamino, fluoromethyl, and fluoromethoxy.

In some embodiments, each R^{A1} is independently selected from the group consisting of deuterium, -F, -Cl, -Br, - OH, -NH₂, -CN, methyl, methoxy, methylthio, methylamino, dimethylamino, trifluoromethyl, and trifluoromethoxy.

In some embodiments, each R^{A1} is independently selected from the group consisting of -F, -Cl, -Br, and methyl.

In some embodiments, Z is selected from the group consisting of a single bond, -S-, -O-, and the following groups optionally substituted with one or more R²: -NH- and -N(C₁₋₆ alkyl)-.

In some embodiments, Z is selected from the group consisting of a single bond, -S-, -O-, -NH-, and -N(C₁₋₄ alkyl)-.

In some embodiments, Z is selected from the group consisting of a single bond, -S-, -O-, -NH-, and -N(C₁₋₃ alkyl)-.

In some embodiments, Z is selected from the group consisting of a single bond, -O-, -NH-, and -N(CH₃)-.

In some embodiments, Z is selected from -O-.

In some embodiments, each R^{z} is independently selected from the group consisting of deuterium, oxo, halogen, - OH, -NH₂, -CN, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino.

In some embodiments, each R^{z} is independently selected from the group consisting of deuterium, oxo, halogen, - OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, each R^{z} is independently selected from the group consisting of deuterium, oxo, -F, -Cl, -Br, -I, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy.

In some embodiments, each R^{z} is independently selected from the group consisting of deuterium, oxo, -F, -Cl, -Br, -OH, -NH₂, -CN, methyl, methoxy, methylamino, dimethylamino, trifluoromethyl, and trifluoromethoxy.

In some embodiments, the heterocyclyl is selected from heterocycloalkyl. In some embodiments, the heterocyclyl is selected from partially unsaturated heterocyclyl.

In some embodiments, the 3- to 12-membered heterocyclyl is selected from the group consisting of 3- to 12-membered heterocycloalkyl and benzo 4- to 6-membered heterocyclyl. In some embodiments, the 3- to 12-membered heterocyclyl C₁₋₆ alkylene is selected from the group consisting of 3- to 12-membered heterocycloalkyl C₁₋₆ alkylene and benzo 4- to 6-membered heterocyclyl C₁₋₆ alkylene.

In some embodiments, R³ is selected from the group consisting of H, deuterium, and the following groups optionally substituted with one or more R^{3a} or R^{C}: C₁₋₆ alkyl, 3- to 10-membered cycloalkyl, 3- to 10-membered heterocyclyl, 6- to 10-membered aryl, 5- to 10-membered heteroaryl, 3- to 10-membered cycloalkyl C₁₋₄ alkylene, 3- to 10-membered heterocyclyl C₁₋₄ alkylene, 6- to 10-membered aryl C₁₋₄ alkylene, and 5- to 10-membered heteroaryl C₁₋₄ alkylene.

In some embodiments, the 3- to 10-membered heterocyclyl is selected from the group consisting of 3- to 10-membered heterocycloalkyl and benzo 4- to 6-membered heterocyclyl. In some embodiments, the 3- to 10-membered heterocyclyl C₁₋₄ alkylene is selected from the group consisting of 3- to 10-membered heterocycloalkyl C₁₋₄ alkylene and benzo 4- to 6-membered heterocyclyl C₁₋₄ alkylene.

In some embodiments, R³ is selected from the group consisting of H, deuterium, and the following groups optionally substituted with one or more R^{3a} or R^{C}: C₁₋₄ alkyl, 3- to 10-membered cycloalkyl, 4- to 10-membered heterocyclyl, phenyl, 5- to 6-membered heteroaryl, 3- to 10-membered cycloalkyl C₁₋₃ alkylene, 4- to 10-membered heterocyclyl C₁₋₃ alkylene, phenyl C₁₋₃ alkylene, and 5- to 6-membered heteroaryl C₁₋₃ alkylene.

In some embodiments, the 4- to 10-membered heterocyclyl is selected from the group consisting of 4- to 10-membered heterocycloalkyl and benzo 4- to 6-membered heterocyclyl. In some embodiments, the 4- to 10-membered heterocyclyl C₁₋₃ alkylene is selected from the group consisting of 4- to 10-membered heterocycloalkyl C₁₋₃ alkylene and benzo 4- to 6-membered heterocyclyl C₁₋₃ alkylene.

In some embodiments, R³ is selected from the group consisting of 3- to 10-membered heterocyclyl and 3- to 10-membered heterocyclyl C₁₋₃ alkylene optionally substituted with one or more R^{3a} or R^{C}. In some embodiments, R³ is selected from the group consisting of 4- to 10-membered and 4- to 9-membered and 4- to 8-membered and 5- to 9-membered heterocyclyl groups optionally substituted with one or more R^{3a} or R^{C}. In some embodiments, R³ is selected from the group consisting of 4- to 10-membered and 4- to 9-membered and 4- to 8-membered and 5- to 9-membered heterocyclyl C₁₋₃ alkylene groups optionally substituted with one or more R^{3a} or R^{C}.

In some embodiments, R³ is selected from the group consisting of 4- to 10-membered heterocycloalkyl and 4- to 10-membered heterocycloalkyl C₁₋₃ alkylene optionally substituted with one or more R^{3a} or R^{C}. In some embodiments, R³ is selected from the group consisting of 4- to 10-membered and 4- to 9-membered and 4- to 8-membered and 5- to 9-membered heterocycloalkyl groups optionally substituted with one or more R^{3a} or R^{C}. In some embodiments, R³ is selected from the group consisting of 4- to 10-membered and 4- to 9-membered and 4- to 8-membered and 5- to 9-membered heterocycloalkyl C₁₋₃ alkylene groups optionally substituted with one or more R^{3a} or R^{C}.

In some embodiments, R³ is selected from the group consisting of H, deuterium, and the following groups optionally substituted with one or more R^{3a} or R^{C}: C₁₋₄ alkyl, 3- to 8-membered cycloalkyl, 4- to 10-membered heterocyclyl, phenyl, 5- to 6-membered heteroaryl, 3- to 8-membered cycloalkyl C₁₋₃ alkylene, 4- to 10-membered heterocyclyl C₁₋₃ alkylene, phenyl C₁₋₃ alkylene, and 5- to 6-membered heteroaryl C₁₋₃ alkylene.

In some embodiments, R³ is selected from the group consisting of H, deuterium, and the following groups optionally substituted with one or more R^{3a} or R^{C}: C₁₋₄ alkyl, 3- to 8-membered cycloalkyl, 4- to 10-membered heterocyclyl, 5- to 6-membered heteroaryl, 3- to 8-membered cycloalkyl C₁₋₃ alkylene, 4- to 10-membered heterocyclyl C₁₋₃ alkylene, and 5- to 6-membered heteroaryl C₁₋₃ alkylene.

In some embodiments, R³ is selected from the group consisting of H, deuterium, and the following groups optionally substituted with one or more R^{3a} or R^{C}: C₁₋₄ alkyl, 4- to 10-membered heterocyclyl, 3- to 8-membered cycloalkyl C₁₋₃ alkylene, 4- to 10-membered heterocyclyl C₁₋₃ alkylene, and 5- to 6-membered heteroaryl C₁₋₃ alkylene.

In some embodiments, R³ is selected from the group consisting of H, deuterium, and the following groups optionally substituted with one or more R^{3a} or R^{C}: C₁₋₄ alkyl, 4- to 10-membered heterocycloalkyl, 3- to 8-membered cycloalkyl C₁₋₃ alkylene, 4- to 10-membered heterocycloalkyl C₁₋₃ alkylene, and 5- to 6-membered heteroaryl C₁₋₃ alkylene.

In some embodiments, R³ is selected from the group consisting of H, deuterium, and the following groups optionally substituted with one or more R^{3a} or R^{C}: methyl, ethyl, n-propyl, isopropyl, n-butyl, isobutyl, sec-butyl, tert-butyl, cyclopropyl, cyclobutyl, cyclopentyl, cyclohexyl, azetidinyl, tetrahydropyrrolyl, morpholinyl, piperidinyl, piperazinyl, hexahydro-1H-pyrrolizinyl, 5-azaspiro[2.4]heptanyl, tetrahydro-1'H,3'H-pyrrolo[cyclopropane-1,2'-pyrrolidine], cyclopropyl C₁₋₃ alkylene, cyclobutyl C₁₋₃ alkylene, cyclopentyl C₁₋₃ alkylene, cyclohexyl C₁₋₃ alkylene, azetidinyl C₁₋₃ alkylene, tetrahydropyrrolyl C₁₋₃ alkylene, morpholinyl C₁₋₃ alkylene, piperidinyl C₁₋₃ alkylene, piperazinyl C₁₋₃ alkylene, hexahydro-1H-pyrrolizinyl C₁₋₃ alkylene, hexahydro-1H-pyrrolo[2,1-c][1,4]oxazinyl, hexahydro-1H-pyrrolo[2,1-c][1,4]oxazinyl C₁₋₃ alkylene, 5-azaspiro[2.4]heptanyl C₁₋₃ alkylene, tetrahydro-1'H,3'H-pyrrolo[cyclopropane-1,2'-pyrrolidine]C₁₋₃ alkylene, and imidazolyl C₃ alkylene.

In some embodiments, R³ is selected from the group consisting of H, deuterium, and the following groups optionally substituted with one or more R^{3a} or R^{C}: methyl, ethyl, n-propyl, isopropyl, n-butyl, isobutyl, sec-butyl, tert-butyl, cyclobutyl, cyclohexyl, azetidinyl, tetrahydropyrrolyl, piperidinyl, piperazinyl, cyclopropyl C₁₋₃ alkylene, cyclobutyl C₁₋₃ alkylene, cyclopentyl C₁₋₃ alkylene, azetidinyl C₁₋₃ alkylene, tetrahydropyrrolyl C₁₋₃ alkylene, morpholinyl C₁₋₃ alkylene, piperidinyl C₁₋₃ alkylene, piperazinyl C₁₋₃ alkylene, hexahydro-1H-pyrrolizinyl C₁₋₃ alkylene, hexahydro-1H-pyrrolo[2,1-c][1,4]axazinyl C₁₋₃ alkylene, 5-azaspiro[2.4]heptanyl C₁₋₃ alkylene, tetrahydro-1'H,3'H-pyrrolo[cyclopropane-1,2'-pyrrolidine]C₁₋₃ alkylene, and imidazolyl C₁₋₃ alkylene.

In some embodiments, R³ is selected from the group consisting of H, deuterium, and the following groups optionally substituted with one or more R^{3a} or R^{C}: methyl, ethyl, n-propyl, isopropyl, n-butyl, isobutyl, sec-butyl, 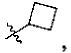 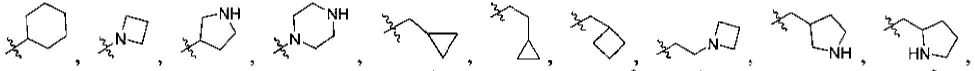 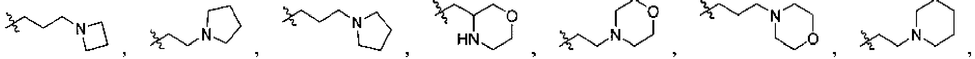 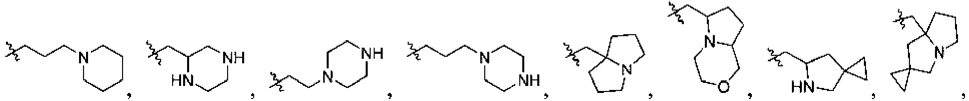 and

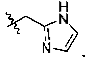

**In** some embodiments, R³ is selected from hexahydro-1H-pyrrolizinyl C₁₋₃ alkylene optionally substituted with one or more R^{3a} or R^{C}. In some embodiments, R³ is selected from 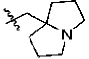 optionally substituted with one or more R^{3a} or R^{C}. In some embodiments, R³ is selected from 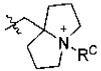 optionally substituted with one or more R^{3a}. In some embodiments, R³ is selected from 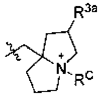 optionally substituted with one or more R^{3a}. In some embodiments, R³ is selected from 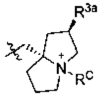 optionally substituted with one or more R^{3a}. In some embodiments, R³ is selected from 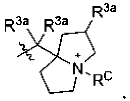 In some embodiments, R³ is selected from

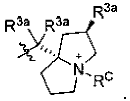

**In** some embodiments, R³ is selected from tetrahydropyrrolyl C₁₋₃ alkylene optionally substituted with one or more R^{3a} or R^{C}. In some embodiments, R³ is selected from 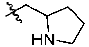 optionally substituted with one or more R^{3a} or R^{C}. **In** some embodiments, R³ is selected from 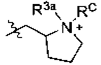 optionally substituted with one or more R^{3a}. In some embodiments, R³ is selected from 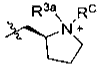 optionally substituted with one or more R^{3a}. In some embodiments, R³ is selected from 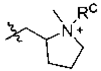 optionally substituted with one or more R^{3a}. In some embodiments, R³ is selected from 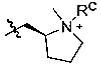 optionally substituted with one or more R^{3a}. In some embodiments, R³ is selected from 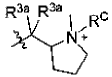 . In some embodiments, R³ is selected from

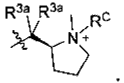

**In** some embodiments, R³ is optionally substituted with one or more R^{3a}. In some embodiments, R³ is not substituted with R^{C}.

In some embodiments, R³ is selected from the group consisting of H, deuterium, methyl,

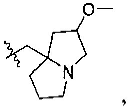

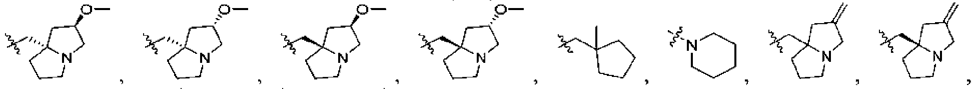

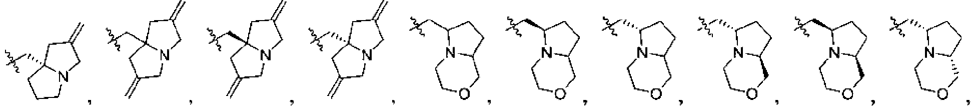

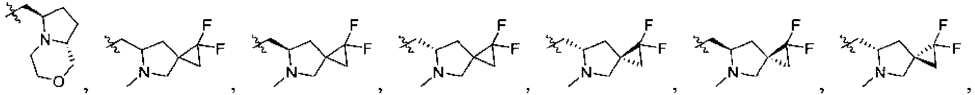

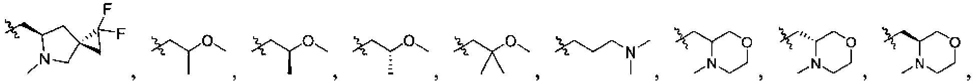

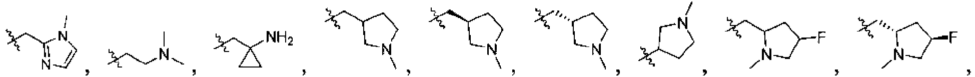

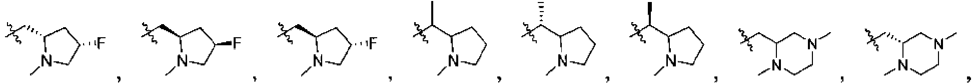

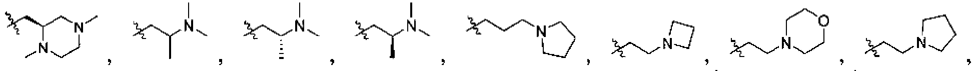

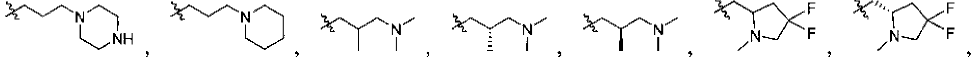

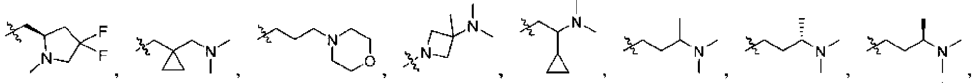

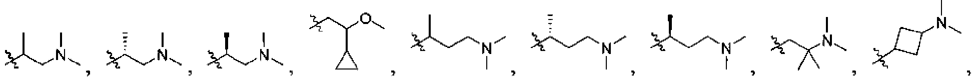

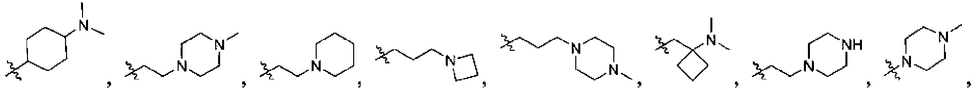

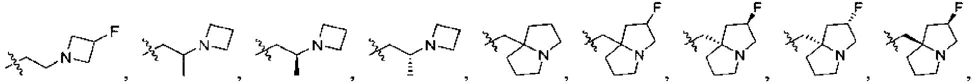

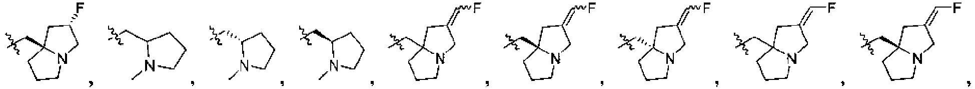

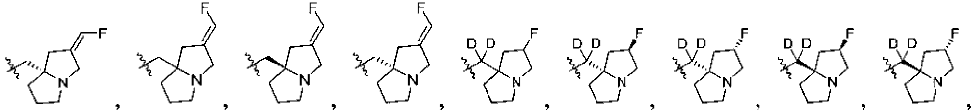

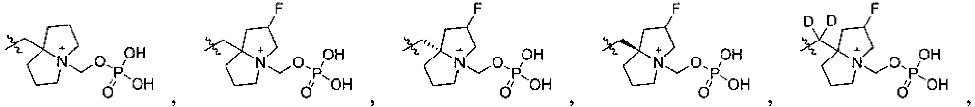

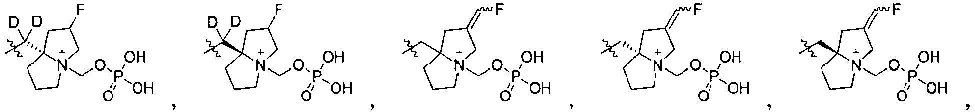

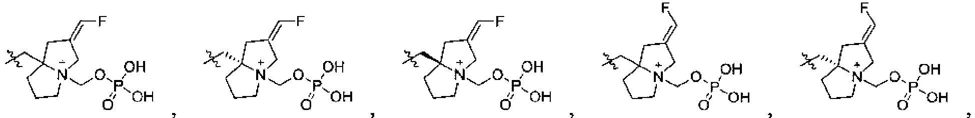

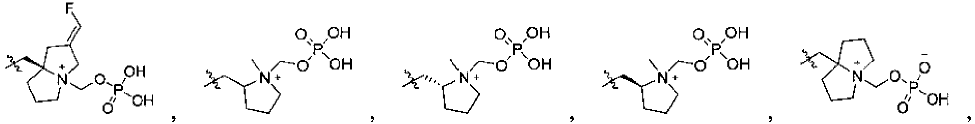

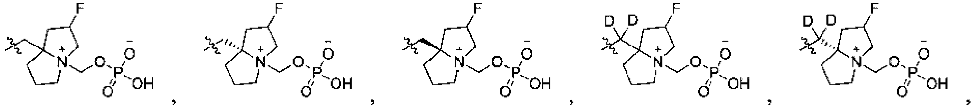

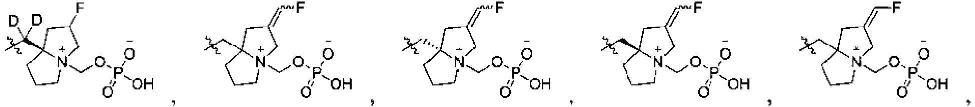

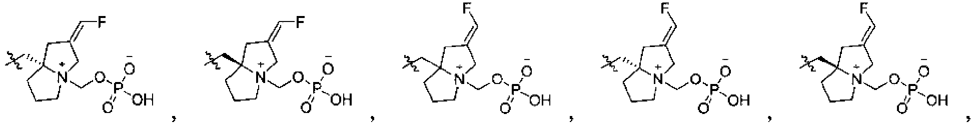

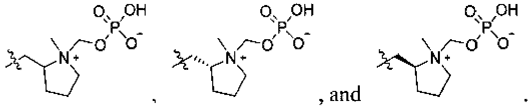

**In** some embodiments, R³ is selected from the group consisting of 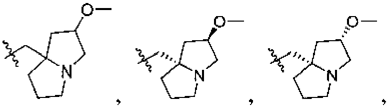 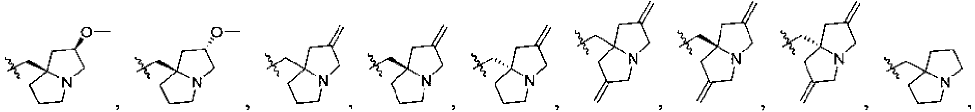 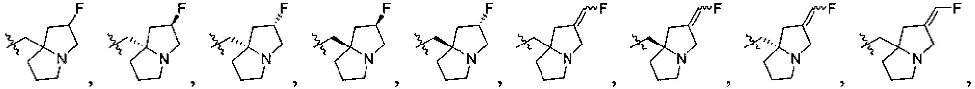 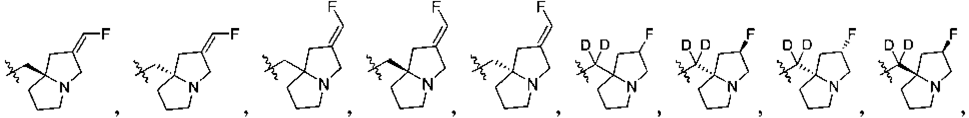 and 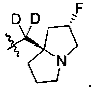 **In** some embodiments, R³ is selected from the group consisting of 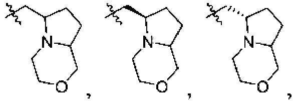 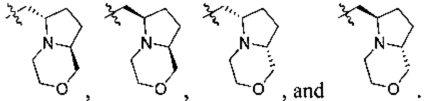 In some embodiments, R³ is selected from the group consisting of 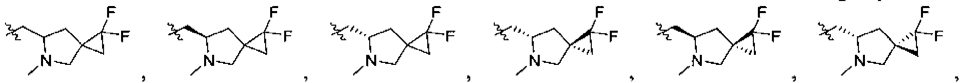 and 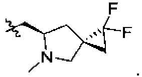 In some embodiments, R³ is selected from the group consisting of 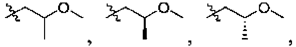 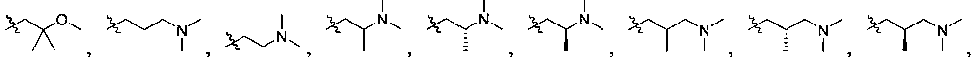 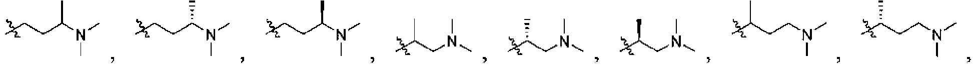 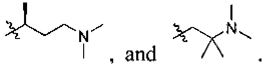 In some embodiments, R³ is selected from the group consisting of 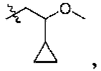 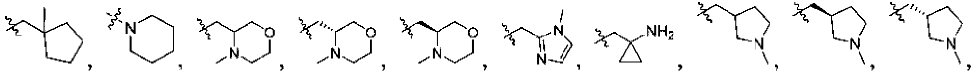 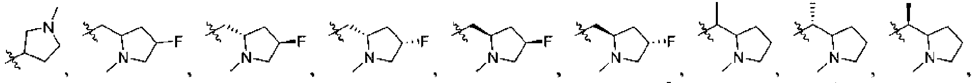 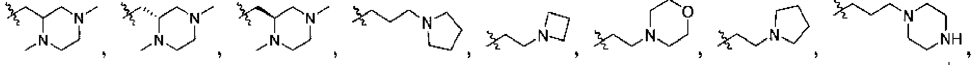 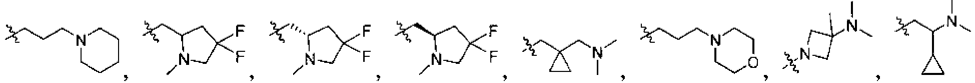 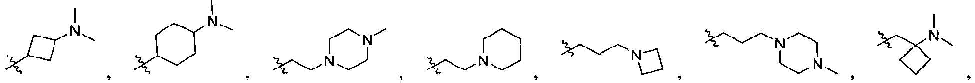 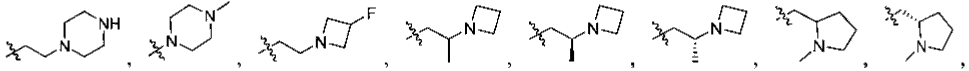 and 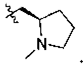 **In** some embodiments, R³ is selected from the group consisting of 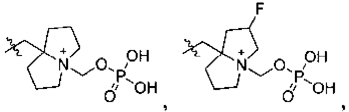 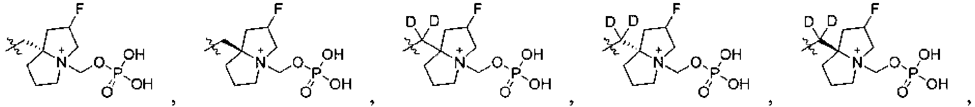 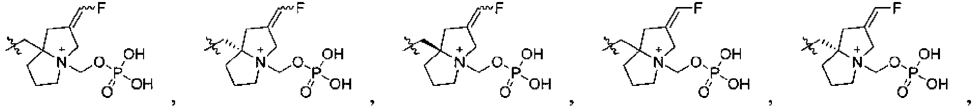 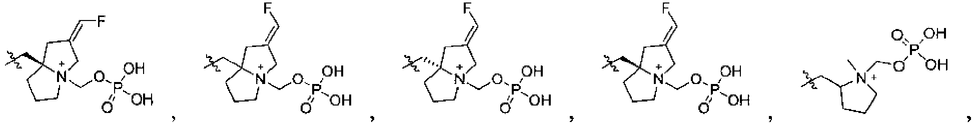 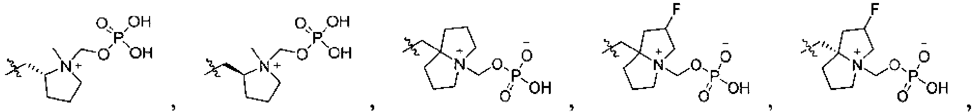 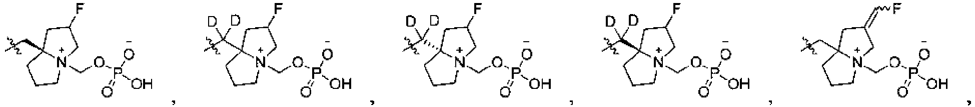 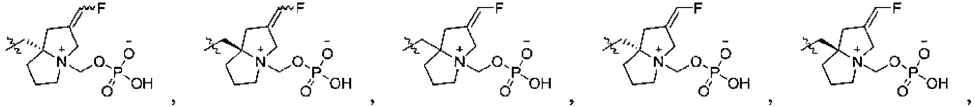 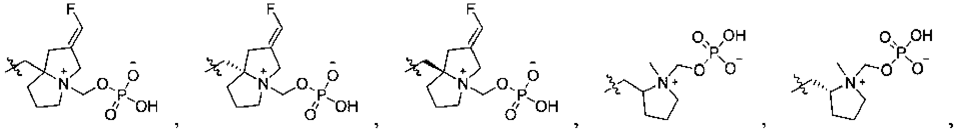 and

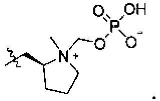

In some embodiments, the position of each R^{3a} substitution is independently selected from a cyclic moiety and/or an alkylene moiety in R³. In some embodiments, the position of each R^{3a} substitution is a cyclic moiety in R³. In some embodiments, the position of each R^{3a} substitution is an alkylene moiety in R³.

In some embodiments, each R^{3a} is independently selected from the group consisting of deuterium, oxo, halogen, - OH, -NH₂, -CN, and the following groups optionally substituted with one or more R^{3b}: =NH, =CH₂, =N(C₁₋₄ alkyl), =CH(C₁₋₄ alkyl), =C(C₁₋₄ alkyl)₂, C₁₋₄ alkyl, C₁₋₄ alkoxy, C₁₋₄ alkylthio, C₁₋₄ alkylamino, and di-C₁₋₄ alkylamino.

In some embodiments, each R^{3a} is independently selected from the group consisting of deuterium, oxo, -F, -Cl, -Br, -I, -OH, -NH₂, -CN, and the following groups optionally substituted with one or more R^{3b}: =NH, =CH₂, methyl, ethyl, isopropyl, methoxy, ethoxy, methylthio, methylamino, ethylamino, and dimethylamino.

In some embodiments, each R^{3a} is independently selected from the group consisting of deuterium, oxo, -F, -Cl, -Br, -OH, -NH₂, -CN, =CH₂, =CHF, =CF₂, methyl, methoxy, methylamino, dimethylamino, trifluoromethyl, trifluoromethoxy, and dimethylaminomethylene.

In some embodiments, each R^{3b} is independently selected from the group consisting of deuterium, oxo, halogen, - OH, -NH₂, -CN, C₁₋₃ alkoxy, C₁₋₃ alkylthio, C₁₋₃ alkylamino, and di-C₁₋₃ alkylamino.

In some embodiments, each R^{3b} is independently selected from the group consisting of deuterium, oxo, -F, -Cl, -Br, -I, -OH, -NH₂, -CN, methoxy, ethoxy, methylthio, methylamino, ethylamino, dimethylamino, and diethylamino.

In some embodiments, each R^{3b} is independently selected from the group consisting of -F, -Cl, -NH₂, methyl, methoxy, methylamino, and dimethylamino.

In some embodiments, ring B is optionally substituted with one or more R^{C} and optionally substituted with one or more R^{B}; ring A is optionally substituted with one or more R^{C} and optionally substituted with one or more R^{A}; R³ is optionally substituted with one or more R^{C} and optionally substituted with one or more R^{3a}. In some embodiments, ring B is optionally substituted with one R^{C} and optionally substituted with one or more R^{B}; ring A is optionally substituted with one R^{C} and optionally substituted with one or more R^{A}; R³ is optionally substituted with one R^{C} and optionally substituted with one or more R^{3a}.

In some embodiments, ring B is substituted with one or more R^{C} and optionally substituted with one or more R^{B}. In some embodiments, ring B is substituted with one R^{C} and optionally substituted with one or more R^{B}.

In some embodiments, ring B is substituted with one or more R^{C} and optionally substituted with one or more R^{B}; ring A is not substituted with R^{C} and optionally substituted with one or more R^{A}; R³ is not substituted with R^{C} and optionally substituted with one or more R^{3a}. In some embodiments, ring B is substituted with one R^{C} and optionally substituted with one or more R^{B}; ring A is not substituted with R^{C} and optionally substituted with one or more R^{A}; R³ is not substituted with R^{C} and optionally substituted with one or more R^{3a}.

In some embodiments, ring A is substituted with one or more R^{C} and optionally substituted with one or more R^{A}. In some embodiments, ring A is substituted with one R^{C} and optionally substituted with one or more R^{A}.

In some embodiments, ring A is substituted with one or more R^{C} and optionally substituted with one or more R^{A}; ring B is not substituted with R^{C} and optionally substituted with one or more R^{B}; R³ is not substituted with R^{C} and optionally substituted with one or more R^{3a}. In some embodiments, ring A is substituted with one R^{C} and optionally substituted with one or more R^{A}; ring B is not substituted with R^{C} and optionally substituted with one or more R^{B}; R³ is not substituted with R^{C} and optionally substituted with one or more R^{3a}.

In some embodiments, R³ is substituted with one or more R^{C} and optionally substituted with one or more R^{3a}. In some embodiments, R³ is substituted with one R^{C} and optionally substituted with one or more R^{3a}.

In some embodiments, R³ is substituted with one or more R^{C} and optionally substituted with one or more R^{3a}; ring A is not substituted with R^{C} and optionally substituted with one or more R^{A}; ring B is not substituted with R^{C} and optionally substituted with one or more R^{B}. In some embodiments, R³ is substituted with one R^{C} and optionally substituted with one or more R^{3a}; ring A is not substituted with R^{C} and optionally substituted with one or more R^{A}; ring B is not substituted with R^{C} and optionally substituted with one or more R^{B}.

In some embodiments, the compound of formula (I) comprises one, two, three, four, five, or six R^{C}.

In some embodiments, the compound of formula (I) comprises one or two R^{C}.

In some embodiments, the compound of formula (I) has one and only one R^{C}.

In some embodiments, each R^{C} is independently selected from the group consisting of -NR^{C1}C(O)R^{C2}, - NR^{C1}C(O)OR^{C2}, -NR^{C1}C(O)OC₁₋₄ alkylene OC(O)R^{C2}, -NR^{C1}C(O)OC₁₋₄ alkylene NR^{C1}C(O)R^{C2}, - NR^{C1}C(O)NR^{C1}R^{C2}, -NR^{C1}C(O)NR^{C1}C₁₋₄ alkylene NR^{C1}C(O)R^{C2}, -NR^{C1}C(O)NR^{C1}C₁₋₄ alkylene OC(O)R^{C2}, - OC(O)R^{C2}, -OC(O)OR^{C2}, -OC(O)OC₁₋₄ alkylene OC(O)R^{C2}, -OC(O)OC₁₋₄ alkylene NR^{C1}C(O)R^{C2}, - OC(O)NR^{C1}R^{C2}, -OC(O)NR^{C1}C₁₋₄ alkylene OC(O)R^{C2}, -OC(O)NR^{C1}C₁₋₄ alkylene NR^{C1}C(O)R^{C2}, -P(O)(OR^{C2})₂, - C₁₋₄ alkylene P(O)(OR^{C2})₂, -C₁₋₄ alkylene NR^{C1}P(O)(OR^{C2})₂, -C₁₋₄ alkylene OP(O)(OR^{C2})₂, -NR^{C1}P(O)(OR^{C2})₂, - NR^{C1}C₁₋₄ alkylene P(O)(OR^{C2})₂, -NR^{C1}C₁₋₄ alkylene OP(O)(OR^{C2})₂, -NR^{C1}C₁₋₄ alkylene NR^{C1}P(O)(OR^{C2})₂, - OP(O)(OR^{C2})₂, -OC₁₋₄ alkylene P(O)(OR^{C2})₂, -OC₁₋₄ alkylene OP(O)(OR^{C2})₂, -OC₁₋₄ alkylene NRP(O)(OR^{C2})₂, - P(O)HOR^{C2}, -C₁₋₄ alkylene P(O)HOR^{C2}, -C₁₋₄ alkylene NR^{C1} P(O)HOR^{C2}, -C₁₋₄ alkylene OP(O)HOR^{C2}, - NR^{C1}P(O)HOR^{C2}, -NR^{C1}C₁₋₄ alkylene P(O)HOR^{C2}, -NR^{C1}C₁₋₄ alkylene OP(O)HOR^{C2}, -NR^{C1}C₁₋₄ alkylene NR^{C1}P(O)HOR^{C2}, -OP(O)HOR^{C2}, -OC₁₋₄ alkylene P(O)HOR^{C2}, -OC₁₋₄ alkylene OP(O)HOR^{C2}, and -OC₁₋₄ alkylene NR^{C1}P(O)HOR^{C2}.

In some embodiments, each R^{C} is independently selected from the group consisting of -NR^{C1}C(O)R^{C2}, - NR^{C1}C(O)OR^{C2}, -NR^{C1}C(O)OC₁₋₃ alkylene OC(O)R^{C2}, -NR^{C1}C(O)OC₁₋₃ alkylene NR^{C1}C(O)R^{C2}, - NR^{C1}C(O)NR^{C1}R^{C2}, -NR^{C1}C(O)NR^{C1}C₁₋₃ alkylene NR^{C1}C(O)R^{C2}, -NR^{C1}C(O)NR^{C1}C₁₋₃ alkylene OC(O)R^{C2}, - OC(O)R^{C2}, -OC(O)OR^{C2}, -OC(O)OC₁₋₃ alkylene OC(O)R^{C2}, -OC(O)OC₁₋₃ alkylene NR^{C1}C(O)R^{C2}, - OC(O)NR^{C1}R^{C2}, -OC(O)NR^{C1}C₁₋₃ alkylene OC(O)R^{C2}, -OC(O)NR^{C1}C₁₋₃ alkylene NR^{C1}C(O)R^{C2}, -P(O)(OR^{C2})₂, - C₁₋₃ alkylene P(O)(OR^{C2})₂, -C₁₋₃ alkylene NR^{C1}P(O)(OR^{C2})₂, -C₁₋₃ alkylene OP(O)(OR^{C2})₂, -NR^{C1}P(O)(OR^{C2})₂, - NR^{C1}C₁₋₃ alkylene P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, - OP(O)(OR^{C2})₂, -OC₁₋₃ alkylene P(O)(OR^{C2})₂, -OC₁₋₃ alkylene OP(O)(OR^{C2})₂, -OC₁₋₃ alkylene NR^{C1}P(O)(OR^{C2})₂, - P(O)HOR^{C2}, -C₁₋₃ alkylene P(O)HOR^{C2}, -C₁₋₃ alkylene NR^{C1}P(O)HOR^{C2}, -C₁₋₃ alkylene OP(O)HOR^{C2}, - NR^{C1}P(O)HOR^{C2}, -NR^{C1}C₁₋₃ alkylene P(O)HOR^{C2}, -NR^{C1}C₁₋₃ alkylene OP(O)HOR^{C2}, -NR^{C1}C₁₋₃ alkylene NR^{C1}P(O)HOR^{C2}, -OP(O)HOR^{C2}, -OC₁₋₃ alkylene P(O)HOR^{C2}, -OC₁₋₃ alkylene OP(O)HOR^{C2}, and -OC₁₋₃ alkylene NR^{C1}P(O)HOR^{C2}.

In some embodiments, each R^{C} is independently selected from the group consisting of -NR^{C1}C(O)R^{C2}, - NR^{C1}C(O)OR^{C2}, -NR^{C1}C(O)OC₁₋₃ alkylene OC(O)R^{C2}, -NR^{C1}C(O)OC₁₋₃ alkylene NR^{C1}C(O)R^{C2}, -OC(O)R^{C2}, - OC(O)OR^{C2}, -OC(O)OC₁₋₃ alkylene OC(O)R^{C2}, -OC(O)OC₁₋₃ alkylene NR^{C1}C(O)R^{C2}, -OC(O)NR^{C1}R^{C2}, - OC(O)NR^{C1}C₁₋₃ alkylene OC(O)R^{C2}, -OC(O)NR^{C1}C₁₋₃ alkylene NR^{C1}C(O)R^{C2}, -C₁₋₃ alkylene NR^{C1}P(O)(OR^{C2})₂, - C₁₋₃ alkylene OP(O)(OR^{C2})₂, -NR^{C1}P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, -OP(O)(OR^{C2})₂, -OC₁₋₃ alkylene OP(O)(OR^{C2})₂, -OC₁₋₃ alkylene NR^{C1}P(O)(OR^{C2})₂, -C₁₋₃ alkylene NR^{C1}P(O)HOR^{C2}, -C₁₋₃ alkylene OP(O)HOR^{C2}, -NR^{C1}P(O)HOR^{C2}, -NR^{C1}C₁₋₃ alkylene OP(O)HOR^{C2}, - NR^{C1}C₁₋₃ alkylene NR^{C1}P(O)HOR^{C2}, -OP(O)HOR^{C2}, -OC₁₋₃ alkylene OP(O)HOR^{C2}, and -OC₁₋₃ alkylene NR^{C1}P(O)HOR^{C2}.

In some embodiments, each R^{C} is independently selected from the group consisting of -NR^{C1}C(O)R^{C2}, - NR^{C1}C(O)OR^{C2}, -NR^{C1}C(O)OC₁₋₃ alkylene OC(O)R^{C2}, -OC(O)R^{C2}, -OC(O)OR^{C2}, -OC(O)OC₁₋₃ alkylene OC(O)R^{C2}, -OC(O)NR^{C1}R^{C2}, -OC(O)NR^{C1}C₁₋₃ alkylene OC(O)R^{C2}, -OC(O)NR^{C1}C₁₋₃ alkylene NR^{C1}C(O)R^{C2}, -C₁₋₃ alkylene NR^{C1}P(O)(OR^{C2})₂, -C₁₋₃ alkylene OP(O)(OR^{C2})₂, -NR^{C1}P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, -OP(O)(OR^{C2})₂, -OC₁₋₃ alkylene OP(O)(OR^{C2})₂, -OC₁₋₃ alkylene NR^{C1}P(O)(OR^{C2})₂, -C₁₋₃ alkylene NR^{C1}P(O)HOR^{C2}, -C₁₋₃ alkylene OP(O)HOR^{C2}, -NR^{C1}P(O)HOR^{C2}, -NR^{C1}C₁₋₃ alkylene OP(O)HOR^{C2}, -NR^{C1}C₁₋₃ alkylene NR^{C1}P(O)HOR^{C2}, -OP(O)HOR^{C2}, -OC₁₋₃ alkylene OP(O)HOR^{C2}, and -OC₁₋₃ alkylene NR^{C1}P(O)HOR^{C2}.

In some embodiments, each R^{C} is independently selected from the group consisting of -NR^{C1}C(O)R^{C2}, - NR^{C1}C(O)OR^{C2}, -OC(O)R^{C2}, -OC(O)OR^{C2}, -OC(O)OC₁₋₃ alkylene OC(O)R^{C2}, -OC(O)NR^{C1}R^{C2}, -C₁₋₃ alkylene OP(O)(OR^{C2})₂, -NR^{C1}P(O)(OR^{C2})₂, -NR^{C1}C₁₋₃ alkylene OP(O)(OR^{C2})₂, -OP(O)(OR^{C2})₂, -OC₁₋₃ alkylene OP(O)(OR^{C2})₂, and -NR^{C1}P(O)HOR^{C2}.

In some embodiments, each R^{C} is independently selected from the group consisting of -NR^{C1}C(O)OR^{C2}, -C₁₋₂ alkylene OP(O)(OR^{C2})₂, -NR^{C1}P(O)(OR^{C2})₂, -NR^{C1}C₁₋₂ alkylene OP(O)(OR^{C2})₂, and -NR^{C1}P(O)HOR^{C2}.

In some embodiments, each R^{C} is independently selected from the group consisting of -NR^{C1}C(O)OR^{C2}, -C₁₋₂ alkylene OP(O)(OH)₂, -NR^{C1}P(O)(OH)₂, -NR^{C1}P(O)(OR^{C2})₂, -NR^{C1}C₁₋₂ alkylene OP(O)(OH)₂, and - NR^{C1}P(O)HOR^{C2}.

In some embodiments, each R^{C} is independently selected from the group consisting of -NR^{C1}C(O)OR^{C2}, - P(O)(OR^{C2})₂, -C₁₋₄ alkylene P(O)(OR^{C2})₂, -C₁₋₄ alkylene NR^{C1}P(O)(OR^{C2})₂, -C₁₋₄ alkylene OP(O)(OR^{C2})₂, - NR^{C1}P(O)(OR^{C2})₂, -NR^{C1}C₁₋₄ alkylene P(O)(OR^{C2})₂, -NR^{C1}C₁₋₄ alkylene OP(O)(OR^{C2})₂, -NR^{C1}C₁₋₄ alkylene NR^{C1}P(O)(OR^{C2})₂, -OP(O)(OR^{C2})₂, -OC₁₋₄ alkylene P(O)(OR^{C2})₂, -OC₁₋₄ alkylene OP(O)(OR^{C2})₂, -OC₁₋₄ alkylene NR^{C1}P(O)(OR^{C2})₂, -P(O)HOR^{C2}, -C₁₋₄ alkylene P(O)HOR^{C2}, -C₁₋₄ alkylene NR^{C1} P(O)HOR^{C2}, -C₁₋₄ alkylene OP(O)HOR^{C2}, -NR^{C1}P(O)HOR^{C2}, -NR^{C1}C₁₋₄ alkylene P(O)HOR^{C2}, -NR^{C1}C₁₋₄ alkylene OP(O)HOR^{C2}, -NR^{C1}C₁₋₄ alkylene NR^{C1}P(O)HOR^{C2}, -OP(O)HOR^{C2}, -OC₁₋₄ alkylene P(O)HOR^{C2}, -OC₁₋₄ alkylene OP(O)HOR^{C2}, and -OC₁₋₄ alkylene NR^{C1}P(O)HOR^{C2}.

In some embodiments, each R^{C} is independently selected from the group consisting of -NR^{C1}C(O)OR^{C2}, - P(O)(OR^{C2})₂, -C₁₋₃ alkylene P(O)(OR^{C2})₂, -C₁₋₃ alkylene NR^{C1}P(O)(OR^{C2})₂, -C₁₋₃ alkylene OP(O)(OR^{C2})₂, - NR^{C1}P(O)(OR^{C2})₂, -NR^{C1}C₁₋₃ alkylene P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, -OP(O)(OR^{C2})₂, -OC₁₋₃ alkylene P(O)(OR^{C2})₂, -OC₁₋₃ alkylene OP(O)(OR^{C2})₂, -OC₁₋₃ alkylene NR^{C1}P(O)(OR^{C2})₂, -P(O)HOR^{C2}, -C₁₋₃ alkylene P(O)HOR^{C2}, -C₁₋₃ alkylene NR^{C1}P(O)HOR^{C2}, -C₁₋₃ alkylene OP(O)HOR^{C2}, -NR^{C1}P(O)HOR^{C2}, -NR^{C1}C₁₋₃ alkylene P(O)HOR^{C2}, -NR^{C1}C₁₋₃ alkylene OP(O)HOR^{C2}, -NR^{C1}C₁₋₃ alkylene NR^{C1}P(O)HOR^{C2}, -OP(O)HOR^{C2}, -OC₁₋₃ alkylene P(O)HOR^{C2}, -OC₁₋₃ alkylene OP(O)HOR^{C2}, and -OC₁₋₃ alkylene NR^{C1}P(O)HOR^{C2}.

In some embodiments, each R^{C} is independently selected from the group consisting of -NR^{C1}C(O)OR^{C2}, -C₁₋₃ alkylene NR^{C1}P(O)(OR^{C2})₂, -C₁₋₃ alkylene OP(O)(OR^{C2})₂, -NR^{C1}P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, -OP(O)(OR^{C2})₂, -OC₁₋₃ alkylene OP(O)(OR^{C2})₂, -OC₁₋₃ alkylene NR^{C1}P(O)(OR^{C2})₂, -C₁₋₃ alkylene NR^{C1}P(O)HOR^{C2}, -C₁₋₃ alkylene OP(O)HOR^{C2}, -NR^{C1}P(O)HOR^{C2}, -NR^{C1}C₁₋₃ alkylene OP(O)HOR^{C2}, -NR^{C1}C₁₋₃ alkylene NR^{C1}P(O)HOR^{C2}, -OP(O)HOR^{C2}, -OC₁₋₃ alkylene OP(O)HOR^{C2}, and -OC₁₋₃ alkylene NR^{C1}P(O)HOR^{C2}.

In some embodiments, each R^{C} is independently selected from the group consisting of -NR^{C1}C(O)OR^{C2}, -C₁₋₃ alkylene NR^{C1}P(O)(OH)₂, -C₁₋₃ alkylene NR^{C1}P(O)(OR^{C2})₂, -C₁₋₃ alkylene OP(O)(OH)₂, -C₁₋₃ alkylene OP(O)(OR^{C2})₂, -NR^{C1}P(O)(OH)₂, -NR^{C1}P(O)(OR^{C2})₂, -NR^{C1}C₁₋₃ alkylene OP(O)(OH)₂, -NR^{C1}C₁₋₃ alkylene OP(O)(OR^{C2})₂, -NR^{C1}C₁₋₃ alkylene NR^{C1}P(O)(OH)₂, -NR^{C1}C₁₋₃ alkylene NR^{C1}P(O)(OR^{C2})₂, -OP(O)(OH)₂, - OP(O)(OR^{C2})₂, -OC₁₋₃ alkylene OP(O)(OH)₂, -OC₁₋₃ alkylene OP(O)(OR^{C2})₂, -OC₁₋₃ alkylene NR^{C1}P(O)(OH)₂, - OC₁₋₃ alkylene NR^{C1}P(O)(OR^{C2})₂, -C₁₋₃ alkylene NR^{C1}P(O)HOH, -C₁₋₃ alkylene NR^{C1}P(O)HOR^{C2}, -C₁₋₃ alkylene OP(O)HOH, -C₁₋₃ alkylene OP(O)HOR^{C2}, -NR^{C1}P(O)HOH, -NR^{C1}P(O)HOR^{C2}, -NR^{C1}C₁₋₃ alkylene OP(O)HOH, - NR^{C1}C₁₋₃ alkylene OP(O)HOR^{C2}, -NR^{C1}C₁₋₃ alkylene NR^{C1}P(O)HOH, -NR^{C1}C₁₋₃ alkylene NR^{C1}P(O)HOR^{C2}, - OP(O)HOH, -OP(O)HOR^{C2}, -OC₁₋₃ alkylene OP(O)HOH, -OC₁₋₃ alkylene OP(O)HOR^{C2}, -OC₁₋₃ alkylene NR^{C1}P(O)HOH, and -OC₁₋₃ alkylene NR^{C1}P(O)HOR^{C2}.

In some embodiments, each R^{C} is independently selected from the group consisting of -NR^{C1}C(O)OR^{C2}, -C₁₋₂ alkylene NR^{C1}P(O)(OH)₂, -C₁₋₂ alkylene OP(O)(OH)₂, -NR^{C1}P(O)(OH)₂, -NR^{C1}P(O)(OR^{C2})₂, -NR^{C1}C₁₋₂ alkylene OP(O)(OH)₂, -NR^{C1}C₁₋₂ alkylene NR^{C1}P(O)(OH)₂, -OP(O)(OH)₂, -OP(O)(OR^{C2})₂, -OC₁₋₂ alkylene OP(O)(OH)₂, - OC₁₋₂ alkylene NR^{C1}P(O)(OH)₂, -C₁₋₂ alkylene NR^{C1}P(O)HOH, -C₁₋₂ alkylene OP(O)HOH, -NR^{C1}P(O)HOH, - NR^{C1}P(O)HOR^{C2}, -NR^{C1}C₁₋₃ alkylene OP(O)HOH, -NR^{C1}C₁₋₃ alkylene NR^{C1}P(O)HOH, -OP(O)HOH, - OP(O)HOR^{C2}, -OC₁₋₃ alkylene OP(O)HOH, and -OC₁₋₃ alkylene NR^{C1}P(O)HOH.

In some embodiments, each R^{C} is independently selected from the group consisting of

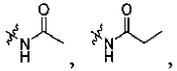

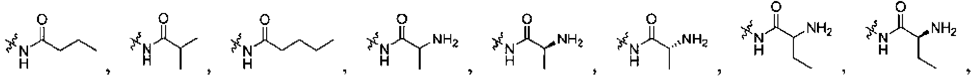

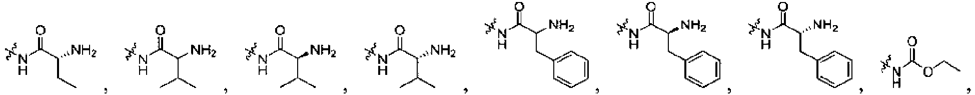

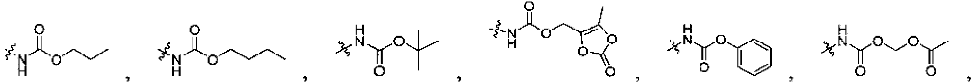

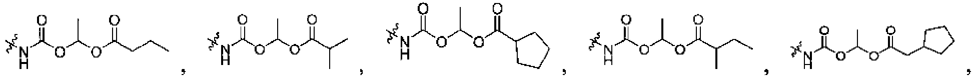

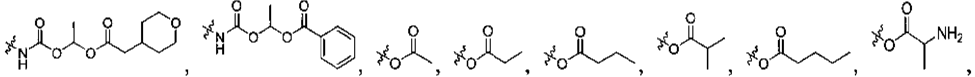

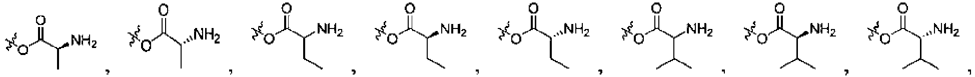

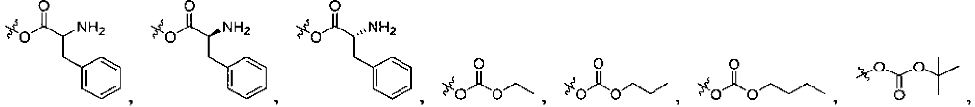

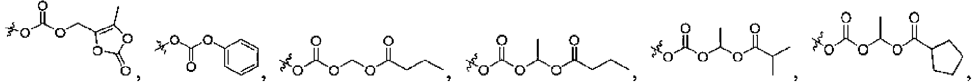

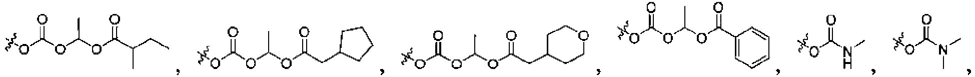

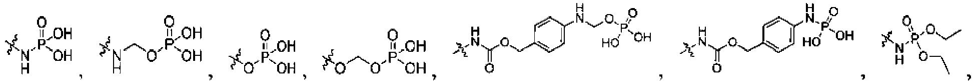

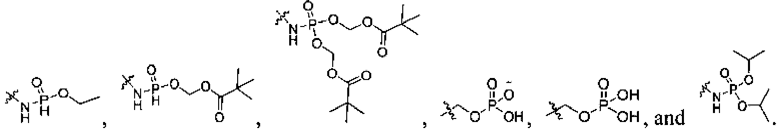

**In** some embodiments, each R^{C} is independently selected from the group consisting of 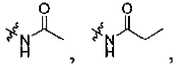 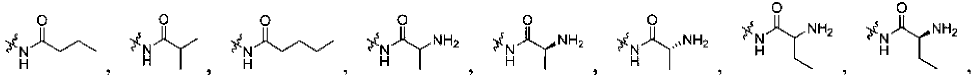 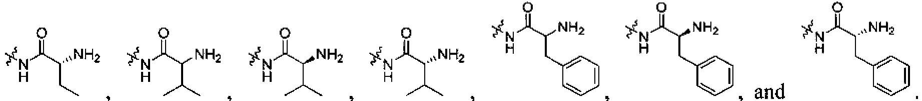 In some embodiments, each R^{C} is independently selected from the group consisting of 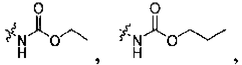 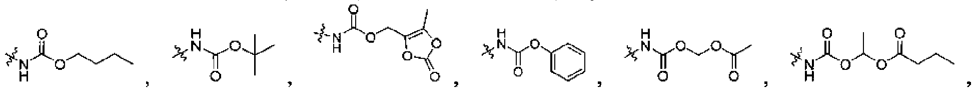 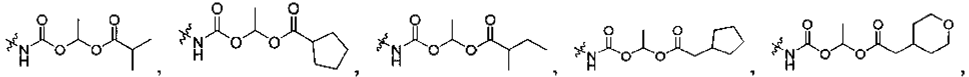 and 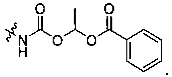 In some embodiments, each R^{C} is independently selected from the group consisting of 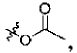 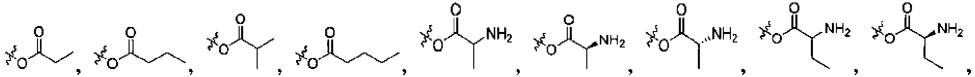 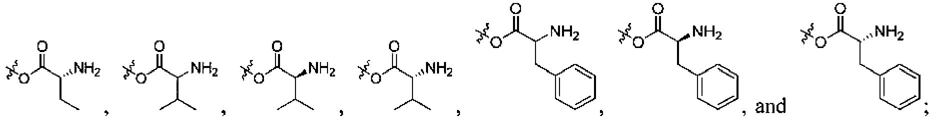 in some embodiments, each R^{C} is independently selected from the group consisting of 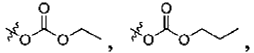 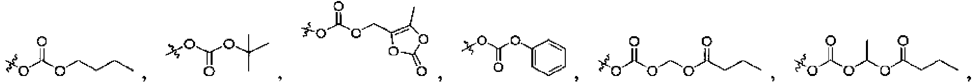 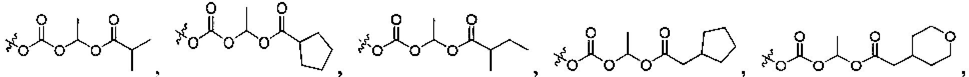 and 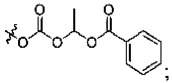 in some embodiments, each R^{C} is independently selected from the group consisting of 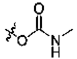 and 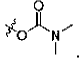 In some embodiments, each R^{C} is independently selected from the group consisting of

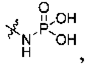

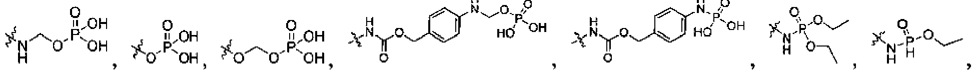

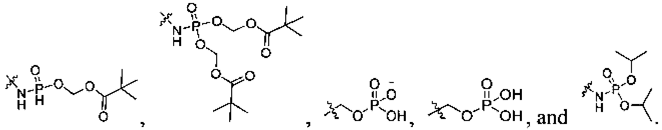

In some embodiments, R^{C1} is independently selected from the group consisting of H and C₁₋₄ alkyl.

In some embodiments, R^{C1} is independently selected from the group consisting of H and C₁₋₃ alkyl.

In some embodiments, R^{C1} is independently selected from the group consisting of H, methyl, ethyl, and isopropyl.

In some embodiments, R^{C1} is independently selected from the group consisting of H and methyl.

In some embodiments, R^{C1} is independently selected from H.

In some embodiments, R^{C2} is independently selected from the group consisting of H and the following groups optionally substituted with one or more R^{C3}: C₁₋₁₀ alkyl, C₁₋₁₀ heteroalkyl, 3- to 10-membered cycloalkyl, 3- to 10-membered heterocyclyl, 6- to 10-membered aryl, 5- to 10-membered heteroaryl, 3- to 10-membered cycloalkyl C₁₋₆ alkylene, 3- to 10-membered heterocyclyl C₁₋₆ alkylene, 6- to 10-membered aryl C₁₋₆ alkylene, and 5- to 10-membered heteroaryl C₁₋₆ alkylene.

In some embodiments, R^{C2} is independently selected from the group consisting of H and the following groups optionally substituted with one or more R^{C3}: C₁₋₈ alkyl, C₁₋₈ heteroalkyl, 3- to 8-membered cycloalkyl, 3- to 8-membered heterocyclyl, 6- to 10-membered aryl, 5- to 10-membered heteroaryl, 3- to 8-membered cycloalkyl C₁₋₄ alkylene, 3- to 8-membered heterocyclyl C₁₋₄ alkylene, 6- to 10-membered aryl C₁₋₄ alkylene, and 5- to 10-membered heteroaryl C₁₋₄ alkylene.

In some embodiments, R^{C2} is independently selected from the group consisting of H and the following groups optionally substituted with one or more R^{C3}: C₁₋₆ alkyl, C₁₋₆ heteroalkyl, 3- to 6-membered cycloalkyl, 3- to 6-membered heterocyclyl, phenyl, 5- to 6-membered heteroaryl, 3- to 6-membered cycloalkyl C₁₋₃ alkylene, 3- to 6-membered heterocyclyl C₁₋₃ alkylene, phenyl C₁₋₃ alkylene, and 5- to 6-membered heteroaryl C₁₋₃ alkylene.

In some embodiments, the "heteroalkyl" moiety in R^{C2} is selected from the group consisting of alkoxy, alkylamino, dialkylamino, alkoxyalkylene, alkylaminoalkylene, dialkylaminoalkylene, alkoxyalkyleneoxy, alkoxyalkyleneamino, alkylaminoalkyleneoxy, alkylaminoalkyleneamino, dialkylaminoalkyleneoxy, and dialkylaminoalkyleneamino.

In some embodiments, R^{C2} is independently selected from the group consisting of H and the following groups optionally substituted with one or more R^{C3}: C₁₋₆ alkyl, C₁₋₆ alkoxy, C₁₋₆ alkylamino, di-C₁₋₃ alkylamino, C₁₋₃ alkoxy C₁₋₃ alkylene, C₁₋₃ alkylamino C₁₋₃ alkylene, di-C₁₋₂ alkylamino C₁₋₂ alkylene, C₁₋₃ alkoxy C₁₋₃ alkyleneoxy, C₁₋₃ alkoxy C₁₋₃ alkyleneamino, C₁₋₃ alkylamino C₁₋₃ alkyleneoxy, C₁₋₃ alkylamino C₁₋₃ alkyleneamino, di-C₁₋₂ alkylamino C₁₋₂ alkyleneoxy, di-C₁₋₂ alkylamino C₁₋₂ alkyleneamino, 3- to 6-membered cycloalkyl, 3- to 6-membered heterocyclyl, phenyl, 5- to 6-membered heteroaryl, 3- to 6-membered cycloalkyl C₁₋₃ alkylene, 3- to 6-membered heterocyclyl C₁₋₃ alkylene, phenyl C₁₋₃ alkylene, and 5- to 6-membered heteroaryl C₁₋₃ alkylene.

In some embodiments, R^{C2} is independently selected from the group consisting of H and the following groups optionally substituted with one or more R^{C3}: C₁₋₆ alkyl, C₁₋₆ alkoxy, C₁₋₆ alkylamino, di-C₁₋₃ alkylamino, C₁₋₄ alkoxy C₁₋₂ alkylene, C₁₋₄ alkylamino C₁₋₂ alkylene, di-C₁₋₂ alkylamino C₁₋₂ alkylene, C₁₋₄ alkoxy C₁₋₂ alkyleneoxy, C₁₋₄ alkoxy C₁₋₂ alkyleneamino, C₁₋₄ alkylamino C₁₋₂ alkyleneoxy, C₁₋₄ alkylamino C₁₋₂ alkyleneamino, di-C₁₋₂ alkylamino C₁₋₂ alkyleneoxy, di-C₁₋₂ alkylamino C₁₋₂ alkyleneamino, 3- to 6-membered cycloalkyl, 3- to 6-membered heterocyclyl, phenyl, 5- to 6-membered heteroaryl, 3- to 6-membered cycloalkyl C₁₋₃ alkylene, 3- to 6-membered heterocyclyl C₁₋₃ alkylene, phenyl C₁₋₃ alkylene, and 5- to 6-membered heteroaryl C₁₋₃ alkylene.

In some embodiments, R^{C2} is independently selected from the group consisting of H and the following groups optionally substituted with one or more R^{C3}: C₁₋₆ alkyl, C₁₋₆ alkoxy, C₁₋₆ alkylamino, di-C₁₋₃ alkylamino, C₁₋₅ alkoxymethylene, C₁₋₅ alkylaminomethylene, di-C₁₋₂ alkylaminomethylene, C₁₋₅, alkoxymethyleneoxy, C₁₋₅ alkoxymethyleneamino, C₁₋₅ alkylaminomethyleneoxy, C₁₋₅ alkylaminomethyleneamino, di-C₁₋₂ alkylaminomethyleneoxy, di-C₁₋₂ alkylaminomethyleneamino, 3- to 6-membered cycloalkyl, 3- to 6-membered heterocyclyl, phenyl, 5- to 6-membered heteroaryl, 3- to 6-membered cycloalkyl C₁₋₃ alkylene, 3- to 6-membered heterocyclyl C₁₋₃ alkylene, phenyl C₁₋₃ alkylene, and 5- to 6-membered heteroaryl C₁₋₃ alkylene.

In some embodiments, R^{C2} is independently selected from the group consisting of H and the following groups optionally substituted with one or more R^{C3}: C₁₋₆ alkyl, 3- to 6-membered cycloalkyl, 4- to 6-membered heterocyclyl, phenyl, 5- to 6-membered heteroaryl, 3- to 6-membered cycloalkyl C₁₋₃ alkylene, 4- to 6-membered heterocyclyl C₁₋₃ alkylene, phenyl C₁₋₃ alkylene, and 5- to 6-membered heteroaryl C₁₋₃ alkylene.

In some embodiments, R^{C2} is independently selected from the group consisting of H and the following groups optionally substituted with one or more R^{C3}: methyl, ethyl, n-propyl, isopropyl, n-butyl, isobutyl, sec-butyl, tert-butyl, cyclopropyl, cyclobutyl, cyclopentyl, cyclohexyl, azetidinyl, oxetanyl, pyrrolidinyl, tetrahydrofuranyl, piperidinyl, tetrahydropyranyl, piperazinyl, morpholinyl, dioxolyl, phenyl, pyrrolyl, pyrazolyl, imidazolyl, pyridinyl, pyridazinyl, pyrimidinyl, pyrazinyl, cyclopropyl C₁₋₃ alkylene, cyclobutyl C₁₋₃ alkylene, cyclopentyl C₁₋₃ alkylene, cyclohexyl C₁₋₃ alkylene, azetidinyl C₁₋₃ alkylene, oxetanyl C₁₋₃ alkylene, pyrrolidinyl C₁₋₃ alkylene, tetrahydrofuranyl C₁₋₃ alkylene, piperidinyl C₁₋₃ alkylene, tetrahydropyranyl C₁₋₃ alkylene, piperazinyl C₁₋₃ alkylene, morpholinyl C₁₋₃ alkylene, dioxolyl C₁₋₃ alkylene, phenyl C₁₋₃ alkylene, pyrrolyl C₁₋₃ alkylene, pyrazolyl C₁₋₃ alkylene, imidazolyl C₁₋₃ alkylene, pyridinyl C₁₋₃ alkylene, pyridazinyl C₁₋₃ alkylene, pyrimidinyl C₁₋₃ alkylene, and pyrazinyl C₁₋₃ alkylene.

In some embodiments, R^{C2} is independently selected from the group consisting of H and the following groups optionally substituted with one or more R^{C3}: methyl, ethyl, n-propyl, isopropyl, n-butyl, isobutyl, see-butyl, tert-butyl, cyclopentyl, phenyl, cyclopentyl C₁₋₃ alkylene, tetrahydropyranyl C₁₋₃ alkylene, dioxolyl C₁₋₃ alkylene, and phenyl C₁₋₃ alkylene.

In some embodiments, R^{C2} is independently selected from the group consisting of H and the following groups optionally substituted with one or more R^{C3}: methyl, ethyl, n-propyl, isopropyl, n-butyl, isobutyl, sec-butyl, tert-butyl, cyclopentyl, phenyl, cyclopentylmethylene, 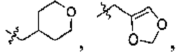 benzyl, and phenylethylidene.

In some embodiments, R^{C2} is independently selected from the group consisting of 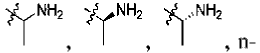 propyl, isopropyl, 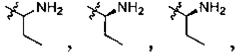 n-butyl, isobutyl, sec-butyl, tert-butyl, 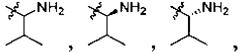 cyclopentyl, phenyl, cyclopentylmethylene, 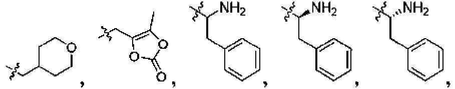 H, methyl, ethyl,

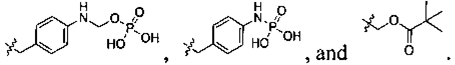

In some embodiments, each R^{C3} is independently selected from the group consisting of deuterium, oxo, halogen, - OH, -CN, -NH₂, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, di-C₁₋₃ haloalkylamino, -P(O)(OH)₂, -C₁₋₃ alkylene P(O)(OH)₂, -C₁₋₃ alkylene OP(O)(OH)₂, -C₁₋₃ alkylene NR^{C1}P(O)(OH)₂, -NR^{C1}P(O)(OH)₂, -NR^{C1}C₁₋₃ alkylene P(O)(OH)₂, - NR^{C1}C₁₋₃ alkylene OP(O)(OH)₂, -NR^{C1}C₁₋₃ alkylene NR^{C1}P(O)(OH)₂, -OP(O)(OH)₂, -OC₁₋₃ alkylene P(O)(OH)₂, - OC₁₋₃ alkylene OP(O)(OH)₂, -OC₁₋₃ alkylene NR^{C1}P(O)(OH)₂, -P(O)HOH, -C₁₋₃ alkylene P(O)HOH, -C₁₋₃ alkylene OP(O)HOH, -C₁₋₃ alkylene NR^{C1}P(O)HOH, -NR^{C1}P(O)HOH, -NR^{C1}C₁₋₃ alkylene P(O)HOH, -NR^{C1}C₁₋₃ alkylene OP(O)HOH, -NR^{C1}C₁₋₃ alkylene NR^{C1}P(O)HOH, -OP(O)HOH, -OC₁₋₃ alkylene P(O)HOH, -OC₁₋₃ alkylene OP(O)HOH, -OC₁₋₃ alkylene NR^{C1}P(O)HOH, -C(O)NR^{C1}C₁₋₄ alkyl, -NR^{C1}C(O)C₁₋₄ alkyl, -NR^{C1}C(O)OC₁₋₄ alkyl, -NR^{C1}C(O)NR^{C1}C₁₋₄ alkyl, -C(O)OC₁₋₄ alkyl, -OC(O)C₁₋₄ alkyl, -OC(O)OC₁₋₄ alkyl, and -OC(O)NR^{C1}C₁₋₄ alkyl.

In some embodiments, each R^{C3} is independently selected from the group consisting of deuterium, oxo, -F, -Cl, -Br, -I, -OH, -CN, -NH₂, methyl, ethyl, isopropyl, methoxy, methylthio, methylamino, dimethylamino, diethylamino, fluoromethyl, fluoromethoxy, -C₁₋₂ alkylene OP(O)(OH)₂, -C₁₋₂ alkylene NR^{C1}P(O)(OH)₂, -NR^{C1}P(O)(OH)₂, - NR^{C1}C₁₋₂ alkylene OP(O)(OH)₂, -NR^{C1}C₁₋₂ alkylene NR^{C1}P(O)(OH)₂, -OP(O)(OH)₂, -OC₁₋₂ alkylene OP(O)(OH)₂, -OC₁₋₂ alkylene NR^{C1}P(O)(OH)₂, -C₁₋₂ alkylene OP(O)HOH, -C₁₋₂ alkylene NR^{C1}P(O)HOH, -NR^{C1}P(O)HOH,-NR^{C1}C₁₋₂ alkylene OP(O)HOH, -NR^{C1}C₁₋₂ alkylene NR^{C1}P(O)HOH, -OP(O)HOH, -OC₁₋₂ alkylene OP(O)HOH, - OC₁₋₂ alkylene NR^{C1}P(O)HOH, -C(O)NR^{C1}C₁₋₄ alkyl, -NR^{C1}C(O)C₁₋₄ alkyl, -C(O)OC₁₋₄ alkyl, and -OC(O)C₁₋₄ alkyl.

In some embodiments, each R^{C3} is independently selected from the group consisting of deuterium, oxo, -F, -Cl, -Br, -OH, -NH₂, methyl, methoxy, methylthio, methylamino, dimethylamino, trifluoromethyl, trifluoromethoxy, - NHP(O)(OH)₂, -NHC₁₋₂ alkylene OP(O)(OH)₂, -NHC₁₋₂ alkylene NHP(O)(OH)₂, -OP(O)(OH)₂, -OC₁₋₂ alkylene OP(O)(OH)₂, -OC₁₋₂ alkylene NHP(O)(OH)₂, -NHP(O)HOH, -NHC₁₋₂ alkylene OP(O)HOH, -NHC₁₋₂ alkylene NHP(O)HOH, -OP(O)HOH, -OC₁₋₂ alkylene OP(O)HOH, -OC₁₋₂ alkylene NHP(O)HOH, -C(O)NR^{C1}C₁₋₄ alkyl, - NR^{C1}C(O)C₁₋₄ alkyl, -C(O)OC₁₋₄ alkyl, and -OC(O)C₁₋₄ alkyl.

In some embodiments, each R^{C3} is independently selected from the group consisting of deuterium, oxo, -F, -Cl, -Br, -OH, -NH₂, methyl, methoxy, methylthio, methylamino, dimethylamino, trifluoromethyl, trifluoromethoxy, - NHP(O)(OH)₂, -NHC₁₋₂ alkylene OP(O)(OH)₂, -NHC₁₋₂ alkylene NHP(O)(OH)₂, -OP(O)(OH)₂, -OC₁₋₂ alkylene OP(O)(OH)₂, -OC₁₋₂ alkylene NHP(O)(OH)₂, -NR^{C1}C(O)C₁₋₄ alkyl, -C(O)OC₁₋₄ alkyl, and -OC(O)C₁₋₄ alkyl.

In some embodiments, each R^{C3} is independently selected from the group consisting of deuterium, oxo, -F, -Cl, - OH, -NH₂, methyl,

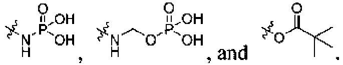

In some embodiments, R⁴ is selected from the group consisting of H, deuterium, halogen, -CN, C₁₋₄ alkyl, C₁₋₄ alkoxy, C₁₋₄ haloalkyl, and C₁₋₄ haloalkoxy.

In some embodiments, R⁴ is selected from the group consisting of H, deuterium, halogen, -CN, C₁₋₃ alkyl, C₁₋₃ alkoxy, C₁₋₃ haloalkyl, and C₁₋₃ haloalkoxy.

In some embodiments, R⁴ is selected from the group consisting of H, deuterium, -F, -Cl, -Br, -I, -CN, methyl, methoxy, difluoromethyl, trifluoromethyl, and trifluoromethoxy.

In some embodiments, R⁴ is selected from the group consisting of H, deuterium, -F, and -Cl.

In some embodiments, the compound of formula (I) is selected from an onium ion compound. In some embodiments, the compound of formula (I) is selected from a mono-onium ion compound. In some embodiments, the compound of formula (I) is selected from a bis-onium ion compound. In some embodiments, the compound of formula (I) is selected from a nitrogen-onium ion compound. In some embodiments, the compound of formula (I) is selected from an onium ion compound formed in the ring A moiety. In some embodiments, the compound of formula (I) is selected from an onium ion compound formed in the ring B moiety. In some embodiments, the compound of formula (I) is selected from an onium ion compound formed in the R³ moiety.

In some embodiments, the compound of formula (I) is selected from an onium ion compound, wherein each R^{C} is independently selected from the group consisting of -C₁₋₆ alkylene P(O)(OR^{C2})₂, -C₁₋₆ alkylene NR^{C1}P(O)(OR^{C2})₂, -C₁₋₆ alkylene OP(O)(OR^{C2})₂, -C₁₋₆ alkylene P(O)HOR^{C2}, -C₁₋₆ alkylene NR^{C1}P(O)HOR^{C2}, and -C₁₋₆ alkylene OP(O)HOR^{C2}.

In some embodiments, the compound of formula (I) is selected from an onium ion compound, wherein each R^{C} is independently selected from the group consisting of -C₁₋₄ alkylene P(O)(OR^{C2})₂, -C₁₋₄ alkylene NR^{C1}P(O)(OR^{C2})₂, -C₁₋₄ alkylene OP(O)(OR^{C2})₂, -C₁₋₄ alkylene P(O)HOR^{C2}, -C₁₋₄ alkylene NR^{C1}P(O)HOR^{C2}, and -C₁₋₄ alkylene OP(O)HOR^{C2}.

In some embodiments, the compound of formula (I) is selected from an onium ion compound, wherein each R^{C} is independently selected from the group consisting of -C₁₋₂ alkylene P(O)(OH)₂, -C₁₋₂ alkylene NHP(O)(OH)₂, and - C₁₋₂ alkylene OP(O)(OH)₂.

In some embodiments, the compound of formula (I) or a pharmaceutically acceptable salt thereof is selected from an onium ion compound or a pharmaceutically acceptable salt thereof.

In some embodiments, the compound of formula (I) is selected from an onium ion compound, and the pharmaceutically acceptable salt of the compound of formula (I) is selected from an inner salt.

In some embodiments, the compound of formula (I) is selected from an onium ion compound, and the pharmaceutically acceptable salt of the compound of formula (I) is selected from a hydrochloride.

In some embodiments, the compound of formula (I) is selected from an onium ion compound, and the pharmaceutically acceptable salt of the compound of formula (I) is selected from a monohydrochloride.

In some embodiments, the C₁₋₁₂ is selected from the group consisting of C₁₋₁₀, C₁₋₈, C₁₋₆, C₁₋₄, C₁₋₃, and C₁₋₂.

In some embodiments, the C₁₋₆ alkyl is selected from the group consisting of C₁₋₄ alkyl, C₁₋₃ alkyl, and C₁₋₂ alkyl.

In some embodiments, the C₁₋₆ alkylene is selected from the group consisting of C₁₋₄ alkylene, C₁₋₃ alkylene, and C₁₋₂ alkylene.

In some embodiments, the halogen is selected from the group consisting of -F, -Cl, -Br, and -I.

In some embodiments, the halo is selected from the group consisting of fluoro, chloro, and bromo. In some embodiments, the halo is selected from the group consisting of fluoro and chloro. In some embodiments, the halo is selected from fluoro.

In some embodiments, the "3- to 12-membered" is selected from the group consisting of 3- to 10-membered, 3- to 8-membered, 3- to 6-membered, 4- to 10-membered, 4- to 9-membered, 4- to 8-membered, 4- to 7-membered, 4- to 6-membered, 5- to 10-membered, 5- to 8-membered, and 5- to 6-membered.

In some embodiments, the heteroalkylene contains 1 or 2 heteroatoms selected from the group consisting of N, O, and S.

In some embodiments, the heteroalkylene contains 1 or 2 heteroatoms selected from the group consisting of N and O.

In some embodiments, the heteroalkylene contains 1 N atom and 1 O atom.

In some embodiments, the heteroalkylene contains 1 N atom.

In some embodiments, the heteroalkylene contains 1 O atom.

In some embodiments, the heterocycloalkyl contains 1 or 2 heteroatoms selected from the group consisting of N and O.

In some embodiments, the heterocycloalkyl contains 1 N atom.

In some embodiments, the heterocycloalkyl contains 1 O atom.

In some embodiments, the heterocycloalkyl contains 1 N atom and 1 O atom.

In some embodiments, the heterocyclyl or heteroaryl contains 1 or 2 heteroatoms selected from the group consisting of N, O, and S.

In some embodiments, the heterocyclyl or heteroaryl contains 1 or 2 N atoms.

In some embodiments, the heterocyclyl or heteroaryl contains 1 N atom and 1 O atom.

In some embodiments, the heterocyclyl or heteroaryl contains 1 N atom and 1 S atom.

In some embodiments, the heterocyclyl or heterocycloalkyl includes a monocyclic ring, a spiro ring, a fused ring, and a bridged ring. In some embodiments, the heterocyclyl or heterocycloalkyl includes a monocyclic ring, a fused ring, and a spiro ring.

In some embodiments, the "one or more" in the present disclosure may refer to an integer ranging from one to ten. For example, "one or more" refers to one, two, three, four, five, six, seven, eight, nine, or ten; or "one or more" refers to one, two, three, four, five, or six; or "one or more" refers to one, two, three, or four; or "one or more" refers to one, two, or three; or "one or more" refers to one or two.

The present disclosure relates to compounds of formulas (II), (III), (III-1), (III-1a), (IV-1), (IV-2), and (IV-3) or pharmaceutically acceptable salts thereof, 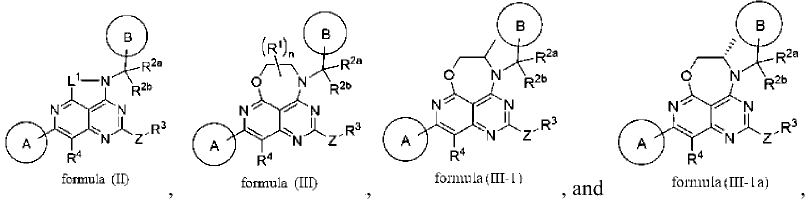 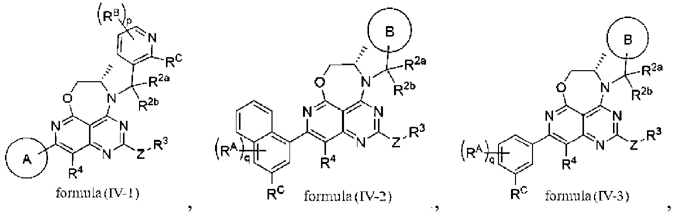 and 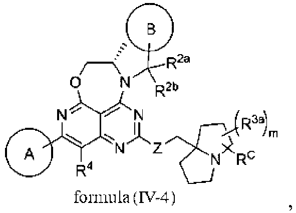 wherein the moieties L¹, R¹, R^{2a}, R^{2b}, R³, R⁴, R^{A}, R^{B}, R^{C}, Z, ring A, and ring B are as defined above; n is selected from the group consisting of 0, 1, 2, 3, 4, and 5; p is selected from the group consisting of 0, 1, 2, 3, 4, and 5; q is selected from the group consisting of 0, 1, 2, 3, 4, and 5; m is selected from the group consisting of 0, 1, 2, 3, 4, and 5.

In some embodiments, n is selected from the group consisting of 0, 1, 2, and 3.

In some embodiments, m is selected from the group consisting of 0, 1, 2, and 3.

In some embodiments, p is selected from the group consisting of 0, 1, 2, and 3.

In some embodiments, q is selected from the group consisting of 0, 1, 2, and 3.

In some embodiments, the present disclosure relates to compounds of formulas (A), (A-1), (B), (B-1), (C), (D), (E), (E-1), (F), (F-1), (G), and (H) or pharmaceutically acceptable salts thereof, 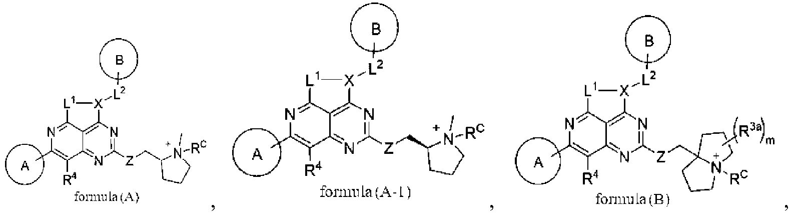 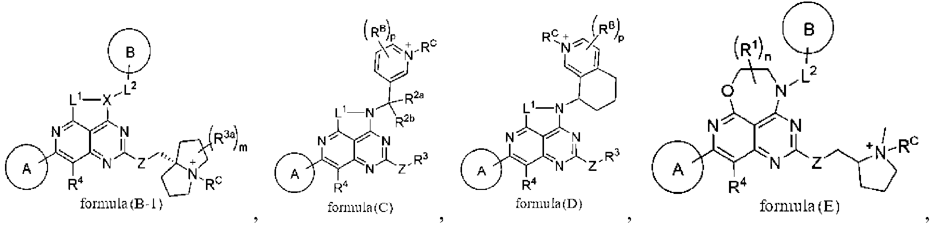 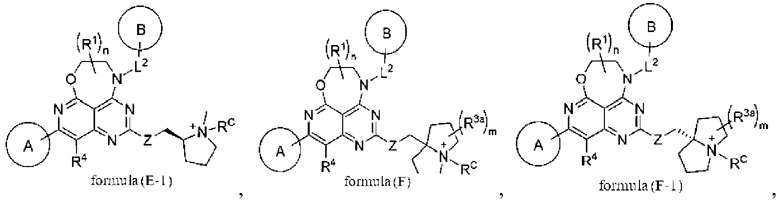 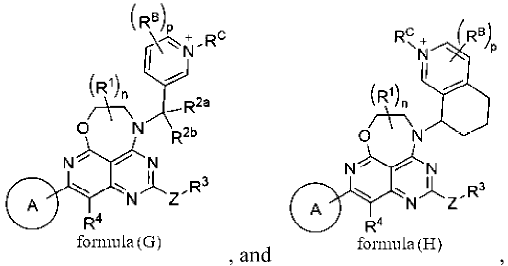 wherein the moieties L¹, L², X, R^{3a}, R^{2a}, R^{2b}, R³, R⁴, R^{B}, R^{C}, R¹, Z, ring A, and ring B are as defined above; n is selected from the group consisting of 0, 1, 2, 3, 4, and 5; m is selected from the group consisting of 0, 1, 2, 3, 4, and 5; p is selected from the group consisting of 0, 1, 2, 3, 4, and 5.

In some embodiments, R^{C} is selected from the group consisting of -C₁₋₆ alkylene P(O)(OR^{C2})₂, -C₁₋₆ alkylene NR^{C1}P(O)(OR^{C2})₂, -C₁₋₆ alkylene OP(O)(OR^{C2})₂, -C₁₋₆ alkylene P(O)HOR^{C2}, -C₁₋₆ alkylene NR^{C1}P(O)HOR^{C2}, and -C₁₋₆ alkylene OP(O)HOR^{C2}.

In some embodiments, R^{C} is selected from the group consisting of -C₁₋₄ alkylene P(O)(OR^{C2})₂, -C₁₋₄ alkylene NR^{C1}P(O)(OR^{C2})₂, -C₁₋₄ alkylene OP(O)(OR^{C2})₂, -C₁₋₄ alkylene P(O)HOR^{C2}, -C₁₋₄ alkylene NR^{C1}P(O)HOR^{C2}, and -C₁₋₄ alkylene OP(O)HOR^{C2}.

In some embodiments, R^{C} is selected from the group consisting of -C₁₋₂ alkylene P(O)(OH)₂, -C₁₋₂ alkylene NHP(O)(OH)₂, and -C₁₋₂ alkylene OP(O)(OH)₂.

In some embodiments, n is selected from the group consisting of 0, 1, 2, and 3.

In some embodiments, m is selected from the group consisting of 0, 1, 2, and 3.

In some embodiments, p is selected from the group consisting of 0, 1, 2, and 3.

In some embodiments, the compound disclosed herein does not include a compound of a formula selected from the group consisting of the following formulas, or a pharmaceutically acceptable salt thereof:

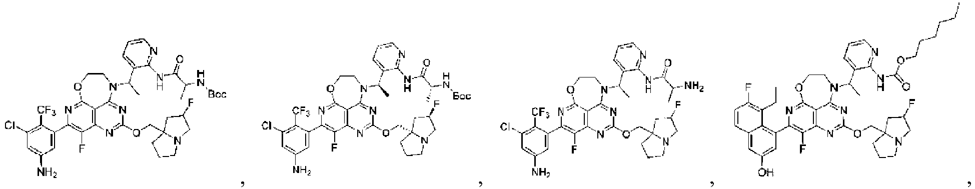

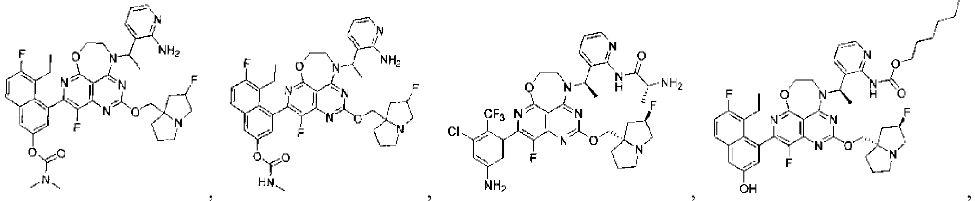

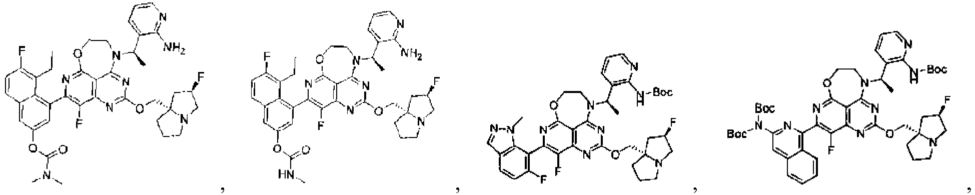

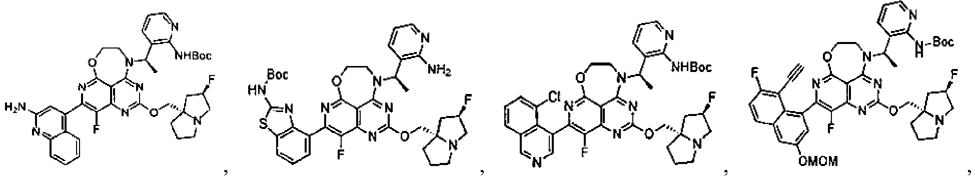

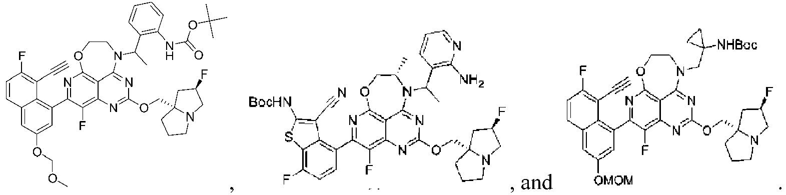

In some embodiments, the present disclosure encompasses the variables defined above and embodiments thereof, as well as any combination thereof.

The present disclosure further relates to a compound of a formula selected from the group consisting of the following formulas, or a pharmaceutically acceptable salt thereof:

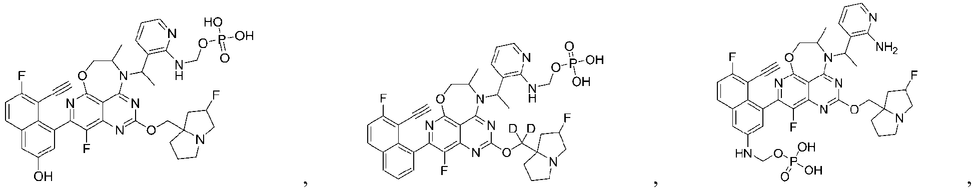

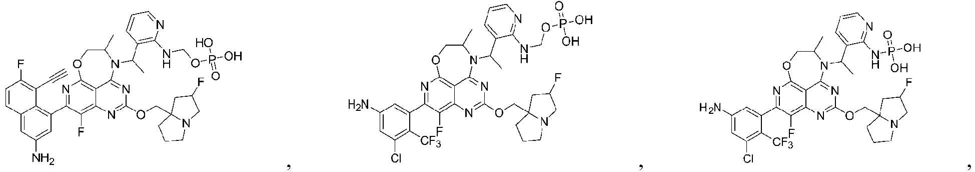

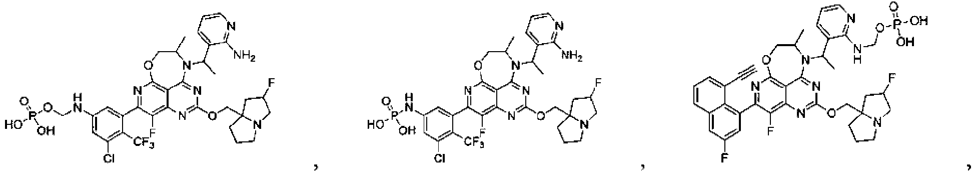

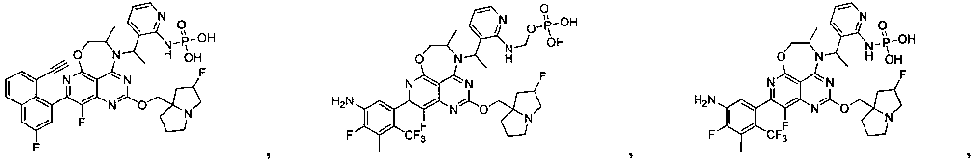

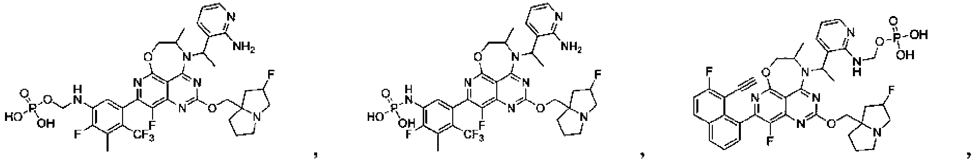

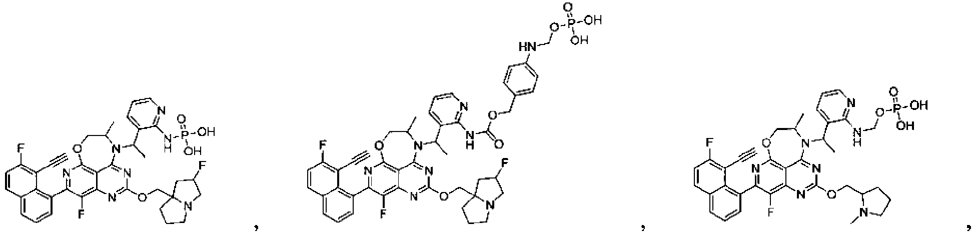

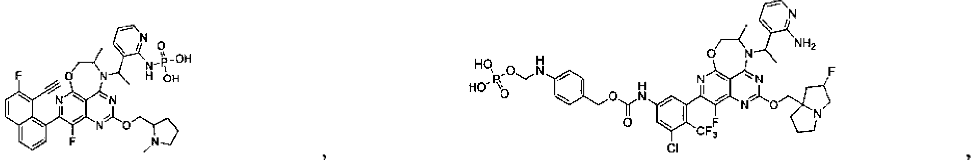

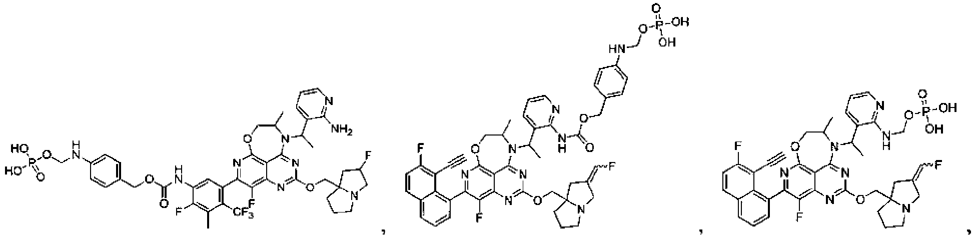

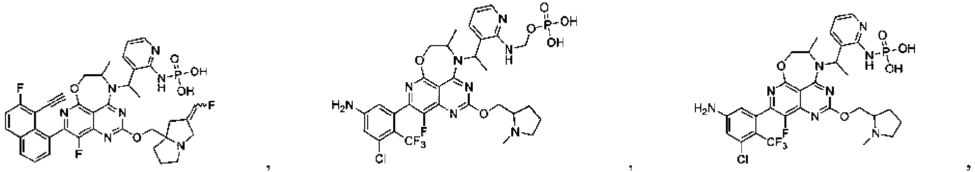

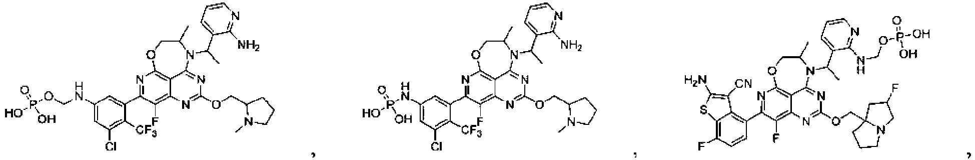

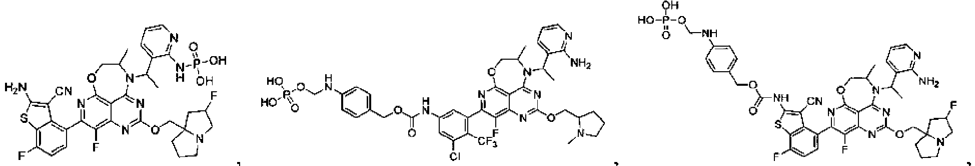

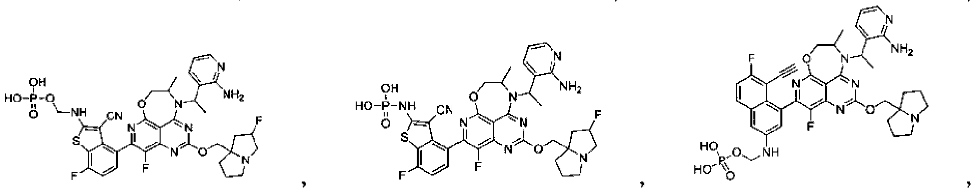

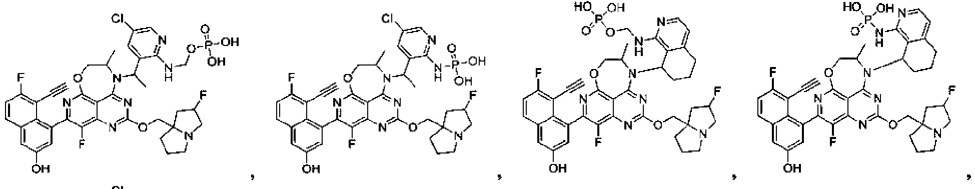

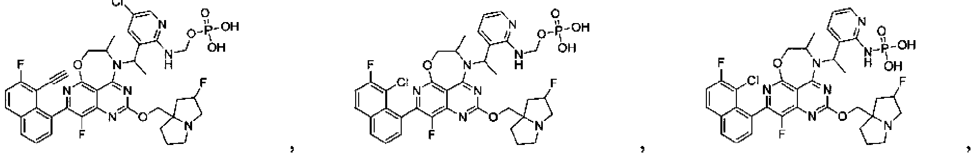

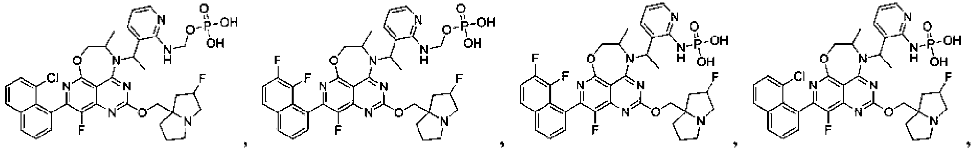

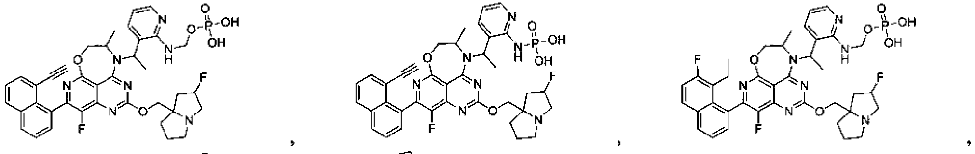

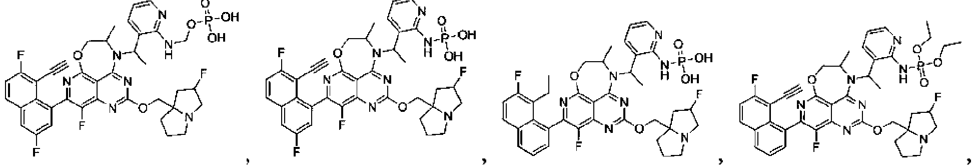

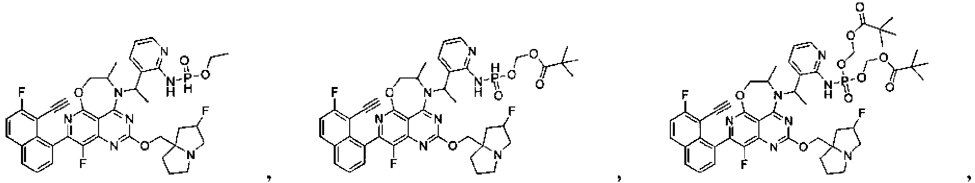

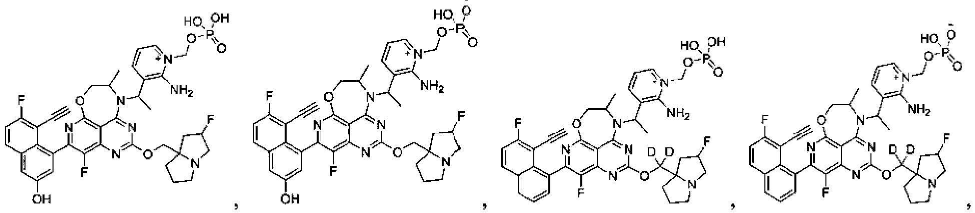

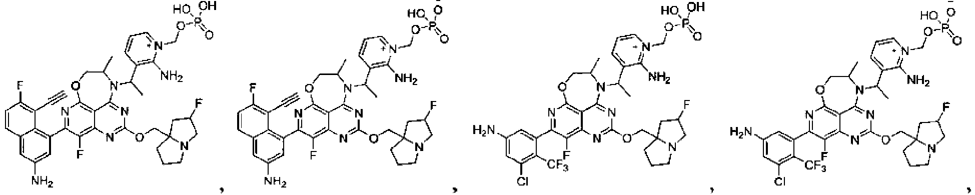

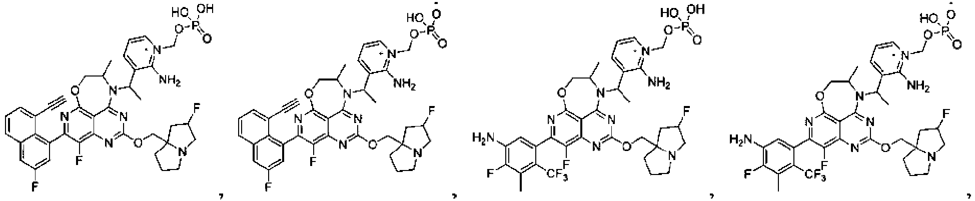

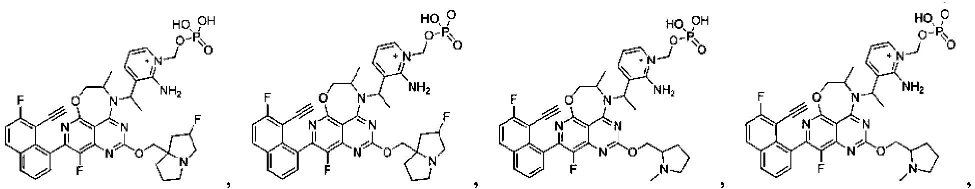

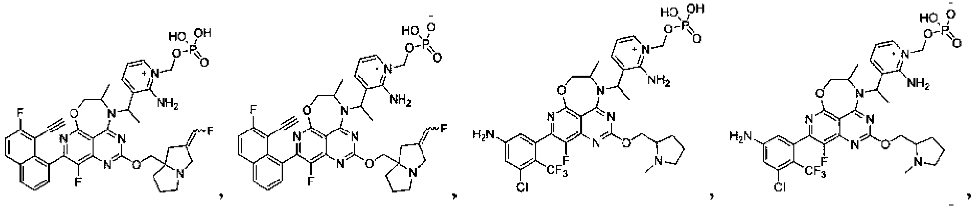

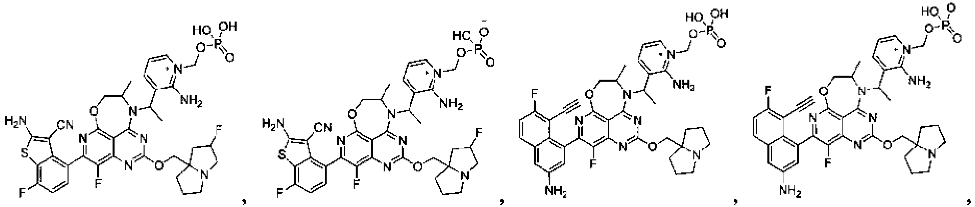

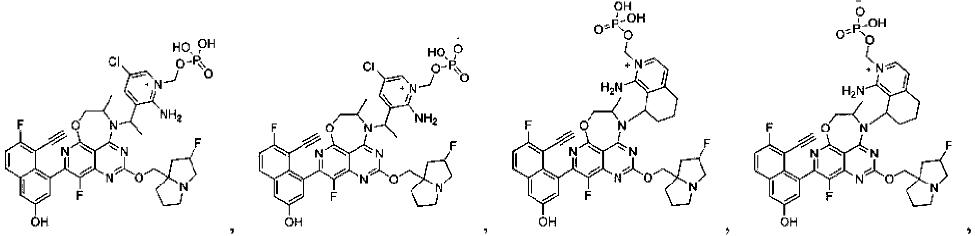

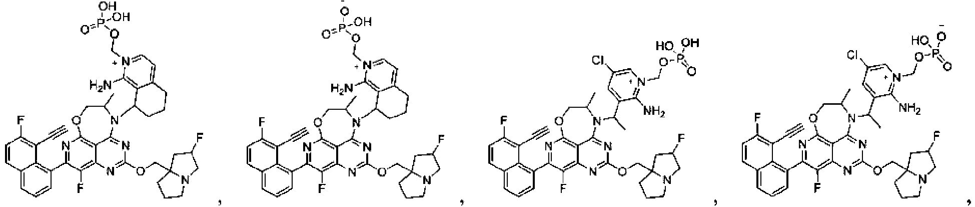

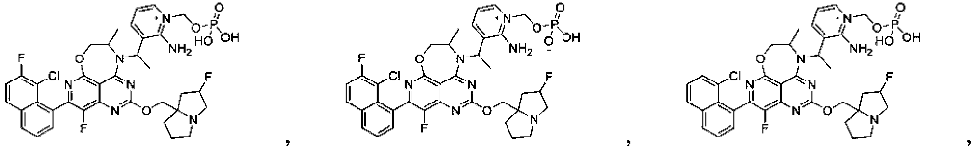

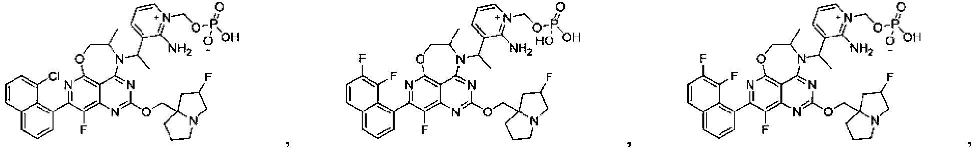

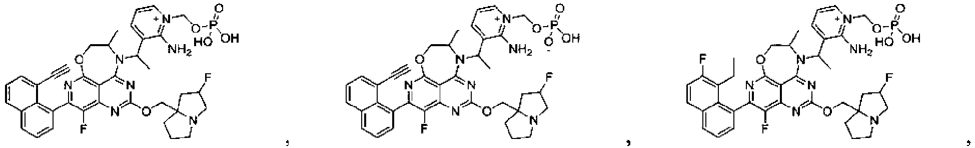

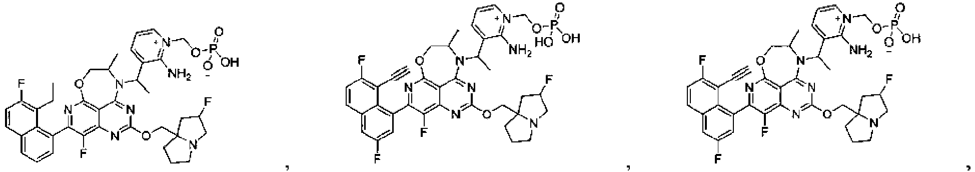

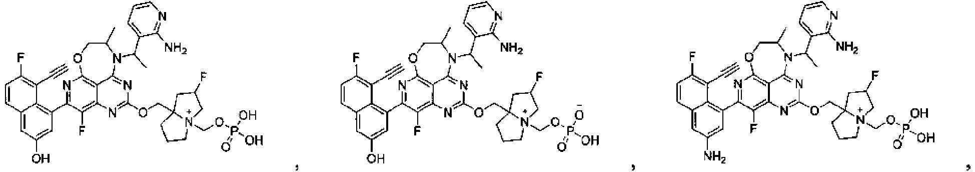

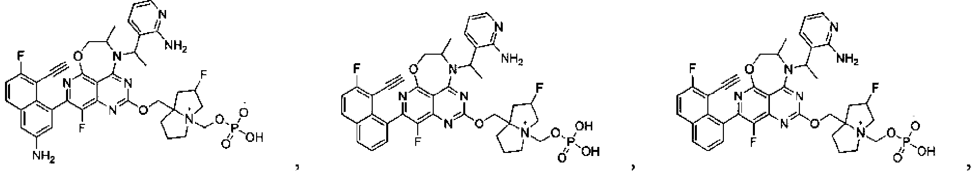

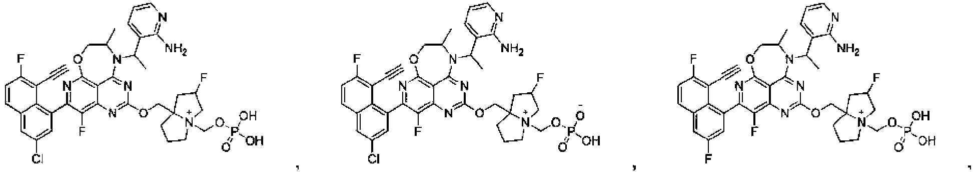

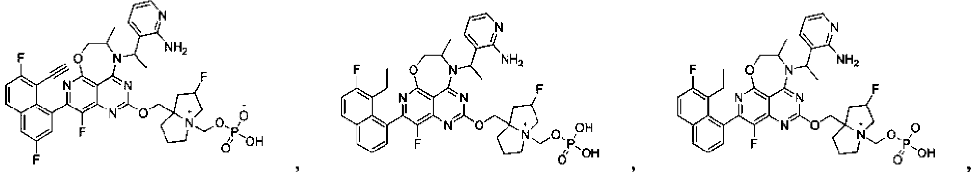

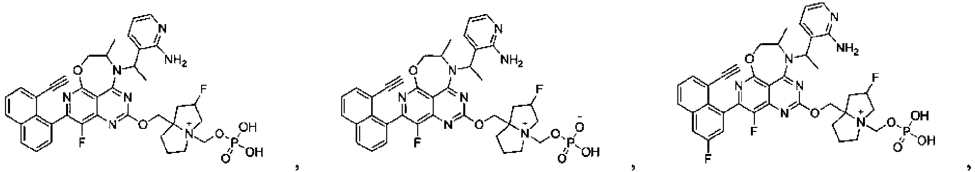

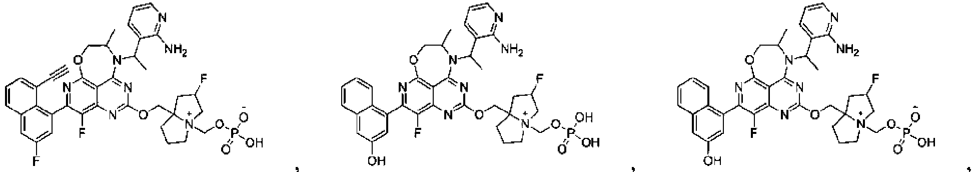

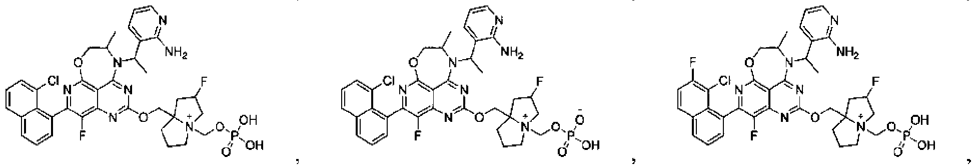

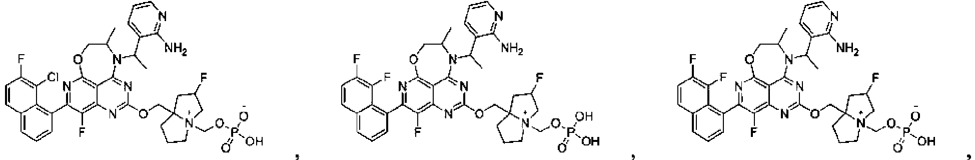

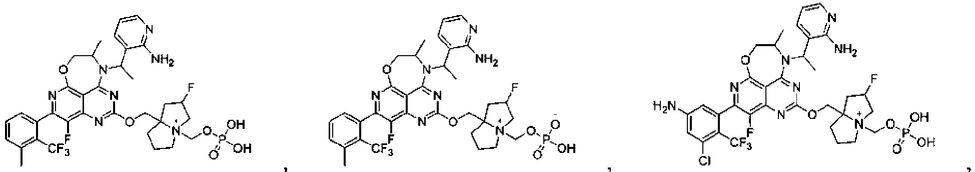

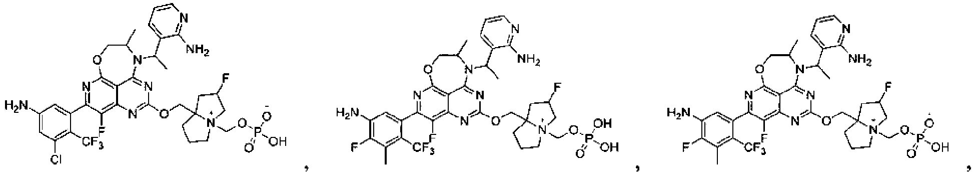

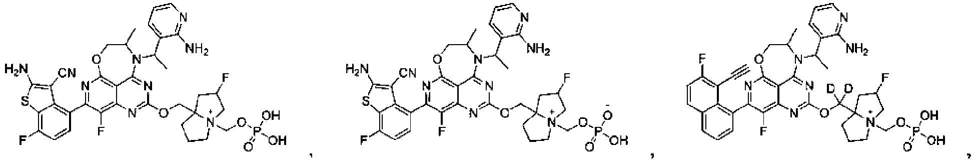

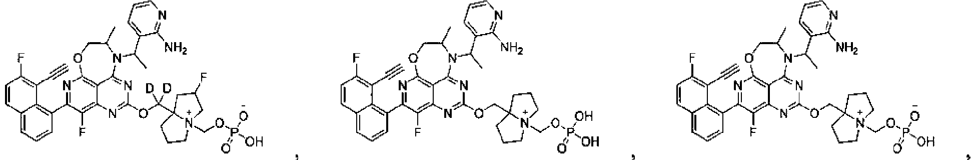

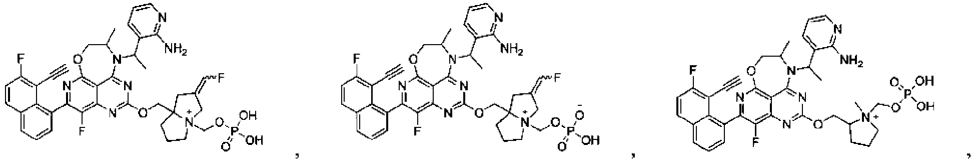

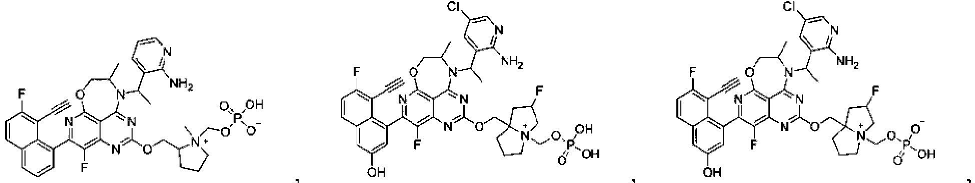

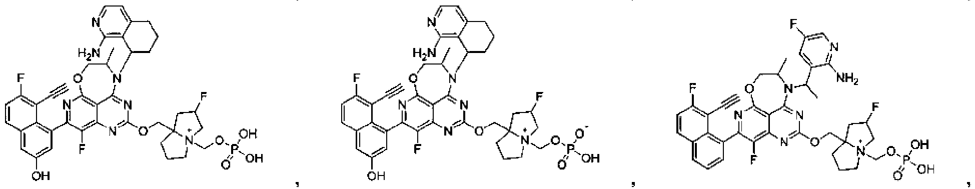

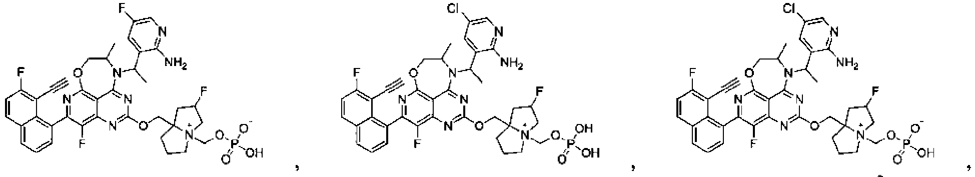

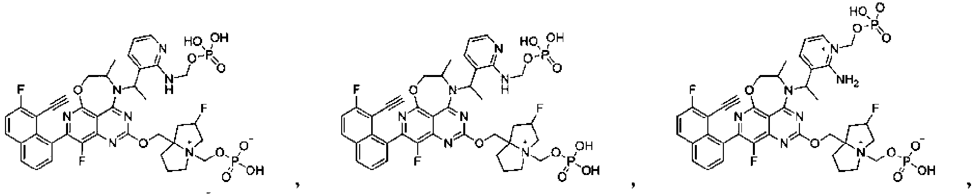

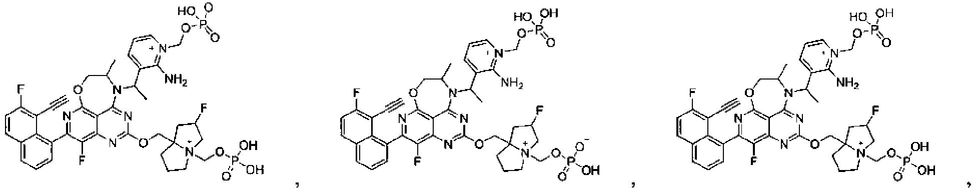

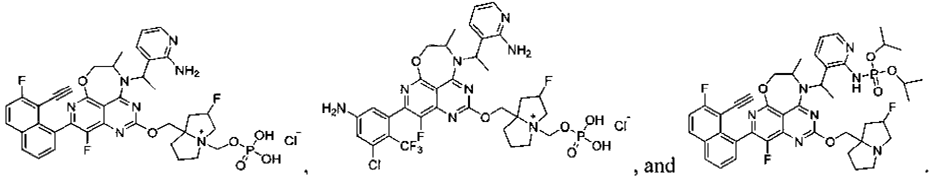

The present disclosure further relates to a compound of a formula selected from the group consisting of the following formulas, or a pharmaceutically acceptable salt thereof:

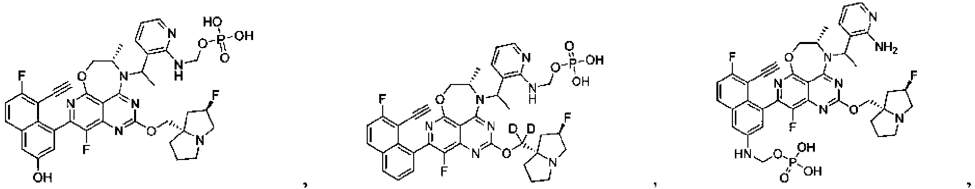

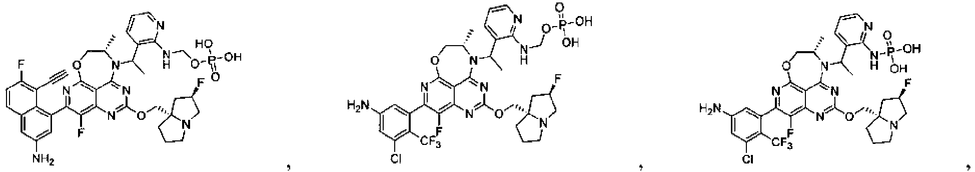

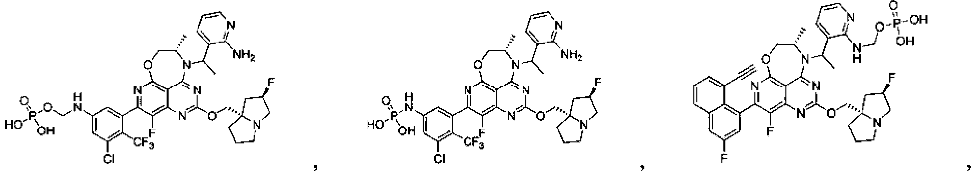

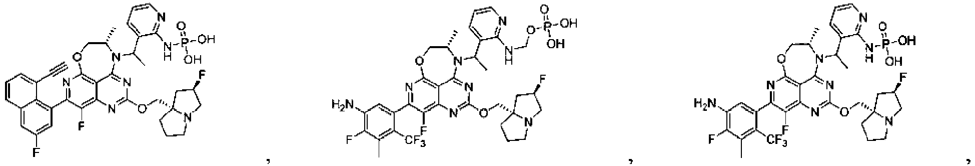

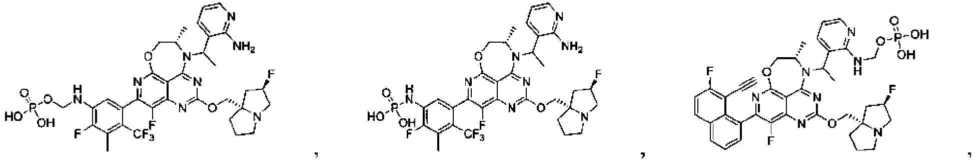

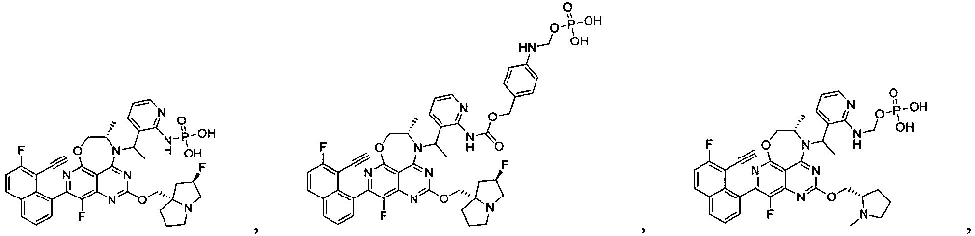

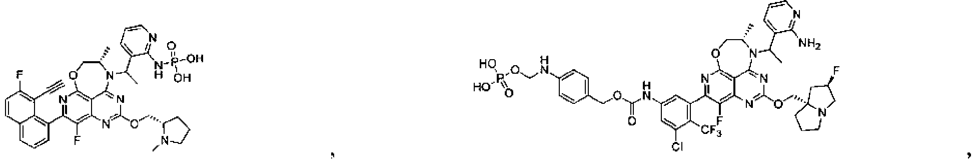

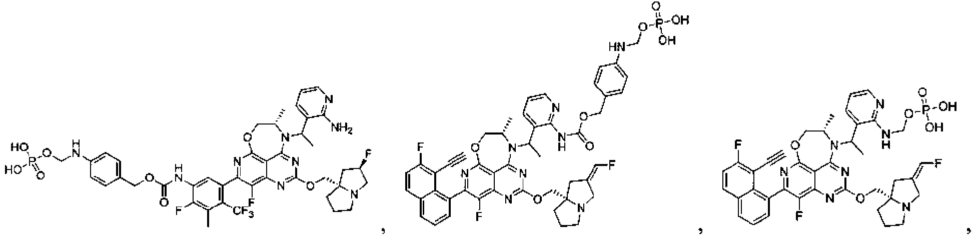

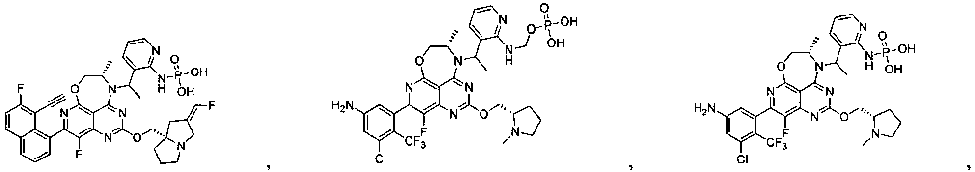

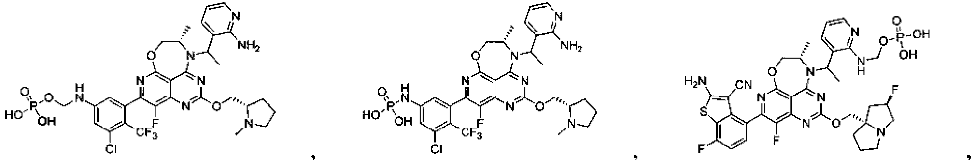

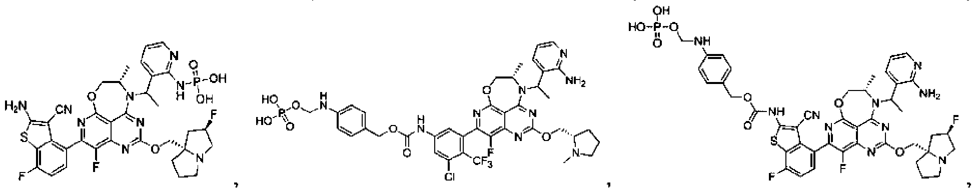

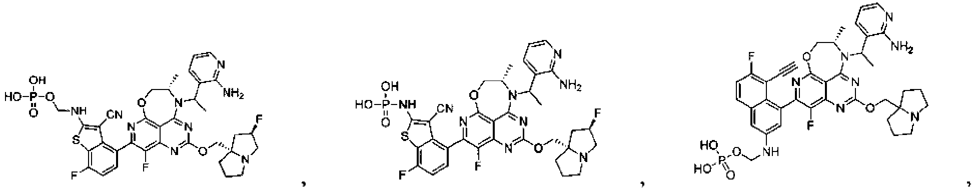

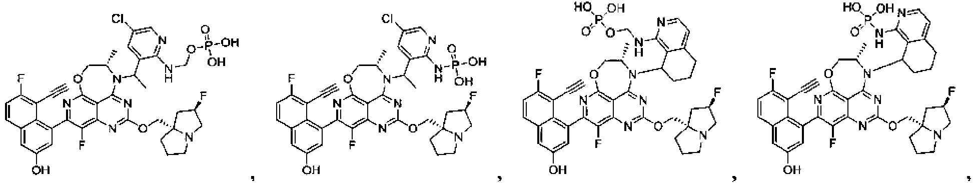

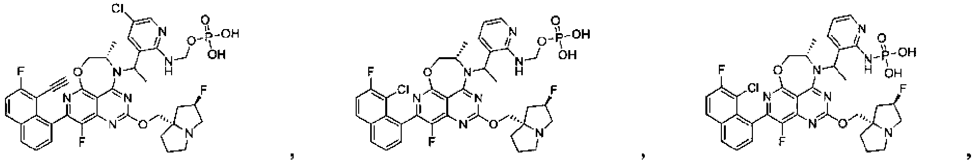

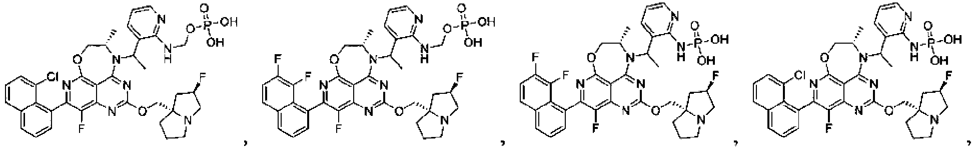

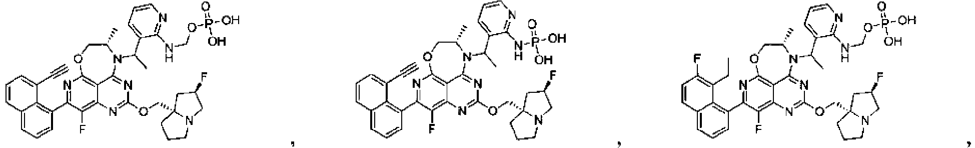

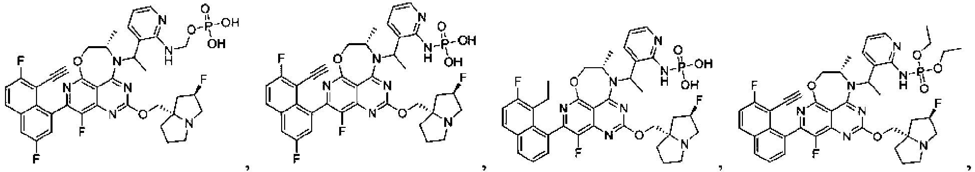

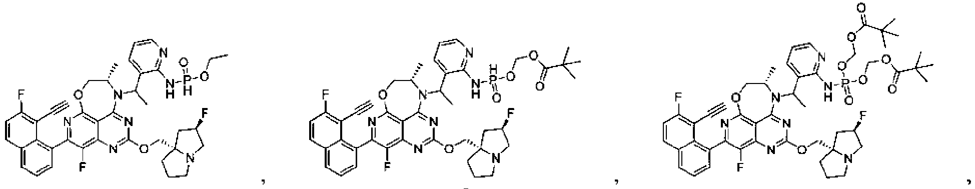

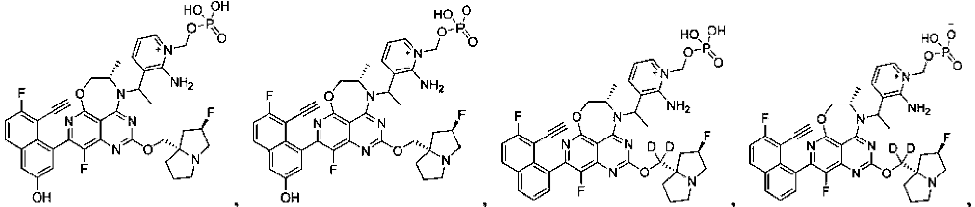

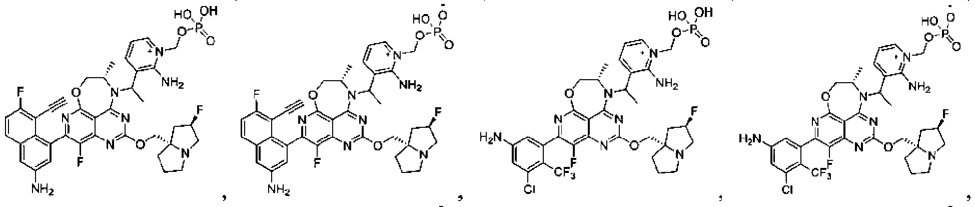

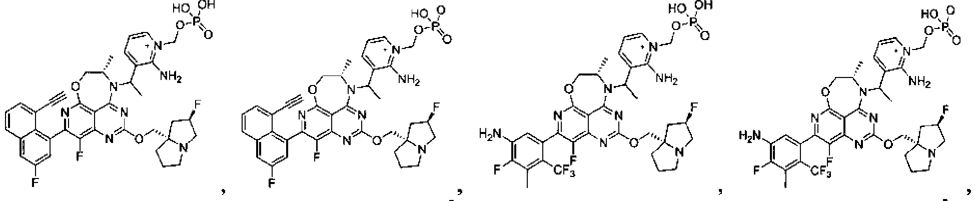

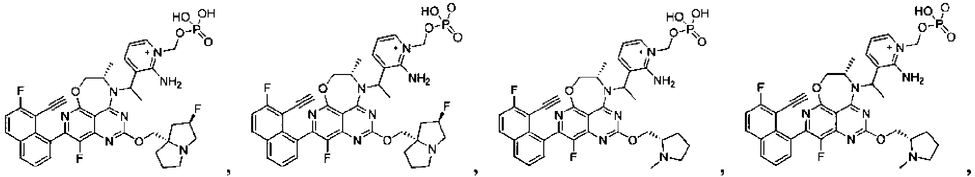

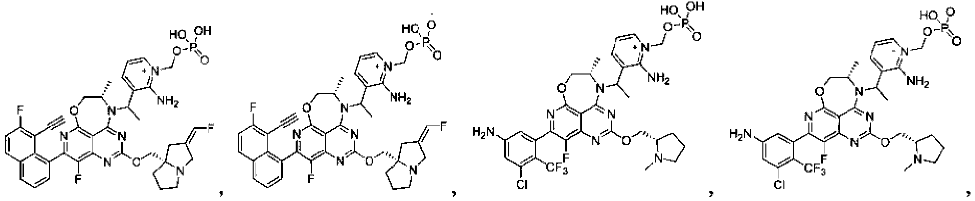

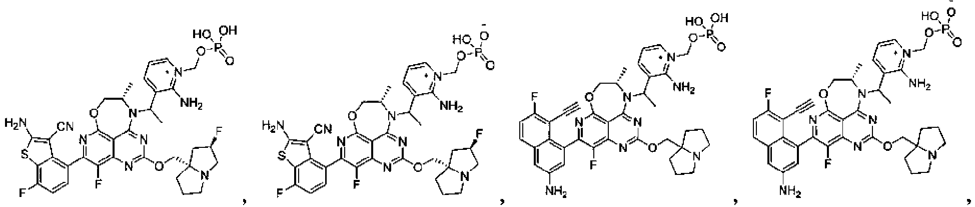

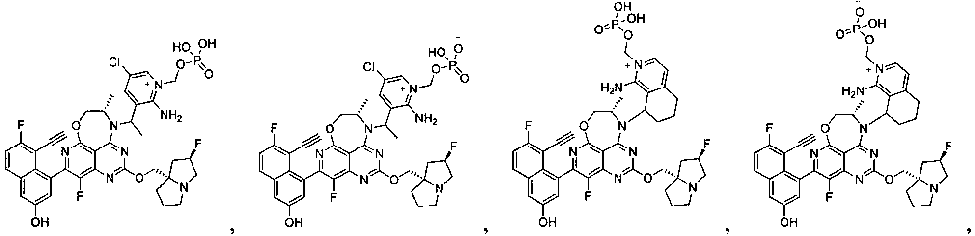

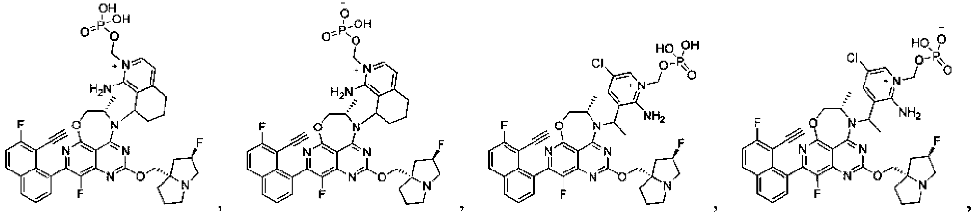

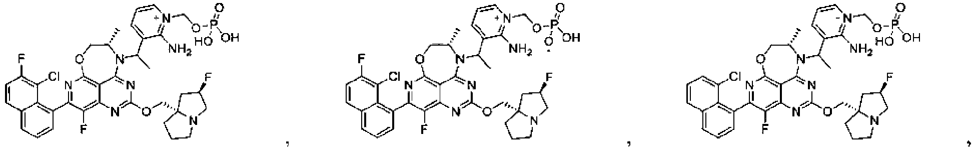

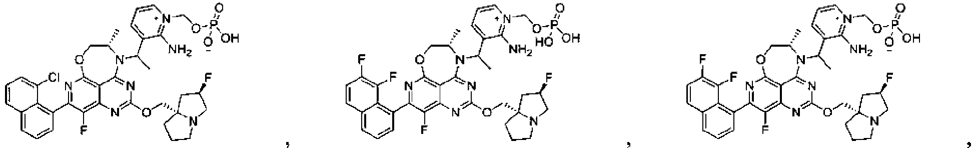

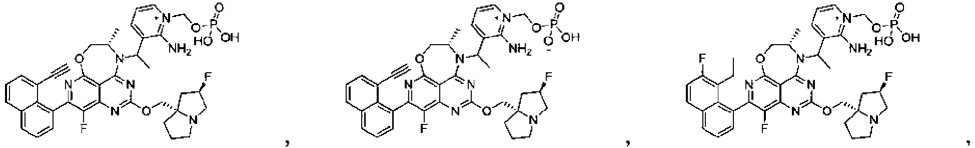

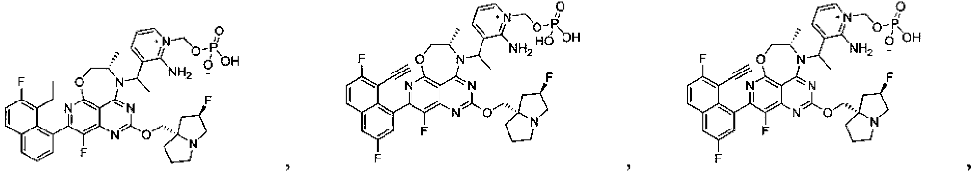

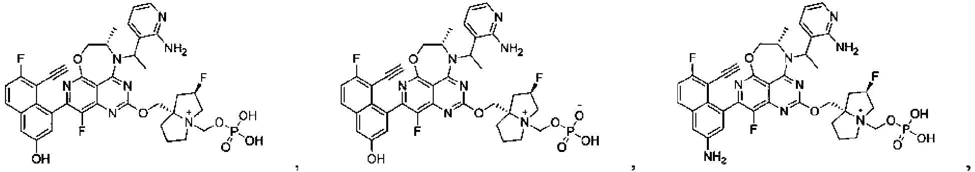

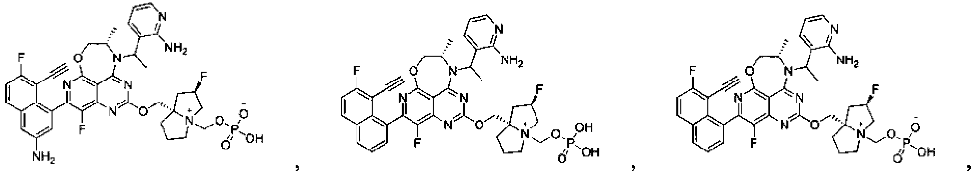

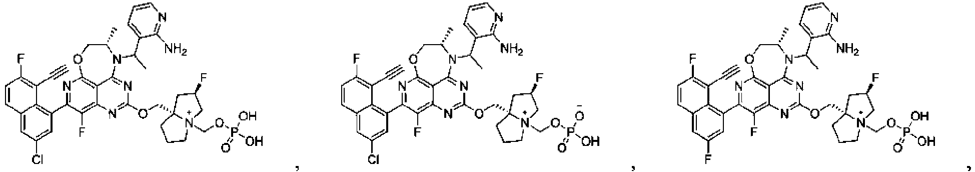

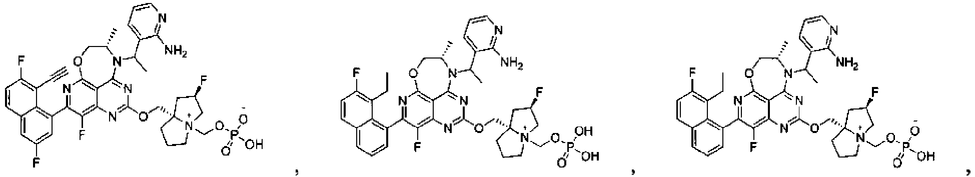

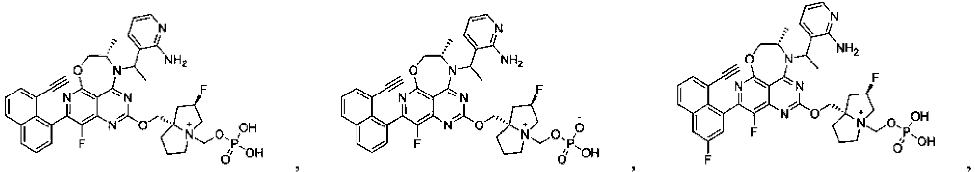

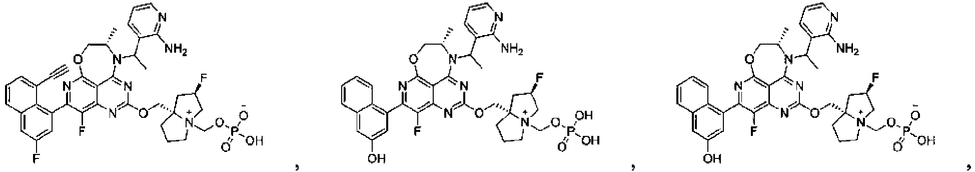

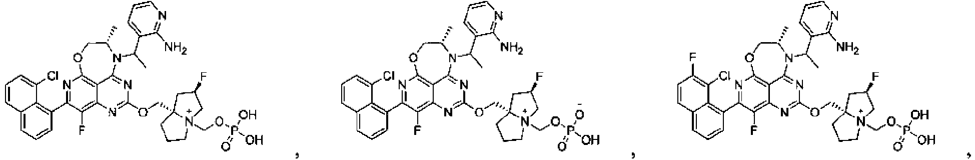

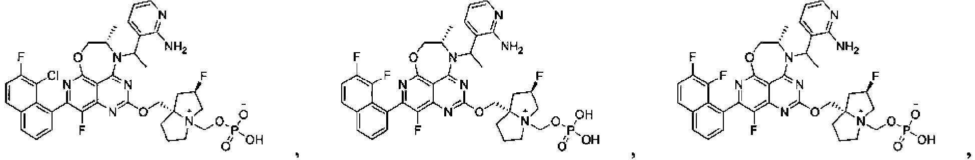

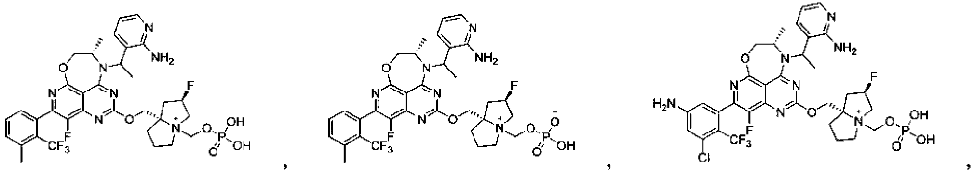

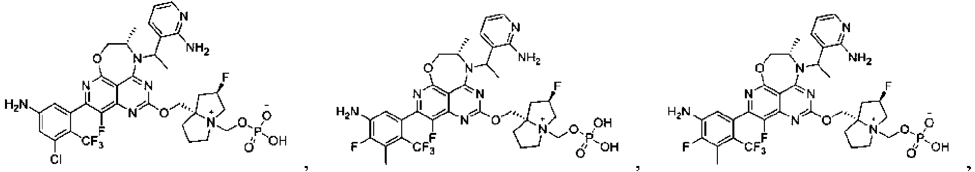

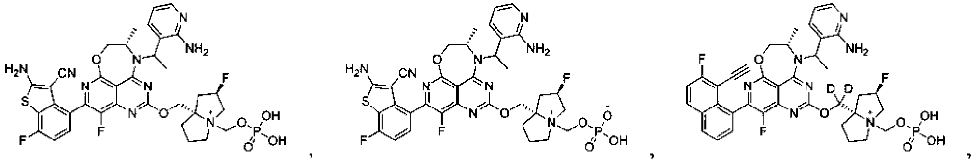

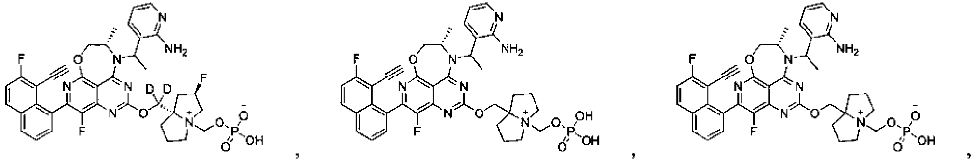

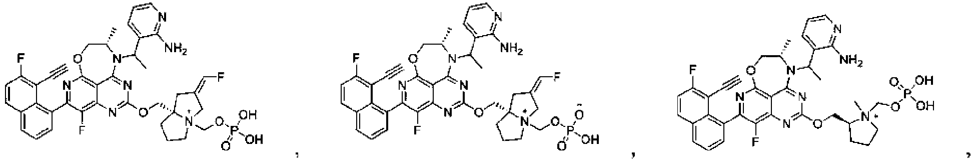

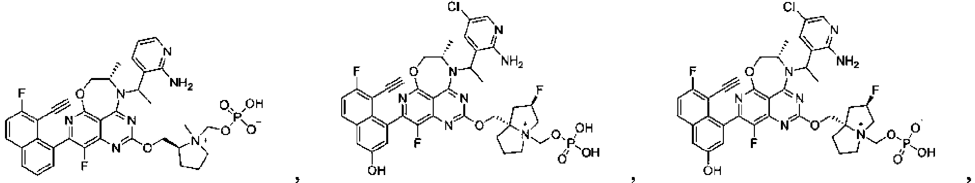

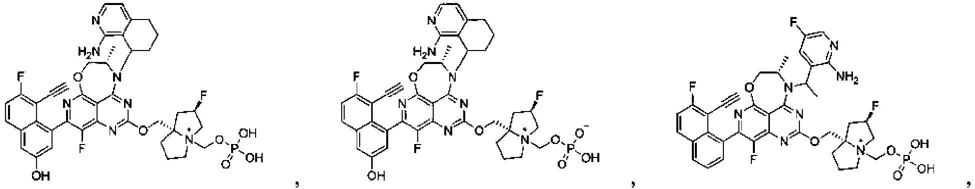

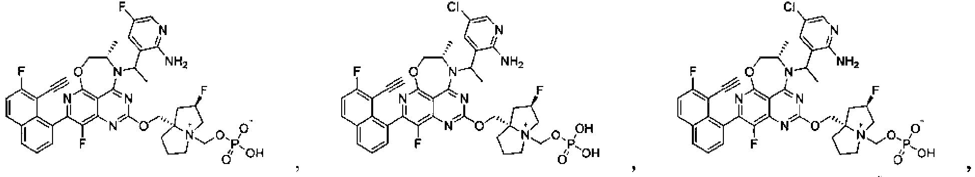

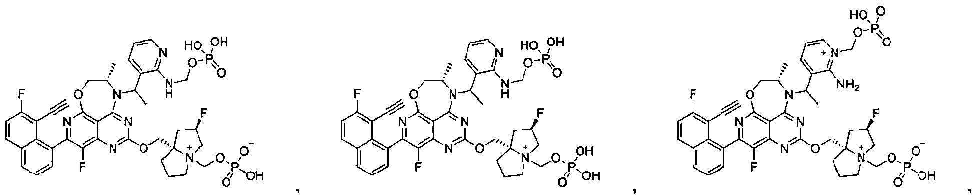

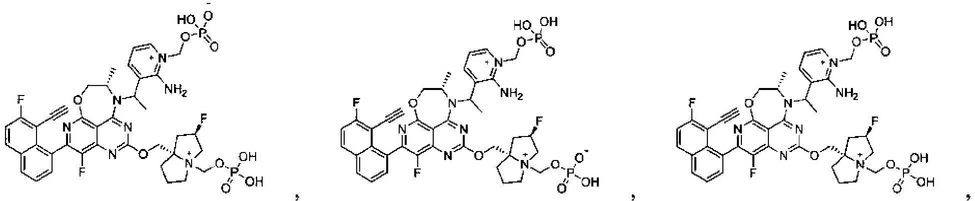

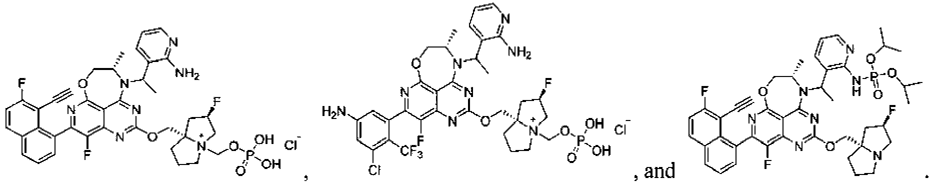

The present disclosure further relates to a compound of a formula selected from the group consisting of the following formulas, or a pharmaceutically acceptable salt thereof: 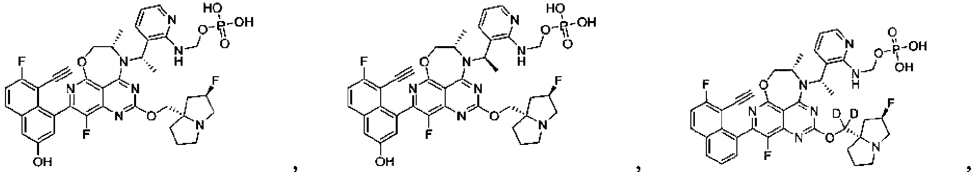 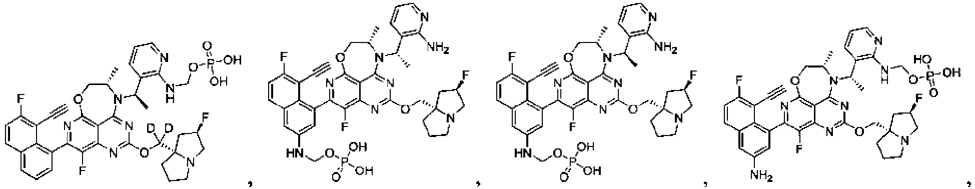 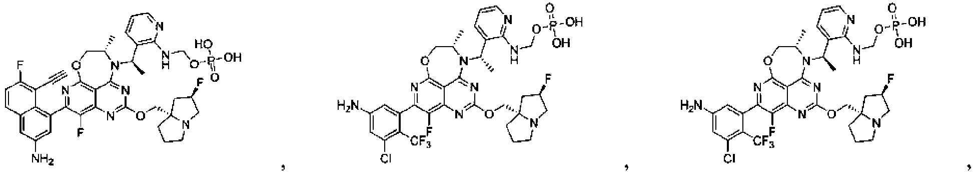 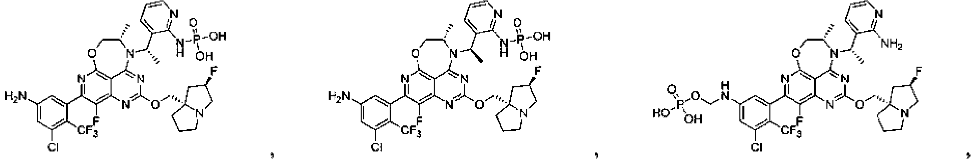 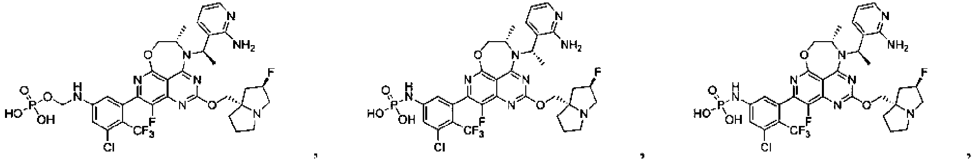 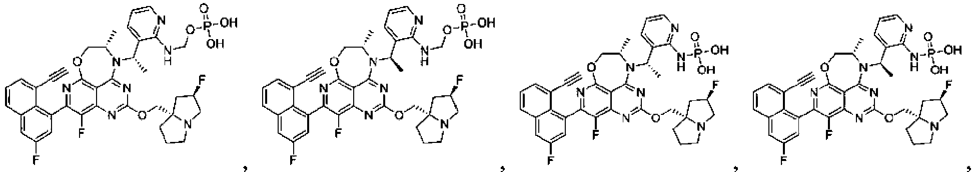 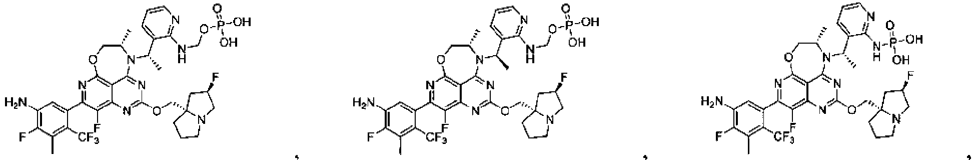 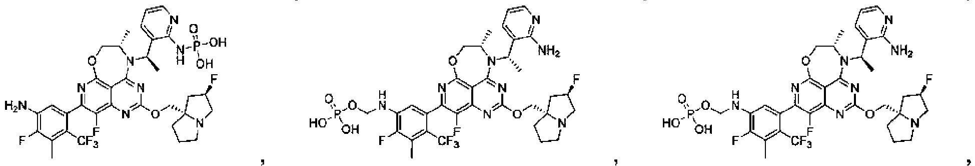 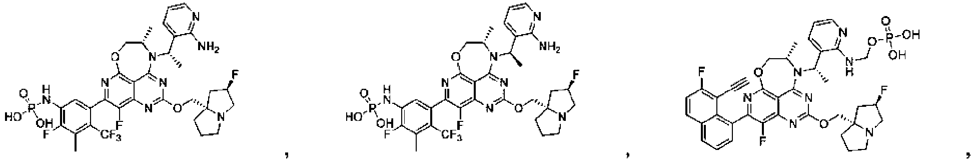 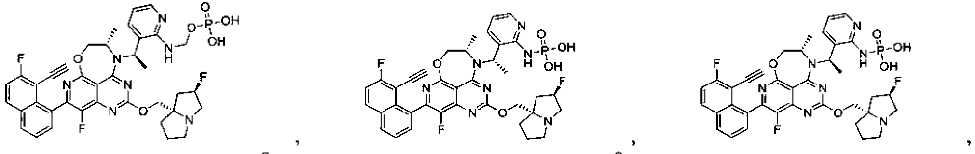 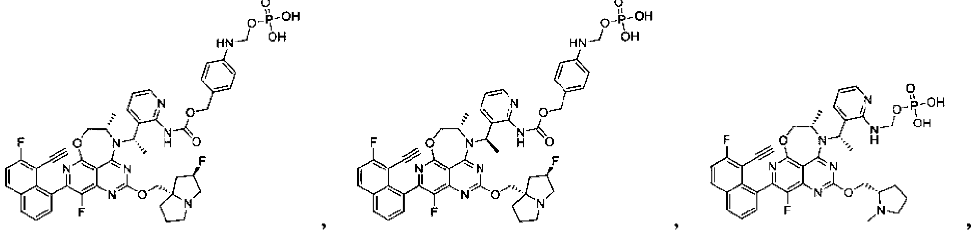 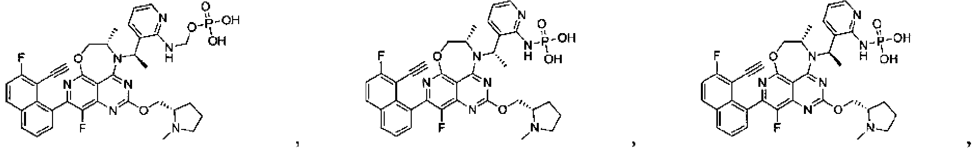 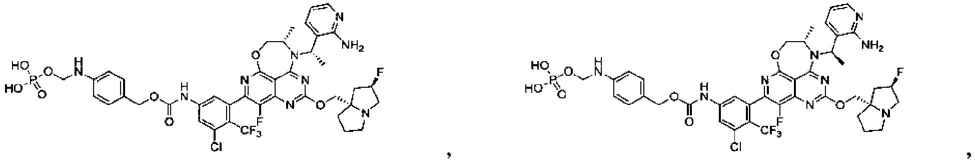 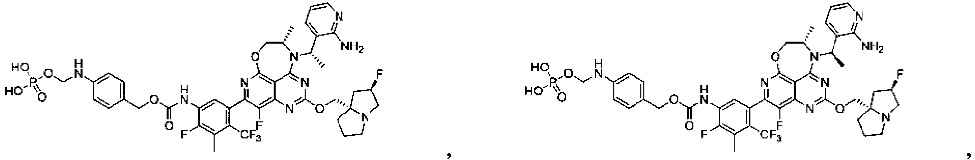 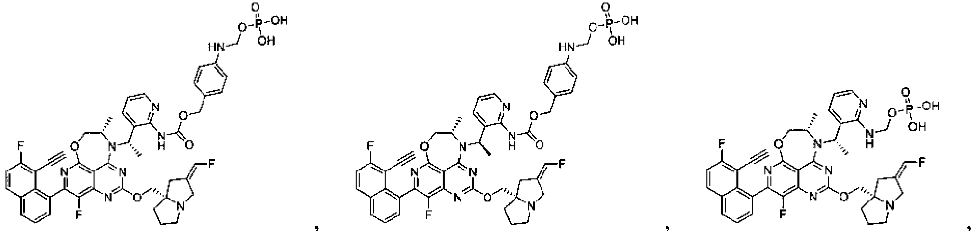 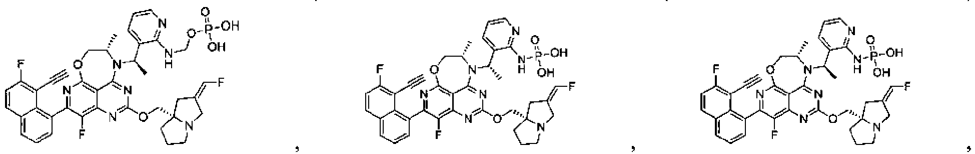 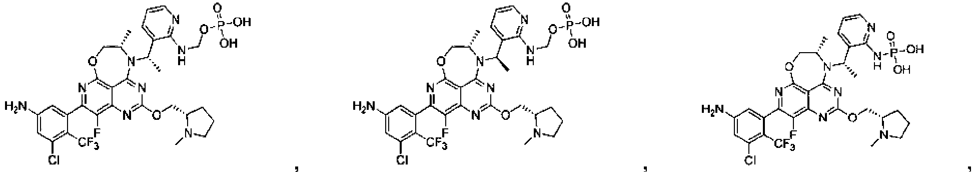 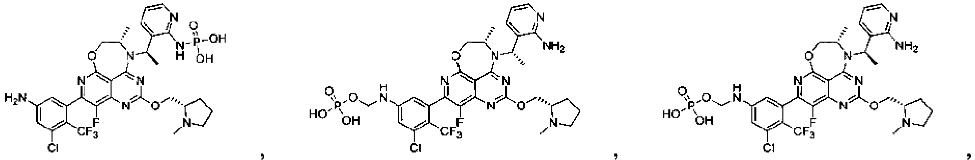 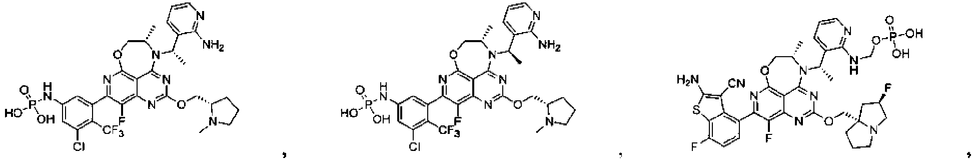 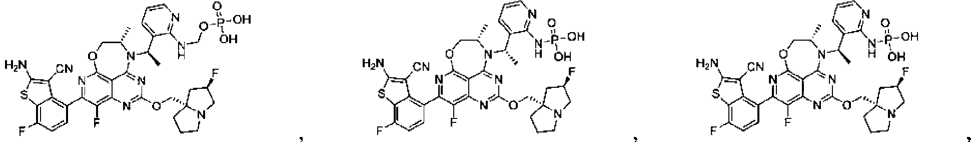 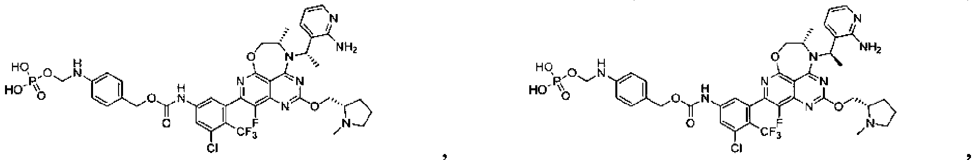 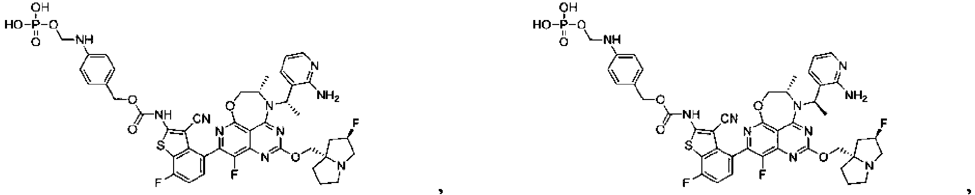 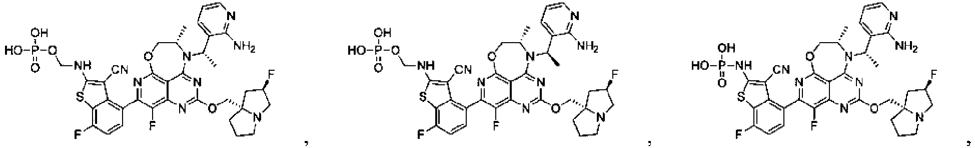 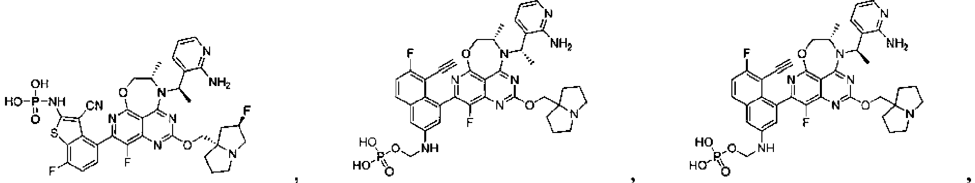 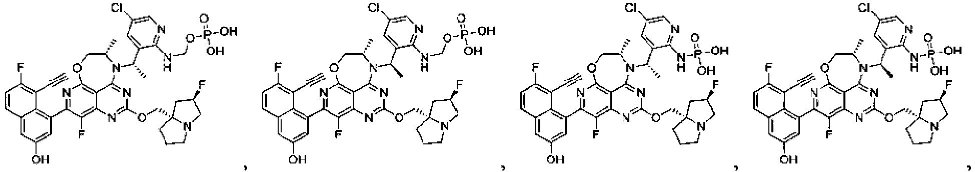 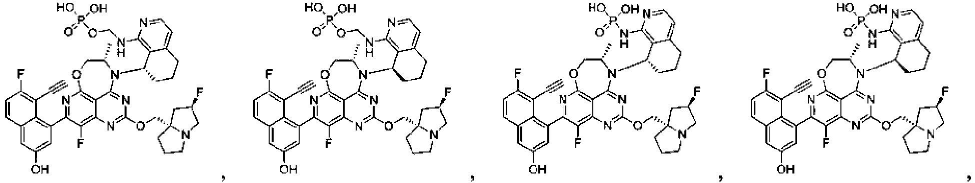 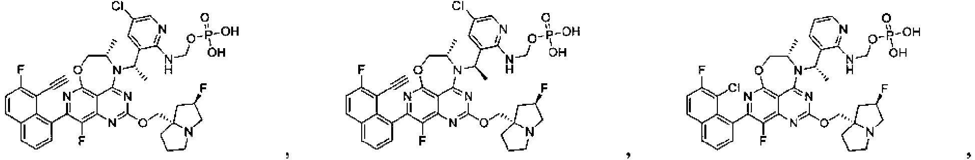 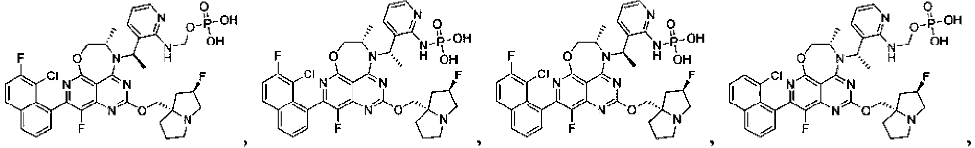 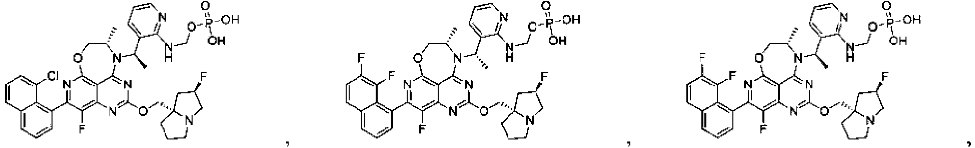 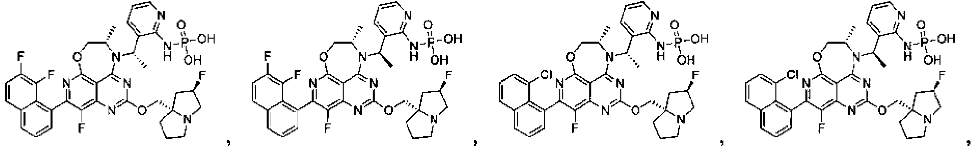 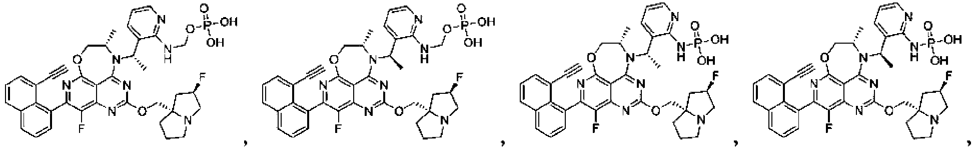 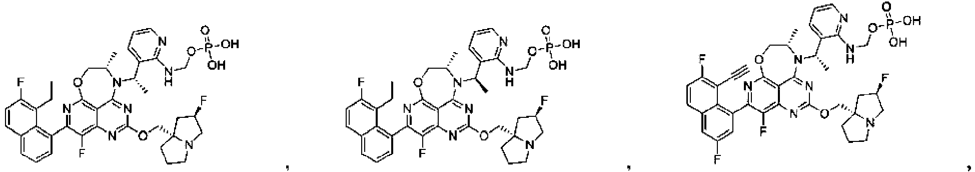 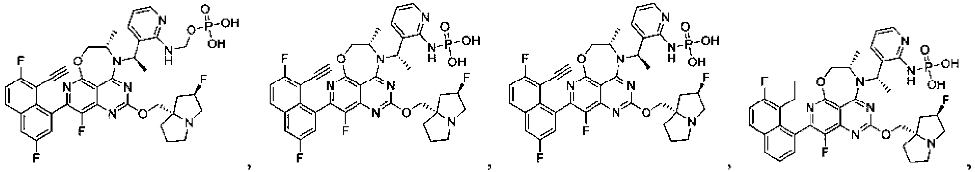 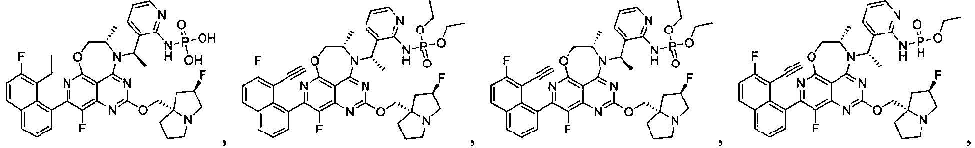 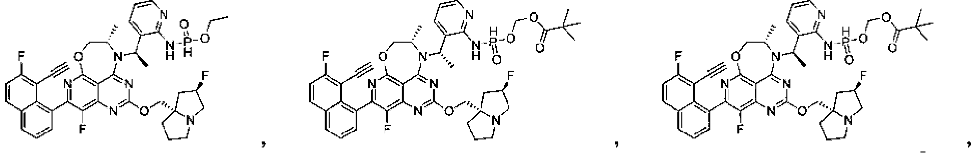 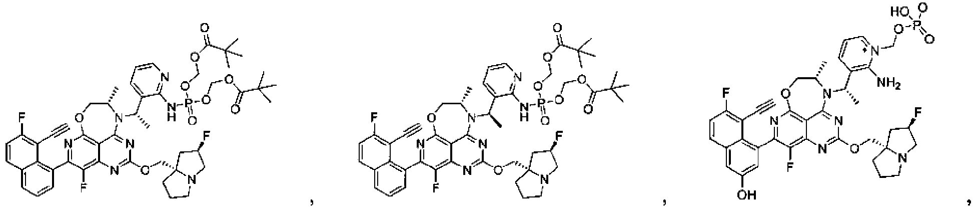 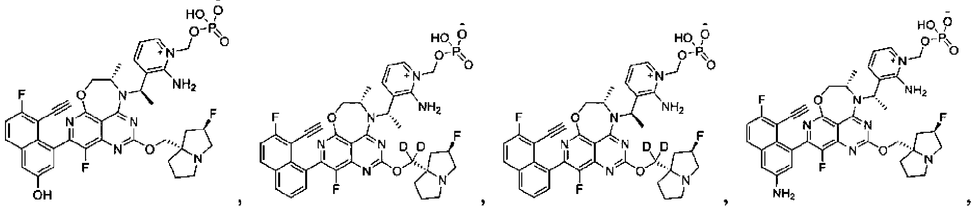 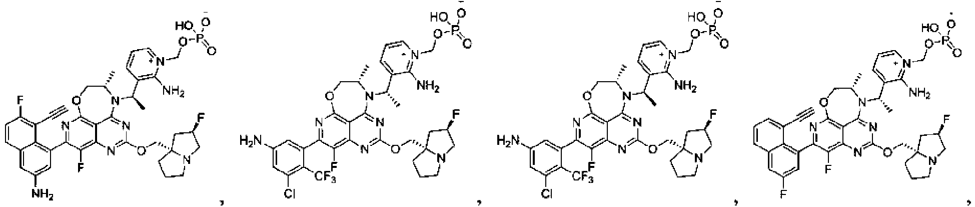 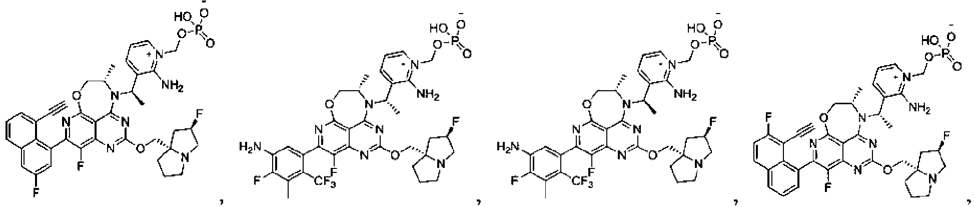 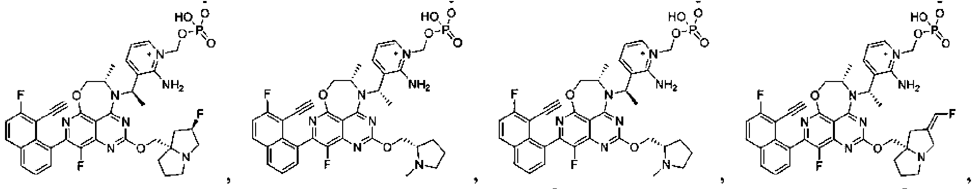 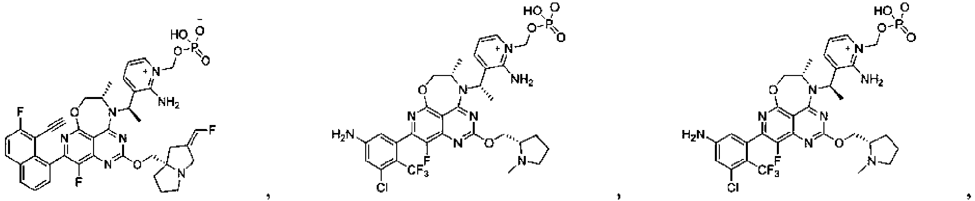 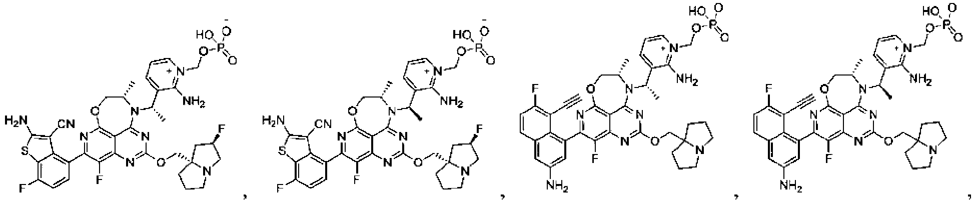 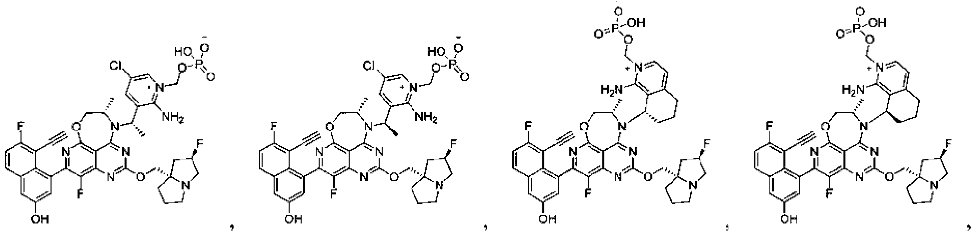 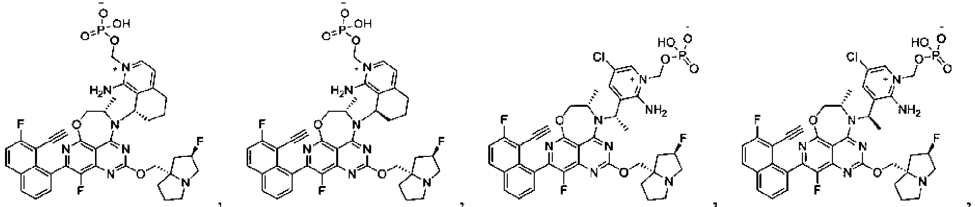 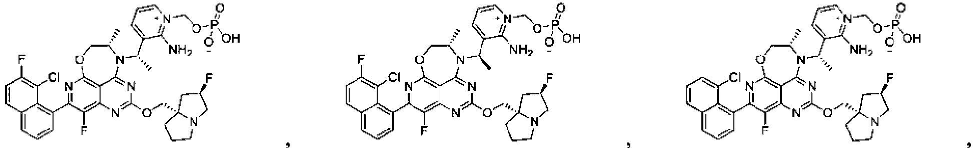 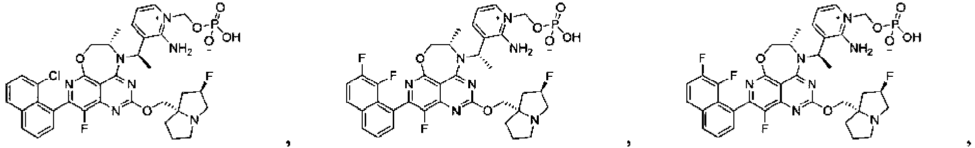 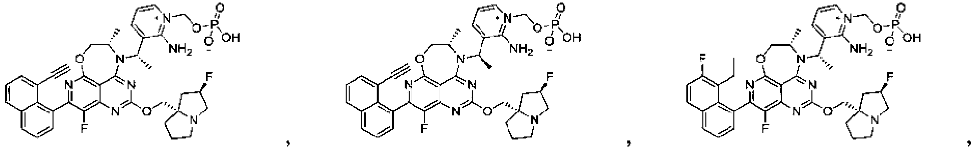 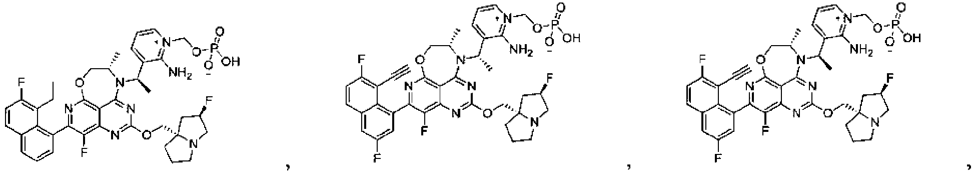 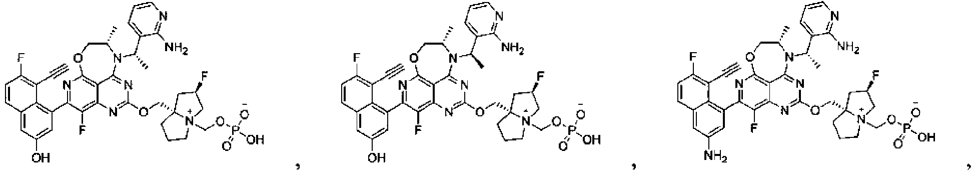 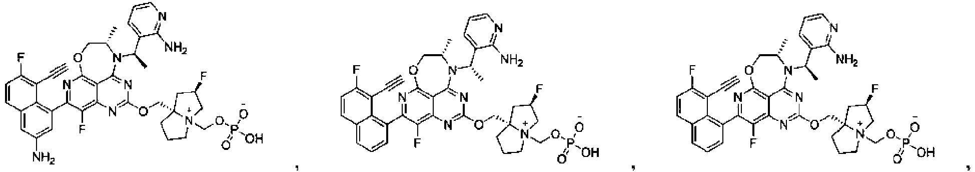 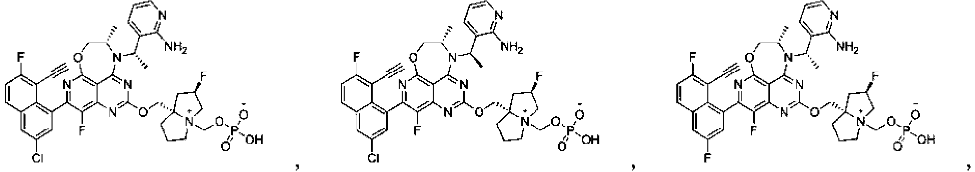 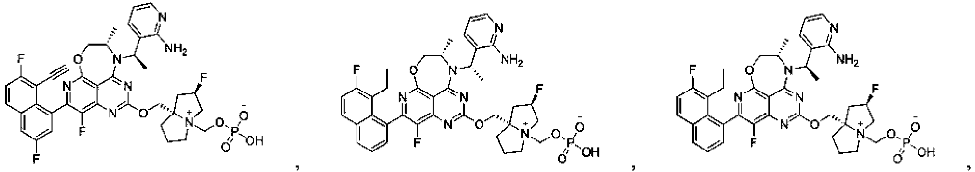 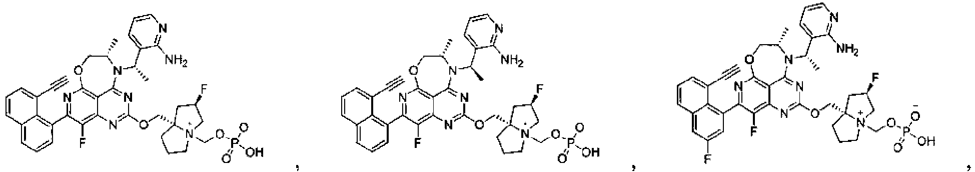 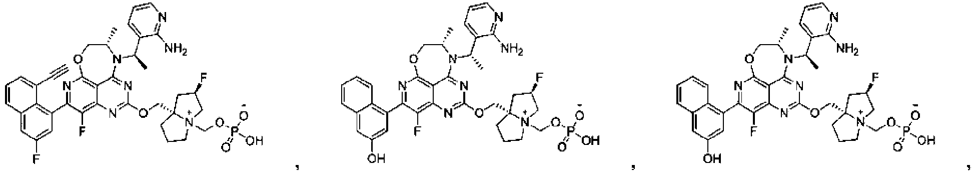 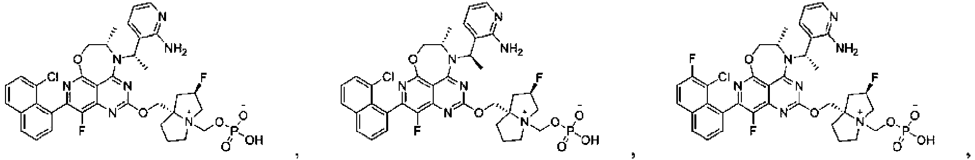 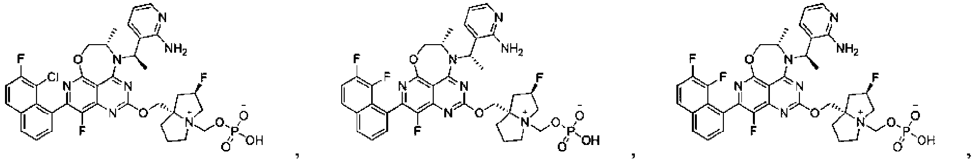 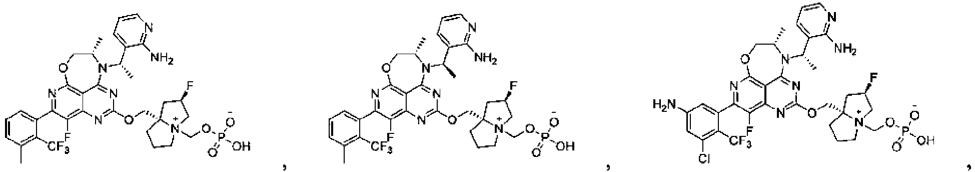 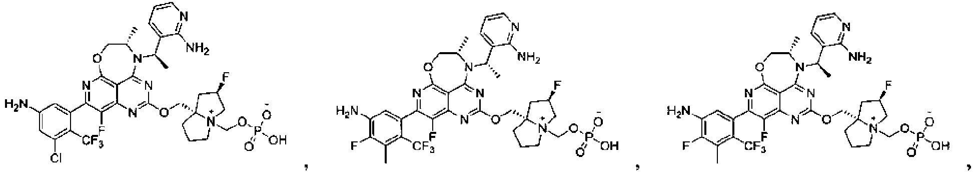 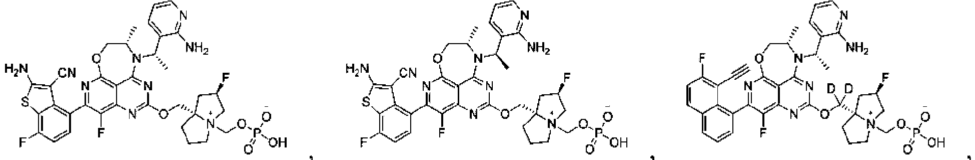 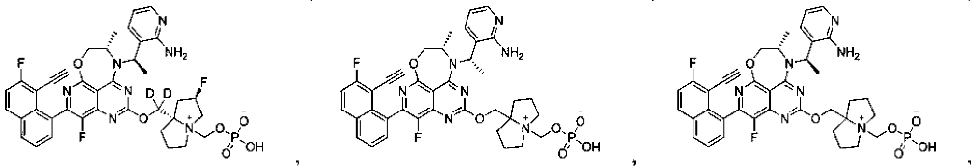 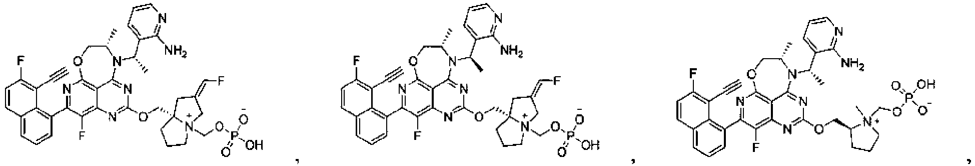 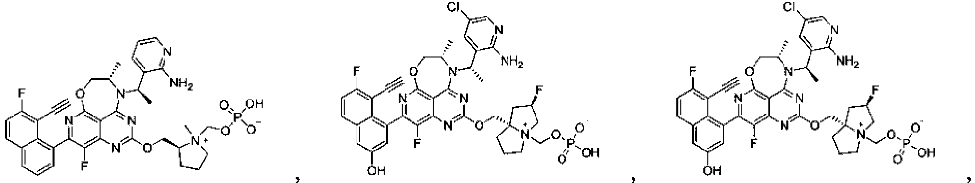 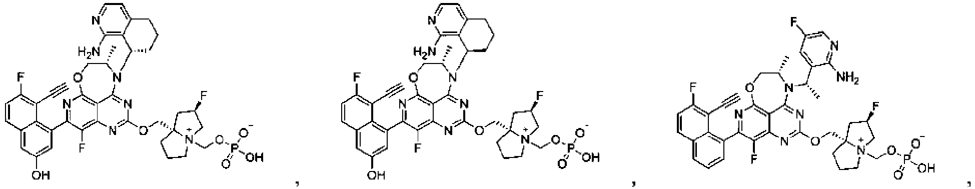 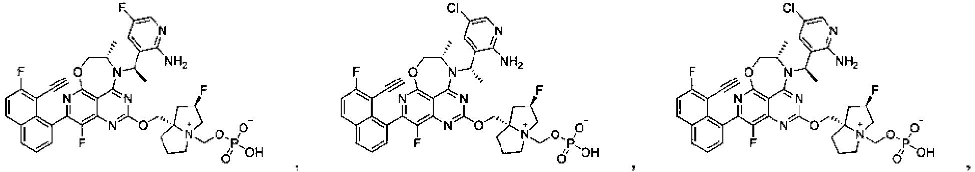 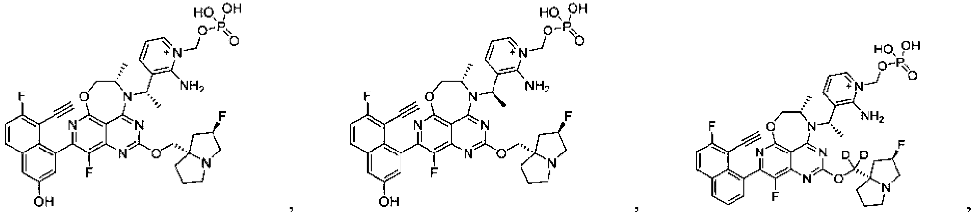 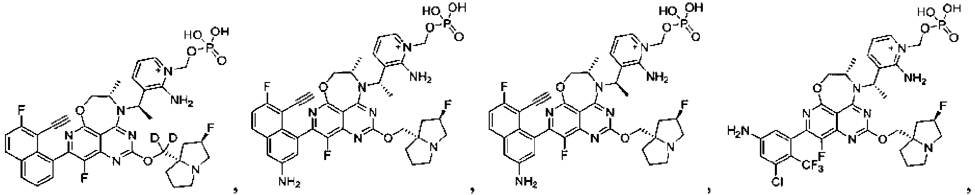 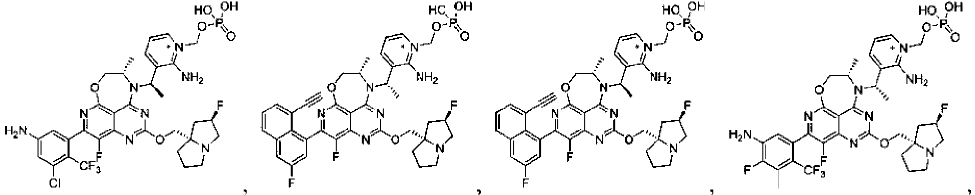 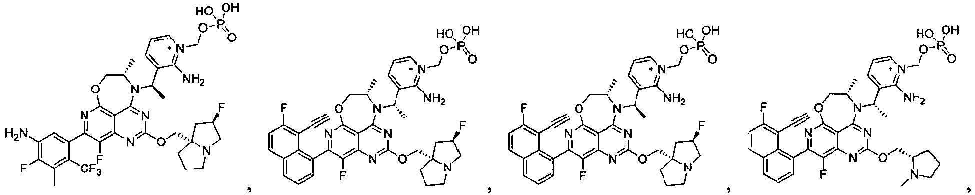 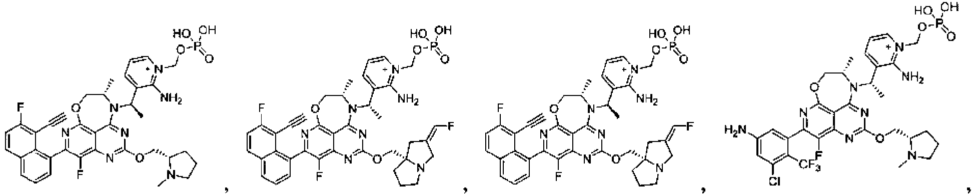 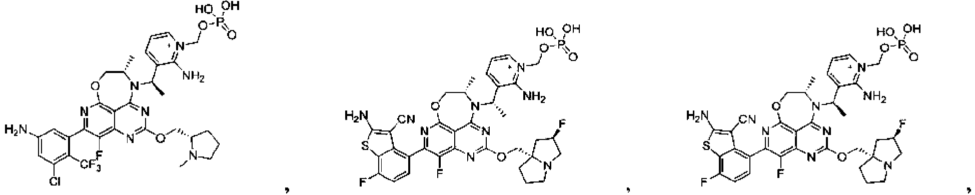 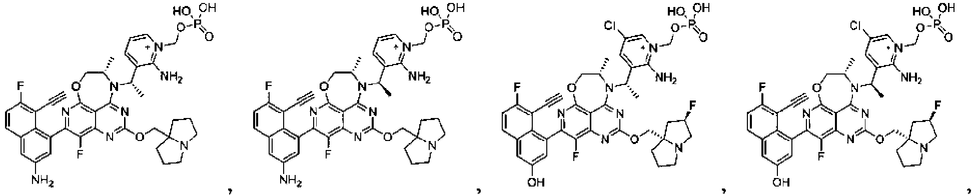 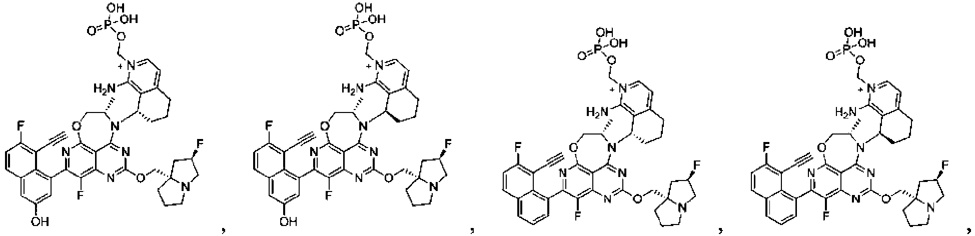 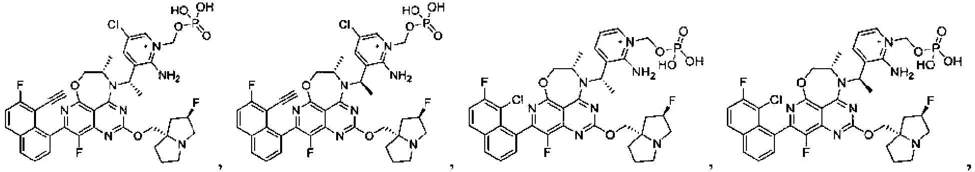 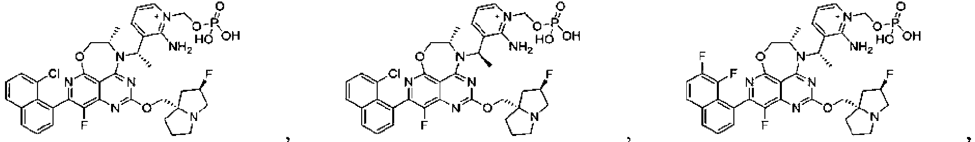 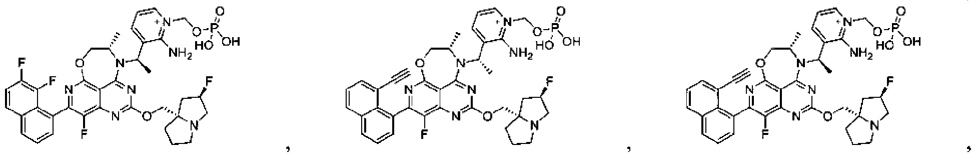 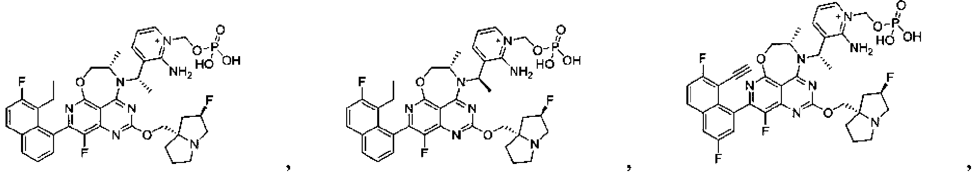 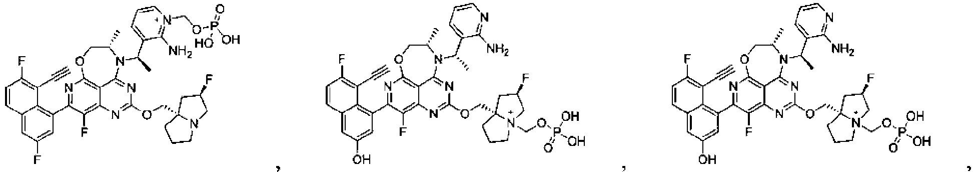 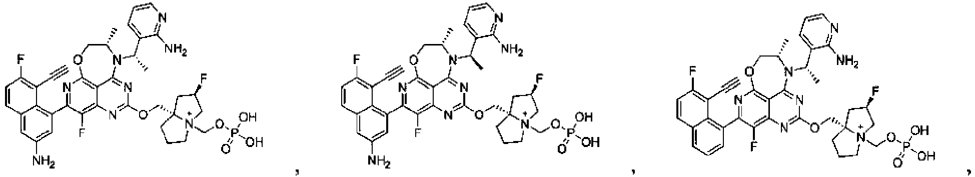 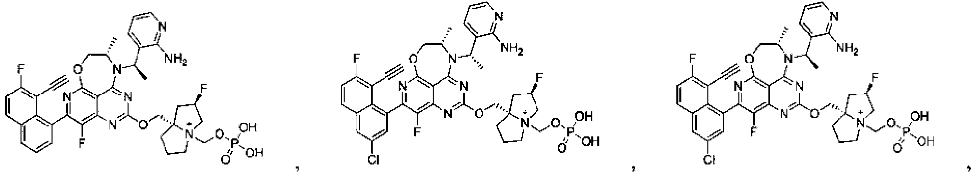 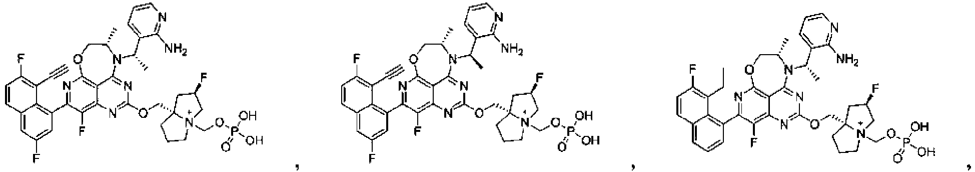 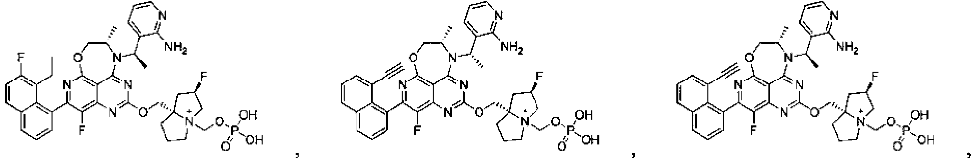 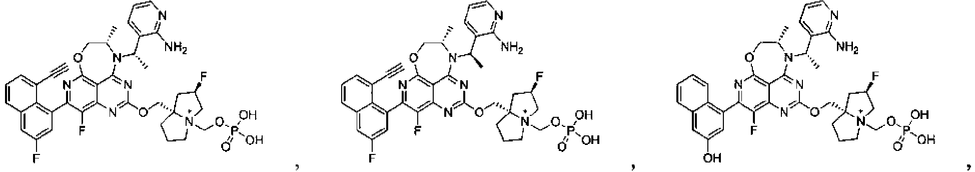 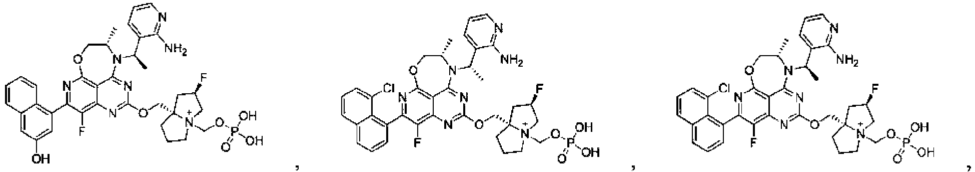 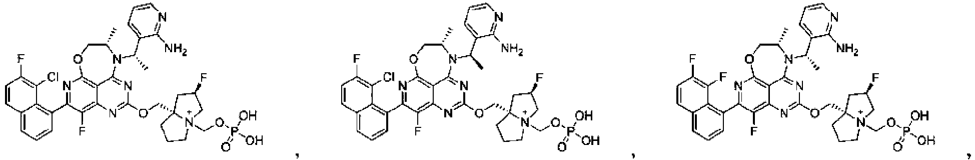 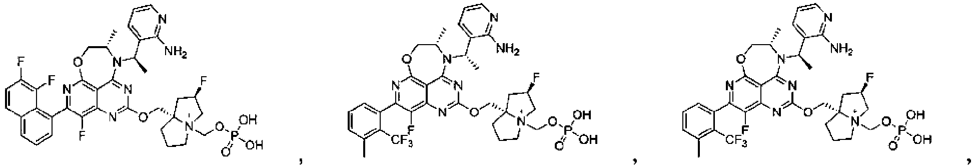 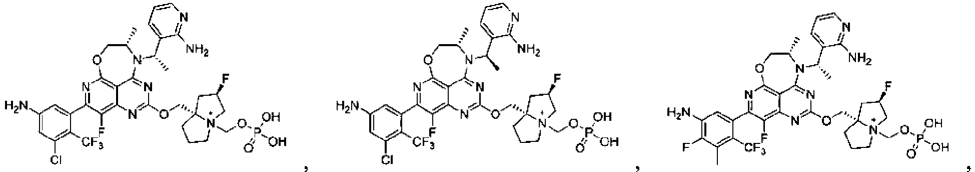 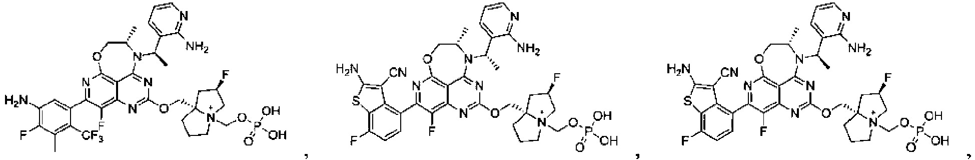 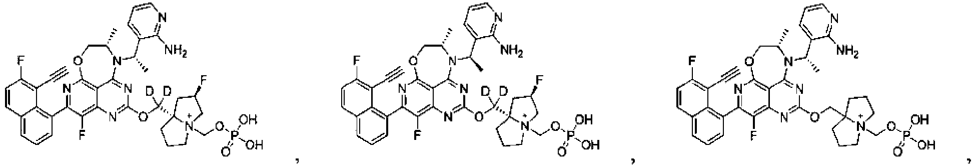 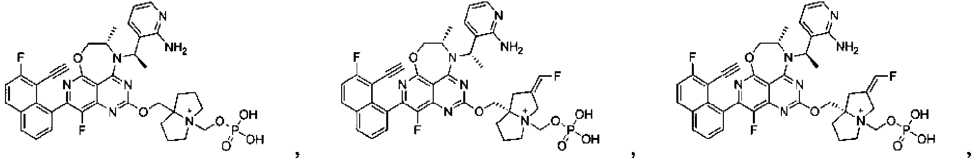 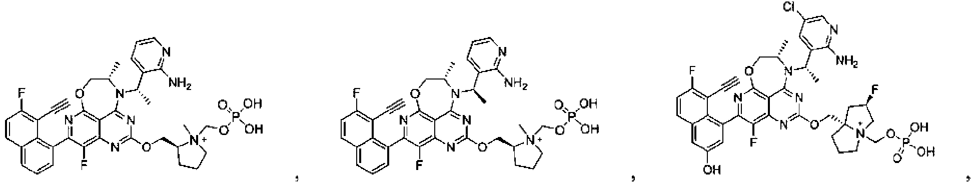 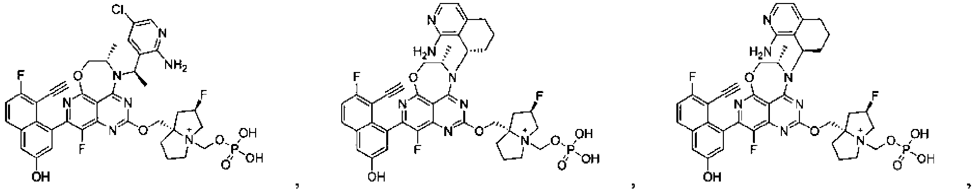 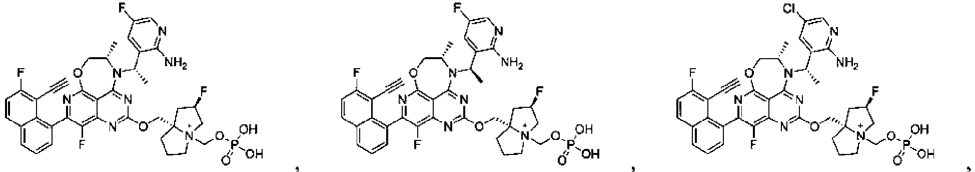 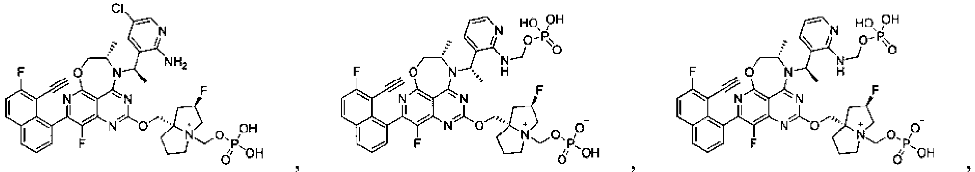 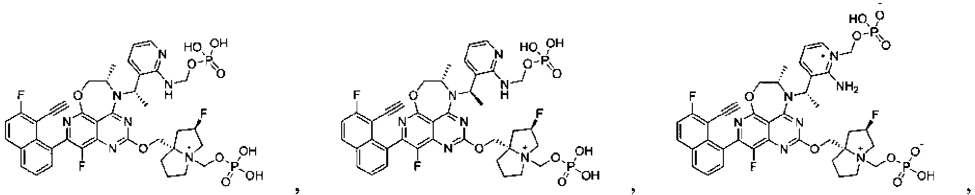 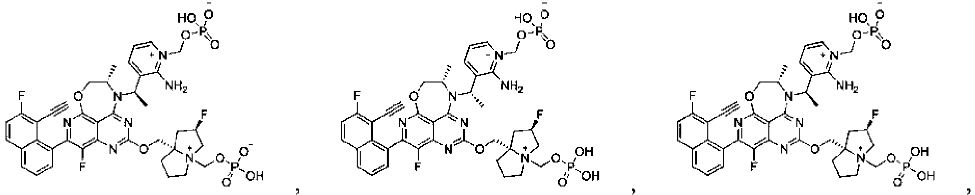 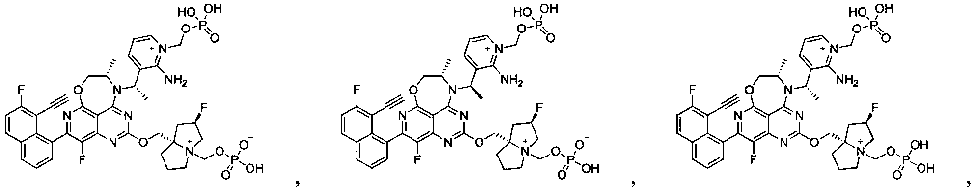 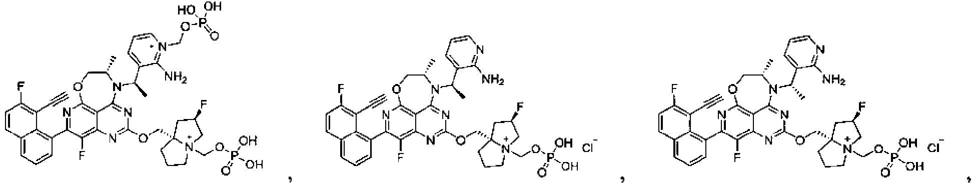 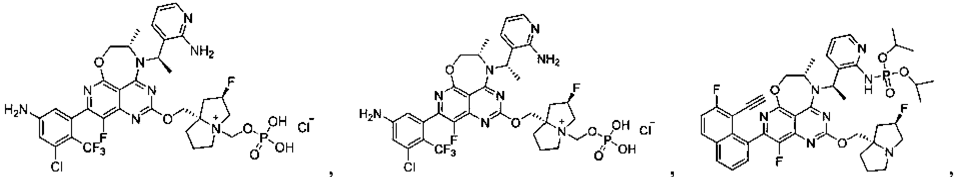 and

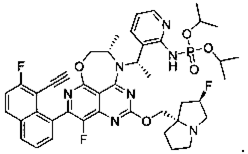

In another aspect, the present disclosure relates to a pharmaceutical composition, which comprises the compound or the pharmaceutically acceptable salt thereof of the present disclosure described above. In some embodiments, the pharmaceutical composition of the present disclosure further comprises a pharmaceutically acceptable excipient.

In another aspect, the present disclosure relates to a method for treating a disease in a mammal, comprising administering to a mammal, preferably a human, in need of such treatment a therapeutically effective amount of the compound or the pharmaceutically acceptable salt thereof of the present disclosure, or the pharmaceutical composition of the present disclosure.

In another aspect, the present disclosure relates to use of the compound or the pharmaceutically acceptable salt thereof of the present disclosure, or the pharmaceutical composition of the present disclosure for preparing a medicament for treating a disease.

In another aspect, the present disclosure relates to use of the compound or the pharmaceutically acceptable salt thereof of the present disclosure, or the pharmaceutical composition of the present disclosure for treating a disease.

In another aspect, the present disclosure relates to the compound or the pharmaceutically acceptable salt thereof of the present disclosure, or the pharmaceutical composition of the present disclosure for use in treating a disease.

In some embodiments of the present disclosure, the disease is preferably a Kras-associated disease.

In some embodiments of the present disclosure, the Kras-associated disease is preferably a Kras-mutated cancer.

In some embodiments of the present disclosure, the Kras-associated disease is selected from a cancer (e.g., pancreatic cancer, non-small cell lung cancer, colon cancer, or multiple myeloma).

In some embodiments of the present disclosure, the Kras-associated disease is preferably a Kras ^{G12D}-associated disease.

In some embodiments of the present disclosure, the Kras ^{G12D} -associated disease is preferably a Kras ^{G12D}-mutated cancer.

In some embodiments of the present disclosure, the Kras ^{G12D}-associated disease is selected from a cancer (e.g., pancreatic cancer).

In some embodiments of the present disclosure, the Kras-associated disease is preferably a Kras ^{G12V}-associated disease.

In some embodiments of the present disclosure, the Kras ^{G12V}-associated disease is preferably a Kras ^{G12V}-mutated cancer.

In some embodiments of the present disclosure, the Kras ^{G12V}-associated disease is selected from a cancer (e.g., pancreatic cancer).

In some embodiments of the present disclosure, the Kras-associated disease is preferably a Kras ^{G12C}-associated disease.

In some embodiments of the present disclosure, the Kras ^{G12C}-associated disease is preferably a Kras ^{G12C}-mutated cancer.

In some embodiments of the present disclosure, the Kras ^{G12C}-associated disease is selected from a cancer (e.g., non-small cell lung cancer).

In some embodiments of the present disclosure, the Kras-associated disease is preferably a Kras ^{G12A}-associated disease.

In some embodiments of the present disclosure, the Kras ^{G12A}-associated disease is preferably a Kras ^{G12A}-mutated cancer.

In some embodiments of the present disclosure, the Kras ^{G12A}-associated disease is selected from a cancer (e.g., multiple myeloma).

In some embodiments of the present disclosure, the Kras-associated disease is preferably a Kras ^{G13D}-associated disease.

In some embodiments of the present disclosure, the Kras ^{G13D}-associated disease is preferably a Kras ^{G13D}-mutated cancer.

In some embodiments of the present disclosure, the Kras ^{G13D}-associated disease is selected from a cancer (e.g., colon cancer).

### Technical Effects

The compounds of the present disclosure or the pharmaceutically acceptable salts thereof include those compounds of the examples which have relatively high Kras inhibitory activity (e.g., Kras ^{G12D} and/or Kras ^{G12V} nucleotide exchange inhibitory activity), cell proliferation inhibitory activity (e.g., AsPc-1 cells and/or Capan-1 cells and/or NCI-H358 cells and/or HCT-116 cells and/or RPMI-8226 cells), and *in vivo* anti-tumor activity (e.g., AsPc-1 cell NOD-SCID mouse subcutaneous xenograft tumor model, NCI-H358 cell CB17-SCID mouse subcutaneous xenograft tumor model, Capan-1 cell nude mouse subcutaneous xenograft tumor model), while showing good stability, bioavailability, and/or other pharmacokinetic parameters in *in vitro* liver microsome stability and *in vivo* pharmacokinetics (e.g., mouse and/or rat and/or dog) studies. The compounds of the present disclosure or the pharmaceutically acceptable salts thereof include those compounds of the examples which have good safety, e.g., a larger safety window.

### Definitions

Unless otherwise stated, the following terms used in the present disclosure shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

The term "substituted" means that any one or more hydrogen atoms or any one or more lone pair electrons on a specific atom are replaced with a substituent, as long as the valence of the specific atom is normal and the substitution results in a stable compound. When the substituent is oxo (i.e., =O), it means that two hydrogen atoms are substituted; oxo substitutions do not occur on aromatic groups.

Non-limiting examples of the "substituent" described herein include, but are not limited to, hydroxy, sulfydryl, halogen, amino, nitro, nitroso, cyano, an azide group, a sulfoxide group, a sulfone group, a sulfonamide group, carboxy, a carboxaldehyde group, an imine group, alkyl, halo-alkyl, cycloalkyl, halo-cycloalkyl, alkenyl, haloalkenyl, cycloalkenyl, halo-cycloalkenyl, alkynyl, halo-alkynyl, cycloalkynyl, halo-cycloalkynyl, heteroalkyl, halo-heteroalkyl, alkoxy, alkylthio, aryl, aryloxy, arylthio, arylalkyl, arylalkoxy, arylalkylthio, heteroaryl, heteroaryloxy, heteroarylthio, heteroarylalkyl, heteroarylalkoxy, heteroarylalkylthio, heterocyclyl, heterocyclyloxy, heterocyclylthio, heterocyclylalkylene, heterocyclylalkoxy, heterocyclylalkylthio, acyl, acyloxy, a carbamate group, an amido group, ureido, an epoxy group, an ester group, and the like, wherein the groups are optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "replaced" means that a particular atom or group can be replaced with another atom or group as specified. For example, 1 or 2 or 3 of the -CH₂- in -CH₂CH₂CH₂- may be replaced with O, S, or NH to give -O-CH₂-CH₂-, - O-CH₂-, -CH₂-O-CH₂-, -CH₂-O-, -CH₂-CH₂-O-, -O-, or the like.

The term "optional" or "optionally" means that the subsequently described event or circumstance may or may not occur, and the description includes instances where the event or circumstance occurs and instances where it does not. For example, ethyl "optionally" substituted with halogen means that the ethyl may be unsubstituted (CH₂CH₃), monosubstituted (e.g., CH₂CH₂F), polysubstituted (e.g., CHFCH₂F, CH₂CHF₂, etc.), or fully substituted (CF₂CF₃). It will be appreciated by those skilled in the art that for any group comprising one or more substituents, no substitution or substitution pattern that is sterically impossible and/or cannot be synthesized is introduced.

The "or" and "alternatively" herein may be used interchangeably with "and/or".

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R) occurs more than once in the constitution or structure of a compound, the definition of the variable in each case is independent. Therefore, for example, if a group is substituted with 2 R, the definition of each R is independent.

When the number of connecting groups is 0, for example, -(CH₂)₀-, it means that the connecting group is a covalent bond.

When a variable is selected from a covalent bond, it means that the two groups are directly connected. For example, in A-L-Z, when L represents a covalent bond, it means that the structure is actually A-Z.

When the direction for connection of the listed connecting group is not specified, the direction for connecting is arbitrary. For example, in A-L-Z, when the connecting group L is -M-W-, it means that the structure may be A-M-W-Z or A-W-M-Z.

When a bond of a substituent is crosslinked to two atoms on a ring, the substituent can be bonded to any atoms on the ring. For example, a structural unit 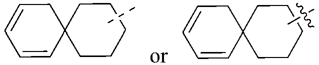 represents that substitution may occur in any one position of cyclohexyl or cyclohexadienyl.

The term "halo" or "halogen" refers to fluorine, chlorine, bromine, and iodine.

The term "hydroxy" refers to an -OH group.

The term "cyano" refers to a -CN group.

The term "sulfydryl" refers to an -SH group.

The term "amino" refers to an -NH₂ group.

The term "nitro" refers to an -NO₂ group.

The term "alkylene" refers to saturated linear or branched divalent hydrocarbyl with a general formula of CₙH₂ₙ and usually has 1 to 12, 1 to 8, 1 to 6, 1 to 4, 1 to 3, or 1 to 2 carbon atoms. For example, the term "C₁₋₆ alkylene" refers to alkylene containing 1 to 6 carbon atoms. Non-limiting examples of alkylene include, but are not limited to, methylene (-CH₂-), ethylene (-CH₂CH₂-), propylene (-CH₂CH₂CH₂- or -CH₂CH(CH₃)-), butylene (-CH₂CH₂CH₂CH₂-, -CH₂CH(CH₃)CH₂-, or -CH₂CH₂CH(CH₃)-), and the like. The alkylene is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "heteroalkylene" refers to alkylene in which one or more carbon atoms (and the hydrogen atoms to which they are connected) are each independently replaced with the same or different heteroatom groups. Unless otherwise indicated, the heteroalkylene contains 1, 2, or 3 heteroatom groups, non-limiting examples of which include O, S, N, and NH, and usually has 1 to 12, 1 to 8, 1 to 6, 1 to 4, 1 to 3, or 1 to 2 carbon atoms. For example, the term "C₁₋₆ heteroalkylene" refers to heteroalkylene containing 1 to 6 carbon atoms and 1-3 heteroatom groups. The heteroatom group can be placed in any position (e.g., internal or terminal position) on the heteroalkylene, including a position where the alkylene is connected to the rest of the molecule. Usually, where more than one heteroatom group is present, the heteroatoms are not adjacent to each other. Non-limiting examples of heteroalkylene include, but are not limited to, -OCH₂-, -OCH₂CH₂-, -OCH₂CH₂CH₂-, -CH₂OCH₂-, -OCH₂O-, -OCH₂CH₂O-, -OCH₂OCH₂CH₂-, - SCH₂-, -SCH₂CH₂-, -SCH₂CH₂CH₂-, -CH₂SCH₂-, -SCH₂S-, -SCH₂CH₂S-, -SCH₂SCH₂CH₂-, -NHCH₂-, - NHCH₂CH₂-, -NHCH₂CH₂CH₂-, -CH₂NHCH₂-, -N(CH₃)CH₂-, -CH₂N(CH₃)-, -OCH₂NH-, -OCH₂CH₂NH-, - OCH₂NHCH₂CH₂-, -OCH₂N(CH₃)CH₂-, and the like. The heteroalkylene is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkyl" refers to saturated hydrocarbyl with a general formula of CₙH₂ₙ₊₁ and usually has 1 to 12, 1 to 8, 1 to 6, 1 to 4, 1 to 3, or 1 to 2 carbon atoms. The alkyl may be linear or branched and usually has 1 to 12, 1 to 8, 1 to 6, 1 to 4, or 1 to 3 carbon atoms. For example, the term "C₁₋₆ alkyl" refers to alkyl containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). The alkyl is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy. Similarly, the alkyl moieties (i.e., alkyl) of alkoxy, alkylamino, dialkylamino, alkylsulfonyl, and alkylthio have the same definition as described above.

The term "heteroalkyl" refers to alkyl in which one or more carbon atoms (and the hydrogen atoms to which they are connected) are each independently replaced with the same or different heteroatom groups. Unless otherwise indicated, the heteroalkyl contains 1, 2, or 3 heteroatom groups, non-limiting examples of which include O, S, N, and NH, and usually has 1 to 12, 1 to 8, 1 to 6, 1 to 4, 1 to 3, or 1 to 2 carbon atoms. For example, the term "C₁₋₆ heteroalkyl" refers to heteroalkyl containing 1 to 6 carbon atoms and 1-3 heteroatom groups. The heteroatom group can be placed in any position (e.g., internal or terminal position) on the heteroalkyl, including a position where the heteroalkyl is connected to the rest of the molecule. Usually, where more than one heteroatom group is present, the heteroatom groups are not adjacent to each other. Exemplary heteroalkyl includes, but is not limited to, alkoxy, alkoxyalkylene, alkylamino, alkylaminoalkylene, dialkylamino, dialkylaminoalkylene, and the like. The heteroalkyl is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkoxy" refers to -O-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkylamino" refers to -NH-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "dialkylamino" refers to -N(alkyl)₂ and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkylsulfonyl" refers to -SO₂-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkylthio" refers to -S-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkenyl" refers to linear or branched unsaturated aliphatic hydrocarbyl consisting of carbon atoms and hydrogen atoms and having at least one double bond, and usually has 2 to 12, 2 to 8, 2 to 6, 2 to 4, or 2 to 3 carbon atoms. Non-limiting examples of the alkenyl include, but are not limited to, ethenyl, 1-propenyl, 2-propenyl, 1-butenyl, isobutenyl, 1,3-butadienyl, and the like. The alkenyl is optionally substituted with one or more substituents selected from the group consisting of the following substituents: oxo, hydroxy, amino, nitro, halogen, cyano, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkenylene" refers to a divalent form of alkenyl. The alkenylene usually has 2 to 12, 2 to 8, 2 to 6, 2 to 4, or 2 to 3 carbon atoms. Non-limiting examples of the alkenylene include, but are not limited to, ethenylene, 1-propenylidene, 2-propenylidene, 1-butenylidene, isobutenylidene, 1,3-butadienylene, and the like. The alkenylene is optionally substituted with one or more substituents selected from the group consisting of the following substituents: oxo, hydroxy, amino, nitro, halogen, cyano, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "heteroalkenyl" refers to alkenyl in which one or more carbon atoms (and the hydrogen atoms to which they are connected) are each independently replaced with the same or different heteroatom groups. Unless otherwise indicated, the heteroalkenyl contains 1, 2, or 3 heteroatom groups, non-limiting examples of which include O, S, N, and NH, and usually has 1 to 12, 1 to 8, 1 to 6, 1 to 4, 1 to 3, 2 to 12, 2 to 8, 2 to 6, 2 to 4, or 2 to 3 carbon atoms. For example, the term "C₁₋₄ heteroalkenyl" refers to heteroalkenyl containing 1 to 4 carbon atoms and 1-3 heteroatom groups. The heteroatom group can be placed in any position (e.g., internal or terminal position) on the heteroalkenyl, including a position where the heteroalkenyl is connected to the rest of the molecule. Usually, where more than one heteroatom group is present, the heteroatom groups are not adjacent to each other. Exemplary heteroalkenyl includes, but is not limited to, CH₂=N-, alkenyl-O-, alkenyl-NH-, alkenyl-S-, alkenyl-O-alkylene-, alkyl-O-alkenylene-, alkenyl-NH-alkylene-, alkyl-NH-alkenylene-, alkenyl-S-alkylene-, alkyl-S-alkenylene-, alkenyl-CH=N-, alkenyl-N=CH-, alkyl-CH=N-alkenylene-, alkenyl-CH=N-alkylene-, and alkyl-NH-alkenylene-O-. In some embodiments, the double bond in the heteroalkenyl is a carbon-carbon double bond. The heteroalkenyl is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "heteroalkenylene" refers to a divalent form of heteroalkenyl. Unless otherwise indicated, the heteroalkenylene contains 1, 2, or 3 heteroatom groups, non-limiting examples of which include O, S, N, and NH, and usually has 1 to 12, 1 to 8, 1 to 6, 1 to 4, 1 to 3, 2 to 12, 2 to 8, 2 to 6, 2 to 4, or 2 to 3 carbon atoms. For example, the term "C₁₋₄ heteroalkenylene" refers to heteroalkenylene containing 1 to 4 carbon atoms and 1-3 heteroatom groups. The heteroatom group can be placed in any position (e.g., internal or terminal position) on the heteroalkenylene, including a position where the heteroalkenylene is connected to the rest of the molecule. Usually, where more than one heteroatom group is present, the heteroatom groups are not adjacent to each other. Exemplary heteroalkenylene includes, but is not limited to, -alkenylene-O-, -alkenylene-NH-, -alkenylene-S-, -alkenylene-O-alkylene-, -alkylene-O-alkenylene-, -alkenylene-NH-alkylene-, -alkylene-NH-alkenylene-, -alkenylene-S-alkylene-, -alkylene-S-alkenylene-, -alkenylene-CH=N-, -alkenylene-N=CH-, -alkylene-CH=N-alkenylene-, -alkenylene-CH=N-alkylene-, and -alkylene-NH-alkenylene-O-. In some embodiments, the double bond in the heteroalkenylene is a carbon-carbon double bond. The heteroalkenylene is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkynyl" refers to linear or branched unsaturated aliphatic hydrocarbyl consisting of carbon atoms and hydrogen atoms and having at least one triple bond, and usually has 2 to 12, 2 to 8, 2 to 6, 2 to 4, or 2 to 3 carbon atoms. Non-limiting examples of the alkynyl include, but are not limited to, ethynyl (-C≡CH), 1-propynyl (-C≡C-CH₃), 2-propynyl (-CH₂-C≡CH), 1,3-butadiynyl (-C≡C-C≡CH), and the like. The alkynyl is optionally substituted with one or more substituents selected from the group consisting of the following substituents: oxo, hydroxy, amino, nitro, halogen, cyano, alkenyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "cycloalkyl" refers to a carbocyclic ring that is fully saturated and may exist as a monocyclic ring, a bridged ring, or a spiro ring. Unless otherwise indicated, the carbocyclic ring is usually a 3- to 10-membered ring, a 4- to 8-membered ring, a 5- to 8-membered ring, or a 5- to 6-membered ring. Non-limiting examples of cycloalkyl include, but are not limited to, cyclopropanyl, cyclobutanyl, cyclopentanyl, cyclohexanyl, norbornyl (bicyclo[2.2.1]heptyl), bicyclo[2.2.2]octyl, adamantyl, and the like. The cycloalkyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "cycloalkenyl" refers to a non-aromatic carbocyclic ring that is not fully saturated, has at least one double bond, and may exist as a monocyclic ring, a bridged ring, or a spiro ring. Unless otherwise indicated, the carbocyclic ring is usually a 3- to 10-membered ring, a 4- to 8-membered ring, a 5- to 8-membered ring, or a 5- to 6-membered ring. Non-limiting examples of the cycloalkenyl include, but are not limited to, cyclopentenyl, cyclopentadienyl, cyclohexenyl, cyclohexadienyl, cycloheptenyl, cycloheptadienyl, and the like. The cycloalkenyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, - C(O)N(alkyl)₂, -NHC(O)-alkyl, -C(O)-alkyl, -S(O)-alkyl, -S(O)₂-alkyl, -S(O)₂NH₂, -S(O)₂NH-alkyl, - S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "heterocyclyl" refers to a non-aromatic ring that is fully saturated or partially unsaturated (but not a fully unsaturated heteroaromatic group) and may exist as a monocyclic ring, a bridged ring, a fused ring, or a spiro ring. Unless otherwise indicated, the heterocyclic ring is usually a 3- to 12-membered, 3- to 10-membered, 4- to 8-membered, 5- to 8-membered, 5- to 6-membered, 3- to 7-membered, or 4- to 6-membered ring containing 1 to 3 heteroatoms (preferably 1 or 2 heteroatoms) independently selected from the group consisting of sulfur, oxygen, nitrogen, phosphorus, silicon, and/or boron. Non-limiting examples of the heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, and the like. The heterocyclyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, - C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "heterocycloalkenyl" refers to a non-aromatic ring that is partially unsaturated (but not a fully unsaturated heteroaromatic group) and may exist as a monocyclic ring, a bridged ring, a fused ring, or a spiro ring. Unless otherwise indicated, the heterocycloalkenyl is usually a 3- to 12-membered, 3- to 10-membered, 4- to 8-membered, 5- to 8-membered, 5- to 6-membered, 3- to 7-membered, or 4- to 6-membered ring containing 1 to 3 heteroatoms (preferably 1 or 2 heteroatoms) independently selected from the group consisting of sulfur, oxygen, nitrogen, phosphorus, silicon, and/or boron. Non-limiting examples of heterocycloalkenyl include, but are not limited to, dihydrofuranyl, dihydropyrrolidinyl, dihydrothienyl, dihydroindolyl, tetrahydroquinolinyl (e.g., 1,2,3,4-tetrahydroquinolinyl), tetrahydroisoquinolinyl (e.g., 5,6,7,8-tetrahydroisoquinolinyl), and the like. The heterocycloalkenyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, - C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "heterocycloalkyl" refers to a cyclic group that is fully saturated and may exist as a monocyclic ring, a bridged ring, or a spiro ring. Unless otherwise indicated, the heterocyclic ring is usually a 3- to 12-membered, 3- to 10-membered, 4- to 8-membered, 5- to 8-membered, 5- to 6-membered, 3- to 7-membered, or 4- to 6-membered ring containing 1 to 3 heteroatoms (preferably 1 or 2 heteroatoms) independently selected from the group consisting of sulfur, oxygen, nitrogen, phosphorus, silicon, and/or boron. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. The heterocycloalkyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, - C(O)N(alkyl)₂, -NHC(O)-alkyl, -C(O)-alkyl, -S(O)-alkyl, -S(O)₂-alkyl, -S(O)₂NH₂, -S(O)₁NH-alkyl, - S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "aryl" refers to an all-carbon aromatic monocyclic or fused polycyclic group having a conjugated π-electron system. For example, the aryl may have 6-20 carbon atoms, 6-14 carbon atoms, or 6-12 carbon atoms. Non-limiting examples of the aryl include, but are not limited to, phenyl, naphthyl, anthryl, and the like. The aryl is optionally substituted with one or more substituents selected from the group consisting of the following substituents: hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, - C(O)N(alkyl)₂, -NHC(O)-alkyl, -C(O)-alkyl, -S(O)-alkyl, -S(O)₂-alkyl, -S(O)₂NH₂, -S(O)₂NH-alkyl, - S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "heteroaryl" refers to an aromatic monocyclic or fused polycyclic system containing at least one ring atom selected from the group consisting of N, O, and S, with the remaining ring atoms being C, and usually has a 5- to 14-membered, 5- to 12-membered, 5- to 10-membered, 5- to 8-membered, 5- to 7-membered, or 5- to 6-membered ring. Preferably, heteroaryl has a single 4- to 8-membered ring, in particular a 5- to 6-membered ring, or has a plurality of fused rings containing 5 to 14 ring atoms, in particular 5 to 10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like. The heteroaryl is optionally substituted with one or more substituents selected from the group consisting of the following substituents: hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, - C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, - S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The group -L¹- in the present disclosure is correspondingly connected to the lower group and the right group that are connected to this group in the general formula in a left-to-right or right-to-left reading order, e.g., the left-to-right reading order. Specifically, for example, when -L¹- is -OCH₂CH₂-, the structure 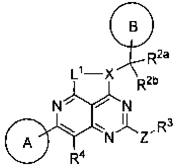 corresponds to

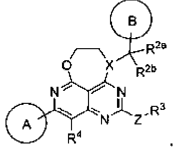

The term "treat" or "treatment" means administering the compound or formulation described in the present disclosure to ameliorate or eliminate a disease or one or more symptoms associated with the disease, including:
(i) inhibiting a disease or disease state, i.e., arresting its progression; and
(ii) alleviating a disease or disease state, i.e., causing the regression of the disease or disease state.

The term "prevent", "preventing", or "prevention" means administering the compound or formulation described herein to prevent a disease or one or more symptoms associated with the disease, and includes preventing the occurrence of the disease or disease state in a mammal, particularly when such a mammal is predisposed to the disease state but has not yet been diagnosed with it.

The term "therapeutically effective amount" refers to an amount of the compound of the present disclosure for (i) treating or preventing a specific disease, condition, or disorder, (ii) relieving, ameliorating, or eliminating one or more symptoms of a specific disease, condition, or disorder, or (iii) preventing or delaying the onset of one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrates, wax, water-soluble and/or water-swellable polymers, hydrophilic or hydrophobic materials, gelatin, oil, solvents, and water.

The word "comprise" and variations thereof such as "comprises" or "comprising" should be understood in an open, non-exclusive sense, i.e., "including but not limited to".

The compounds and intermediates of the present disclosure may also be present in different tautomeric forms, and all such forms are included within the scope of the present disclosure. The term "tautomer" or "tautomeric form" refers to structural isomers of different energies that can interconvert via a low energy barrier. For example, a proton tautomer (also referred to as a prototropic tautomer) includes interconversion via proton transfer, such as keto-enol isomerization and imine-enamine isomerization. A specific example of a proton tautomer is an imidazole moiety in which a proton can transfer between two ring nitrogens. A valence tautomer includes the interconversion via recombination of some bonding electrons.

The present disclosure also includes isotopically labeled compounds of the present disclosure, which are identical to those recited herein but have one or more atoms replaced by an atom having an atomic mass or mass number different from the atomic mass or mass number generally found in nature. Examples of isotopes of the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl, and the like.

The compound of the present disclosure may be asymmetrical, for example, having one or more stereoisomers.

Unless otherwise stated, all stereoisomers are included, for example, enantiomers and diastereoisomers. The compound containing asymmetric carbon atoms of the present disclosure may be isolated in an optically active pure form or in a racemic form. The optically pure form can be resolved from a racemic mixture or can be synthesized using a chiral raw material or a chiral reagent. Non-limiting examples of stereoisomers include, but are not limited to:

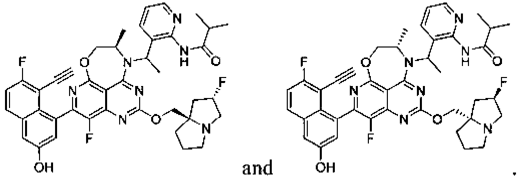

The compounds of the present disclosure may have one or more atropisomers, which, unless otherwise stated, refer to optically active isomers resulting from the hindrance of free rotation between single bonds. The compounds containing a chiral axis of the present disclosure can be isolated in a racemic form. When the energy barrier for the single bond free rotation of the compounds containing a chiral axis of the present disclosure is sufficiently high, the atropisomers of the compounds may be isolated in an optically active pure form.

The pharmaceutical composition of the present disclosure can be prepared by combining the compound of the present disclosure with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present disclosure include, but are not limited to, oral, rectal, topical, inhalational, parenteral, sublingual, intravaginal, intranasal, intraocular, intraperitoneal, intramuscular, subcutaneous, and intravenous administration.

The pharmaceutical composition of the present disclosure can be manufactured using methods well known in the art, such as conventional mixing, dissolving, granulating, dragee-making, levigating, emulsifying, lyophilizing, etc.

In some embodiments, the pharmaceutical composition is in an oral form. For oral administration, the pharmaceutical composition can be formulated by mixing the active compounds with pharmaceutically acceptable excipients well known in the art. These excipients enable the compounds of the present disclosure to be formulated into tablets, pills, pastilles, dragees, capsules, gels, slurries, suspensions, etc. for oral administration to a patient.

A solid oral composition can be prepared by conventional mixing, filling, or tableting. For example, the solid oral composition can be obtained by the following method: mixing the active compound with solid excipients, optionally grinding the resulting mixture, adding additional suitable excipients if desired, and then processing the mixture into granules to obtain the core parts of tablets or dragees. Suitable excipients include, but are not limited to: binders, diluents, disintegrants, lubricants, glidants, sweeteners, flavoring agents, etc.

The pharmaceutical composition may also be suitable for parenteral administration, such as sterile solutions, suspensions, or lyophilized products in suitable unit dosage forms.

In all administration methods of the compound of general formula I described herein, the daily dose is 0.01 mg/kg of body weight to 200 mg/kg of body weight. The compounds of the present disclosure can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present disclosure.

The chemical reactions in the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthetic procedure or a reaction process based on the existing embodiments.

The compound of formula (I) disclosed herein, e.g., the compound of formula (III), may be prepared by those skilled in the art of organic synthesis via route 1, wherein the moieties n, R¹, R^{2a}, R^{2b}, R³, R⁴, Z, ring A, and ring B are as defined above.

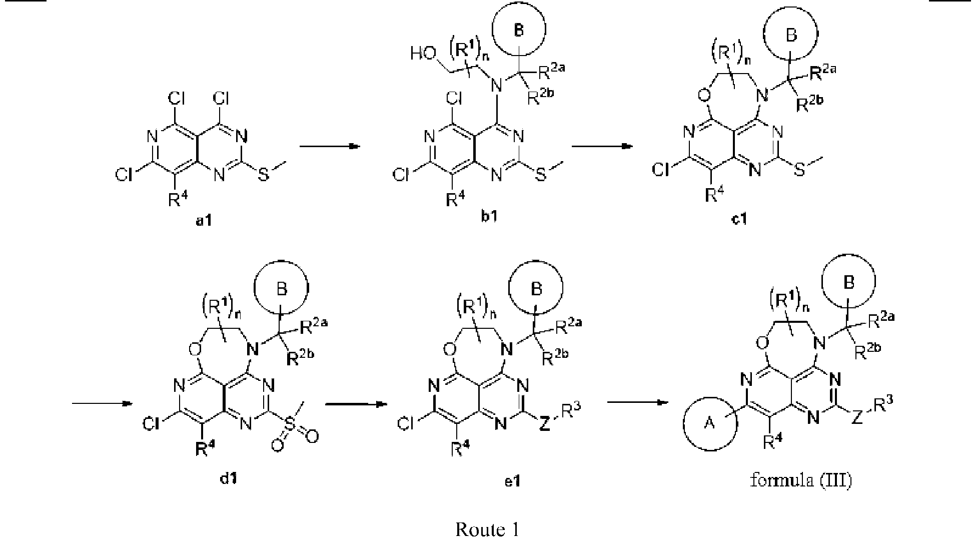

The compound of formula (I) disclosed herein, e.g., the compound of formula (F), may be prepared by those skilled in the art of organic synthesis via route 2 or route 3, wherein the moieties n, m, R¹, L², R^{3a}, R^{C}, R⁴, Z, ring A, and ring B are as defined above.

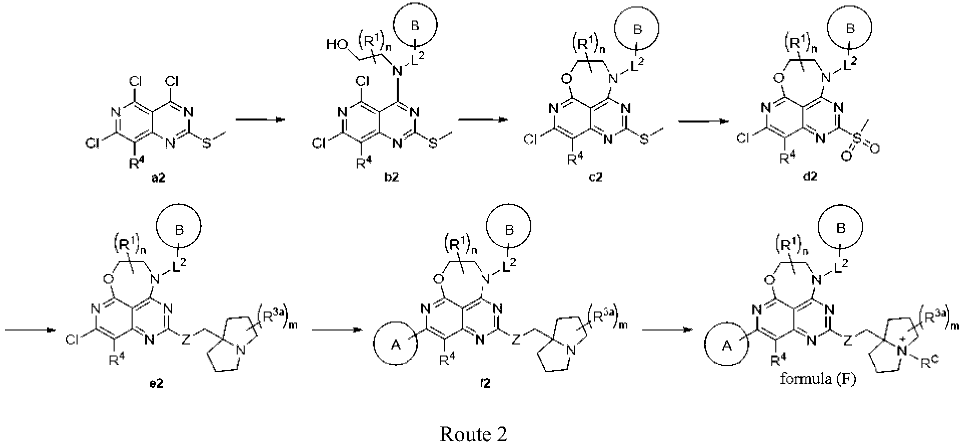

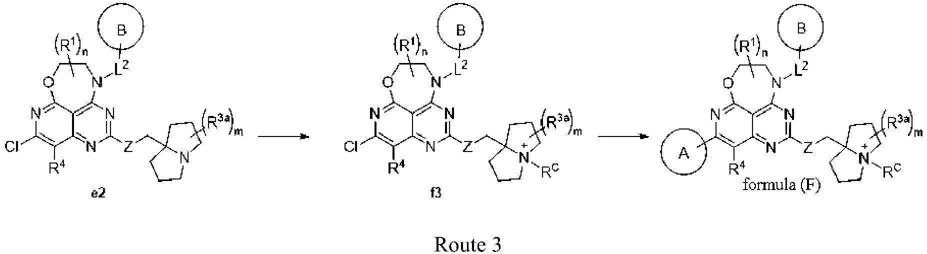

Each of the products from the reactions of the route described above may be obtained by conventional separation techniques including, but not limited to, filtration, distillation, crystallization, chromatography, and the like. The starting materials may be self-synthesized or purchased from commercial establishments (such as, but not limited to, Adrich or Sigma). These materials can be characterized using conventional means, such as physical constants and spectral data. The compounds described herein can be synthesized as a single isomer or a mixture of isomers.

The following abbreviations are used in the present disclosure:
DMF represents N,N-dimethylformamide; PMB represents p-methoxybenzyl; TIPS represents triisopropylsilyl; MOM represents methoxymethyl; DIPEA represents diisopropylethylamine; DMSO represents dimethyl sulfoxide.

The commercially available compounds are under the supplier's catalog names.

For clarity, the present disclosure is further described with the following examples, which are, however, not intended to limit the scope of the present disclosure. Although the present disclosure has been described in detail herein and specific embodiments have also been disclosed, it will be apparent to those skilled in the art that various changes and modifications can be made to the examples without departing from the spirit and scope of the present disclosure.

All the reagents used in the present disclosure are commercially available and can be used without further purification.

### Example

### Example 1 and Example 2

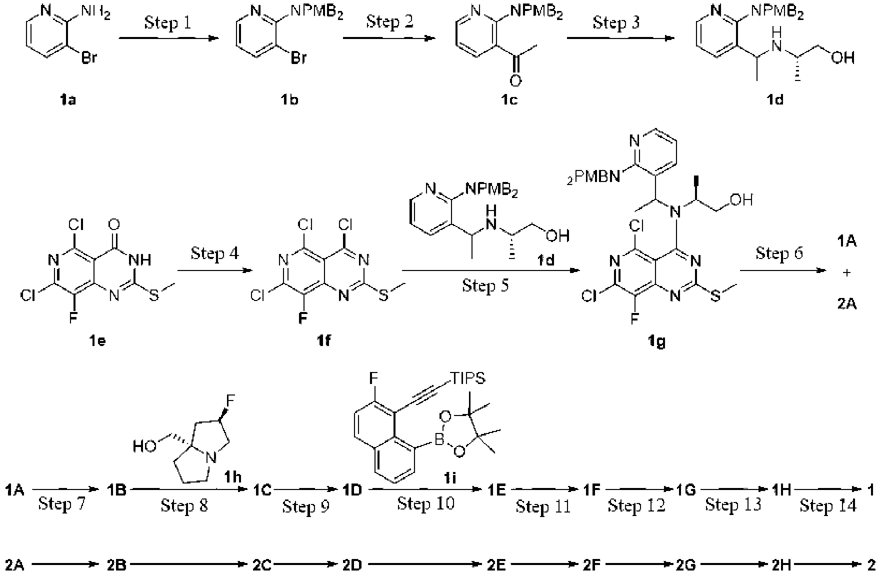

### Step 1:

**1a** (7.25 g) and p-methoxybenzyl chloride (26.3 g) were dispersed in DMF (80 mL), and sodium hydride (60%, 5.9 g) was added under stirring at room temperature. The mixture was stirred for reaction for 2 h. After the reaction was completed, an ammonium chloride solution (20 mL) was added to quench the reaction, and water (100 mL) and ethyl acetate (150 mL) were added. The mixture was stirred, and the phases were separated. The aqueous phase was extracted with ethyl acetate (100 mL × 2), and the organic phases were combined, washed with 5% brine (100 mL × 2), dried over anhydrous sodium sulfate, and filtered. The filtrate was concentrated until no liquid flowed out and purified by column chromatography (ethyl acetate:petroleum ether = 1:100 to 1:10) to give compound **1b**. LC-MS: m/z = 413.16 [M+H]⁺.

### Step 2:

**1b** (1.25 g), bis(diphenylphosphino)palladium(II) dichloride (0.21 g), and tributyl(1-ethoxyvinyl)tin (3.1 mL) were dispersed in DMF (10 mL), and under nitrogen atmosphere, the mixture was heated to 80 °C and stirred for reaction for 8 h. After the reaction was completed, the reaction solution was cooled to room temperature, and the pH was adjusted to acidity with diluted hydrochloric acid. Water (100 mL) and ethyl acetate (100 mL) were added, the mixture was stirred, and the phases were separated. The aqueous phase was extracted with ethyl acetate (100 mL × 2), and the organic phases were combined, washed once with a 10% sodium bicarbonate solution (100 mL) and once with 5% brine (100 mL), dried over anhydrous sodium sulfate, and filtered. The filtrate was concentrated until no liquid flowed out and purified by column chromatography (ethyl acetate:petroleum ether = 1:50 to 1:5) to give compound **1c.** LC-MS: m/z = 377.26 [M+H]⁺.

### Step 3:

**1c** (1.4 g), L-alaninol (0.84 g), and tetraisopropyl titanate (4.5 g) were dispersed in methanol (28 mL), and the mixture was heated to 80 °C and stirred for reaction for 16 h. The reaction solution was cooled to 0 °C, and sodium borohydride (280 mg) was added. The resulting mixture was allowed to be stirred for reaction at room temperature for 4 h. After the reaction was completed, the reaction solution was filtered through celite, and the filtrate was concentrated until no liquid flowed out and purified by column chromatography (methanol:dichloromethane = 1:200 to 1:10) to give compound **1d.** LC-MS: m/z = 436.40 [M+H]⁺.

### Step 4:

**1e** (5 g) was dispersed in acetonitrile (100 mL), and DIPEA (13.8 g) and phosphorus oxychloride (8.19 g) were added sequentially. Under nitrogen atmosphere, the mixture was heated to 80 °C and stirred for reaction for 2 h. After the reaction was completed, the reaction solution was cooled to room temperature and poured into ice water (500 mL), and the mixture was stirred for crystallization for 30 min and filtered. The filter cake was dried under vacuum at 55 °C for 6 h to give compound **1f.**

### Step 5:

**1f** (1.4 g), **1d** (700 mg), and DIPEA (630 mg) were dispersed in tetrahydrofuran (45 mL), and the mixture was stirred for reaction overnight. The reaction solution was concentrated under reduced pressure and purified by column chromatography (ethyl acetate:petroleum ether = 1:20 to 1:5) to give compound **1g.** LC-MS: m/z = 697.31 [M+H]⁺.

### Step 6:

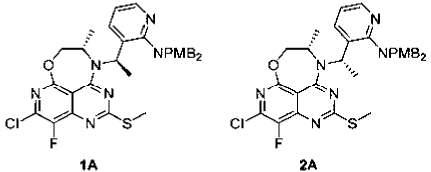

**1g** (1.5 g) and cesium fluoride (3.3 g) were dispersed in DMF (150 mL), and the mixture was heated to 60 °C and stirred for 2 h. After the reaction was completed, the reaction solution was concentrated until no liquid flowed out, and ethyl acetate (100 mL) was added to dissolve the residue. Water (100 mL) was added, and the mixture was stirred, and the phases were separated. The aqueous phase was extracted with ethyl acetate (100 mL × 2). The organic phases were combined, washed with 10% brine (100 mL × 2), dried over anhydrous sodium sulfate, and filtered, and the filtrate was concentrated until no liquid flowed out and purified by preparative SFC (YMC-SA 10 µm 30 × 150 chromatographic column; ethanol-CO₂ (50%-50%, isocratic elution for 0-15 min)) to give **1A** (Rt 10 min, LC-MS: m/z = 661.33 [M+H]⁺) and **2A** (Rt 12 min, LC-MS: m/z = 661.33 [M+H]⁺) in sequence.

### Step 7:

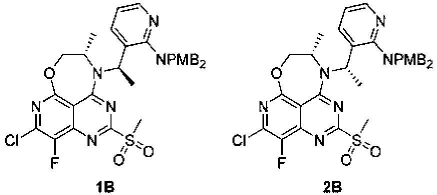

**1A** (6.3 g) was dispersed in dichloromethane (252 mL), and the mixture was cooled to 0-5 °C. m-Chloroperoxybenzoic acid (75%, 8.2 g) was added, and the mixture was stirred for 1 h with the temperature maintained. After the reaction was completed, a 10% sodium thiosulfate solution (100 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out to give compound **1B.** LC-MS: m/z = 693.28 [M+H]⁺.

Referring to the synthesis method of compound **1B,** compound **1A** was replaced with compound **2A** to synthesize compound **2B.** LC-MS: m/z = 693.28 [M+H]⁺.

### Step 8:

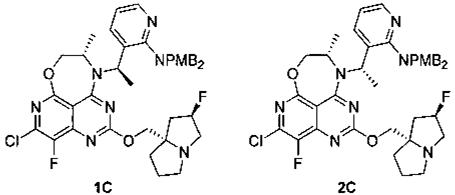

**1B** (6.3 g) and **1h** (4.34 g) were dispersed in tetrahydrofuran (12 mL), and the mixture was cooled to 0 °C. A solution of lithium bis(trimethylsilyl)amide in tetrahydrofuran (1 N, 13.6 mL) was added dropwise, and the mixture was stirred for 1 h with the temperature maintained. After the reaction was completed, a saturated ammonium chloride solution (50 mL) was added to the reaction system to quench the reaction, and water (50 mL) and ethyl acetate (100 mL) were added. The mixture was stirred, and the phases were separated. Extraction was performed with ethyl acetate (80 mL × 2), and the organic phases were combined, washed with 5% brine (80 mL), dried over anhydrous sodium sulfate, and filtered. The filtrate was concentrated until no liquid flowed out and purified by column chromatography (methanol:dichloromethane = 1:200 to 1:30) to give compound **1C.** LC-MS: m/z = 772.38 [M+H]⁺.

Referring to the synthesis method of compound **1C,** compound **1B** was replaced with compound **2B** to synthesize compound **2C.** LC-MS: m/z = 772.33 [M+H]⁺.

### Step 9:

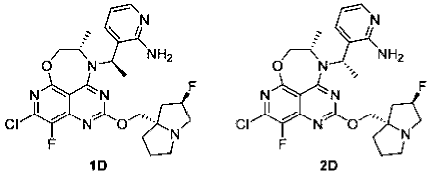

**1C** (8.8 g) was dispersed in trifluoroacetic acid (88 mL), and the mixture was cooled to 0 °C. Methanesulfonic acid (13.2 mL) was added dropwise, and the resulting mixture was stirred for reaction at room temperature for 2 h. After the reaction was completed, the reaction solution was concentrated under reduced pressure until no liquid flowed out, and the residue was dissolved in dichloromethane (200 mL). The pH of the system was adjusted to alkalinity with a saturated sodium bicarbonate solution under an ice bath, and water (100 mL) and dichloromethane (100 mL) were added. The mixture was stirred, and the phases were separated. Extraction was performed with dichloromethane (100 mL × 2), and the organic phases were combined, washed with 10% brine (100 mL), dried over anhydrous sodium sulfate, and filtered. The filtrate was concentrated until no liquid flowed out and purified by column chromatography (methanol:dichloromethane = 1:200 to 1:10) to give compound **1D.** LC-MS: m/z = 532.21 [M+H]⁺.

Referring to the synthesis method of compound **1D,** compound **1C** was replaced with compound **2C** to synthesize compound **2D.** LC-MS: m/z = 532.20 [M+H]⁺.

### Step 10:

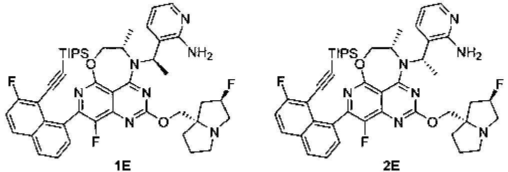

**1D** (1.06 g), **1i** (1.36 g), chloro {[(4-(*N*,*N*-dimethylamino)phenyl]di-tert-butylphosphino}(2'-amino-1,1'-biphenyl-2-yl)palladium(II) (230 mg), potassium phosphate (1.3 g), and water (4.3 mL) were dispersed in 1,4-dioxane (21.3 mL), and under nitrogen atmosphere, the mixture was heated to 90 °C and stirred for reaction for 1 h. The reaction solution was cooled to room temperature and filtered, and the filtrate was concentrated to dryness under reduced pressure and purified by column chromatography (methanol:dichloromethane = 1:200 to 1:10) to give compound **1E.** LC-MS: m/z = 822.52 [M+H]⁺.

Referring to the synthesis method of compound **1E,** compound **1D** was replaced with compound **2D** to synthesize compound **2E.** LC-MS: m/z = 822.52 [M+H]⁺.

### Step 11:

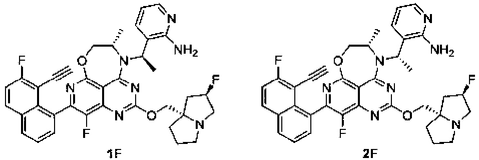

**1E** (4.17 g) and cesium fluoride (7.71 g) were dispersed in DMF (40 mL), and the mixture was stirred for reaction at room temperature for 1 h. After the reaction was completed, the reaction solution was concentrated under reduced pressure until no liquid flowed out, and the residue was dissolved in dichloromethane (100 mL). Water (100 mL) was added, the mixture was stirred, and the phases were separated. The aqueous phase was extracted with dichloromethane (60 mL × 2), and the organic phases were combined, washed with 10% brine (100 mL × 3), dried over anhydrous sodium sulfate, and filtered. The filtrate was concentrated under reduced pressure to dryness, and the residue was purified by column chromatography (methanol:dichloromethane = 1: 100 to 1:10) to give compound **1F.** LC-MS: m/z = 666.31 [M+H]⁺.

Referring to the synthesis method of compound **1F,** compound **1E** was replaced with compound **2E** to synthesize compound **2F.** LC-MS: m/z = 666.30 [M+H]⁺.

### Step 12:

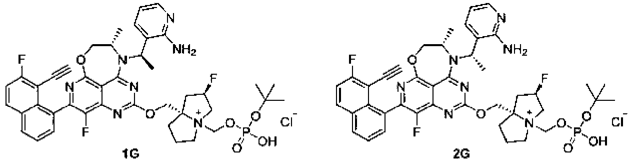

**1F** (270 mg), sodium iodide (900 mg), potassium carbonate (830 mg), and di-tert-butyl chloromethyl phosphate (1040 mg) were dispersed in acetonitrile (50 mL), and the mixture was stirred for reaction at room temperature overnight. The reaction solution was filtered, and the filtrate was purified by column chromatography (methanol:dichloromethane = 1:100 to 1:4) to give compound **1G.** LC-MS: m/z = 832.43 [M]⁺.

Referring to the synthesis method of compound **1G,** compound **1F** was replaced with compound **2F** to synthesize compound **2G.** LC-MS: m/z = 832.41 [M]⁺.

### Step 13:

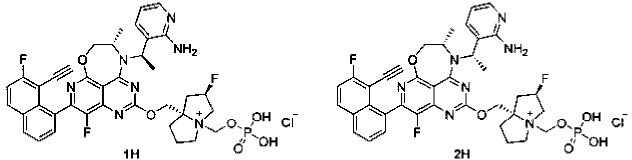

**1G** (200 mg) was dispersed in dichloromethane (10 mL), and a solution of hydrogen chloride in 1,4-dioxane (4 N, 1 mL) was added dropwise to the reaction system. The mixture was stirred for reaction at room temperature for 1 h. After the reaction was completed, the reaction solution was concentrated under reduced pressure until no liquid flowed out to give compound **1H.** LC-MS: m/z = 776.26 [M]⁺.

Referring to the synthesis method of compound **1H,** compound **1G** was replaced with compound **2G** to synthesize compound **2H.** LC-MS: m/z = 776.26 [M]⁺.

### Step 14:

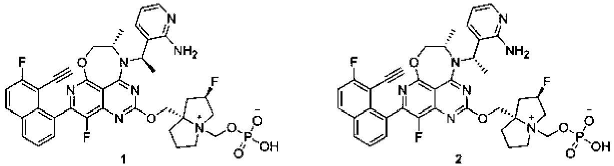

Compound **1H** was purified by preparative liquid chromatography (YMC TA C18 10 µm 30 × 250 chromatographic column; methanol-0.1% aqueous ammonium hydroxide solution (5%-75%/0-70 min gradient elution)) to give compound **1.** LC-MS: m/z = 776.35 [M+H]⁺; ¹H-NMR (500MHz, CD₃OD) *δ* 8.19-8.07 (m, 2H), 8.06-7.97 (m, 1H), 7.86 (d, *J =* 7.5 Hz, 1H), 7.76-7.57 (m, 2H), 7.52-7.39 (m, 1H), 6.89-6.72 (m, 2H), 5.64 (*J =* 55.0 Hz, 1H), 5.29-5.12 (m, 2H), 5.07-4.94 (m, 2H), 4.76-4.48 (m, 2H), 4.45-4.33 (m, 1H), 4.32-4.00 (m, 3H), 3.92-3.78 (m, 1H), 3.68 (s, 0.4H), 3.44 (s, 0.6H), 3.12-2.90 (m, 1H), 2.74-2.31 (m, 5H), 1.85-1.64 (m, 3H), 0.88-0.70 (m, 3H).

Referring to the purification method of compound **1,** compound **1H** was replaced with compound **2H,** and the reaction system was purified to give compound **2.** LC-MS: m/z = 776.35 [M+H]⁺.

### Example 3 and Example 4

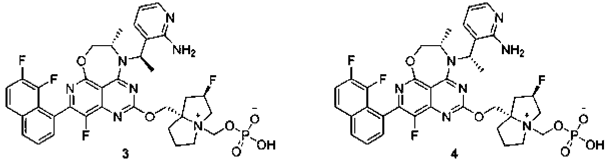

Referring to the preparation process of compound **1** in Example 1, compound 1**i** was replaced with 2-(7,8-difluoronaphthalen-1-yl)-4,4,5,5-tetramethyl-1,3,2-dioxaborolane in step 10 to prepare compound **3.** LC-MS: m/z = 770.41 [M+H]⁺.

Referring to the preparation process of compound **2** in Example 2, compound **1i** was replaced with 2-(7,8-difluoronaphthalen-1-yl)-4,4,5,5-tetramethyl-1,3,2-dioxaborolane in step 10 to prepare compound **4.** LC-MS: m/z = 770.43 [M+H]⁺.

### Example 5 and Example 6

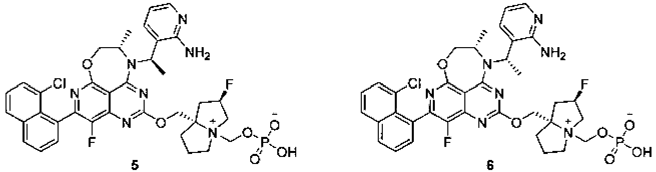

Referring to the preparation process of compound 1 in Example 1, compound **1i** was replaced with 2-(8-chloronaphthalen-1-yl)-4,4,5,5-tetramethyl-1,3,2-dioxaborolane in step 10 to prepare compound **5.** LC-MS: m/z = 768.20 [M+H]⁺.

Referring to the preparation process of compound **2** in Example 2, compound **1i** was replaced with 2-(8-chloronaphthalen-1-yl)-4,4,5,5-tetramethyl-1,3,2-dioxaborolane in step 10 to prepare compound **6.** LC-MS: m/z = 768.18 [M+H]⁺.

### Example 7 and Example 8

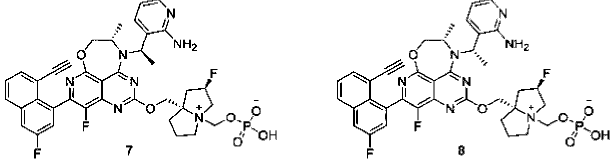

Referring to the preparation process of compound **1** in Example 1, compound **1i** was replaced with ((6-fluoro-8-(4,4,5,5-tetramethyl-1,3,2-dioxaborolan-2-yl)naphthalen-1-yl)ethynyl)triisopropylsilane in step 10 to prepare compound **7.** LC-MS: m/z = 776.26 [M+H]⁺.

Referring to the preparation process of compound **2** in Example 2, compound **1i** was replaced with ((6-fluoro-8-(4,4,5,5-tetramethyl-1,3,2-dioxaborolan-2-yl)naphthalen-1-yl)ethynyl)triisopropylsilane in step 10 to prepare compound **8.** LC-MS: m/z = 776.26 [M+H]⁺.

### Example 9 and Example 10

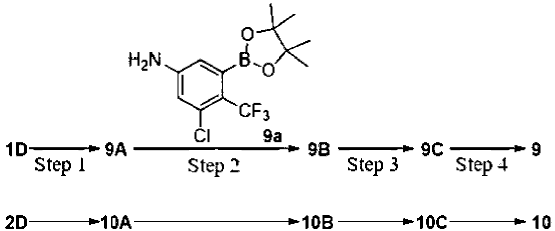

### Step 1:

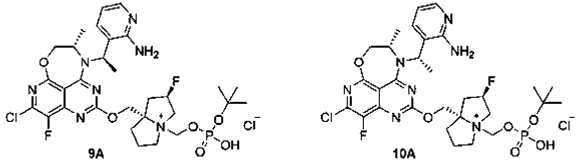

**1D** (0.5 g), sodium iodide (2.1 g), potassium carbonate (1.95 g), and di-tert-butyl chloromethyl phosphate (2.45 g) were dispersed in acetonitrile (25 mL), and the mixture was stirred for reaction overnight at room temperature. The reaction solution was filtered, and the filtrate was purified by column chromatography (methanol:dichloromethane = 1:100 to 1:3) to give compound **9A.** LC-MS: m/z = 698.25 [M]⁺.

Referring to the synthesis method of compound **9A,** compound **1D** was replaced with compound **2D** to synthesize compound **10A.** LC-MS: m/z = 698.24 [M]⁺.

### Step 2:

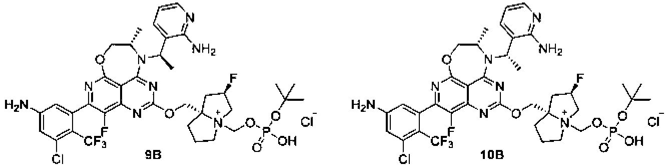

**9A** (315 mg), **9a** (315 mg), chloro{[(4-(*N,N*-dimethylamino)phenyl]di-tert-butylphosphino}(2'-amino-1,1'-biphenyl-2-yl)palladium(II) (52 mg), potassium phosphate (290 mg), and water (6 mL) were dispersed in 1,4-dioxane (30 mL), and under nitrogen atmosphere, the mixture was heated to 90 °C and stirred for reaction for 2 h. The reaction solution was cooled to room temperature and filtered, and the filtrate was concentrated to dryness under reduced pressure and purified by column chromatography (methanol:dichloromethane = 1:200 to 1:10) to give compound **9B.** LC-MS: m/z = 857.35 [M]⁺.

Referring to the synthesis method of compound **9B,** compound **9A** was replaced with compound **10A** to synthesize compound **10B.** LC-MS: m/z = 857.35 [M]⁺.

### Step 3:

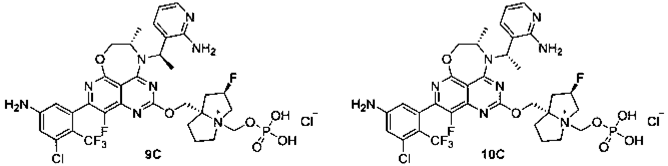

**9B** (290 mg) was dispersed in dichloromethane (8 mL), and a solution of hydrogen chloride in 1,4-dioxane (4 N, 0.8 mL) was added dropwise to the reaction system. The mixture was stirred for reaction at room temperature for 1 h. After the reaction was completed, the reaction solution was concentrated under reduced pressure until no liquid flowed out to give compound **9C.** LC-MS: m/z = 801.23 [M]⁺.

Referring to the synthesis method of compound **9C,** compound **9B** was replaced with compound **10B** to synthesize compound **10C**. LC-MS: m/z = 801.27 [M]⁺.

### Step 4:

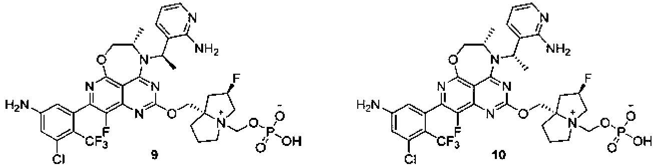

Compound **9C** was purified by preparative liquid chromatography (YMC AQ C18 10 µm 50 × 250 chromatographic column; acetonitrile-0.1% aqueous formic acid solution (15%-75%/0-60 min gradient elution)) to give compound **9.** LC-MS: m/z = 801.23 [M+H]⁺; ¹H-NMR (500MHz, CD₃OD) δ 8.29 (d, *J* = 7.0 Hz, 1H), 8.00 (d, *J =* 6.0 Hz, 1H), 7.08 (t, *J* = 7.0 Hz, 1H), 6.92 (s, 1H), 6.80 (brs, 1H), 6.64-6.33 (m, 1H), 5.66 (dt, *J* = 5.0 Hz, 50.0 Hz, 1H), 5.35-5.24 (m, 1H), 5.23-5.13 (m, 1H), 5.02-4.92 (m, 2H), 4.76-4.65 (m, 1H), 4.50-4.10 (m, 5H), 3.98-3.84 (m, 1H), 3.14-2.96 (m, 1H), 2.78-2.30 (m, 5H), 1.77 (d, *J* = 6.5 Hz, 3H), 0.83 (d, *J* = 7.0 Hz, 3H).

Referring to the purification method of compound **9,** compound **9C** was replaced with compound **10C,** and the reaction system was purified to give compound **10.** LC-MS: m/z = 801.27 [M+H]⁺.

### Example 1l and Example 12

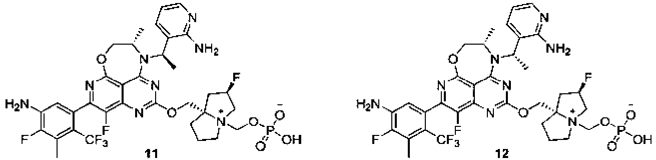

Referring to the preparation process of compound **9** in Example 9, compound **9a** was replaced with 2-fluoro-3-methyl-5-(4,4,5,5-tetramethyl-1,3,2-dioxaborolan-2-yl)-4-(trifluoromethyl)aniline in step 2 to prepare compound **11.** LC-MS: m/z = 799.33 [M+H]⁺).

Referring to the preparation process of compound **10** in Example 10, compound **9a** was replaced with 2-fluoro-3-methyl-5-(4,4,5,5-tetramethyl-1,3,2-dioxaborolan-2-yl)-4-(trifluoromethyl)aniline in step 2 to prepare compound **12.** LC-MS: m/z = 799.34 [M+H]⁺).

### Example 13

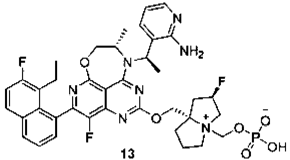

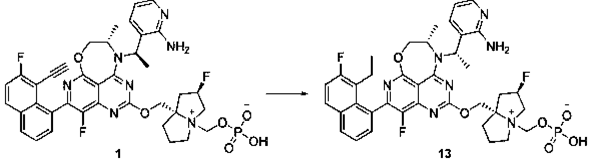

**1** (50 mg) and 10% palladium on carbon (20 mg) were dispersed in methanol (10 mL), and under hydrogen atmosphere, the mixture was purged with hydrogen and stirred for reaction at room temperature overnight. After the reaction was completed, the reaction solution was filtered, and the filtrate was purified by preparative liquid chromatography (YMC TA C18 10 µm 30 × 250 chromatographic column; methanol-0.1% aqueous formic acid solution (20%-80%/0-60 min gradient elution)) to give compound **13.** LC-MS: m/z = 780.33 [M+H]⁺.

### Example 14

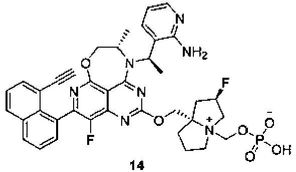

Referring to the preparation process of compound **1** in Example 1, compound **1i** was replaced with triisopropyl((8-(4,4,5,5-tetramethyl-1,3,2-dioxaborolan-2-yl)naphthalen-1-yl)ethynyl)silane in step 10 to prepare compound **14.** LC-MS: m/z = 758.28 [M+H]⁺.

### Example 15

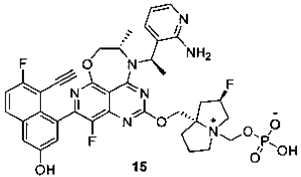

Referring to the preparation process of compound **1** in Example 1, compound **1i** was replaced with ((2-fluoro-6-(methoxymethoxy)-8-(4,4,5,5-tetramethyl-1,3,2-dioxaborolan-2-yl)naphthalen-1-yl)ethynyl)triisopropylsilane in step 10 to prepare compound **15.** LC-MS: m/z = 792.33 [M+H]⁺.

### Example 16

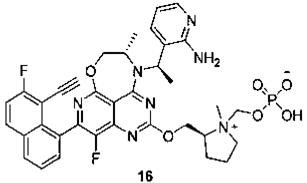

Referring to the preparation process of compound **1** in Example 1, compound **1h** was replaced with *N*-methyl-*L-*prolinol in step 8 to prepare compound **16.** LC-MS: m/z = 732.28 [M+H]⁺.

### Example 17

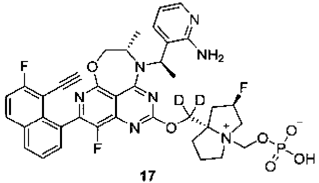

Referring to the preparation process of compound 1 in Example **1,** compound **1h** was replaced with ((2*R*,7a*S*)-2-fluorotetrahydro-1*H*-pyrrolizin-7a(5*H*)-yl)methan-*d*₂-ol in step 8 to prepare compound 17. LC-MS: m/z = 778.36 [M+H]⁺.

### Example 18

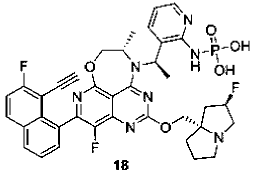

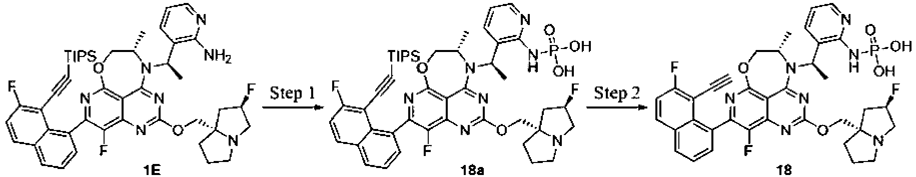

### Step 1:

**1E** (330 mg) and triethylamine (6 mL) were dispersed in dichloromethane (40 mL), and the mixture was cooled to 0 °C. Phosphorus oxychloride (180 mg) was added dropwise, and the mixture was stirred for reaction at room temperature for 1 h. Water (10 mL) was added to the reaction solution, the resulting mixture was stirred, and the phases were separated. The organic phase was washed with a saturated ammonium chloride solution (50 mL × 3), dried over anhydrous sodium sulfate, and filtered. The filtrate was concentrated until no liquid flowed out to give compound **18a.** LC-MS: m/z = 902.53 [M+H]⁺.

### Step 2:

**18a** (200 mg) and cesium fluoride (500 mg) were dispersed in DMF (2.5 mL), and the mixture was stirred for reaction at room temperature for 1 h. Water (15 mL) and dichloromethane (25 mL) were added to the reaction solution, the resulting mixture was stirred, and the phases were separated. The organic phase was dried over anhydrous sodium sulfate and filtered, and the filtrate was concentrated until no liquid flowed out and purified by preparative liquid chromatography (YMC TA C18, 10 µm, 30 × 250 chromatographic column; acetonitrile-0.1% aqueous ammonium hydroxide solution (10%-70%/0-50 min gradient elution)) to give compound **18.** LC-MS: m/z = 746.37 [M+H]⁺.

### Example 19

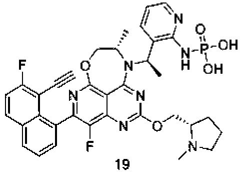

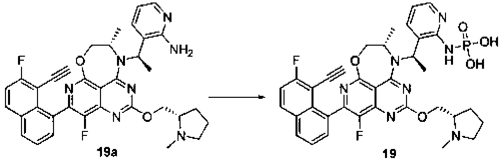

Referring to the preparation process of compound **1F** in Example 1, compound **1h** was replaced with *N*-methyl-*L-*prolinol in step 8 to prepare compound **19a.** LC-MS: m/z = 622.40 [M+H]⁺.

Referring to the preparation method for compound **18a** in Example 18, **1E** was replaced with **19a** to prepare compound 19. LC-MS: m/z = 702.22 [M+H]⁺.

### Example 20

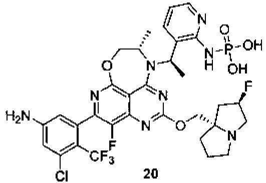

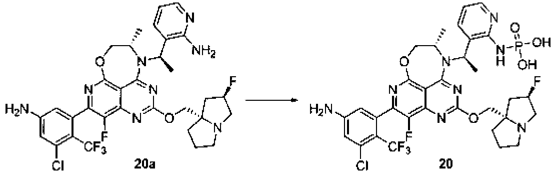

Referring to the preparation process of compound **1E** in Example 1, compound **1i** was replaced with compound **9a** in step 10 to prepare compound **20a.** LC-MS: m/z = 691.23 [M+H]⁺.

Referring to the preparation method for compound **18a** in Example 18, **1E** was replaced with **20a** to prepare compound **20.** LC-MS: m/z = 771.24 [M+H]⁺.

### Example 21

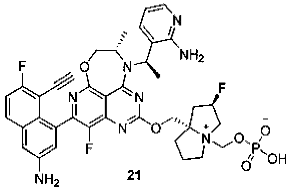

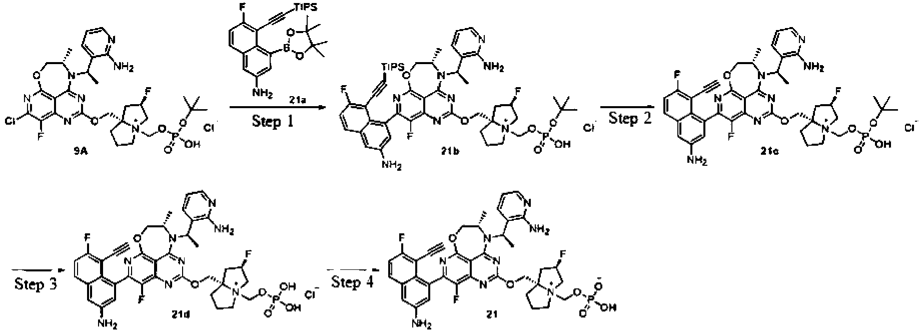

### Step 1:

Referring to the preparation method for compound **9B** in Example 9, **9a** was replaced with 21a to prepare compound **21b.** LC-MS: m/z = 1003.46 [M]⁺.

### Step 2:

Referring to the preparation method for compound **1F** in Example 1, **1E** was replaced with **21b** to prepare compound **21c.** LC-MS: m/z = 847.33 [M]⁺.

### Step 3:

Referring to the preparation method for compound **1H** in Example 1, **1G** was replaced with **21c** to prepare compound **21d.** LC-MS: m/z = 791.30 [M]⁺.

### Step 4:

Compound **21d** was purified by preparative liquid chromatography (YMC AQ C18 10 µm 30 × 250 chromatographic column; methanol-0.1% aqueous acetic acid solution (10%-10%-15%-55%/0-4-5-85 min gradient elution)) to give compound **21.** LC-MS: m/z = 791.30 [M+H]⁺.

### Example 22

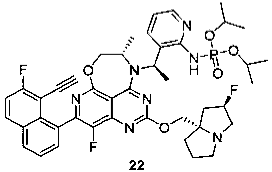

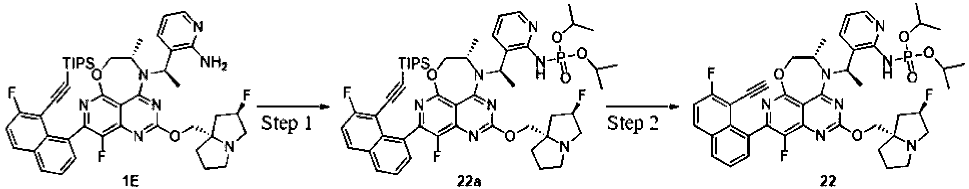

### Step 1:

**1E** (300 mg) and triethylamine (364 mg) were dispersed in dichloromethane (30 mL), and the mixture was cooled to 0 °C. Diisopropyl chlorophosphate (732 mg) was added dropwise, and the mixture was stirred for reaction at room temperature overnight. Water (10 mL) was added to the reaction solution, the resulting mixture was stirred, and the phases were separated. The organic phase was washed with a saturated ammonium chloride solution (50 mL × 3), dried over anhydrous sodium sulfate, and filtered. The filtrate was concentrated until no liquid flowed out to give compound **22a.** LC-MS: m/z = 986.56 [M+H]⁺.

### Step 2:

**22a** (400 mg) and cesium fluoride (920 mg) were dispersed in DMF (20 mL), and the mixture was stirred for reaction at room temperature for 4 h. Water (50 mL) and ethyl acetate (50 mL) were added to the reaction solution, the resulting mixture was stirred, and the phases were separated. The aqueous phase was extracted with ethyl acetate (50 mL), and the organic phases were combined, washed with 10% brine (50 mL × 2), dried over anhydrous sodium sulfate, and filtered. The filtrate was concentrated under reduced pressure until no liquid flowed out, and the residue was purified by preparative liquid chromatography (YMC TA C18 10 µm 50 × 250 chromatographic column; methanol-0.1% aqueous ammonium hydroxide solution (30%-90%-100%/0-60-70 min gradient elution)) to give compound **22.** LC-MS: m/z = 830.48 [M+H]⁺.

### Test Example 1: Experimental Method for Nucleotide Exchange

1. Reagents: His-KRAS G12D (1-169); SOS1^{cat} (564-1049); GDP (purchased from Sigma); Anti-6HIS-Cryptate (purchased from Cisbio); EDA-GTP-DY-647P1 (purchased from Jena Bioscience)
2. Buffer preparation:
   1) assay buffer: HEPES pH 7.4, NaCl, MgCl₂, DTT, BSA, and Igepal;
   2) KRAS G12D working solution: A KRAS G12D working solution containing 100 nM His-KRAS G12D and 2 nM Anti-6HIS-Cryptate was prepared using the assay buffer;
   3) SOS1^{cat} working solution: An SOS1^{cat} working solution containing 20 nM SOS1^{cat} and 200 nM EDA-GTP-DY-647P1 was prepared using the assay buffer;
   4) blank control working solution: A blank control solution containing 2 nM Anti-6HIS-Cryptate was prepared using the assay buffer.

### 3. Testing procedure:

The entire experimental process was completed at room temperature. Using a black clear-bottom 384-well plate, 5 µL of KRAS G12D working solution was added to each well for the experimental and negative groups, while 5 µL of blank control working solution was added to each well for the blank group. The plate was incubated at room temperature for 10 min. Then, the compounds (at an initial concentration of 100 µM, 8 concentration gradients by 1:2 serial dilution) were added to the experimental groups using an ultra microsyringe and incubated at room temperature for 30 min. Finally, 5 µL of SOS1^{cat} working solution was added to each well, and after incubation at room temperature for 15 min, signal values were detected at 665 nm/620 nm using a PerkinElmer Envision HTS multi-label plate reader. The inhibition rate was calculated by the following formula: inhibition rate (%) = (mean value of negative control group - mean value of experimental group)/(mean value of negative control group - mean value of blank group) × 100%. The curves were fitted using the four-parameter logistic model with the logarithm of the compound concentration as the abscissa and the inhibition rate as the ordinate, and IC ₅₀ values were calculated.

### Test Example 2: Assay for Inhibitory Activity Against AsPc-1 Cell Proliferation

AsPc-1 cells in a well-growing state were collected into a centrifuge tube, adjusted to a cell density of 3 × 10⁴ cells/mL, and inoculated onto a 96-well plate (100 µL/well). The compounds were added using a nanoliter pipettor such that the final concentrations of the compounds were 5000 nM-0.31 nM (the addition was performed in duplicate). Meanwhile, a control was set. After 72 h of incubation in the cell incubator, the assay reagent CCK-8 (manufacturer: Dojindo Laboratories; 10 µL/well) was added. After 3 h of incubation in the cell incubator, the absorbance values were measured at 450 nm on a PerkinElmer Envision microplate reader. A four-parameter analysis was performed, a dose-response curve was fitted, and IC₅₀ was calculated.

### Test Example 3: Assay for Inhibitory Activity Against Capan-1 Cell Proliferation

Capan-1 cells in a well-growing state were collected into a centrifuge tube, adjusted to a cell density of 1.5 × 10⁴ cells/mL, and inoculated onto a 96-well plate (100 µL/well). The compounds were added using a nanoliter pipettor such that the final concentrations of the compounds were 20000 nM-9.1 nM (the addition was performed in duplicate). Meanwhile, a control was set. After 5 days of incubation in the cell incubator, the assay reagent CCK-8 (manufacturer: Dojindo Laboratories, Beijing; 10 µL/well) was added. After 4 h of incubation in the cell incubator, the absorbance values were measured at 450 nm on a PerkinElmer Envision microplate reader. A four-parameter analysis was performed, a dose-response curve was fitted, and IC₅₀ was calculated.

### Test Example 4: Assay for Inhibitory Activity Against NCI-H358 Cell Proliferation

NCI-H358 cells in a well-growing state were collected into a centrifuge tube, adjusted to a cell density of 3 × 10⁴ cells/mL, and inoculated onto a 96-well plate (100 µL/well). The compounds were added using a nanoliter pipettor such that the final concentrations of the compounds were 10000 nM-4.6 nM (the addition was performed in duplicate). Meanwhile, a control was set. After 72 h of incubation in the cell incubator, the assay reagent CCK-8 (manufacturer: Dojindo Laboratories, Beijing; 10 µL/well) was added. After 3 h of incubation in the cell incubator, the absorbance values were measured at 450 nm on a PerkinElmer Envision microplate reader. A four-parameter analysis was performed, a dose-response curve was fitted, and IC₅₀ was calculated.

### Test Example 5: Assay for Inhibitory Activity Against HCT-116 Cell Proliferation

HCT-116 cells in a well-growing state were collected into a centrifuge tube, adjusted to a cell density of 1 × 10⁴ cells/mL, and inoculated onto a 96-well plate (100 µL/well). The compounds were added using a nanoliter pipettor such that the final concentrations of the compounds were 20000 nM-9.1 nM (the addition was performed in duplicate). Meanwhile, a control was set. After 5 days of incubation in the cell incubator, the assay reagent CCK-8 (manufacturer: Dojindo Laboratories, Beijing; 10 µL/well) was added. After 2 h of incubation in the cell incubator, the absorbance values were measured at 450 nm on a PerkinElmer Envision microplate reader. A four-parameter analysis was performed, a dose-response curve was fitted, and IC₅₀ was calculated.

### Test Example 6: Assay for Inhibitory Activity Against RPMI-8226 Cell Proliferation

RPMI-8226 cells in a well-growing state were collected into a centrifuge tube, adjusted to a cell density of 3 × 10⁴ cells/mL, and inoculated onto a 96-well plate (100 µL/well). The compounds were added using a nanoliter pipettor such that the final concentrations of the compounds were 10000 nM-4.6 nM (the addition was performed in duplicate). Meanwhile, a control was set. After 72 h of incubation in the cell incubator, the assay reagent CCK-8 (manufacturer: Dojindo Laboratories, Beijing; 10 µL/well) was added. After 4 h of incubation in the cell incubator, the absorbance values were measured at 450 nm on a PerkinElmer Envision microplate reader. A four-parameter analysis was performed, a dose-response curve was fitted, and IC₅₀ was calculated.

### Test Example 7: Assay for Inhibitory Activity Against NCI-H1975 Cell Proliferation

NCI-H1975 cells in a well-growing state were collected into a centrifuge tube, adjusted to a cell density of 1 × 10⁴ cells/mL, and inoculated onto a 96-well plate (100 µL/well). The compounds were added using a nanoliter pipettor such that the final concentrations of the compounds were 20000 nM-9.1 nM (the addition was performed in duplicate). Meanwhile, a control was set. After 5 days of incubation in the cell incubator, the assay reagent CCK-8 (manufacturer: Dojindo Laboratories, Beijing; 10 µL/well) was added. After 2 h of incubation in the cell incubator, the absorbance values were measured at 450 nm on a PerkinElmer Envision microplate reader. A four-parameter analysis was performed, a dose-response curve was fitted, and IC₅₀ was calculated.

The above testing results for some of the compounds of the present disclosure are shown in Table 1.

**Table 1. Testing results for part of compounds**

| Compound | Inhibitory activity IC₅₀ against proliferation of AsPc-1 cells (nM) | Inhibitory activity IC₅₀ against proliferation of Capan-1 cells (nM) | Inhibitory activity IC₅₀ against proliferation of NCI-H358 cells (nM) | Inhibitory activity IC₅₀ against proliferation of HCT-116 cells (nM) | Inhibitory activity IC₅₀ against proliferation of RPMI-8226 cells (nM) | Inhibitory activity IC₅₀ against proliferation of NCI-H1975 cells (nM) |
|---|---|---|---|---|---|---|
| 1 | A | A | A | A | A | >10000 |
| 3 | | A | | | | |
| 5 | | A | | | | |
| 6 | | A | | | | |
| 9 | A | A | | | | |
| 10 | | A | | | | |
| 13 | A | A | | | | |
| 15 | A | A | | | | |
| 16 | | A | | | | |
| 17 | A | A | | | | |
| 18 | A | A | | | | |
| 19 | A | A | | | | |
| 20 | A | A | | | | |
| 21 | A | A | | | | |

| | | | | | | |
|---|---|---|---|---|---|---|
| Note: A represents IC₅₀ ≤ 100 nM; B represents 100 nM < IC₅₀ ≤ 500 nM; C represents 500 nM < IC₅₀ ≤ 999 nM; - represents the IC₅₀ value not detected. | | | | | | |

### Test Example 8: In Vitro Stability in Liver Microsomes

Liver microsome incubation samples (species: human, monkey, rat, and mouse) were each prepared by mixing a PBS buffer (pH = 7.4), a liver microsome solution (0.5 mg/mL), a test compound, and an NADPH + MgCl₂ solution, and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The results are shown in Table 2.

**Table 2. Results for in vitro metabolic stability in liver microsomes**

| **Compound** | Remaining amount % (T = 60 min) (0.5 mg/mL) | |
|---|---|---|
| | Human liver microsome | Mouse liver microsome |
| 1 | > 40 | > 40 |
| 15 | > 40 | > 40 |
| 17 | > 40 | > 40 |

### Test Example 9: Evaluation for In Vivo Pharmacokinetics in Mice

ICR mice weighing 20-24 g were randomized into groups with 9 mice in each group after 3 to 5 days of acclimatization, and intragastric administration of the experimental compounds was performed at a dose of 35 mg/kg.

The test animals (ICR mice) were fasted for 12 h before administration and fed 4 h after administration, and were given free access to water before, after, and during the experiment.

After intragastric administration, about 0.1 mL of blood was collected via the orbital sinus at 0.25 h (15 min), 0.5 h (30 min), 1 h, 2 h, 4 h, 6 h, 8 h, and 10 h (each mouse was subjected to blood collection at 3 to 4 time points, with 3 mice per time point). Following anticoagulation with EDTA-K2, the blood samples were transferred to a centrifuge within 30 min and then centrifuged at 4000 rpm for 10 min at 4 °C to separate the plasma. All the plasma samples were immediately stored after collection at -20 °C for testing.

20 µL of each of the plasma samples to be tested and the standard curve sample were pipetted, and 400 µL of acetonitrile solution containing an internal standard (20 ng/mL) was added. The mixture was shaken and mixed well for 10 min, and centrifuged at 13000 rpm for 10 min. 50 µL of the supernatant was taken and diluted with 100 µL of ultrapure water. After being mixed well, 0.5 µL of the resulting sample was assayed by LC/MS/MS, and a chromatogram was recorded.

Experimental results: The compounds of the present application, e.g., example compounds 1, 3, 5, 9, 17, and 18, have good oral pharmacokinetic parameters (including Tmax, Cmax, AUC, etc.) in mice.

### Test Example 10: Evaluation for In Vivo Pharmacokinetics in Rats

SD rats weighing 210-230 g were randomized into groups with 3 rats in each group after 3 to 5 days of acclimatization, and intragastric administration of an example solution was performed at a dose of 35 mg/kg.

Plasma samples to be tested were prepared by taking blood from the orbit at time points of 0 min, 15 min, 30 min, 1 h, 2 h, 4 h, 6 h, 8 h, 10 h, 24 h, and 32 h.

50 µL of each plasma sample to be tested was taken, and an acetonitrile solution containing an internal standard was added. Supernatants were obtained by protein precipitation, diluted, and then assayed by LC/MS/MS, and fitting was performed using a non-compartmental model.

### Test Example 11: Evaluation for In Vivo Pharmacokinetics in Dogs

Beagle dogs weighing 10-12 kg were randomized into groups with 3 dogs in each group after 3 to 5 days of acclimatization, and intragastric administration of an example solution was performed at a dose of 17.5 mg/kg.

Plasma samples to be tested were prepared by taking blood from the forelimb vein at time points of 0 min, 15 min, 30 min, 1 h, 2 h, 4 h, 6 h, 8 h, 10 h, 24 h, 32 h, and 48 h.

50 µL of each plasma sample to be tested was taken, and an acetonitrile solution containing an internal standard was added. Supernatants were obtained by protein precipitation, diluted, and then assayed by LC/MS/MS, and fitting was performed using a non-compartmental model.

### Test Example 12: Pharmacodynamic Evaluation for AsPc-1 Cell NOD-SCID Mouse Subcutaneous Xenograft Tumor Model

SPF-grade female NOD-SCID mice (source: Jiangsu Huachuang Xinnuo Pharmaceutical Technology Co., Ltd.) were inoculated subcutaneously at the right axilla with AsPc-1 cells at 1 × 10⁷ cells/mouse. When the mean tumor volume reached about 200 mm³, the animals were grouped.

The day of grouping was defined as day 0, and intraperitoneal injection was started on day 1 and performed once daily. The tumor volume and body weight were measured twice to thrice every week, and the data were recorded. The general behavior of the mice was observed and recorded every day. After the experiment was completed, the tumors were extracted, weighed, and photographed.

The detection parameters and the calculation formulas are as follows: $tumor volume TV \left({mm}^{3}\right) = 1 / 2 \times \left(a\times {b}^{2}\right) ,$ where a represents the long diameter of the tumor, and b represents the short diameter of the tumor; $relative tumor volume RTV = TVt / TV 0 ,$ where TV0 represents the tumor volume on day 0, and TVt represents the tumor volume at each measurement; $relative tumor proliferation rate T / C \left(%\right) = \left(TRTV/CRTV\right) \times 100 % ,$ where TRTV represents RTV of the treatment group, and CRTV represents RTV of the vehicle control group; $tumor growth inhibition rate TGI \left(%\right) = \left(1-TW/TW0\right) \times 100 % ;$ where TW represents the tumor weight of the treatment group, and TW0 represents the tumor weight of the vehicle control group; $weight change rate WCR \left(%\right) = \left(Wtt-Wt0\right) / Wt 0 \times 100 % ,$ where Wt0 represents the body weight of the mouse on day 0, and Wtt represents the body weight of the mouse at each measurement.

### Test Example 13: Pharmacodynamic Evaluation for NCI-H358 Cell CB17-SCID Mouse Subcutaneous Xenograft Tumor Model

SPF-grade female CB17-SCID mice (source: Shanghai Lingchang Biotech Co. Ltd.) were inoculated subcutaneously at the right axilla with NCI-H358 cells at 5 × 10⁶ cells/mouse (inoculation by mixing with Matrigel at a ratio of 1:1). When the mean tumor volume reached about 200 mm³, the animals were grouped.

The day of grouping was defined as day 0, and intragastric administration was started on day 0 and performed once daily (1-10 mg/kg). The tumor volume and body weight were measured twice to thrice every week, and the data were recorded. The general behavior of the mice was observed and recorded every day. After the experiment was completed, the tumors were extracted, weighed, and photographed.

The detection parameters and the calculation formulas are as follows: $tumor volume TV \left({mm}^{3}\right) = 1 / 2 \times \left(a\times {b}^{2}\right) ,$ where a represents the long diameter of the tumor, and b represents the short diameter of the tumor; $relative tumor volume RTV = {TV}_{t} / {TV}_{0} ,$ wherein TV₀ represents the tumor volume on day 0, and TVₜ represents the tumor volume at each measurement; $relative tumor proliferation rate T / C \left(%\right) = \left({T}_{RTV}/{C}_{RTV}\right) \times 100 % ,$ where T_{RTV} represents RTV of the treatment group, and C_{RTV} represents RTV of the vehicle control group; $tumor growth inhibition rate TGI \left(%\right) = \left(1-TW/TW0\right) \times 100 % ,$ where TW represents the tumor weight of the treatment group, and TW0 represents the tumor weight of the vehicle control group; $weight change rate WCR \left(%\right) = \left({Wt}_{t}-{Wt}_{0}\right) / {Wt}_{0} \times 100 % ,$ where Wt₀ represents the mouse body weight on day 0, and Wtₜ represents the mouse body weight at each measurement.

Experimental results: After two weeks of administration, some of the compounds of the present application, including example compounds, showed excellent pharmacodynamic indexes in the NCI-H358 cell CB17-SCID mouse subcutaneous xenograft tumor model, including but not limited to, a tumor growth inhibition rate (e.g., a tumor growth inhibition rate greater than 80% or greater than 90%).

### Test Example 14: Pharmacodynamic Evaluation for Capan-1 Cell Nude Mouse Subcutaneous Xenograft Tumor Model

SPF-grade female nude mice (source: Changzhou Cavens Laboratory Animal Ltd.) were inoculated subcutaneously at the right axilla with Capan-1 cells at 2 × 10⁶ cells/mouse. When the mean tumor volume reached about 200-250 mm³, the animals were grouped.

The day of grouping was defined as day 0, and intragastric administration was started on day 0 and performed once daily (20-60 mg/kg). The tumor volume and body weight were measured twice to thrice every week, and the data were recorded. The general behavior of the mice was observed and recorded every day. After the experiment was completed, the tumors were extracted, weighed, and photographed.

The detection parameters and the calculation formulas are as follows: $Tumor volume TV \left({mm}^{3}\right) = 1 / 2 \times \left(a\times {b}^{2}\right) ,$ where a represents the long diameter of the tumor, and b represents the short diameter of the tumor. $Relative tumor volume RTV = {TV}_{t} / {TV}_{0} ,$ wherein TV₀ represents the tumor volume on day 0, and TVₜ represents the tumor volume at each measurement. $Relative tumor proliferation rate T / C \left(%\right) = \left({T}_{RTV}/{C}_{RTV}\right) \times 100 % ,$ where T_{RTV} represents RTV of the treatment group, and C_{RTV} represents RTV of the vehicle control group. $Tumor growth inhibition rate TGI \left(%\right) = \left(1-TW/TW0\right) \times 100 % ,$ where TW represents the tumor weight of the treatment group, and TW0 represents the tumor weight of the vehicle control group. $Weight change rate WCR \left(%\right) = \left({Wt}_{t}-{Wt}_{0}\right) / {Wt}_{0} \times 100 % ,$ where Wt₀ represents the mouse body weight on day 0, and Wtₜ represents the mouse body weight at each measurement.

Experimental results: After 37 days of administration, some of the compounds of the present application, including example compounds, showed excellent pharmacodynamic indexes in the Capan-1 cell nude mouse subcutaneous xenograft tumor model, including but not limited to, a tumor growth inhibition rate (e.g., a tumor growth inhibition rate greater than 80% or greater than 90%).

## Claims

1. A compound of formula (I) or a pharmaceutically acceptable salt thereof, 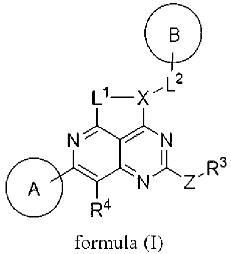 wherein,
X is selected from the group consisting of -N- and -CH- optionally substituted with R^{x};
R^{x} is selected from the group consisting of deuterium, halogen, -OH, -NH₂, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₁₋₄ heteroalkenylene;
each R¹ is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L² is selected from a single bond, and ring B is selected from the group consisting of the following groups optionally substituted with one or more R^{B} or R^{C}: 3- to 12-membered cycloalkenyl, 3- to 12-membered heterocycloalkenyl, 6- to 10-membered aryl, and 5- to 10-membered heteroaryl;
or L² is selected from -C(R^{2a}R^{2b})-, and ring B is selected from the group consisting of the following groups optionally substituted with one or more R^{B} or R^{C}: 3- to 12-membered cycloalkyl, 3- to 12-membered heterocyclyl, 6- to 10-membered aryl, and 5- to 10-membered heteroaryl;
R^{2a} is selected from the group consisting of H, deuterium, halogen, -OH, -NH₂, -CN, and the following groups optionally substituted with one or more R^{2a1}: C₁₋₆ alkyl, C₁₋₆ alkoxy, C₁₋₆ alkylthio, C₁₋₆ alkylamino, di-C₁₋₆ alkylamino, 3- to 12-membered cycloalkyl, and 3- to 12-membered heterocyclyl;
each R^{2a1} is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R^{2b} is selected from the group consisting of H, deuterium, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
or R^{2a} and R^{2b} form =O together;
each R^{B} is independently selected from the group consisting of deuterium, oxo, halogen, -CN, -OH, -NH₂, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
ring A is selected from the group consisting of the following groups optionally substituted with one or more R^{A} or R^{C}: 3- to 12-membered cycloalkyl, 3- to 12-membered cycloalkenyl, 3- to 12-membered heterocyclyl, 6- to 10-membered aryl, and 5- to 10-membered heteroaryl;
each R^{A} is independently selected from the group consisting of deuterium, oxo, halogen, -CN, -OH, -NH₂, and the following groups optionally substituted with one or more R^{A1}: C₁₋₆ alkyl, C₂₋₆ alkenyl, C₂₋₆ alkynyl, C₁₋₆ alkoxy, C₁₋₆ alkylthio, C₁₋₆ alkylamino, di-C₁₋₆ alkylamino, 3- to 12-membered cycloalkyl, 3- to 12-membered heterocyclyl, 6- to 10-membered aryl, and 5- to 10-membered heteroaryl;
each R^{A1} is independently selected from the group consisting of deuterium, oxo, halogen, -OH, -NH₂, -CN, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino;
Z is selected from the group consisting of a single bond, -S-, -O-, and the following groups optionally substituted with one or more R^{z}: -NH- and -N(C₁₋₆ alkyl)-;
each R^{z} is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R³ is selected from the group consisting of H, deuterium, and the following groups optionally substituted with one or more R^{3a} or R^{C}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3a} is independently selected from the group consisting of deuterium, oxo, halogen, -OH, -NH₂, -CN, and the following groups optionally substituted with one or more R^{3b}: =NH, =CH₂, =N(C₁₋₆ alkyl), =CH(C₁₋₆ alkyl), =C(C₁₋₆ alkyl)₂, C₁₋₆ alkyl, C₁₋₆ alkoxy, C₁₋₆ alkylthio, C₁₋₆ alkylamino, and di-C₁₋₆ alkylamino;
each R^{3b} is independently selected from the group consisting of deuterium, oxo, halogen, -OH, -NH₂, -CN, C₁₋₄ alkoxy, C₁₋₄ alkylthio, C₁₋₄ alkylamino, and di-C₁₋₄ alkylamino;
each R^{C} is independently selected from the group consisting of -NR^{C1}C(O)R^{C2}, -NR^{C1}C(O)OR^{C2} - NR^{C1}C(O)OC₁₋₆ alkylene OC(O)R^{C2}, -NR^{C1}C(O)OC₁₋₆ alkylene NR^{C1}C(O)R^{C2}, -NR^{C1}C(O)NR^{C1}R^{C2}, - NR^{C1}C(O)NR^{C1}C₁₋₆ alkylene NR^{C1}C(O)R^{C2}, -NR^{C1}C(O)NR^{C1}C₁₋₆ alkylene OC(O)R^{C2}, -OC(O)R^{C2}, - OC(O)OR^{C2}, -OC(O)OC₁₋₆ alkylene OC(O)R^{C2}, -OC(O)OC₁₋₆ alkylene NR^{C1}C(O)R^{C2}, -OC(O)NR^{C1}R^{C2}, - OC(O)NR^{C1}C₁₋₆ alkylene OC(O)R^{C2}, -OC(O)NR^{C1}C₁₋₆ alkylene NR^{C1}C(O)R^{C2}, -P(O)(OR^{C2})₂, -C₁₋₆ alkylene P(O)(OR^{C2})₂, -C₁₋₆ alkylene NR^{C1}P(O)(OR^{C2})₂, -C₁₋₆ alkylene OP(O)(OR^{C2})₂, -NR^{C1}P(O)(OR^{C2})₂, -NR^{C1}C₁₋₆ alkylene P(O)(OR^{C2})₂, -NR^{C1}C₁₋₆ alkylene OP(O)(OR^{C2})₂, -NR^{C1}C₁₋₆ alkylene NR^{C1}P(O)(OR^{C2})₂, - OP(O)(OR^{C2})₂, -OC₁₋₆ alkylene P(O)(OR^{C2})₂, -OC₁₋₆ alkylene OP(O)(OR^{C2})₂, -OC₁₋₆ alkylene NR^{C1}P(O)(OR^{C2})₂, -P(O)HOR^{C2}, -C₁₋₆ alkylene P(O)HOR^{C2}, -C₁₋₆ alkylene NR^{C1}P(O)HOR^{C2}, -C₁₋₆ alkylene OP(O)HOR^{C2}, -NR^{C1}P(O)HOR^{C2}, -NR^{C1}C₁₋₆ alkylene P(O)HOR^{C2}, -NR^{C1}C₁₋₆ alkylene OP(O)HOR^{C2}, - NR^{C1}C₁₋₆ alkylene NR^{C1}P(O)HOR^{C2}, -OP(O)HOR^{C2}, -OC₁₋₆ alkylene P(O)HOR^{C2}, -OC₁₋₆ alkylene OP(O)HOR^{C2}, and -OC₁₋₆ alkylene NR^{C1}P(O)HOR^{C2};
R^{C1} is independently selected from the group consisting of H and C₁₋₆ alkyl;
R^{C2} is independently selected from the group consisting of H and the following groups optionally substituted with one or more R^{C3}: C₁₋₁₂ alkyl, C₁₋₁₂ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{C3} is independently selected from the group consisting of deuterium, oxo, halogen, -OH, -CN, -NH₂, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, di-C₁₋₄ haloalkylamino, -P(O)(OH)₂, -C₁₋₄ alkylene P(O)(OH)₂, -C₁₋₄ alkylene OP(O)(OH)₂, -C₁₋₄ alkylene NR^{C1}P(O)(OH)₂, -NR^{C1}P(O)(OH)₂, -NR^{C1}C₁₋₄ alkylene P(O)(OH)₂, - NR^{C1}C₁₋₄ alkylene OP(O)(OH)₂, -NR^{C1}C₁₋₄ alkylene NR^{C1}P(O)(OH)₂, -OP(O)(OH)₂, -OC₁₋₄ alkylene P(O)(OH)₂, -OC₁₋₄ alkylene OP(O)(OH)₂, -OC₁₋₄ alkylene NR^{C1}O)(OH)₂, -P(O)HOH, -C₁₋₄alkylene P(O)HOH, -C₁₋₄ alkylene OP(O)HOH, -C₁₋₄ alkylene NR^{C1}P(O)HOH, -NR^{C1}P(O)HOH, -NR^{C1}C₁₋₄ alkylene P(O)HOH, -NR^{C1}C₁₋₄ alkylene OP(O)HOH, -NR^{C1}C₁₋₄ alkylene NR^{C1}P(O)HOH, -OP(O)HOH, -OC₁₋₄ alkylene P(O)HOH, -OC₁₋₄ alkylene OP(O)HOH, -OC₁₋₄ alkylene NR^{C1}P(O)HOH, -C(O)NR^{C1}C₁₋₆ alkyl, - NR^{C1}C(O)C₁₋₆ alkyl, -NR^{C1}C(O)OC₁₋₆ alkyl, -NR^{C1}C(O)NR^{C1}C₁₋₆ alkyl, -C(O)OC₁₋₆ alkyl, -OC(O)C₁₋₆ alkyl, -OC(O)OC₁₋₆ alkyl, and -OC(O)NR^{C1}C₁₋₆ alkyl;
R⁴ is selected from the group consisting of H, deuterium, halogen, -CN, C₁₋₆ alkyl, C₁₋₆ alkoxy, C₁₋₆ haloalkyl, and C₁₋₆ haloalkoxy;
each R^{x}, L¹, R¹, R^{2a}, R^{2a1}, R^{2b}, R^{B}, R^{A}, R^{A1}, R^{z}, R³, R^{3a}, R^{3b}, R^{C}, R^{C1}, R^{C2}, R^{C3}, or R⁴ is independently optionally substituted with one or more substituents;
provided that the compound of formula (I) comprises at least one R^{C}.

2. The compound of formula (I) or the pharmaceutically acceptable salt thereof according to claim 1, wherein X is selected from the group consisting of -N-, -CH-, -C(C₁₋₆ alkyl)-, and -C(C₁₋₆ haloalkyl)-;
or X is selected from the group consisting of -N-, -CH-, -C(C₁₋₃ alkyl)-, and -C(C₁₋₃ haloalkyl)-;
or X is selected from -N-.

3. The compound of formula (I) or the pharmaceutically acceptable salt thereof according to claim 1 or 2, wherein L¹ is selected from the group consisting of -O-, -S-, and the following groups optionally substituted with one or more R¹: -NH-, C₂₋₄ alkylene, C₁₋₃ heteroalkylene, C₂₋₄ alkenylene, and C₁₋₃ heteroalkenylene;
or L¹ is selected from the group consisting of -O- and the following groups optionally substituted with one or more R¹: -NH-, C₂₋₄ alkylene, and C₁₋₃ heteroalkylene;
or L¹ is selected from the group consisting of -O- and the following groups optionally substituted with one or more R¹: -NH-, -CH₂CH₂-, -(CH₂)₃-, -(CH₂)₄-, -OCH₂-, -CH₂O-, -OCH₂CH₂-, -CH₂OCH₂-, -CH₂CH₂O-, - O(CH₂)₃-, -CH₂OCH₂CH₂-, -CH₂CH₂OCH₂-, -CH₂CH₂CH₂O-, -NHCH₂-, -NHCH₂CH₂-, -CH₂NHCH₂-, - CH₂CH₂NH-, -NH(CH₂)₃-, -CH₂NHCH₂CH₂-, -CH₂CH₂NHCH₂-, and -CH₂CH₂CH₂NH-;
or L¹ is selected from the group consisting of the following groups optionally substituted with one or more R¹: -CH₂CH₂-, -(CH₂)₃-, -(CH₂)₄-, -OCH₂-, -OCH₂CH₂-, -O(CH₂)₃-, -NHCH₂-, -NHCH₂CH₂-, and -NH(CH₂)₃-;
or L¹ is selected from the group consisting of the following groups optionally substituted with one or more R¹: -(CH₂)₃-, -OCH₂CH₂-, and -NHCH₂CH₂-;
or L¹ is selected from -OCH₂CH₂- optionally substituted with one or more R¹.

4. The compound of formula (I) or the pharmaceutically acceptable salt thereof according to any one of claims 1-3, wherein each R¹ is independently selected from the group consisting of deuterium, oxo, halogen, -OH, - NH₂, -CN, C₁₋₄ alkyl, C₁₋₄ alkoxy, C₁₋₄ alkylthio, C₁₋₄ alkylamino, di-C₁₋₄ alkylamino, C₁₋₄ haloalkyl, C₁₋₄ haloalkoxy, C₁₋₄ haloalkylthio, C₁₋₄ haloalkylamino, and di-C₁₋₄ haloalkylamino;
or each R¹ is independently selected from the group consisting of deuterium, oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino;
or each R¹ is independently selected from the group consisting of deuterium, oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy;
or each R¹ is independently selected from the group consisting of deuterium, oxo, -F, -Cl, -Br, -I, -OH, -NH₂, -CN, C₁₋₃ alkyl, C₁₋₃ alkoxy, C₁₋₃ haloalkyl, and C₁₋₃ haloalkoxy;
or each R¹ is independently selected from the group consisting of deuterium, oxo, -F, -Cl, -Br, -OH, -NH₂, - CN, methyl, ethyl, isopropyl, methoxy, ethoxy, isopropoxy, -CH₂F, trifluoromethyl, and trifluoromethoxy;
or each R¹ is independently selected from the group consisting of C₁₋₃ alkyl and C₁₋₃ fluoroalkyl;
or each R¹ is independently selected from the group consisting of methyl, ethyl, isopropyl, trifluoromethyl, and -CH₂F.

5. The compound of formula (I) or the pharmaceutically acceptable salt thereof according to any one of claims 1-4, wherein L² is selected from a single bond, and ring B is selected from the group consisting of the following groups optionally substituted with one or more R^{B} or R^{C}: 3- to 10-membered cycloalkenyl, 3- to 10-membered heterocycloalkenyl, 6- to 10-membered aryl, and 5- to 10-membered heteroaryl;
or L² is selected from -C(R^{2a}R^{2b})-, and ring B is selected from the group consisting of the following groups optionally substituted with one or more R^{B} or R^{C}: 3- to 10-membered cycloalkyl, 3- to 10-membered heterocyclyl, 6- to 10-membered aryl, and 5- to 10-membered heteroaryl;
or L² is selected from a single bond, and ring B is selected from 5- to 10-membered heterocycloalkenyl optionally substituted with one or more R^{B} or R^{C};
or L² is selected from -C(R^{2a}R^{2b})-, and ring B is selected from the group consisting of the following groups optionally substituted with one or more R^{B} or R^{C}: 3- to 6-membered cycloalkyl, 4- to 7-membered heterocycloalkyl, phenyl, and 5- to 6-membered heteroaryl;
or L² is selected from a single bond, and ring B is selected from the group consisting of the following groups optionally substituted with one or more R^{B} or R^{C}: indolinyl, cyclopentenopyridinyl, dihydropyrrolopyridinyl, tetrahydroquinolinyl, tetrahydroisoquinolinyl, and tetrahydronaphthyridinyl;
or L² is selected from -C(R^{2a}R^{2b})-, and ring B is selected from the group consisting of the following groups optionally substituted with one or more R^{B} or R^{C}: cyclopropanyl, cyclobutanyl, cyclopentanyl, cyclohexanyl, oxetanyl, azetidinyl, tetrahydrofuranyl, pyrrolidinyl, piperidinyl, piperazinyl, morpholinyl, phenyl, furanyl, pyrrolyl, pyrazolyl, imidazolyl, oxazolyl, isoxazolyl, thienyl, thiazolyl, isothiazolyl, pyranyl, pyridinyl, pyridazinyl, pyrimidinyl, and pyrazinyl;
or L² is selected from a single bond, and ring B is selected from the group consisting of the following groups optionally substituted with one or more R^{B} or R^{C}: cyclopentenylpyridinyl, tetrahydroquinolinyl, and tetrahydroisoquinolinyl;
or L² is selected from -C(R^{2a}R^{2b})-, and ring B is selected from the group consisting of the following groups optionally substituted with one or more R^{B} or R^{C}: cyclopropanyl, cyclobutanyl, cyclopentanyl, oxetanyl, azetidinyl, pyrrolidinyl, piperidinyl, pyrazolyl, phenyl, pyridinyl, pyridazinyl, pyrimidinyl, and pyrazinyl;
or L² is selected from a single bond, and ring B is selected from the group consisting of the following groups optionally substituted with one or more R^{B} or R^{C}: 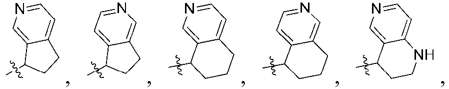 and 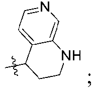
or L² is selected from -C(R^{2a}R^{2b})-, and ring B is selected from the group consisting of the following groups optionally substituted with one or more R^{B} or R^{C}: 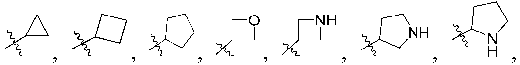 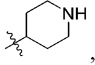 phenyl, 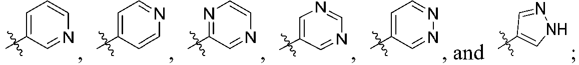
or L² is selected from a single bond, and ring B is selected from the group consisting of 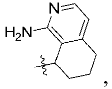 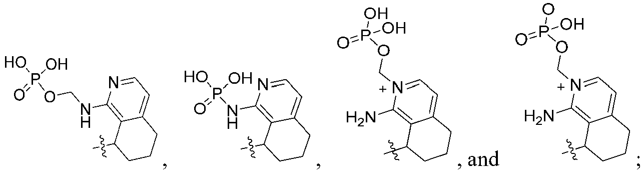
or L² is selected from -C(R^{2a}R^{2b})-, and ring B is selected from the group consisting of cyclopropanyl, 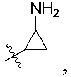 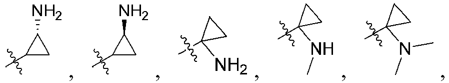 cyclobutanyl, 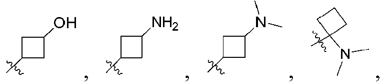 cyclopentanyl,

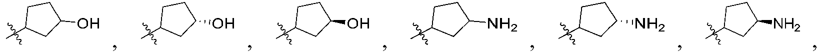

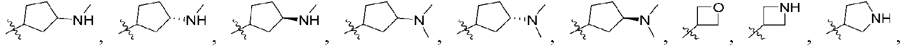

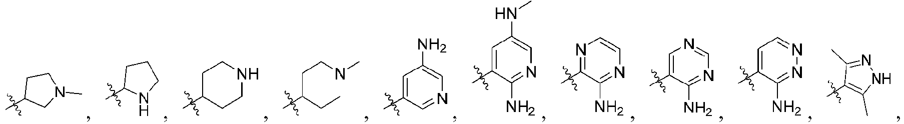

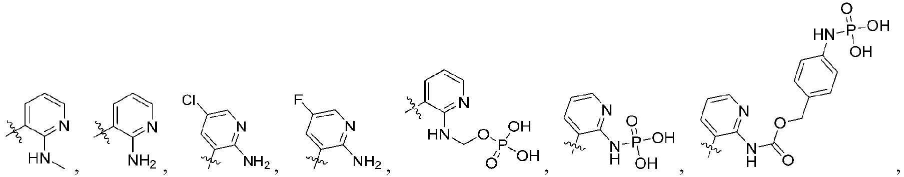

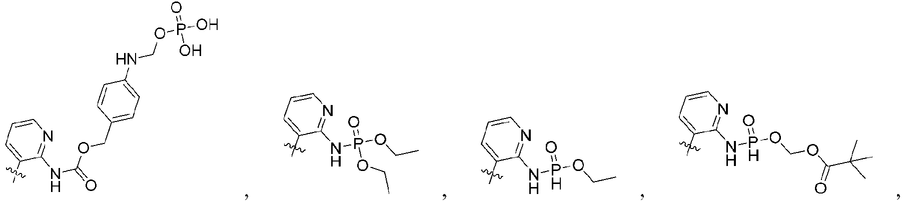

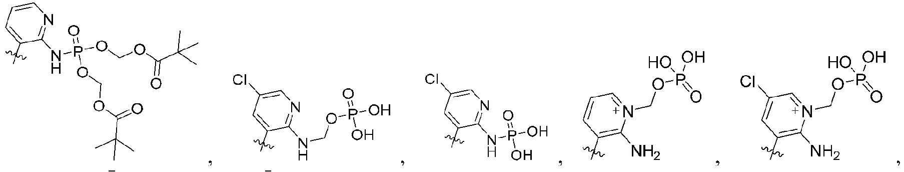

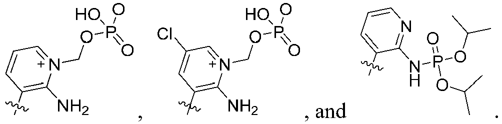

6. The compound of formula (IA) or the pharmaceutically acceptable salt thereof according to any one of claims 1-5, wherein ring A is selected from the group consisting of the following groups optionally substituted with one or more R^{A} or R^{C}: 3- to 6-membered cycloalkyl, 5- to 10-membered cycloalkenyl, 5- to 10-membered heterocyclyl, 6- to 10-membered aryl, and 5- to 10-membered heteroaryl;
or ring A is selected from the group consisting of the following groups optionally substituted with one or more R^{A} or R^{C}: benzo 4- to 6-membered cycloalkenyl, benzo 4- to 6-membered heterocyclyl, 6- to 10-membered aryl, and 5- to 10-membered heteroaryl;
or ring A is selected from the group consisting of the following groups optionally substituted with one or more R^{A} or R^{C}: phenyl, naphthyl, benzocyclohexenyl, benzocyclopentenyl, pyrrolyl, pyrazolyl, imidazolyl, furanyl, oxazolyl, isoxazolyl, thienyl, thiazolyl, isothiazolyl, pyranyl, pyridinyl, pyridazinyl, pyrimidinyl, pyrazinyl, indolyl, benzopyrazolyl, benzimidazolyl, benzothiazolyl, quinolyl, isoquinolyl, benzopyrimidinyl, benzothienyl, pyridopyrazolyl, and pyridopyrrolyl;
or ring A is selected from the group consisting of the following groups optionally substituted with one or more R^{A} or R^{C}: phenyl, naphthyl, pyrazolyl, pyridinyl, indolyl, benzopyrazolyl, benzimidazolyl, benzothiazolyl, quinolyl, isoquinolyl, benzothienyl, pyridopyrazolyl, and pyridopyrrolyl;
or ring A is selected from the group consisting of the following groups optionally substituted with one or more R^{A} or R^{C}: 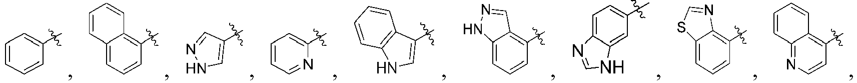 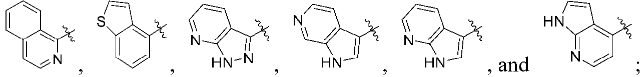
or ring A is selected from the group consisting of

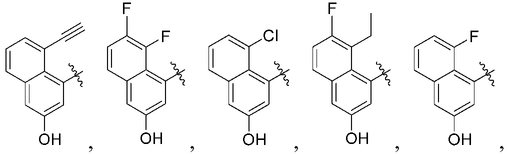

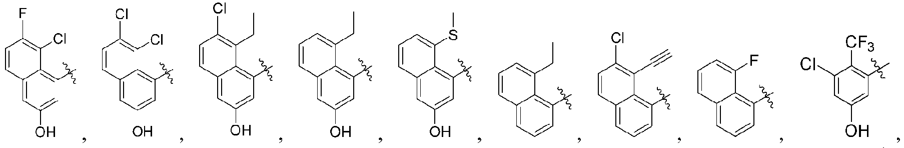

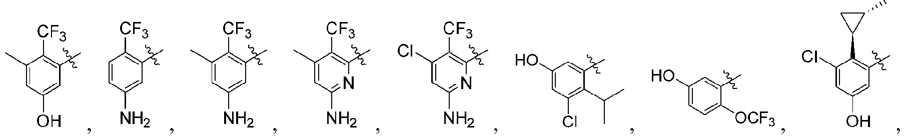

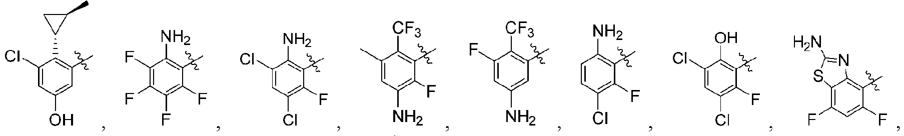

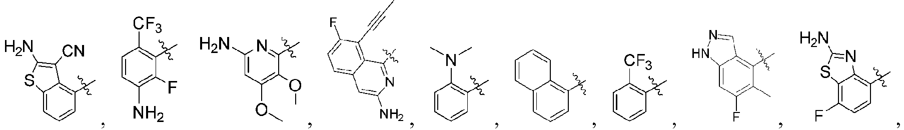

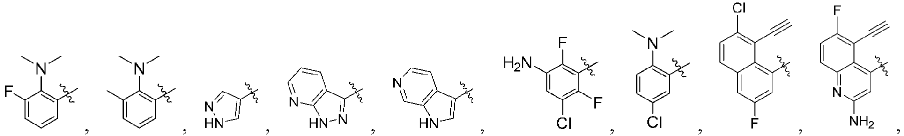

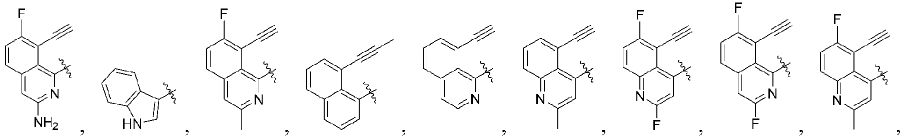

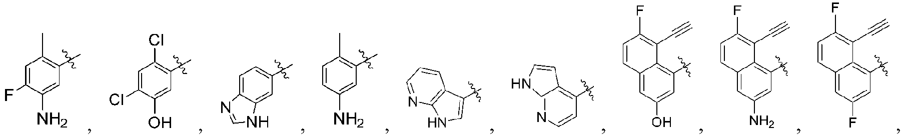

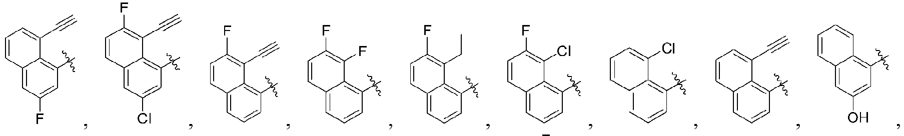

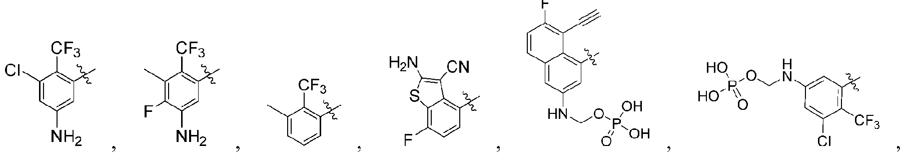

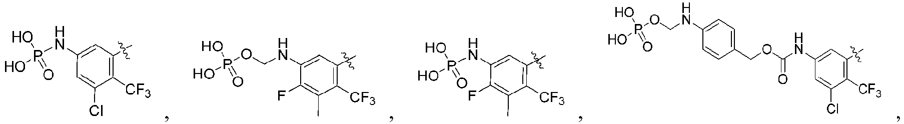

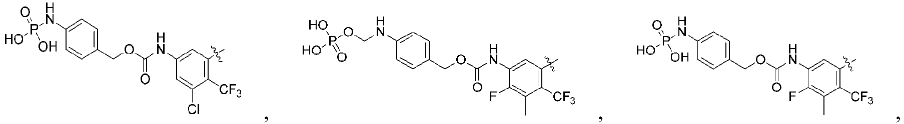

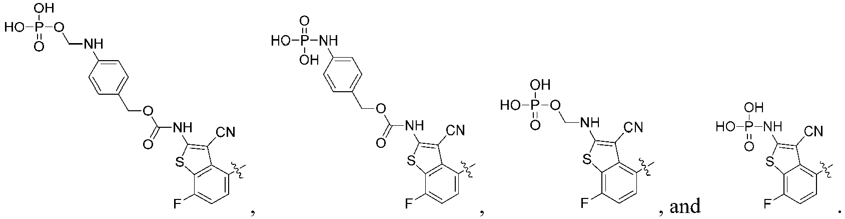

7. The compound of formula (I) or the pharmaceutically acceptable salt thereof according to any one of claims 1-6, wherein Z is selected from the group consisting of a single bond, -S-, -O-, and the following groups optionally substituted with one or more R^{z}: -NH- and -N(C₁₋₆ alkyl)-;
or Z is selected from the group consisting of a single bond, -S-, -O-, -NH-, and -N(C₁₋₄ alkyl)-;
or Z is selected from the group consisting of a single bond, -S-, -O-, -NH-, and -N(C₁₋₃ alkyl)-;
or Z is selected from the group consisting of a single bond, -O-, -NH-, and -N(CH₃)-;
or Z is selected from -O-.

8. The compound of formula (I) or the pharmaceutically acceptable salt thereof according to any one of claims 1-7, wherein R³ is selected from the group consisting of H, deuterium, and the following groups optionally substituted with one or more R^{3a} or R^{C}: C₁₋₆ alkyl, 3- to 10-membered cycloalkyl, 3- to 10-membered heterocyclyl, 6- to 10-membered aryl, 5- to 10-membered heteroaryl, 3- to 10-membered cycloalkyl C₁₋₄ alkylene, 3- to 10-membered heterocyclyl C₁₋₄ alkylene, 6- to 10-membered aryl C₁₋₄ alkylene, and 5- to 10-membered heteroaryl C₁₋₄ alkylene;
or R³ is selected from the group consisting of H, deuterium, and the following groups optionally substituted with one or more R^{3a} or R^{C}: C₁₋₄ alkyl, 3- to 10-membered cycloalkyl, 4- to 10-membered heterocyclyl, phenyl, 5- to 6-membered heteroaryl, 3- to 10-membered cycloalkyl C₁₋₃ alkylene, 4- to 10-membered heterocyclyl C₁₋₃ alkylene, phenyl C₁₋₃ alkylene, and 5- to 6-membered heteroaryl C₁₋₃ alkylene;
or R³ is selected from the group consisting of H, deuterium, and the following groups optionally substituted with one or more R^{3a} or R^{C}: C₁₋₄ alkyl, 3- to 8-membered cycloalkyl, 4- to 10-membered heterocyclyl, phenyl, 5- to 6-membered heteroaryl, 3- to 8-membered cycloalkyl C₁₋₃ alkylene, 4- to 10-membered heterocyclyl C₁₋₃ alkylene, phenyl C₁₋₃ alkylene, and 5- to 6-membered heteroaryl C₁₋₃ alkylene;
or R³ is selected from the group consisting of H, deuterium, and the following groups optionally substituted with one or more R^{3a} or R^{C}: C₁₋₄ alkyl, 3- to 8-membered cycloalkyl, 4- to 10-membered heterocyclyl, 5- to 6-membered heteroaryl, 3- to 8-membered cycloalkyl C₁₋₃ alkylene, 4- to 10-membered heterocyclyl C₁₋₃ alkylene, and 5- to 6-membered heteroaryl C₁₋₃ alkylene;
or R³ is selected from the group consisting of H, deuterium, and the following groups optionally substituted with one or more R^{3a} or R^{C}: C₁₋₄ alkyl, 4- to 10-membered heterocyclyl, 3- to 8-membered cycloalkyl C₁₋₃ alkylene, 4- to 10-membered heterocyclyl C₁₋₃ alkylene, and 5- to 6-membered heteroaryl C₁₋₃ alkylene;
or R³ is selected from the group consisting of H, deuterium, and the following groups optionally substituted with one or more R^{3a} or R^{C}: C₁₋₄ alkyl, 4- to 10-membered heterocycloalkyl, 3- to 8-membered cycloalkyl C₁₋₃ alkylene, 4- to 10-membered heterocycloalkyl C₁₋₃ alkylene, and 5- to 6-membered heteroaryl C₁₋₃ alkylene;
or R³ is selected from the group consisting of H, deuterium, and the following groups optionally substituted with one or more R^{3a} or R^{C}: methyl, ethyl, n-propyl, isopropyl, n-butyl, isobutyl, sec-butyl, tert-butyl, cyclopropyl, cyclobutyl, cyclopentyl, cyclohexyl, azetidinyl, tetrahydropyrrolyl, morpholinyl, piperidinyl, piperazinyl, hexahydro-1H-pyrrolizinyl, 5-azaspiro[2.4]heptanyl, tetrahydro-1'H,3'H-pyrrolo[cyclopropane-1,2'-pyrrolidine], cyclopropyl C₁₋₃ alkylene, cyclobutyl C₁₋₃ alkylene, cyclopentyl C₁₋₃ alkylene, cyclohexyl C₁₋₃ alkylene, azetidinyl C₁₋₃ alkylene, tetrahydropyrrolyl C₁₋₃ alkylene, morpholinyl C₁₋₃ alkylene, piperidinyl C₁₋₃ alkylene, piperazinyl C₁₋₃ alkylene, hexahydro-1H-pyrrolizinyl C₁₋₃ alkylene, hexahydro-1H-pyrrolo[2,1-c][1,4]oxazinyl, hexahydro-1H-pyrrolo[2,1-c][1,4]oxazinyl C₁₋₃ alkylene, 5-azaspiro[2.4]heptanyl C₁₋₃ alkylene, tetrahydro-1'H,3'H-pyrrolo[cyclopropane-1,2'-pyrrolidine]C₁₋₃ alkylene, and imidazolyl C₁₋₃ alkylene;
or R³ is selected from the group consisting of H, deuterium, and the following groups optionally substituted with one or more R^{3a} or R^{C}: methyl, ethyl, n-propyl, isopropyl, n-butyl, isobutyl, sec-butyl, tert-butyl, cyclobutyl, cyclohexyl, azetidinyl, tetrahydropyrrolyl, piperidinyl, piperazinyl, cyclopropyl C₁₋₃ alkylene, cyclobutyl C₁₋₃ alkylene, cyclopentyl C₁₋₃ alkylene, azetidinyl C₁₋₃ alkylene, tetrahydropyrrolyl C₁₋₃ alkylene, morpholinyl C₁₋₃ alkylene, piperidinyl C₁₋₃ alkylene, piperazinyl C₁₋₃ alkylene, hexahydro-1H-pyrrolizinyl C₁₋₃ alkylene, hexahydro-1H-pyrrolo[2,1-c][1,4]oxazinyl C₁₋₃ alkylene, 5-azaspiro[2.4]heptanyl C₁₋₃ alkylene, tetrahydro-1'H,3'H-pyrrolo[cyclopropane-1,2'-pyrrolidine]C₁₋₃ alkylene, and imidazolyl C₁₋₃ alkylene;
or R³ is selected from the group consisting of H, deuterium, and the following groups optionally substituted with one or more R^{3a} or R^{C}: methyl, ethyl, n-propyl, isopropyl, n-butyl, isobutyl, sec-butyl, 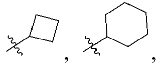 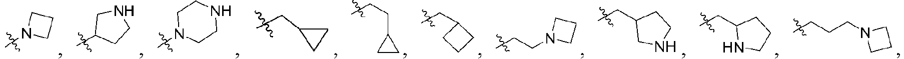 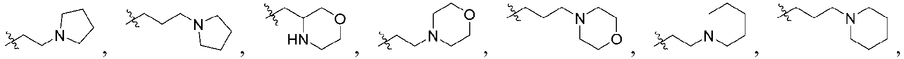 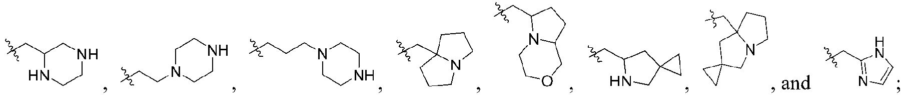
or R³ is selected from the group consisting of H, deuterium, methyl,

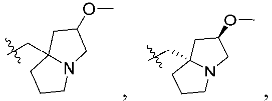

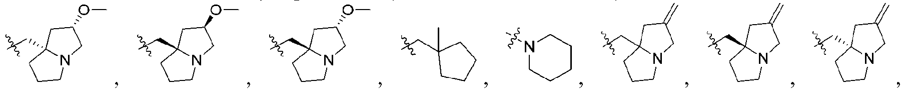

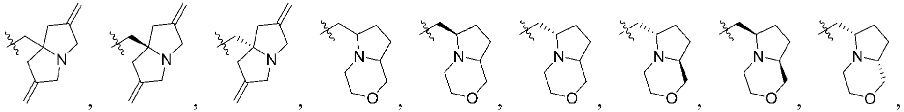

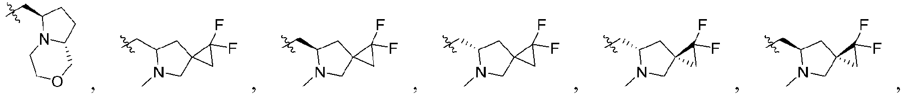

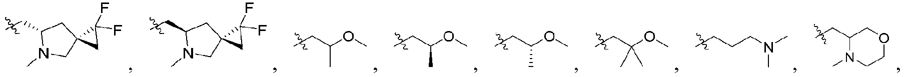

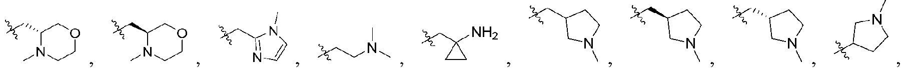

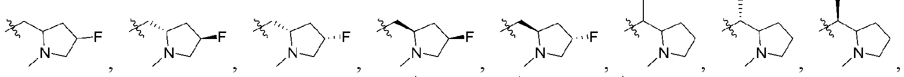

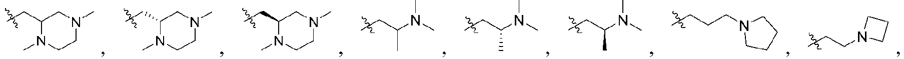

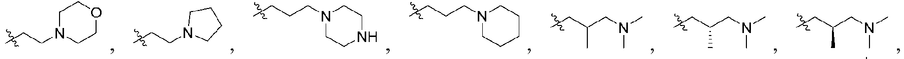

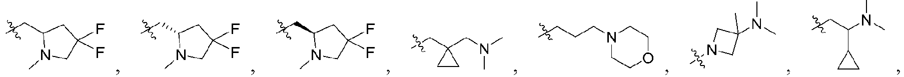

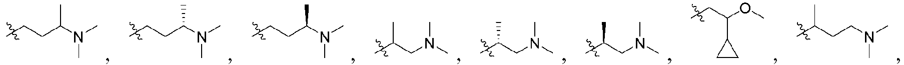

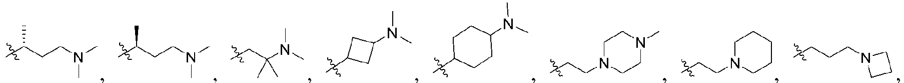

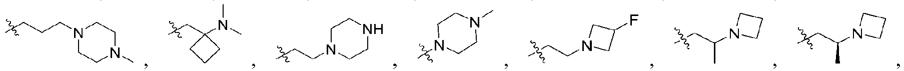

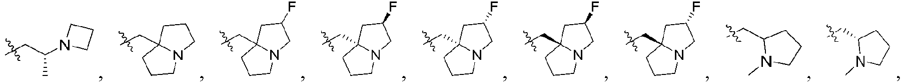

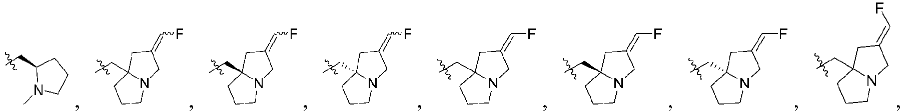

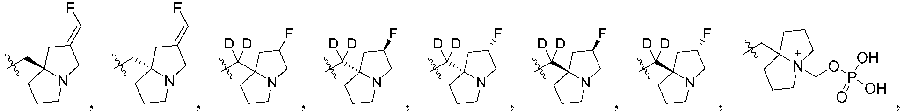

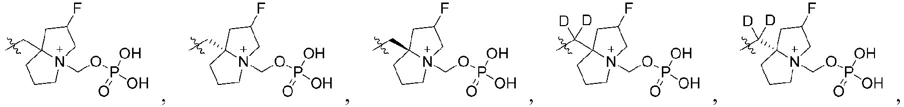

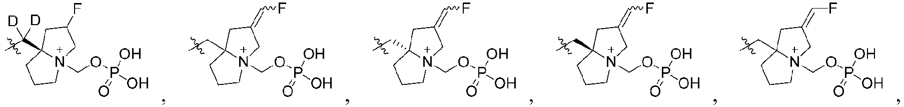

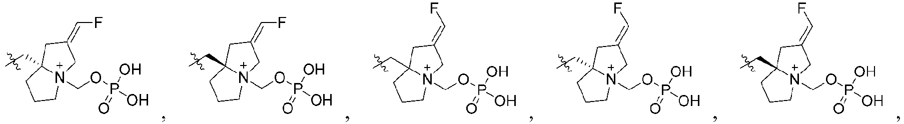

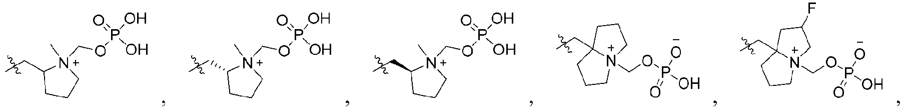

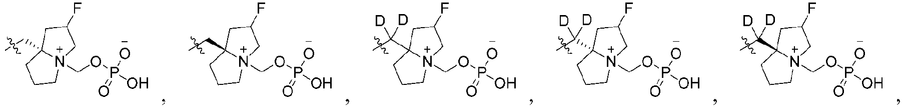

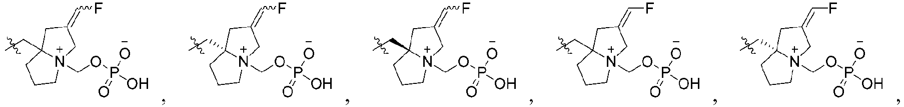

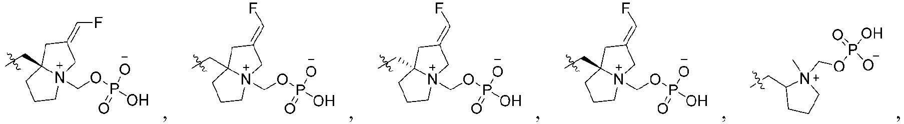

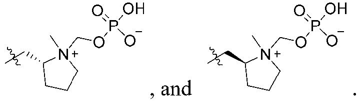

9. The compound of formula (I) or the pharmaceutically acceptable salt thereof according to any one of claims 1-8, wherein each R^{C} is independently selected from the group consisting of -NR^{C1}C(O)R^{C2}, -NR^{C1}C(O)OR^{C2}, -NR^{C1}C(O)OC₁₋₄ alkylene OC(O)R^{C2}, -NR^{C1}C(O)OC₁₋₄ alkylene NR^{C1}C(O)R^{C2}, -NR^{C1}C(O)NR^{C1}R^{C2}, - NR^{C1}C(O)NR^{C1}C₁₋₄ alkylene NR^{C1}C(O)R^{C2}, -NR^{C1}C(O)NR^{C1}C₁₋₄ alkylene OC(O)R^{C2}, -OC(O)R^{C2}, - OC(O)OR^{C2}, -OC(O)OC₁₋₄ alkylene OC(O)R^{C2}, -OC(O)OC₁₋₄ alkylene NR^{C1}C(O)R^{C2}, -OC(O)NR^{C1}R^{C2}, - OC(O)NR^{C1}C₁₋₄ alkylene OC(O)R^{C2}, -OC(O)NR^{C1}C₁₋₄ alkylene NR^{C1}C(O)R^{C2}, -P(O)(OR^{C2})₂, -C₁₋₄ alkylene P(O)(OR^{C2})₂, -C₁₋₄ alkylene NR^{C1}P(O)(OR^{C2})₂, -C₁₋₄ alkylene OP(O)(OR^{C2})₂, -NR^{C1}P(O)(OR^{C2})₂, -NR^{C1}C₁₋₄ alkylene P(O)(OR^{C2})₂, -NR^{C1}C₁₋₄ alkylene OP(O)(OR^{C2})₂, -NR^{C1}C₁₋₄ alkylene NR^{C1}P(O)(OR^{C2})₂, - OP(O)(OR^{C2})₂, -OC₁₋₄ alkylene P(O)(OR^{C2})₂, -OC₁₋₄ alkylene OP(O)(OR^{C2})₂, -OC₁₋₄ alkylene NR^{C1}P(O)(OR^{C2})₂, -P(O)HOR^{C2}, -C₁₋₄ alkylene P(O)HOR^{C2}, -C₁₋₄ alkylene NR^{C1} P(O)HOR^{C2}, -C₁₋₄ alkylene OP(O)HOR^{C2}, -NR^{C1}P(O)HOR^{C2}, -NR^{C1}C₁₋₄ alkylene P(O)HOR^{C2}, -NR^{C1}C₁₋₄ alkylene OP(O)HOR^{C2} - NR^{C1}C₁₋₄ alkylene NR^{C1}P(O)HOR^{C2}, -OP(O)HOR^{C2}, -OC₁₋₄ alkylene P(O)HOR^{C2}, -OC₁₋₄ alkylene OP(O)HOR^{C2}, and -OC₁₋₄ alkylene NR^{C1}P(O)HOR^{C2};
or each R^{C} is independently selected from the group consisting of -NR^{C1}C(O)R^{C2}, -NR^{C1}C(O)OR^{C2} - NR^{C1}C(O)OC₁₋₃ alkylene OC(O)R^{C2}, -NR^{C1}C(O)OC₁₋₃ alkylene NR^{C1}C(O)R^{C2}, -NR^{C1}C(O)NR^{C1}R^{C2}, - NR^{C1}C(O)NR^{C1}C₁₋₃ alkylene NR^{C1}C(O)R^{C2}, -NR^{C1}C(O)NR^{C1}C₁₋₃ alkylene OC(O)R^{C2}, -OC(O)R^{C2}, - OC(O)OR^{C2}, -OC(O)OC₁₋₃ alkylene OC(O)R^{C2}, -OC(O)OC₁₋₃ alkylene NR^{C1}C(O)R^{C2}, -OC(O)NR^{C1}R^{C2}, - OC(O)NR^{C1}C₁₋₃ alkylene OC(O)R^{C2}, -OC(O)NR^{C1}C₁₋₃ alkylene NR^{C1}C(O)R^{C2}, -P(O)(OR^{C2})₂, -C₁₋₃ alkylene P(O)(OR^{C2})₂, -C₁₋₃ alkylene NR^{C1}P(O)(OR^{C2})₂, -C₁₋₃ alkylene OP(O)(OR^{C2})₂, -NR^{C1}P(O)(OR^{C2})₂, -NR^{C1}C₁₋₃ alkylene P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, - OP(O)(OR^{C2})₂, -OC₁₋₃ alkylene P(O)(OR^{C2})₂, -OC₁₋₃ alkylene OP(O)(OR^{C2})₂, -OC₁₋₃ alkylene NR^{C1}P(O)(OR^{C2})₂, -P(O)HOR^{C2}, -C₁₋₃ alkylene P(O)HOR^{C2}, -C₁₋₃ alkylene NR^{C1}P(O)HOR^{C2}, -C₁₋₃ alkylene OP(O)HOR^{C2}, -NR^{C1}P(O)HOR^{C2}, -NR^{C1}C₁₋₃ alkylene P(O)HOR^{C2}, -NR^{C1}C₁₋₃ alkylene OP(O)HOR^{C2} - NR^{C1}C₁₋₃ alkylene NR^{C1}P(O)HOR^{C2}, -OP(O)HOR^{C2}, -OC₁₋₃ alkylene P(O)HOR^{C2}, -OC₁₋₃ alkylene OP(O)HOR^{C2}, and -OC₁₋₃ alkylene NR^{C1}P(O)HOR^{C2};
or each R^{C} is independently selected from the group consisting of -NR^{C1}C(O)R^{C2}, -NR^{C1}C(O)OR^{C2} - NR^{C1}C(O)OC₁₋₃ alkylene OC(O)R^{C2}, -NR^{C1}C(O)OC₁₋₃ alkylene NR^{C1}C(O)R^{C2}, -OC(O)R^{C2}, -OC(O)OR^{C2}, - OC(O)OC₁₋₃ alkylene OC(O)R^{C2}, -OC(O)OC₁₋₃ alkylene NR^{C1}C(O)R^{C2}, -OC(O)NR^{C1}R^{C2}, -OC(O)NR^{C1}C₁₋₃ alkylene OC(O)R^{C2}, -OC(O)NR^{C1}C₁₋₃ alkylene NR^{C1}C(O)R^{C2}, -C₁₋₃ alkylene NR^{C1}P(O)(OR^{C2})₂, -C₁₋₃ alkylene OP(O)(OR^{C2})₂, -NR^{C1}P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, -OP(O)(OR^{C2})₂, -OC₁₋₃ alkylene OP(O)(OR^{C2})₂, -OC₁₋₃ alkylene NR^{C1}P(O)(OR^{C2})₂, -C₁₋₃ alkylene NR^{C1}P(O)HOR^{C2}, -C₁₋₃ alkylene OP(O)HOR^{C2}, -NR^{C1}P(O)HOR^{C2}, -NR^{C1}C₁₋₃ alkylene OP(O)HOR^{C2}, - NR^{C1}C₁₋₃ alkylene NR^{C1}P(O)HOR^{C2}, -OP(O)HOR^{C2}, -OC₁₋₃ alkylene OP(O)HOR^{C2}, and -OC₁₋₃ alkylene NR^{C1}P(O)HOR^{C2};
or each R^{C} is independently selected from the group consisting of -NR^{C1}C(O)R^{C2}, -NR^{C1}C(O)OR^{C2}, - NR^{C1}C(O)OC₁₋₃ alkylene OC(O)R^{C2}, -OC(O)R^{C2}, -OC(O)OR^{C2}, -OC(O)OC₁₋₃ alkylene OC(O)R^{C2}, - OC(O)NR^{C1}R^{C2}, -OC(O)NR^{C1}C₁₋₃ alkylene OC(O)R^{C2}, -OC(O)NR^{C1}C₁₋₃ alkylene NR^{C1}C(O)R^{C2}, -C₁₋₃ alkylene NR^{C1}P(O)(OR^{C2})₂, -C₁₋₃ alkylene OP(O)(OR^{C2})₂, -NR^{C1}P(O)(OR^{C2})₂, -NR^{C1}C₁₋₃ alkylene OP(O)(OR^{C2})₂, -NR^{C1}C₁₋₃ alkylene NR^{C1}P(O)(OR^{C2})₂, -OP(O)(OR^{C2})₂, -OC₁₋₃ alkylene OP(O)(OR^{C2})₂, -OC₁₋₃ alkylene NR^{C1}P(O)(OR^{C2})₂, -C₁₋₃ alkylene NR^{C1}P(O)HOR^{C2}, -C₁₋₃ alkylene OP(O)HOR^{C2}, -NR^{C1}P(O)HOR^{C2}, -NR^{C1}C₁₋₃ alkylene OP(O)HOR^{C2}, -NR^{C1}C₁₋₃ alkylene NR^{C1}P(O)HOR^{C2}, -OP(O)HOR^{C2}, -OC₁₋₃ alkylene OP(O)HOR^{C2}, and -OC₁₋₃ alkylene NR^{C1}P(O)HOR^{C2};
or each R^{C} is independently selected from the group consisting of -NR^{C1}C(O)R^{C2}, -NR^{C1}C(O)OR^{C2}, - OC(O)R^{C2}, -OC(O)OR^{C2}, -OC(O)OC₁₋₃ alkylene OC(O)R^{C2}, -OC(O)NR^{C1}R^{C2}, -C₁₋₃ alkylene OP(O)(OR^{C2})₂, -NR^{C1}P(O)(OR^{C2})₂, -NR^{C1}C₁₋₃ alkylene OP(O)(OR^{C2})₂, -OP(O)(OR^{C2})₂, -OC₁₋₃ alkylene OP(O)(OR^{C2})₂, and - NR^{C1}P(O)HOR^{C2};
or each R^{C} is independently selected from the group consisting of -NR^{C1}C(O)OR^{C2}, -C₁₋₂ alkylene OP(O)(OR^{C2})₂, -NR^{C1}P(O)(OR^{C2})₂, -NR^{C1}C₁₋₂ alkylene OP(O)(OR^{C2})₂, and -NR^{C1}P(O)HOR^{C2};
or each R^{C} is independently selected from the group consisting of -NR^{C1}C(O)OR^{C2}, -C₁₋₂ alkylene OP(O)(OH)₂, -NR^{C1}P(O)(OH)₂, -NR^{C1}P(O)(OR^{C2})₂, -NR^{C1}C₁₋₂ alkylene OP(O)(OH)₂, and -NR^{C1}P(O)HOR^{C2}; or each R^{C} is independently selected from the group consisting of

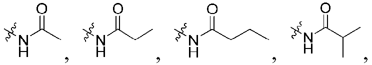

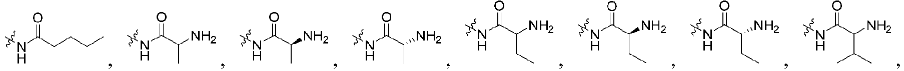

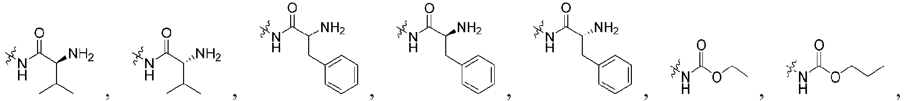

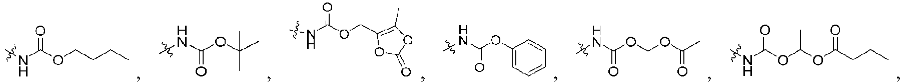

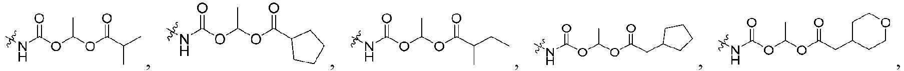

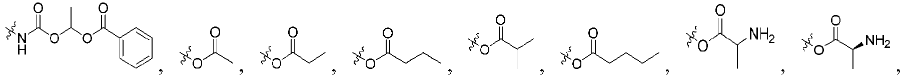

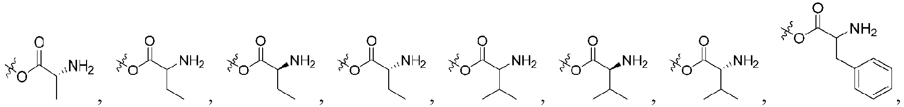

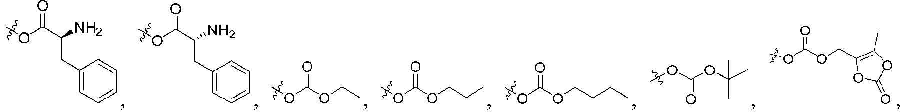

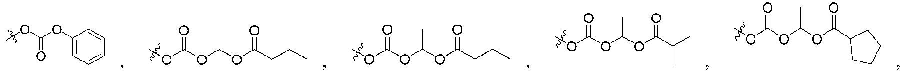

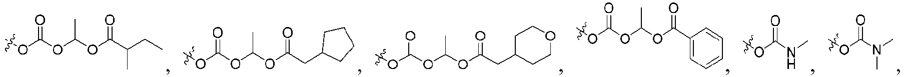

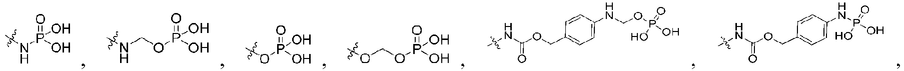

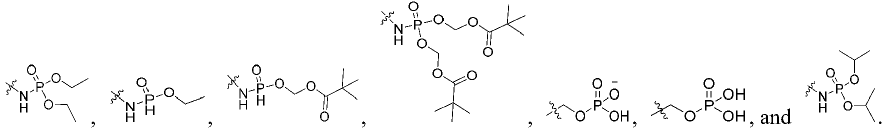

10. The compound of formula (I) or the pharmaceutically acceptable salt thereof according to any one of claims 1-9, wherein R⁴ is selected from the group consisting of H, deuterium, halogen, -CN, C₁₋₄ alkyl, C₁₋₄ alkoxy, C₁₋₄ haloalkyl, and C₁₋₄ haloalkoxy;
or R⁴ is selected from the group consisting of H, deuterium, halogen, -CN, C₁₋₃ alkyl, C₁₋₃ alkoxy, C₁₋₃ haloalkyl, and C₁₋₃ haloalkoxy;
or R⁴ is selected from the group consisting of H, deuterium, -F, -Cl, -Br, -I, -CN, methyl, methoxy, difluoromethyl, trifluoromethyl, and trifluoromethoxy;
or R⁴ is selected from the group consisting of H, deuterium, -F, and -Cl.

11. The compound of formula (I) or the pharmaceutically acceptable salt thereof according to any one of claims 1-10, being selected from the group consisting of compounds of formulas (II), (III), (III-1), (III-1a), (IV-1), (IV-2), and (IV-3) or pharmaceutically acceptable salts thereof, 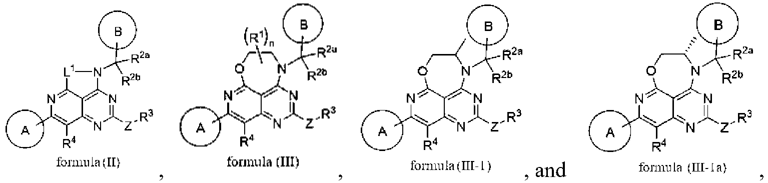 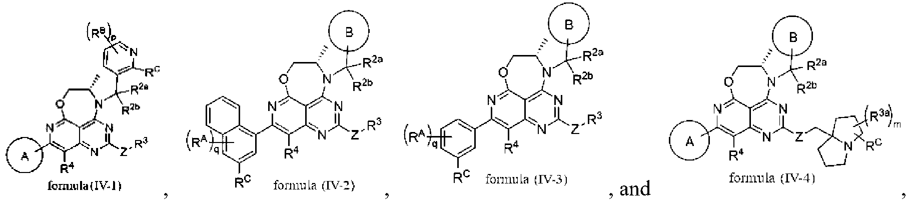
wherein the moieties L¹, R¹, R^{2a}, R^{2b}, R³, R⁴, R^{A}, R^{B}, R^{C}, Z, ring A, and ring B are as defined in claim 1;
n is selected from the group consisting of 0, 1, 2, 3, 4, and 5;
p is selected from the group consisting of 0, 1, 2, 3, 4, and 5;
q is selected from the group consisting of 0, 1, 2, 3, 4, and 5;
m is selected from the group consisting of 0, 1, 2, 3, 4, and 5.

12. The compound of formula (I) or the pharmaceutically acceptable salt thereof according to any one of claims 1-10, being selected from the group consisting of compounds of formulas (A), (A-1), (B), (B-1), (C), (D), (E), (E-1), (F), (F-1), (G), and (H) or pharmaceutically acceptable salts thereof, 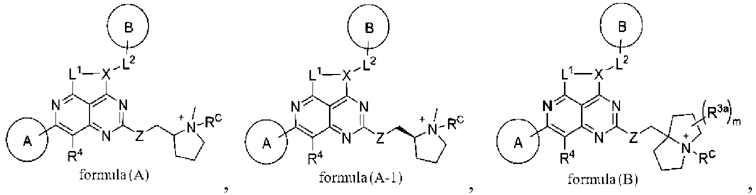 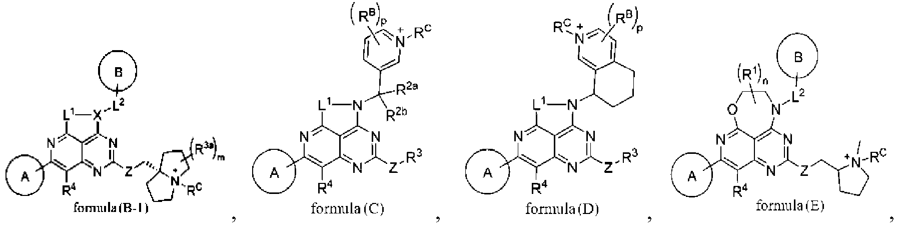 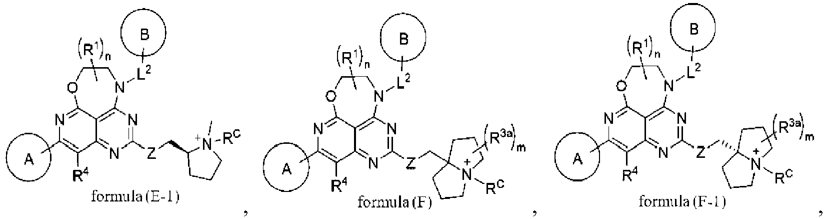 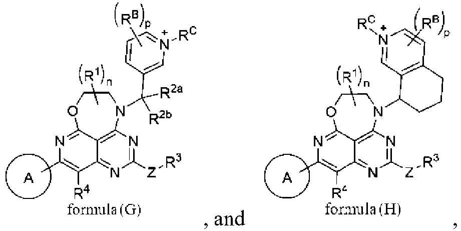
wherein the moieties L¹, L², X, R^{3a}, R^{2a}, R^{2b}, R³, R⁴, R^{B}, R^{C}, R¹, Z, ring A, and ring B are as defined in claim 1;
n is selected from the group consisting of 0, 1, 2, 3, 4, and 5;
m is selected from the group consisting of 0, 1, 2, 3, 4, and 5;
p is selected from the group consisting of 0, 1, 2, 3, 4, and 5.

13. The compound of formula (I) or the pharmaceutically acceptable salt thereof according to any one of claims 1-12, being selected from the group consisting of the following compounds or pharmaceutically acceptable salts thereof: 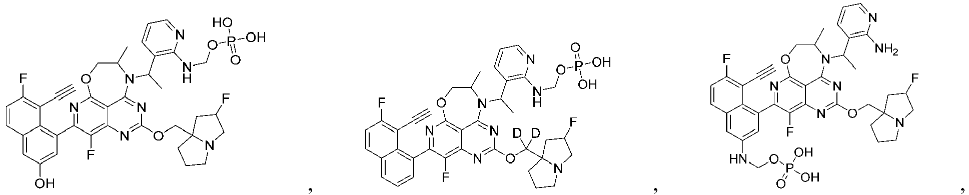 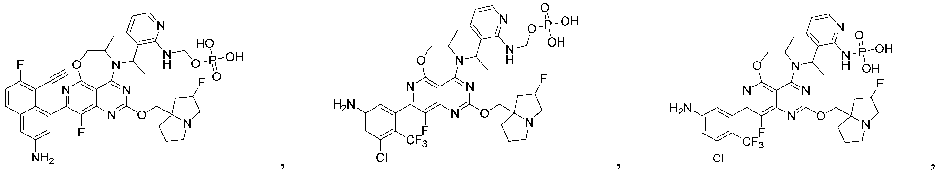 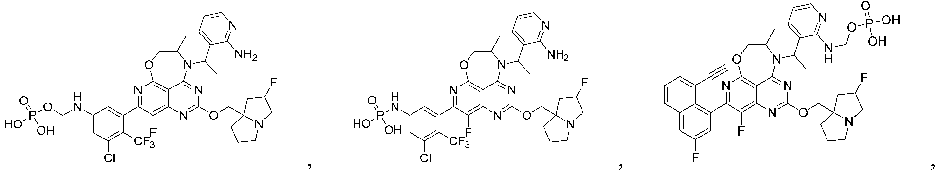 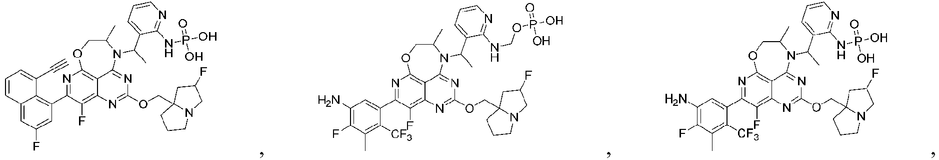 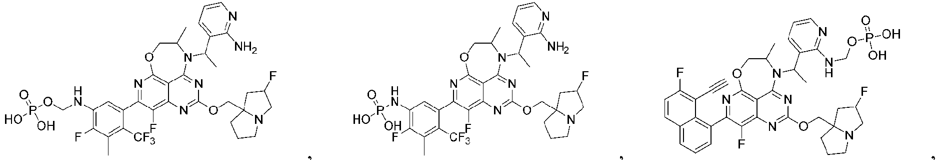 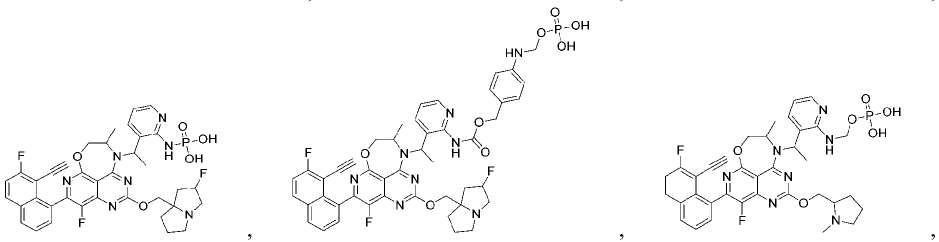 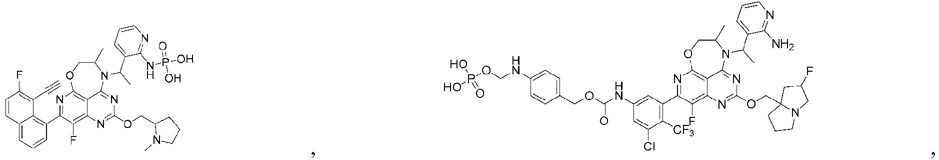 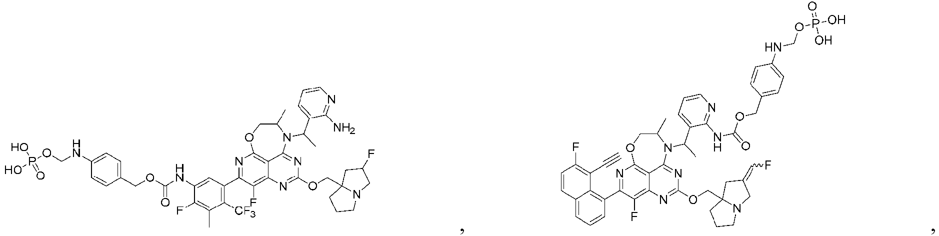 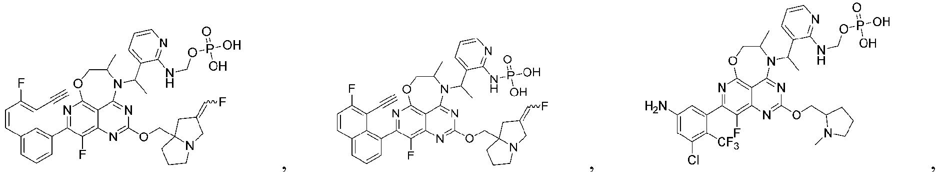 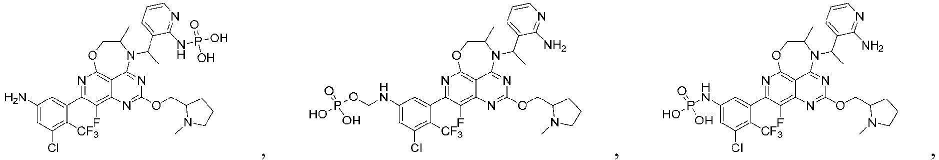 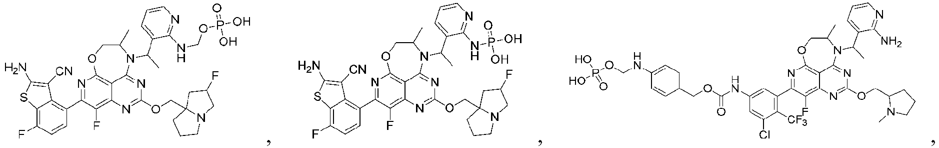 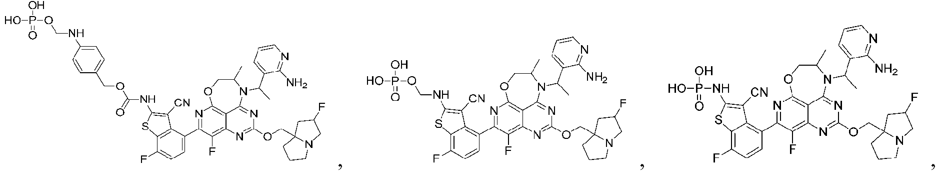 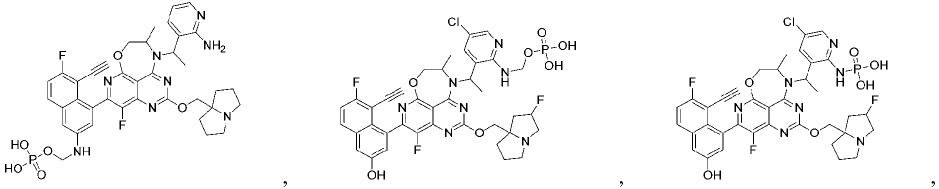 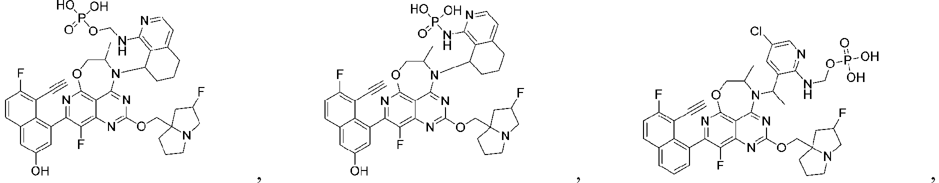 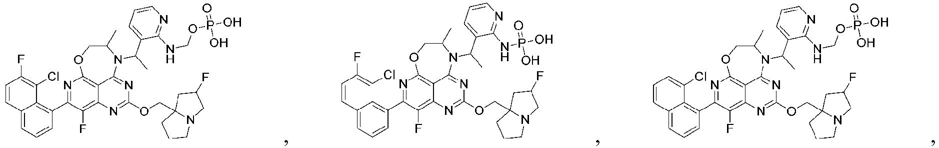 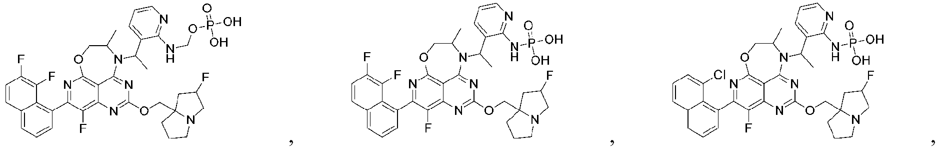 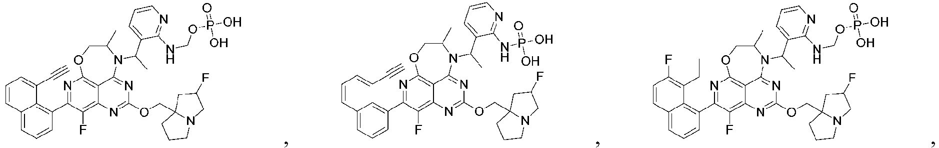 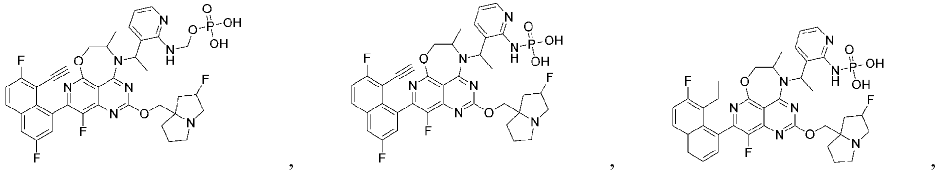 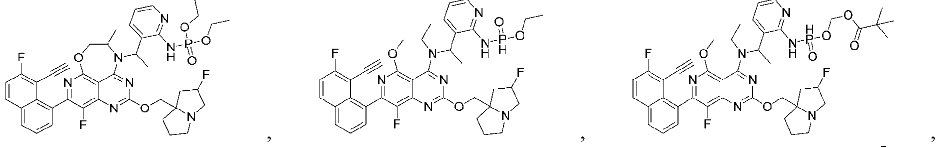 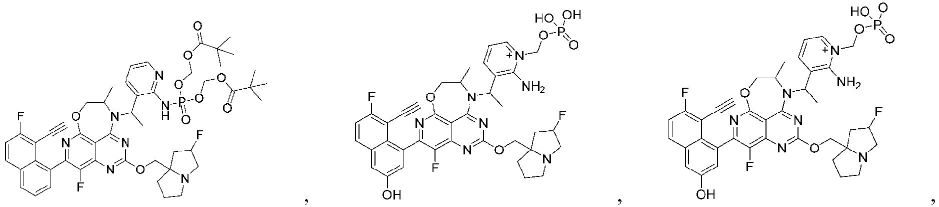 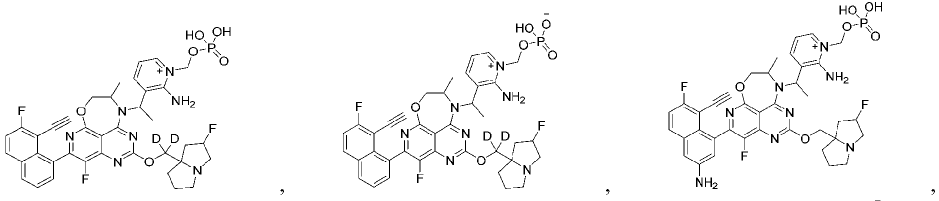 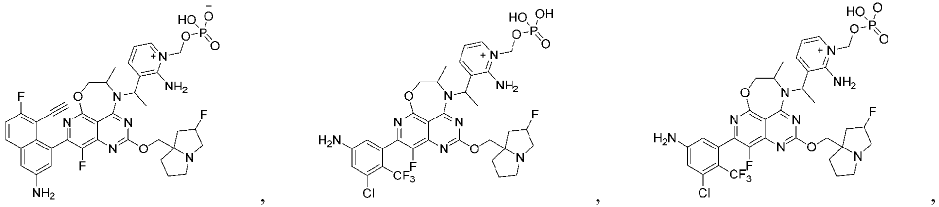 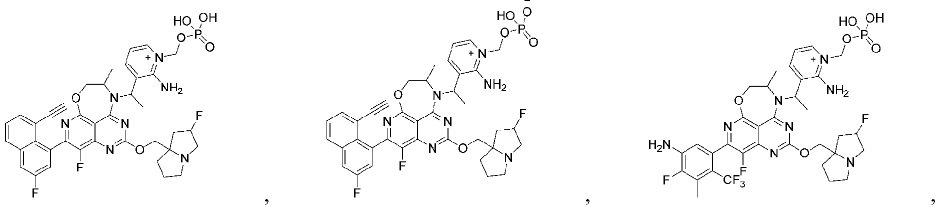 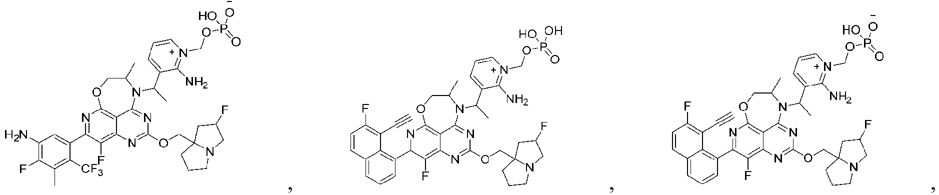 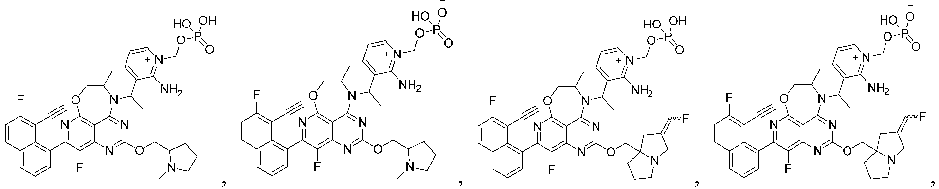 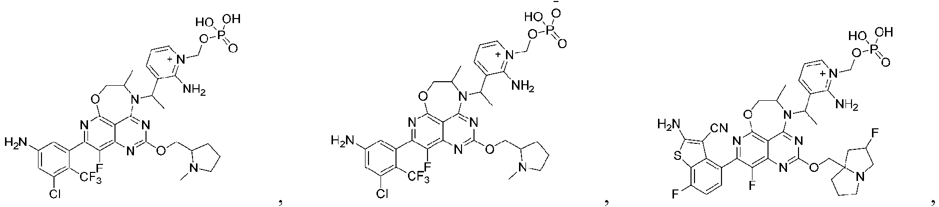 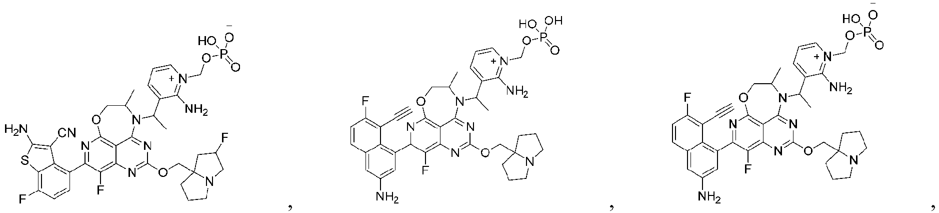 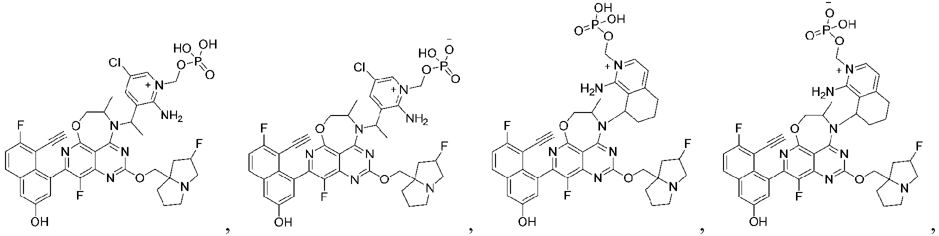 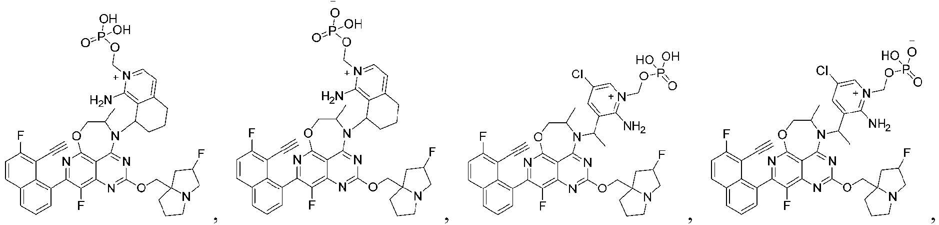 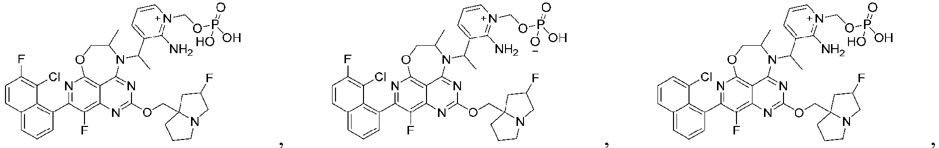 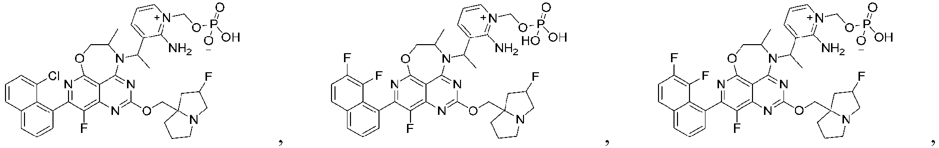 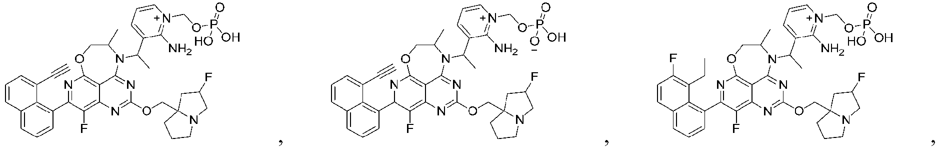 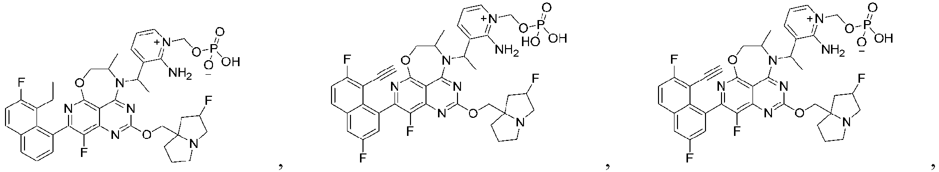 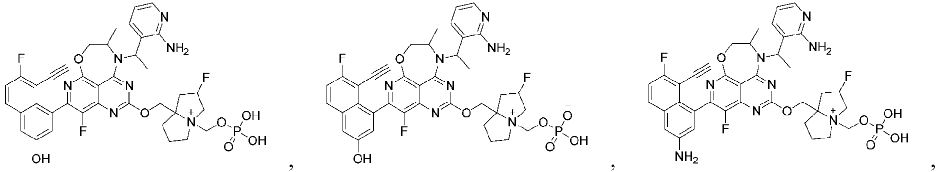 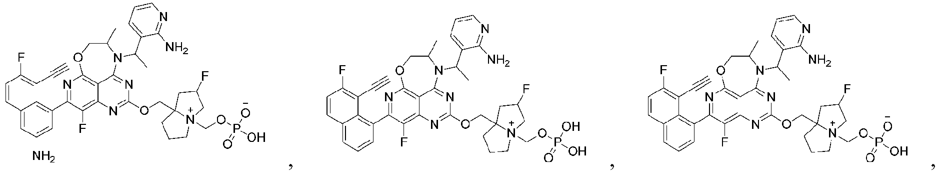 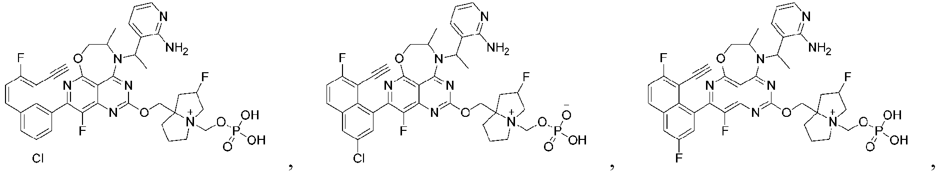 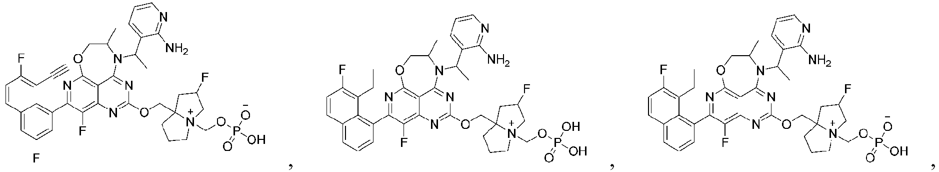 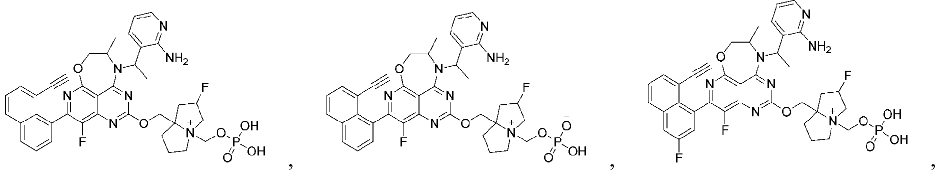 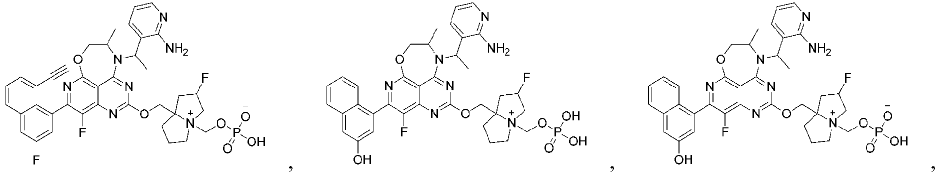 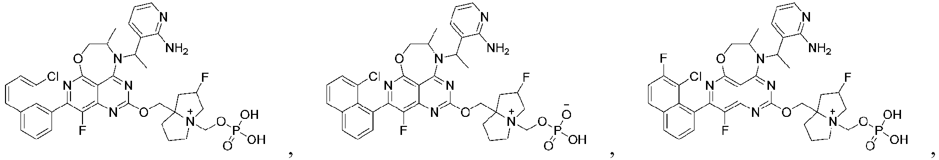 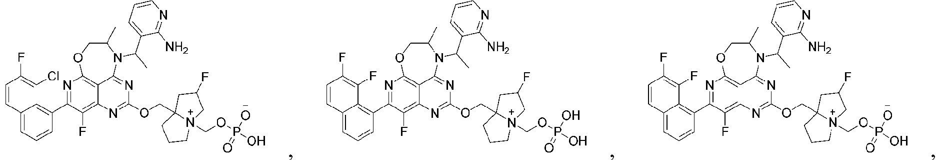 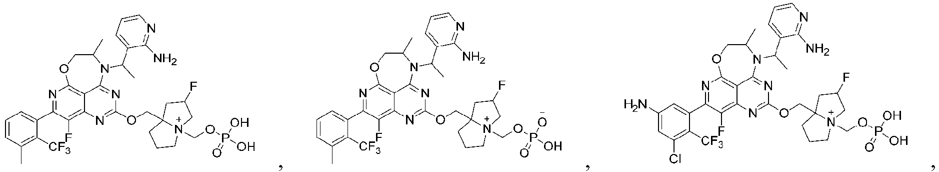 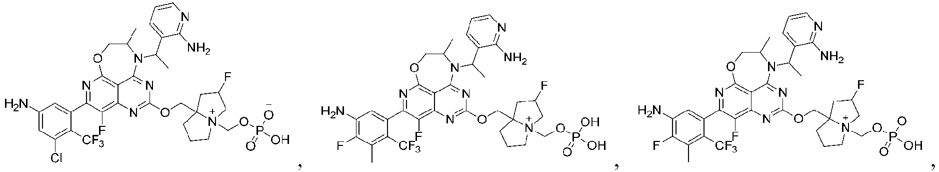 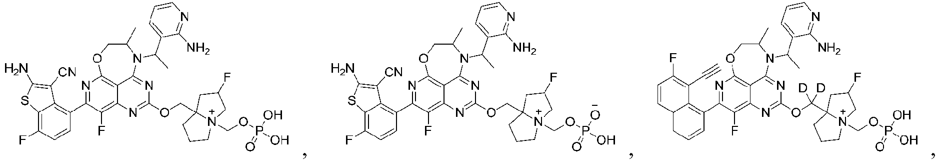 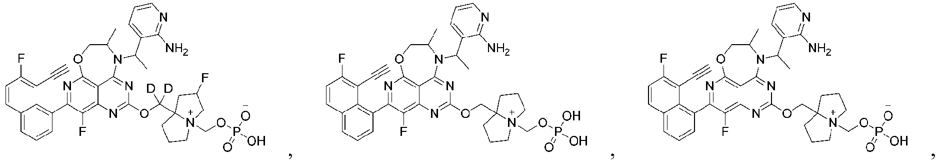 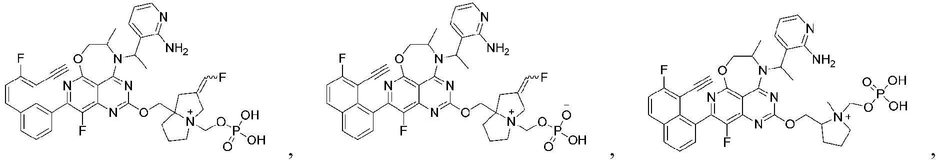 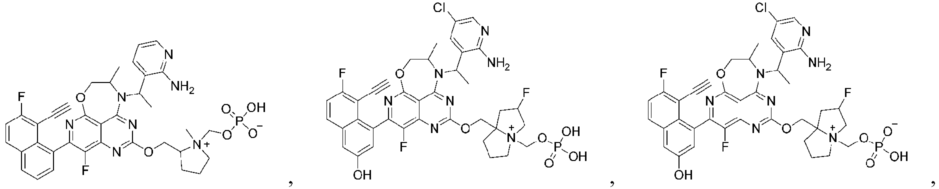 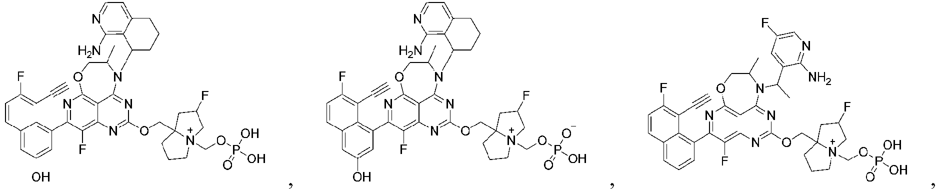 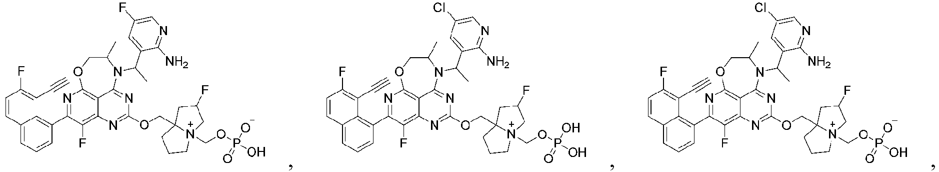 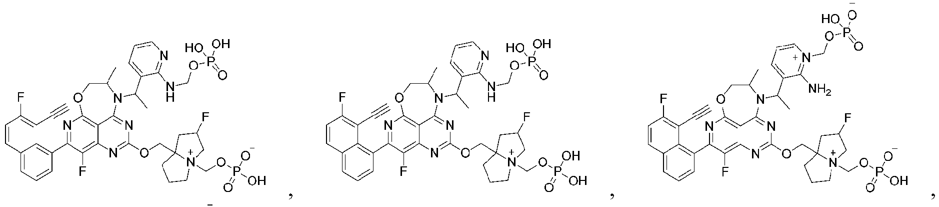 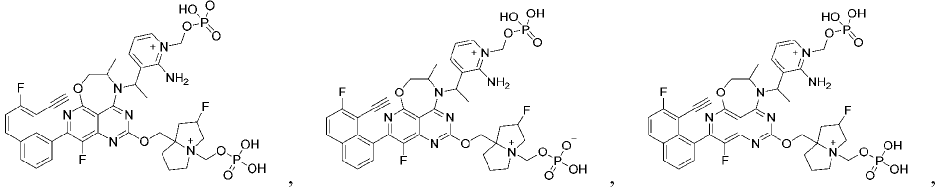 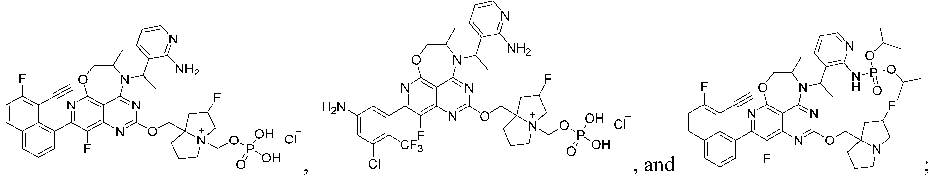
or being selected from the group consisting of the following compounds or pharmaceutically acceptable salts thereof: 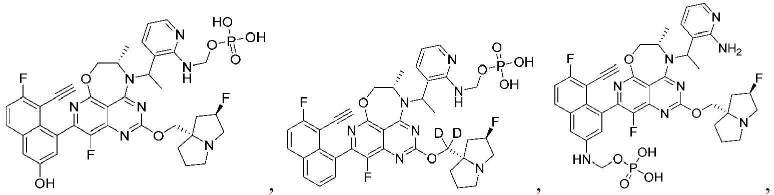 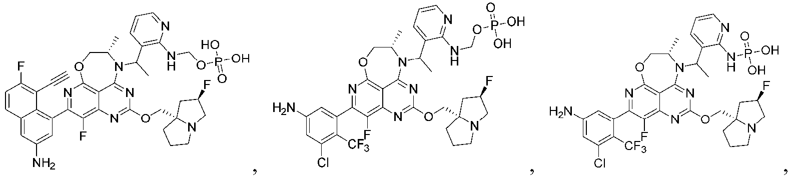 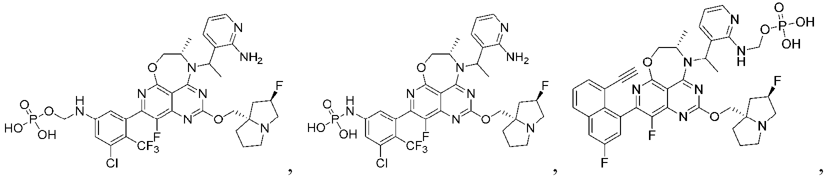 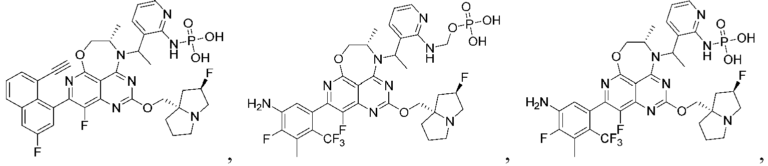 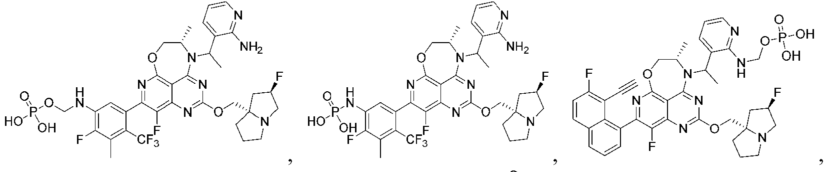 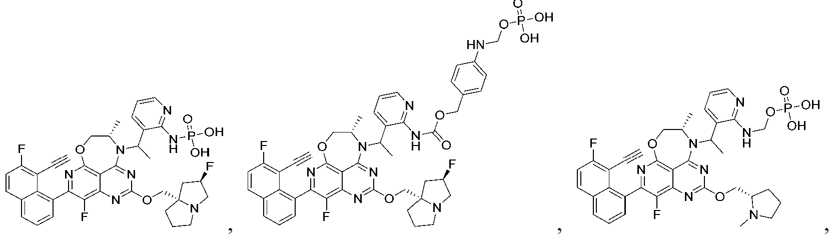 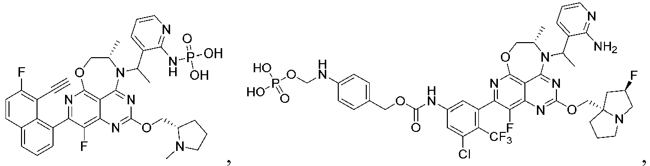 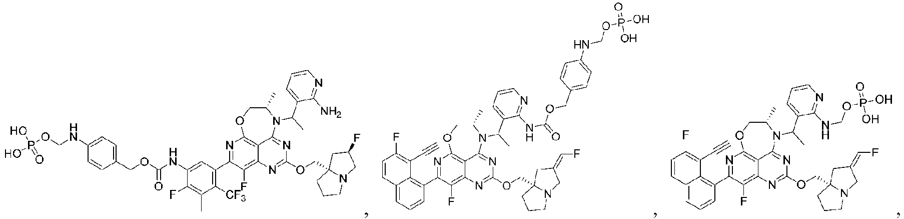 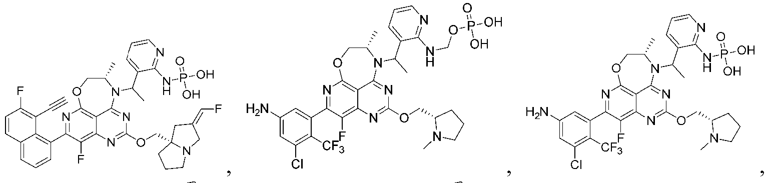 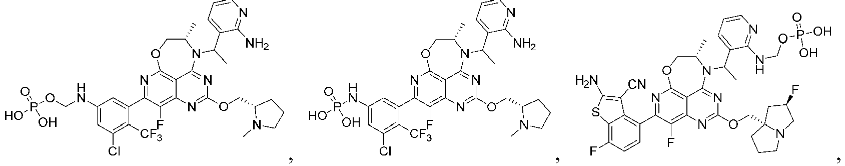 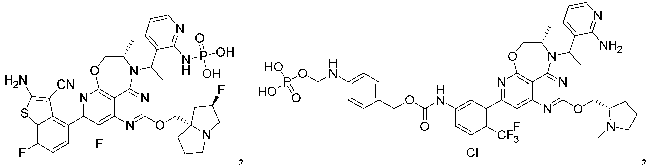 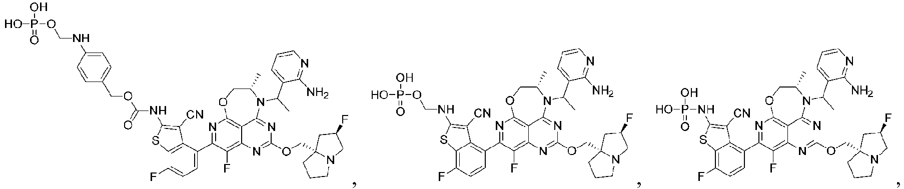 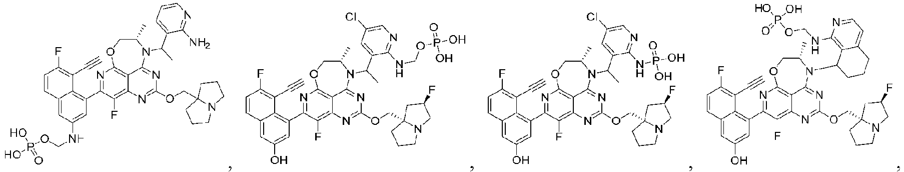 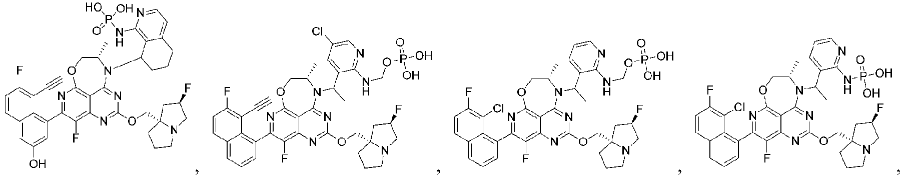 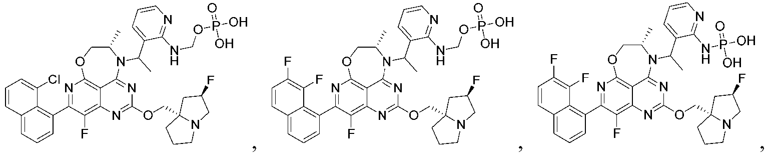 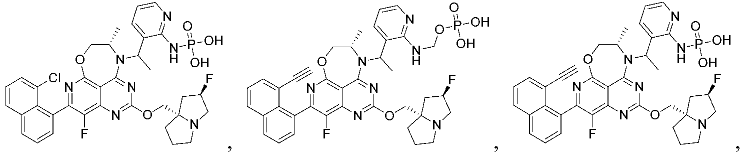 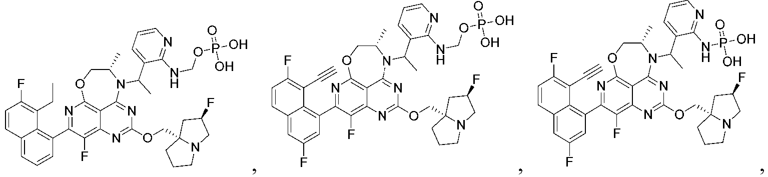 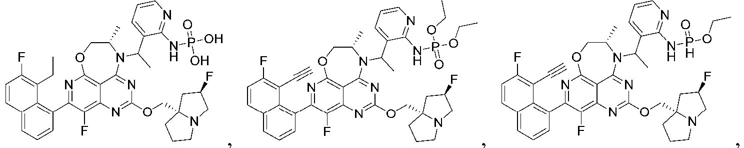 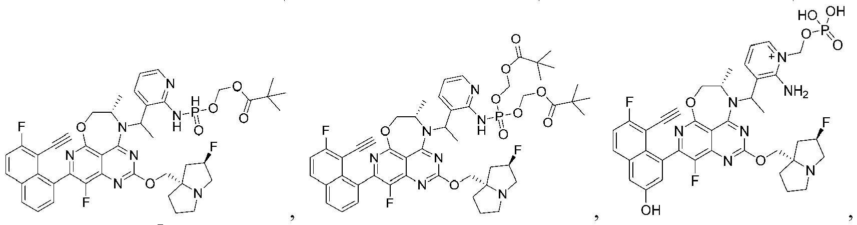 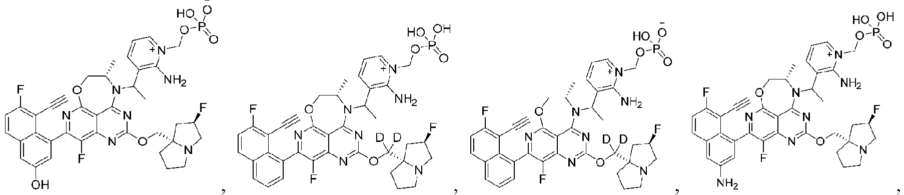 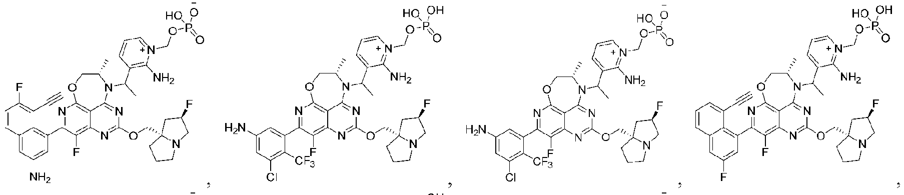 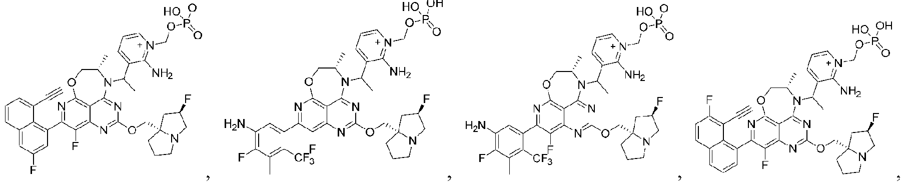 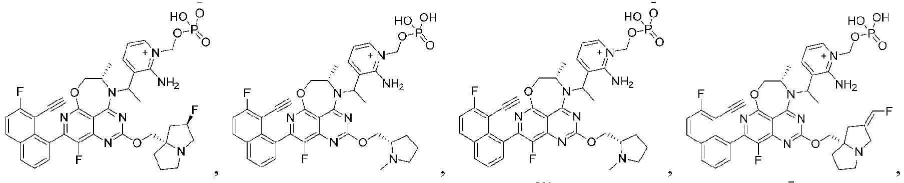 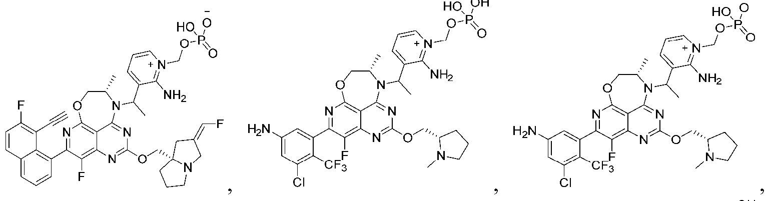 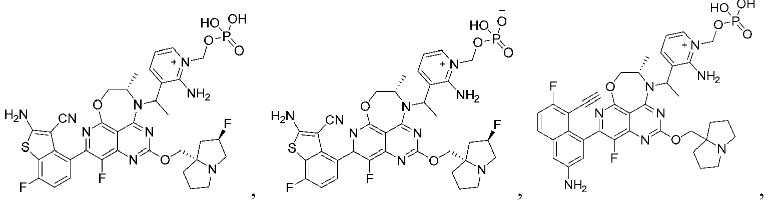 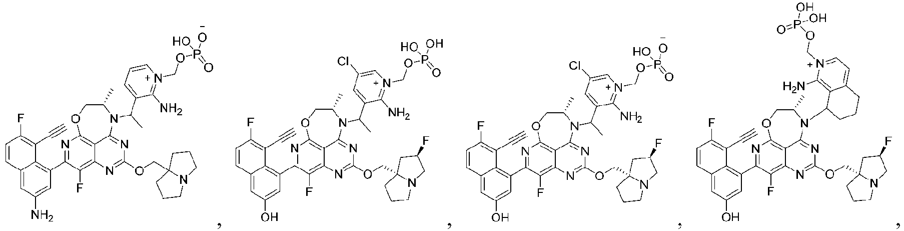 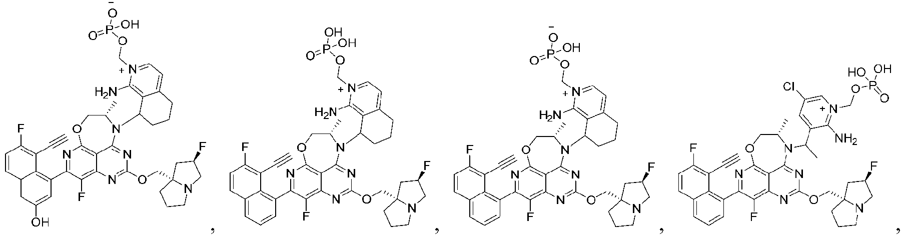 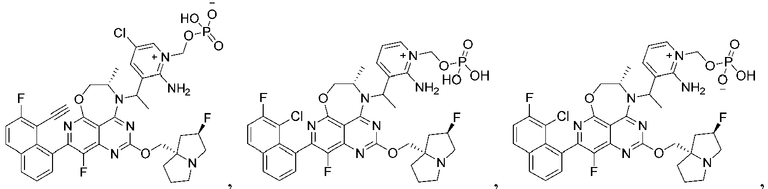 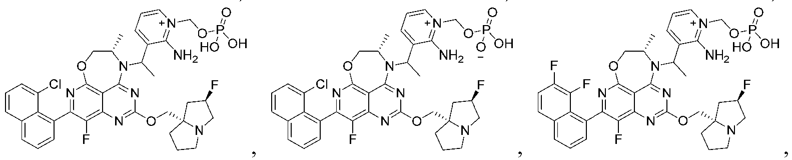 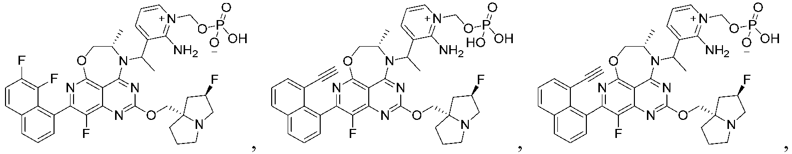 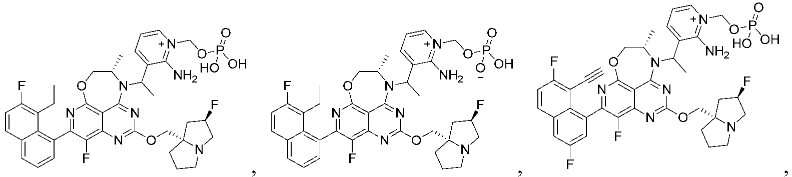 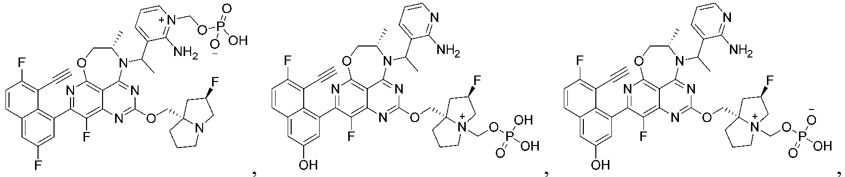 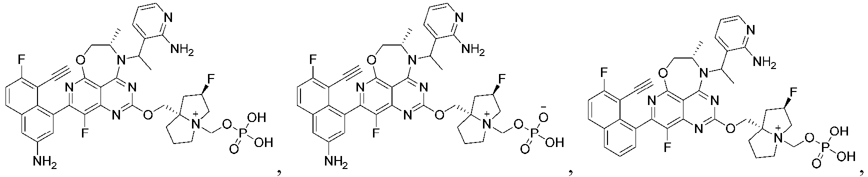 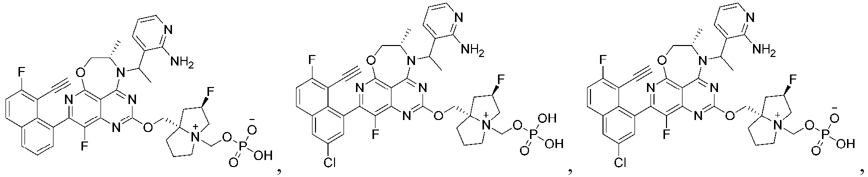 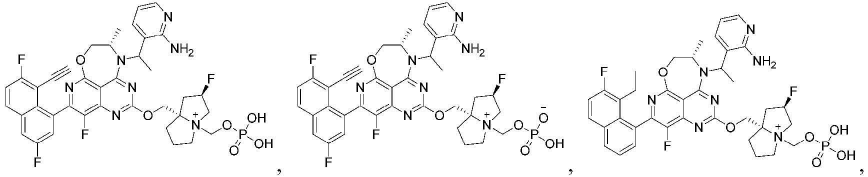 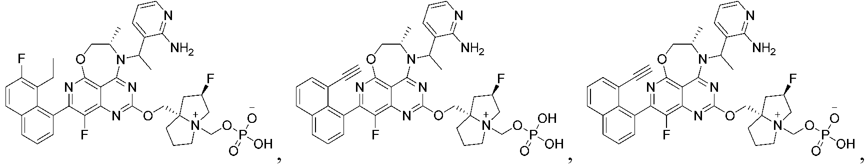 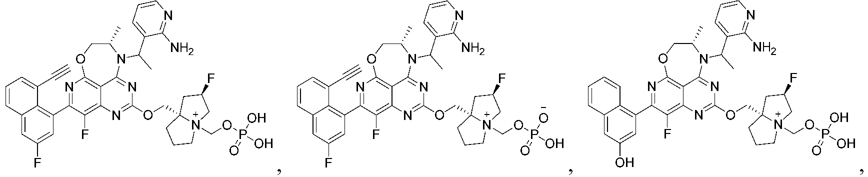 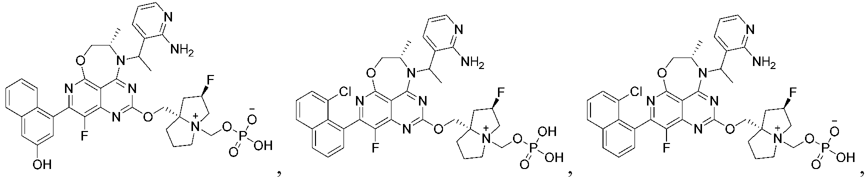 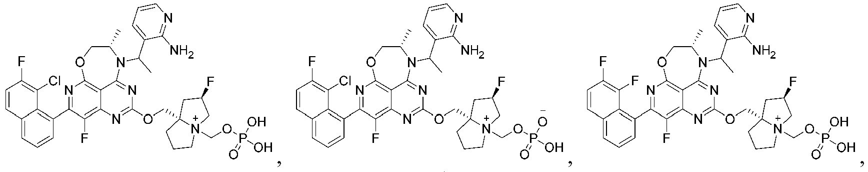 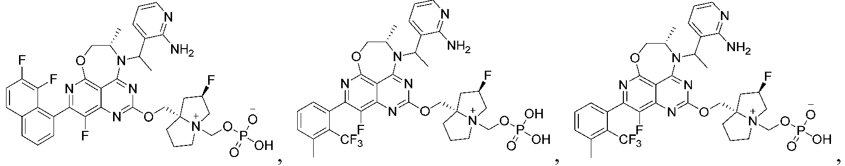 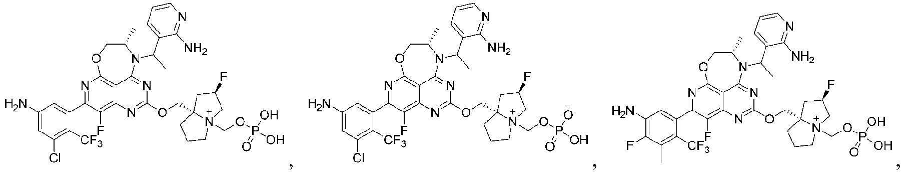 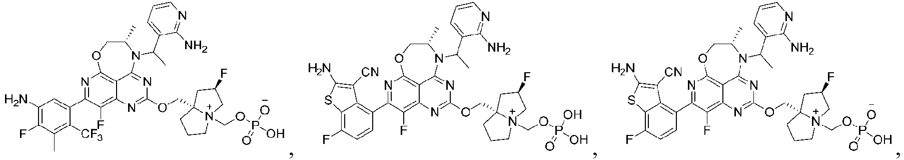 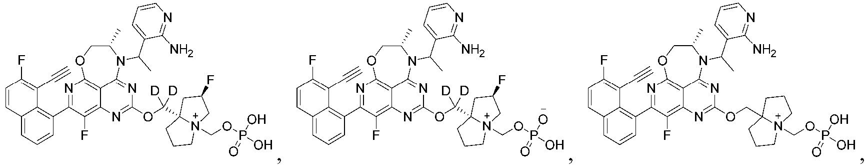 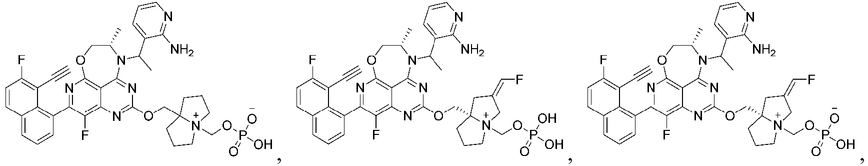 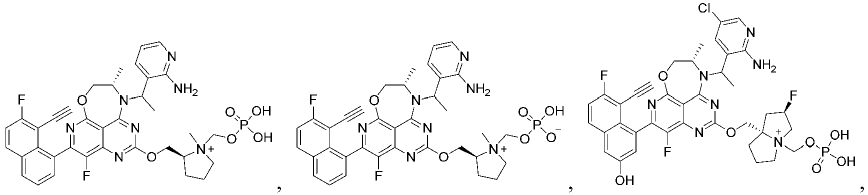 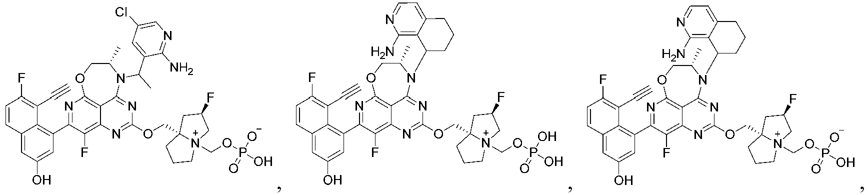 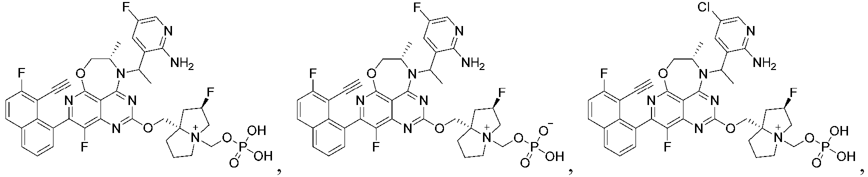 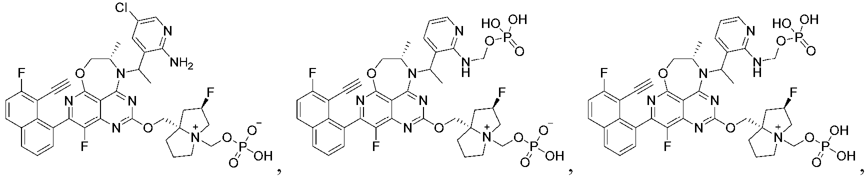 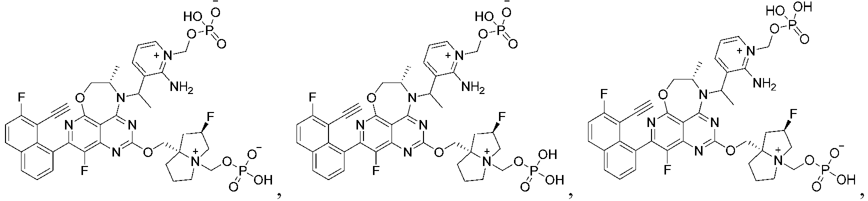 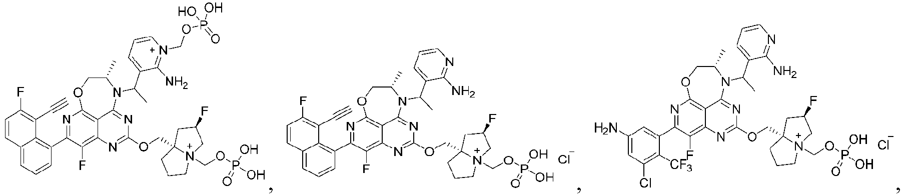 and 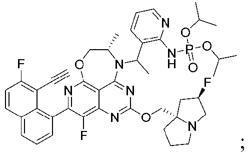
or being selected from the group consisting of the following compounds or pharmaceutically acceptable salts thereof: 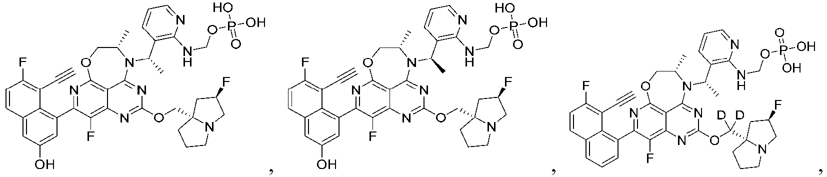 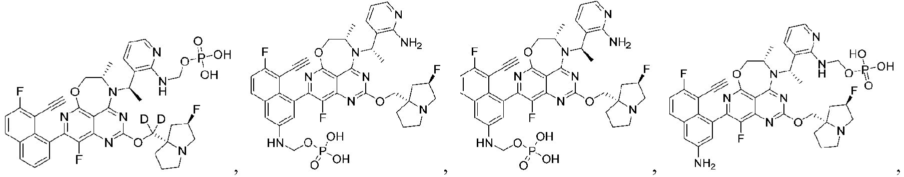 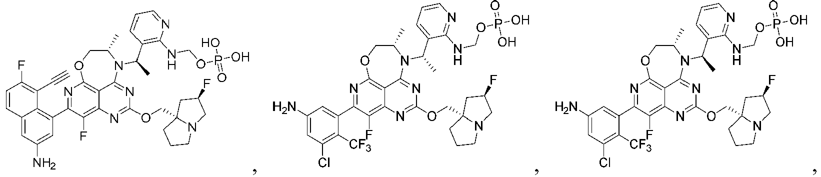 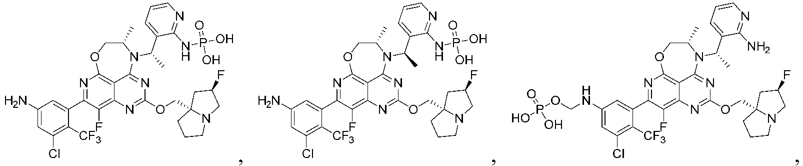 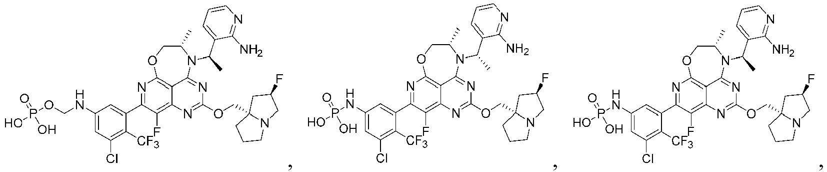 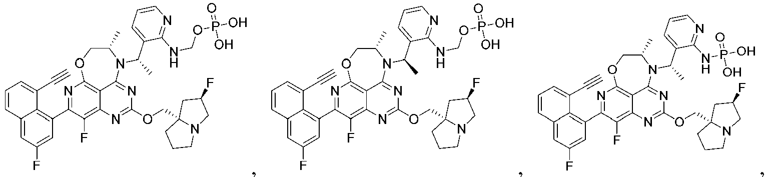 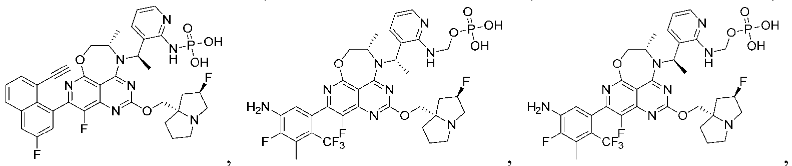 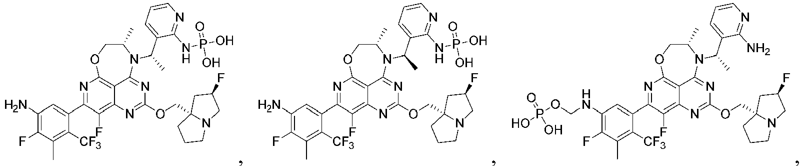 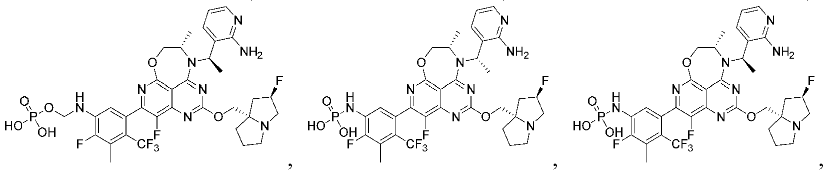 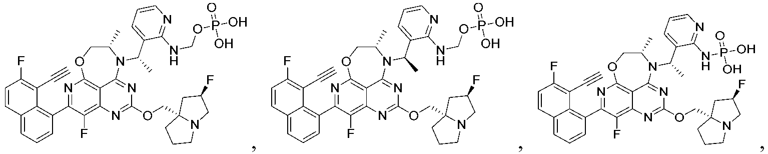 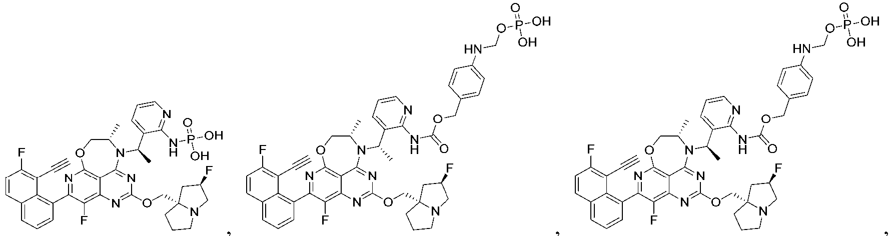 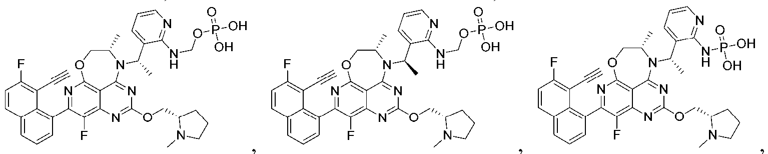 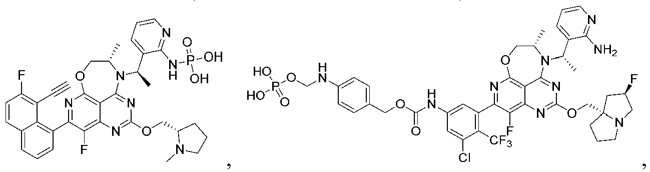 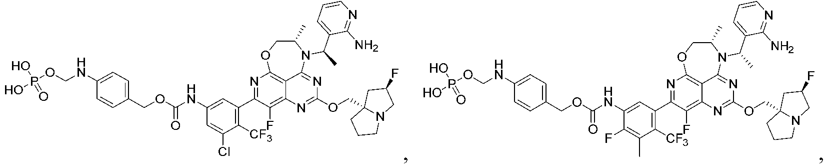 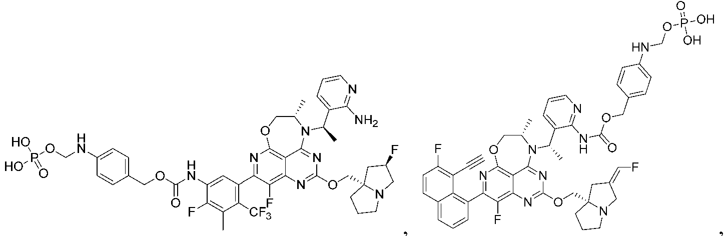 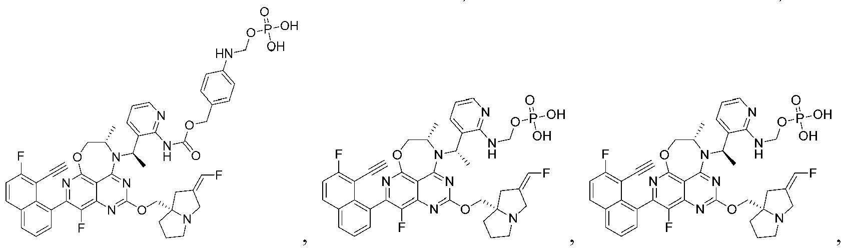 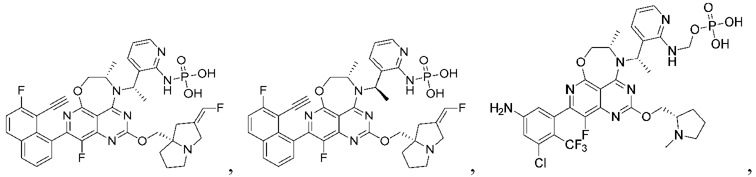 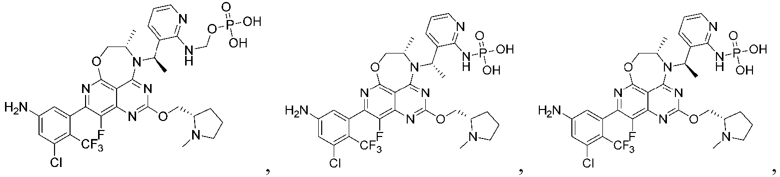 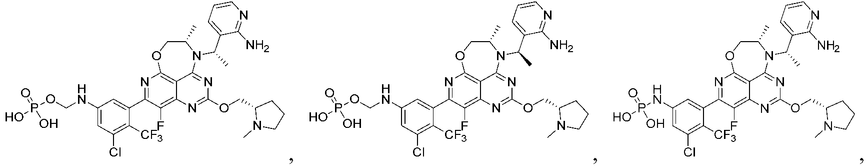 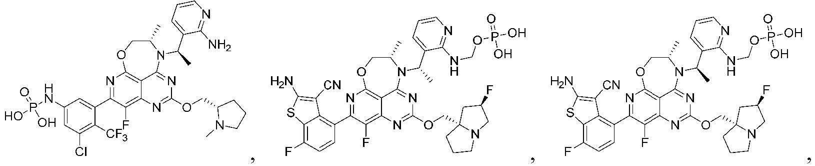 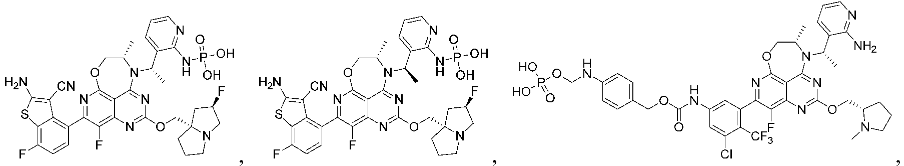 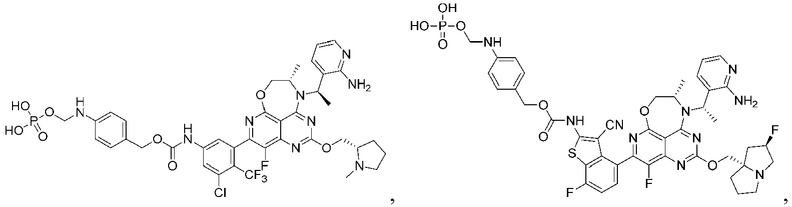 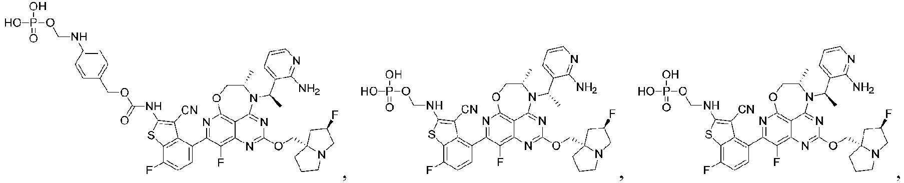 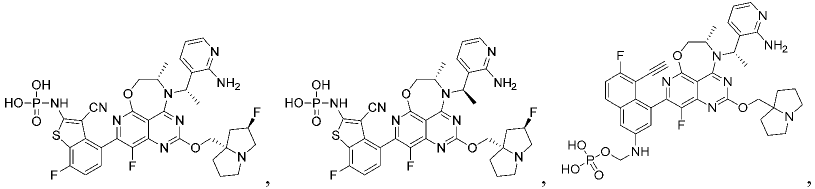 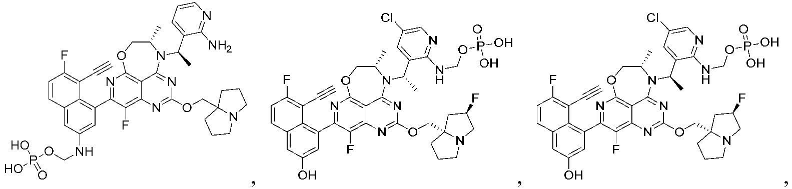 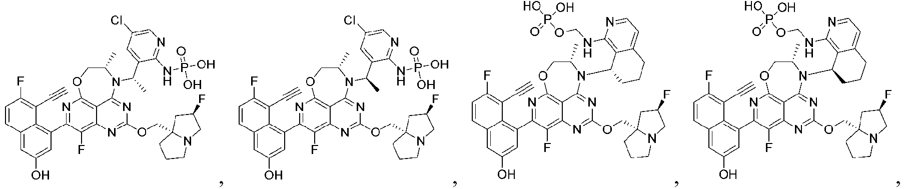 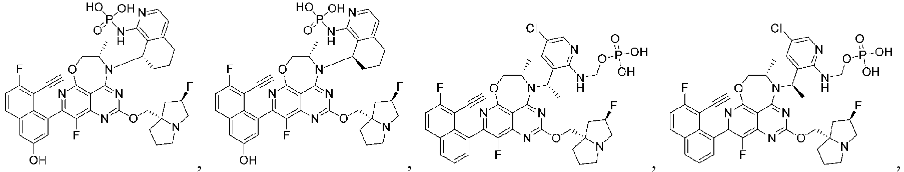 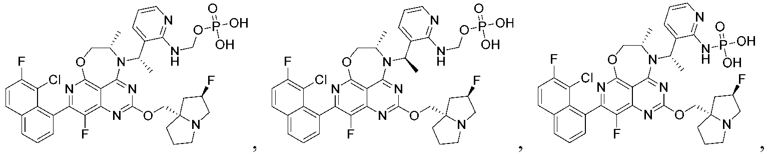 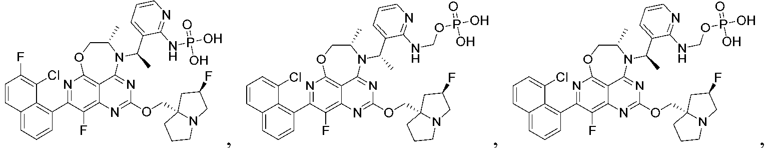 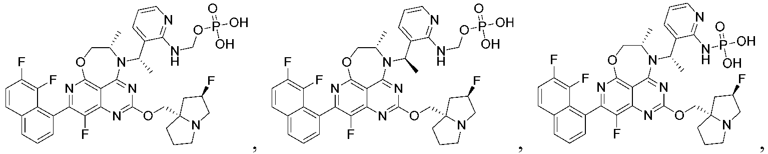 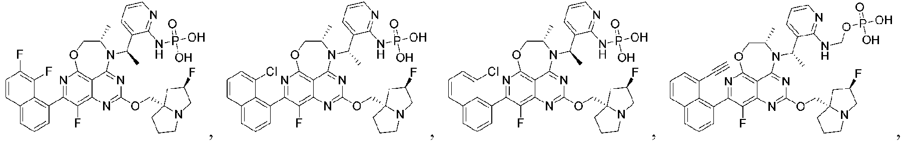 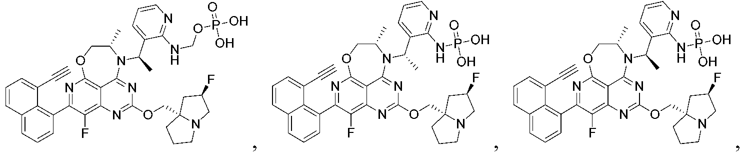 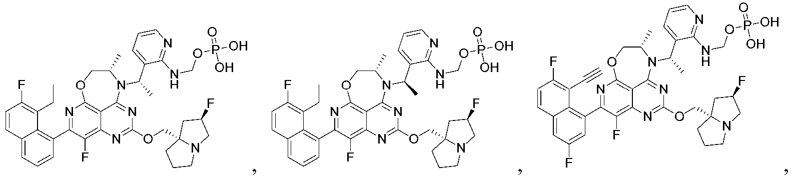 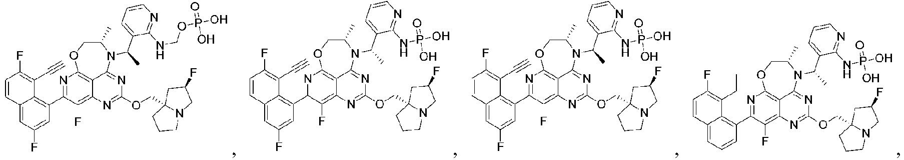 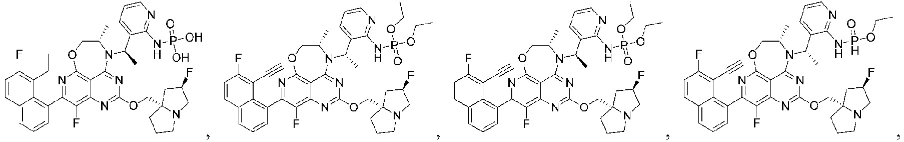 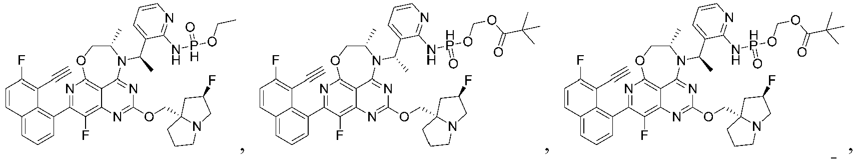 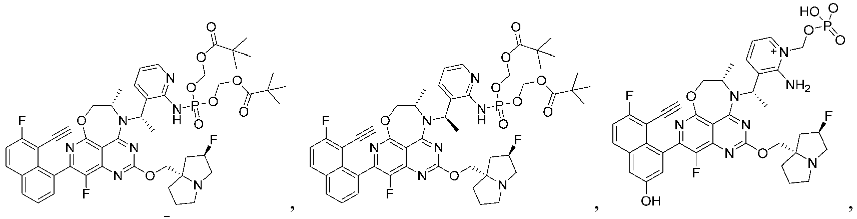 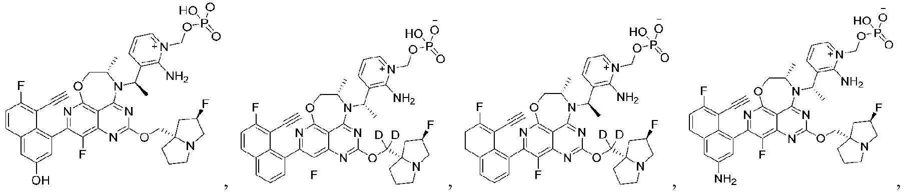 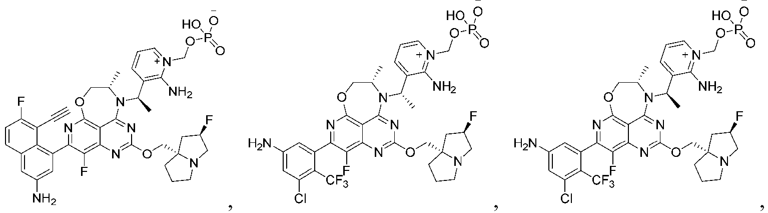 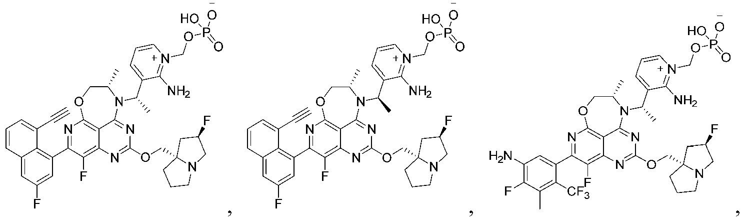 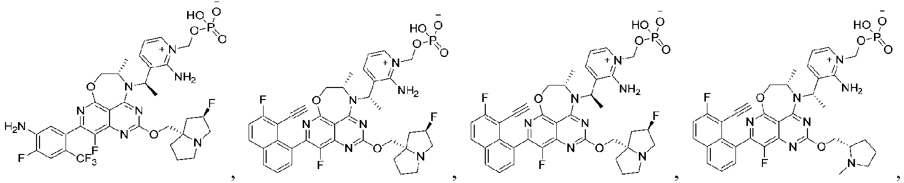 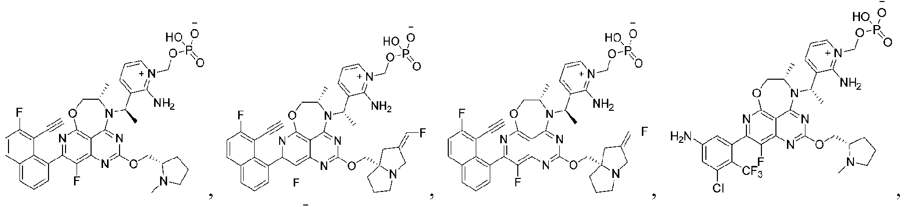 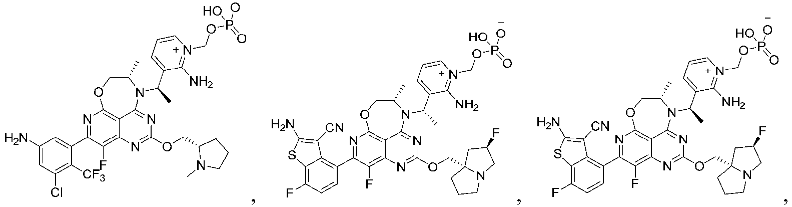 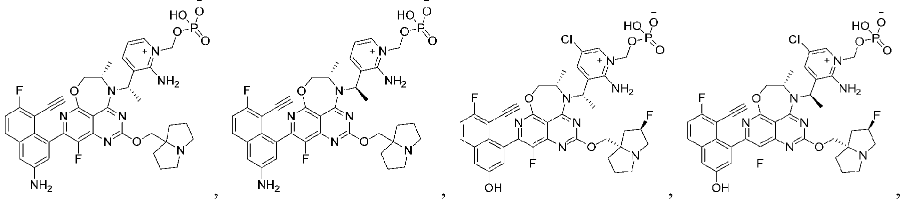 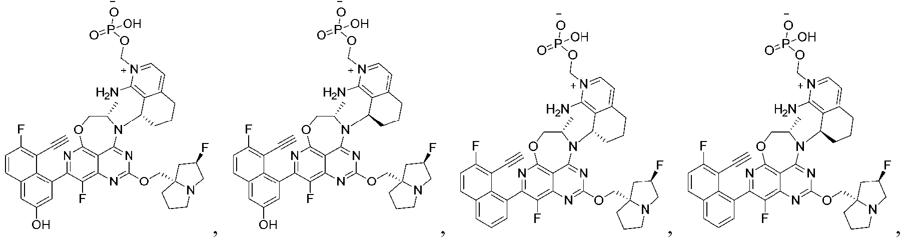 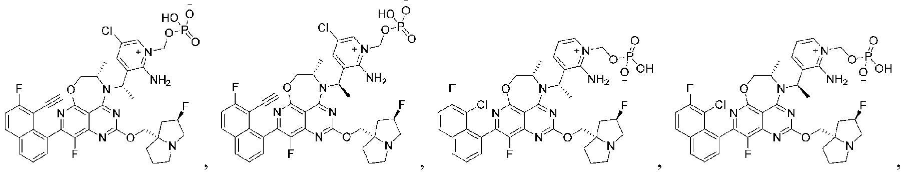 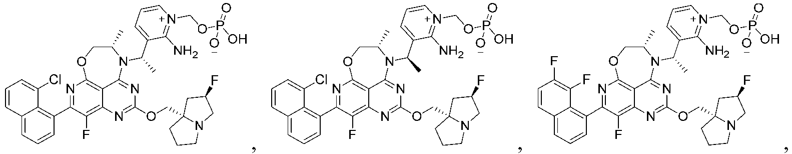 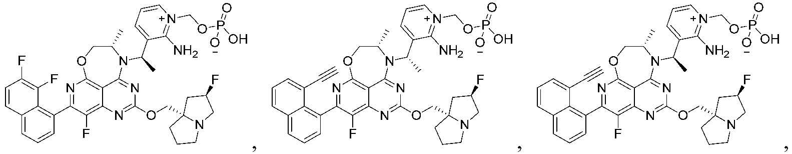 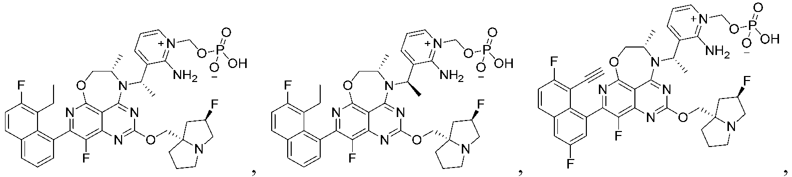 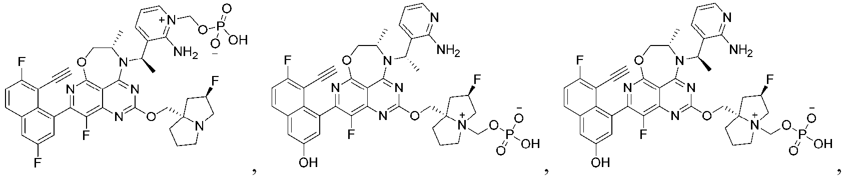 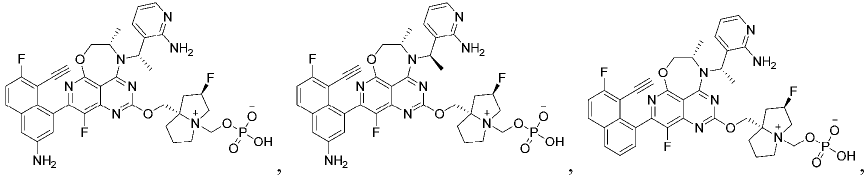 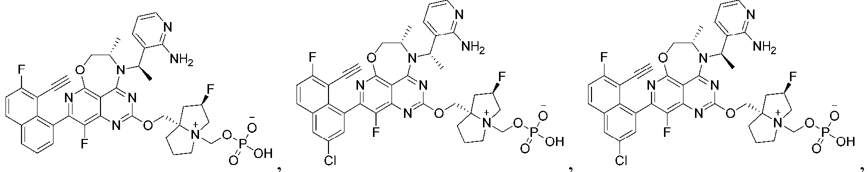 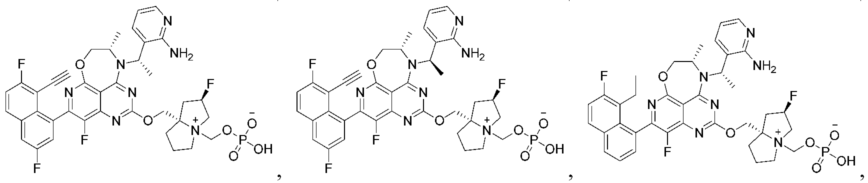 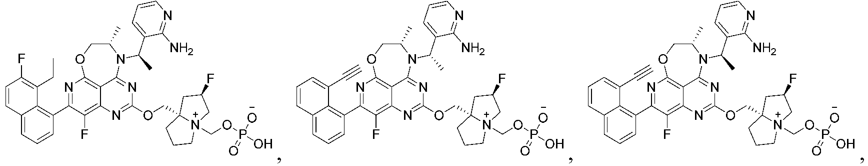 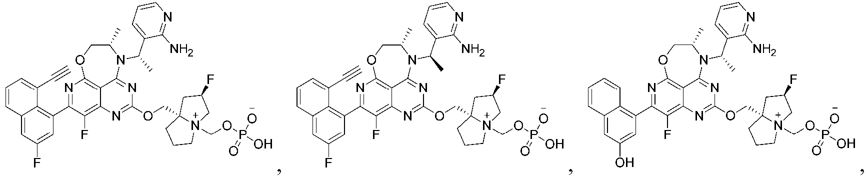 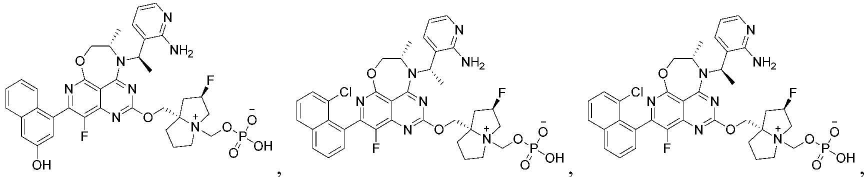 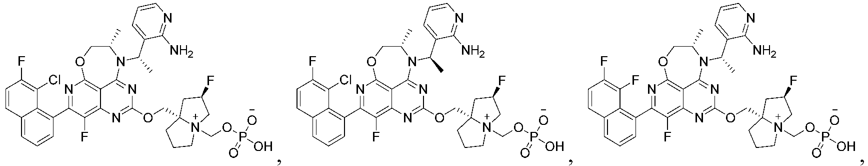 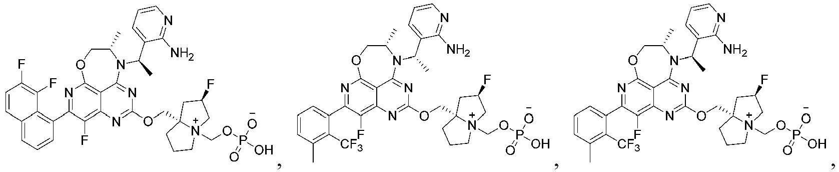 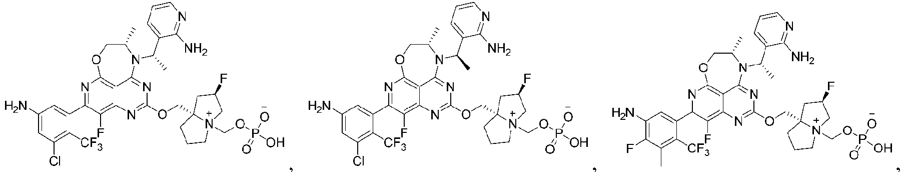 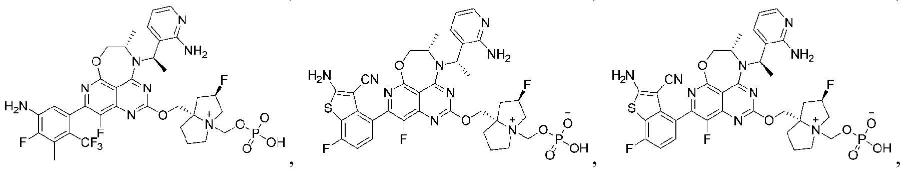 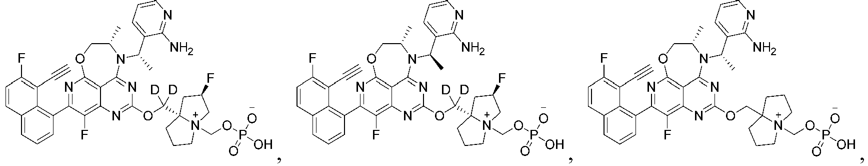 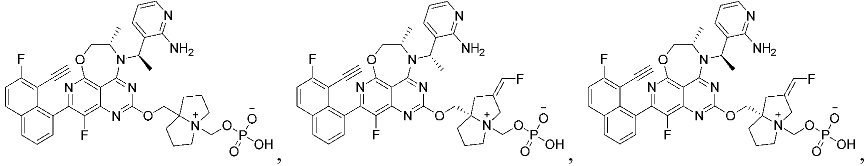 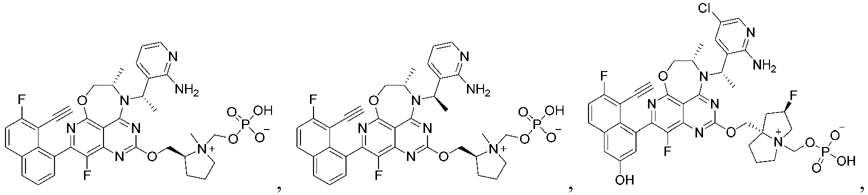 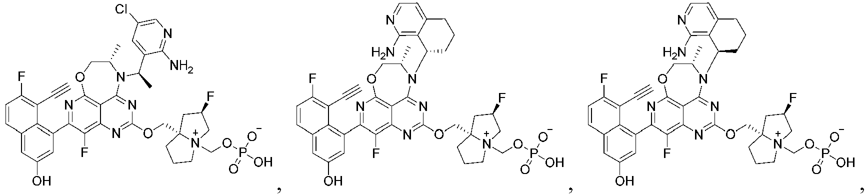 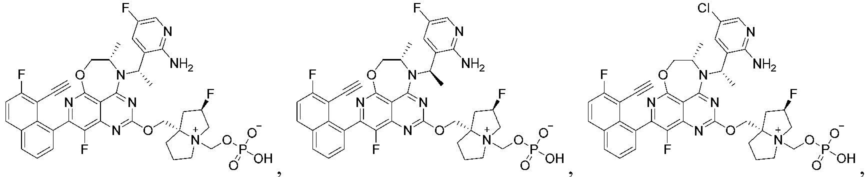 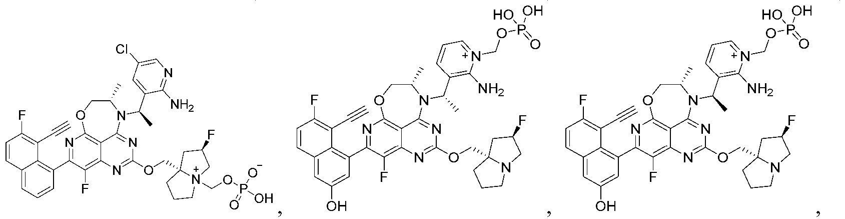 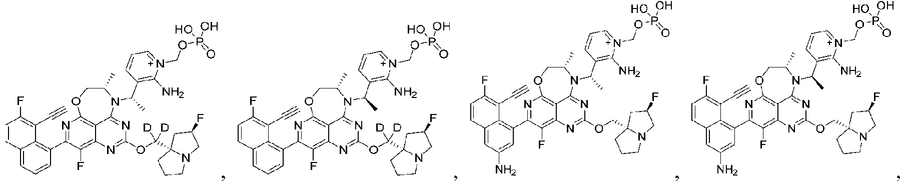 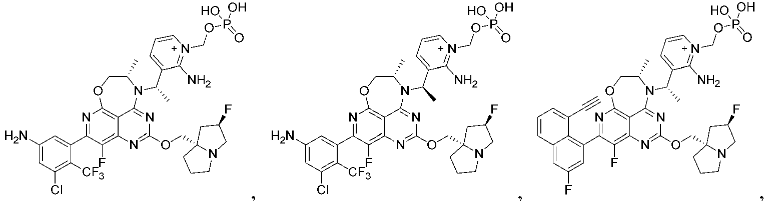 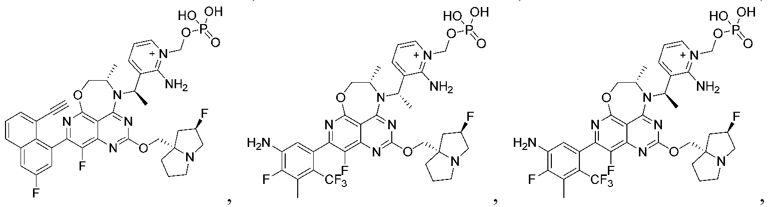 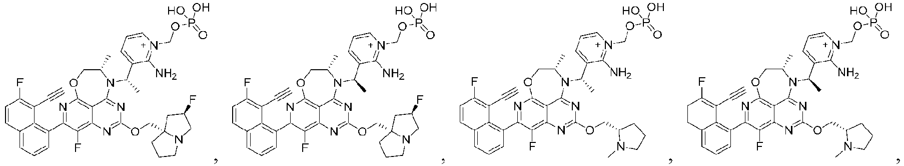 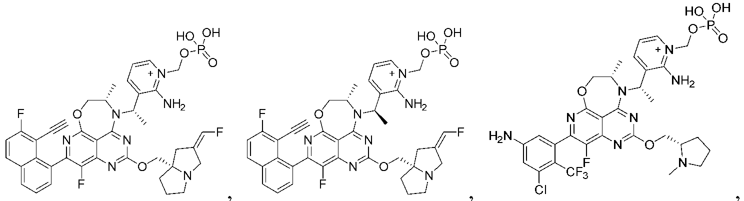 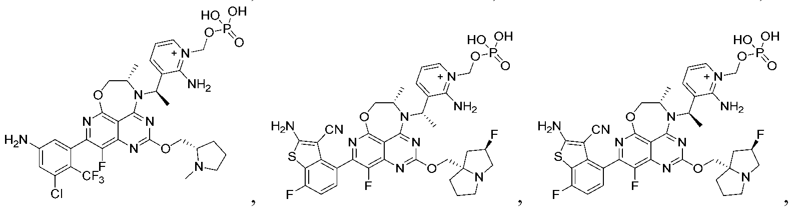 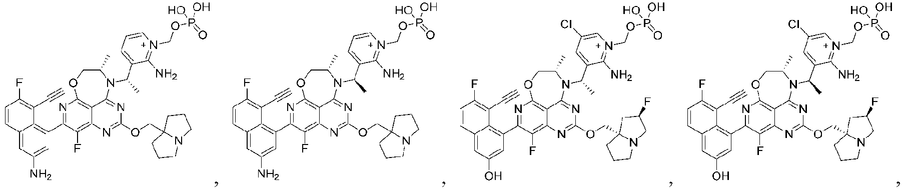 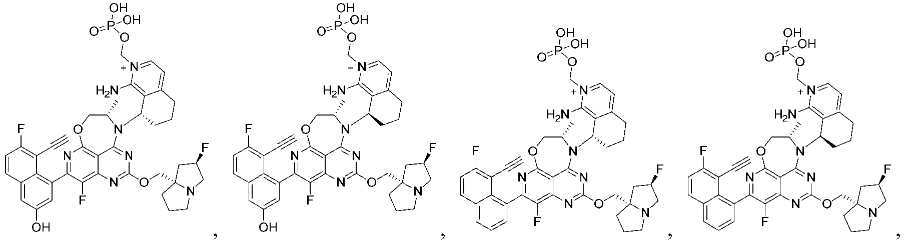 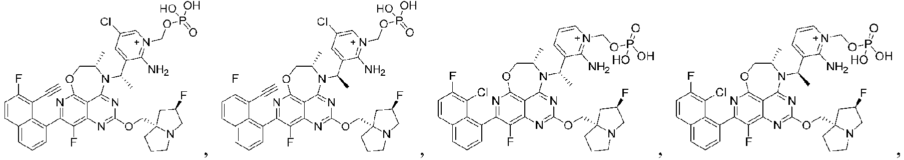 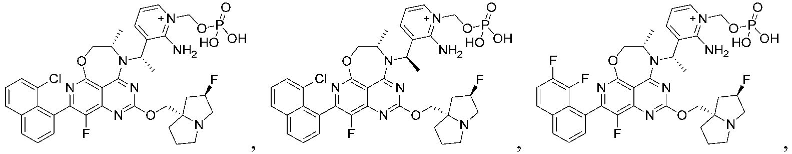 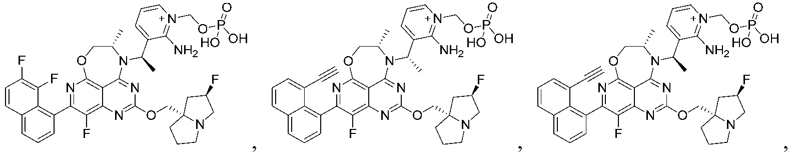 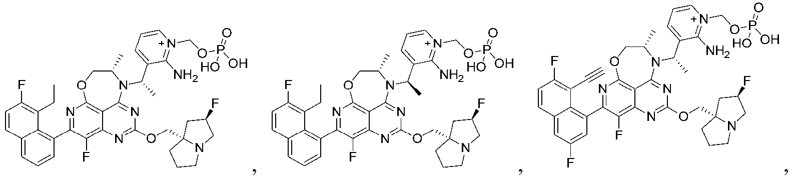 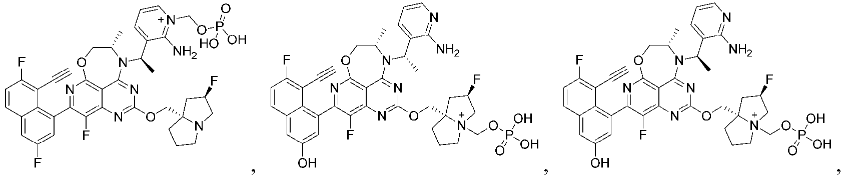 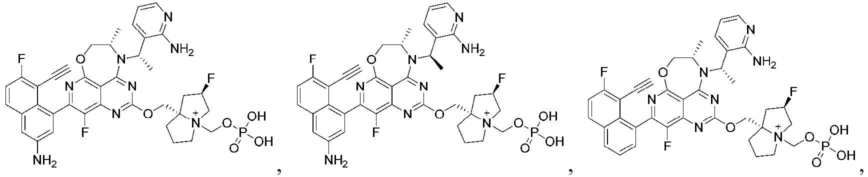 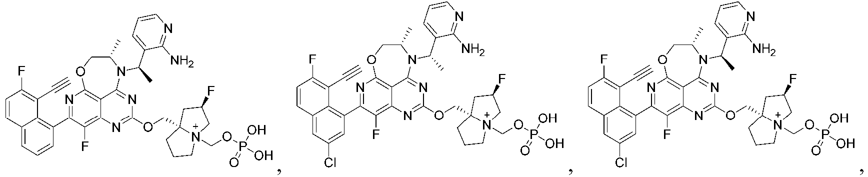 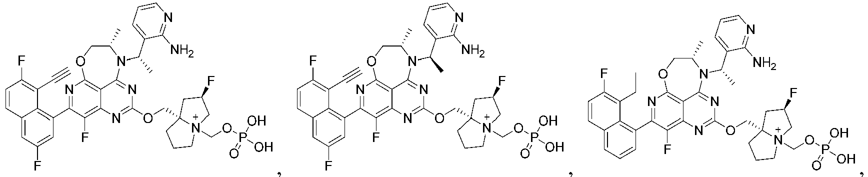 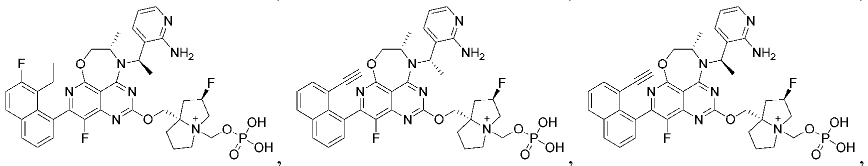 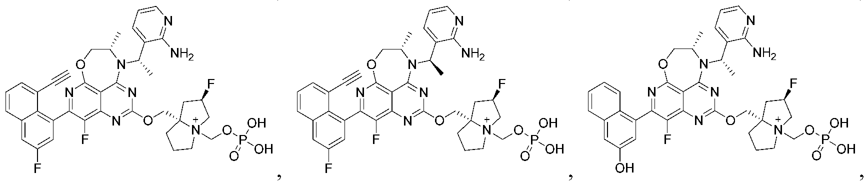 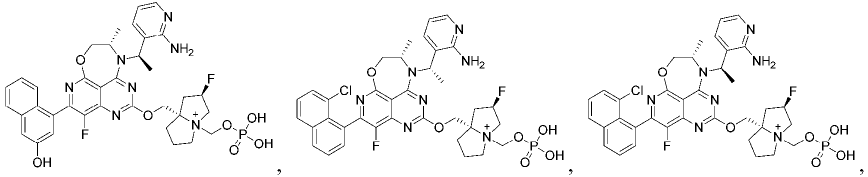 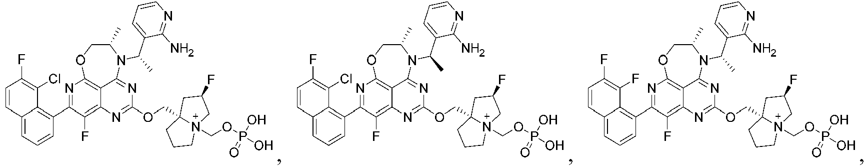 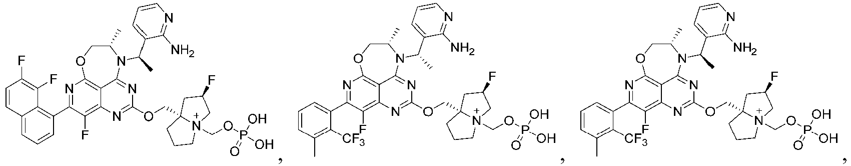 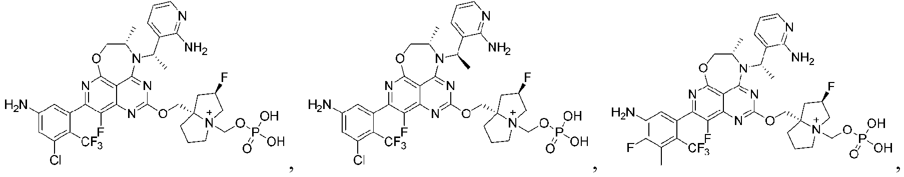 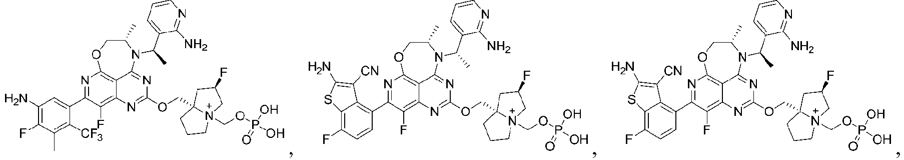 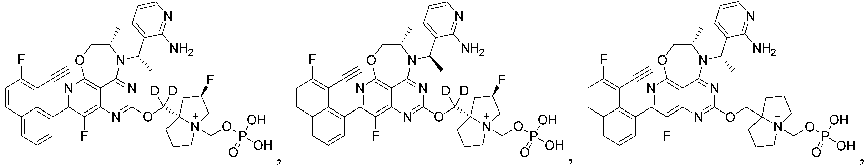 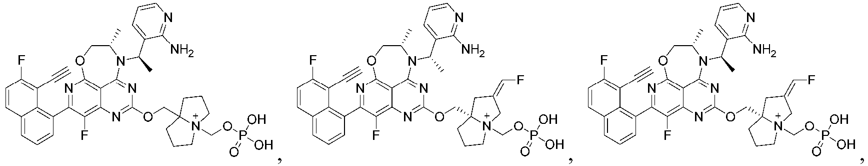 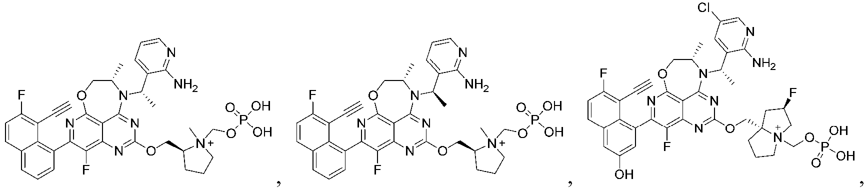 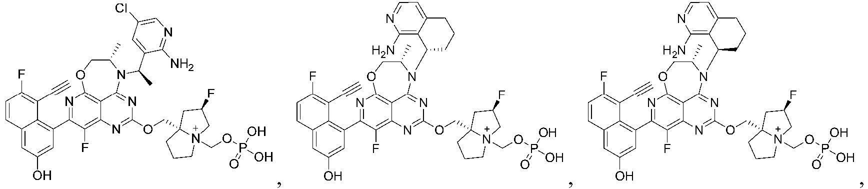 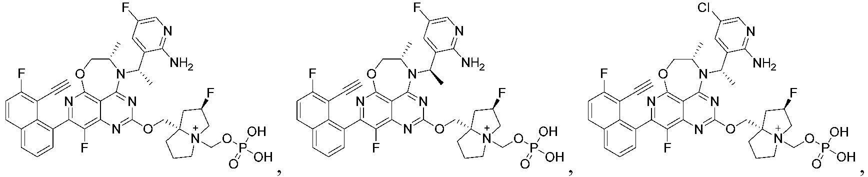 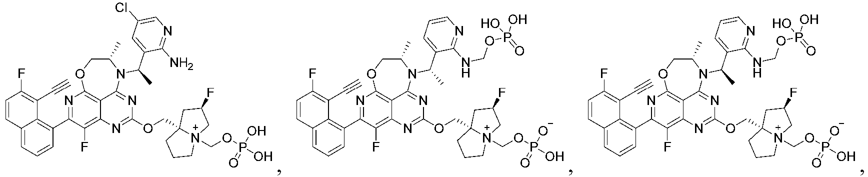 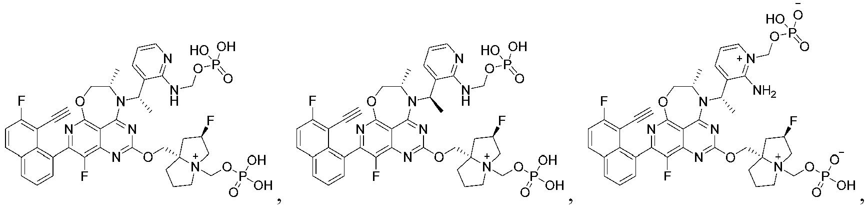 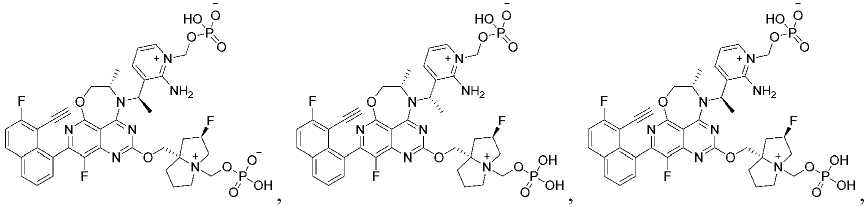 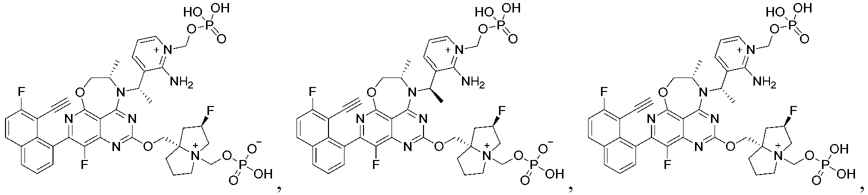 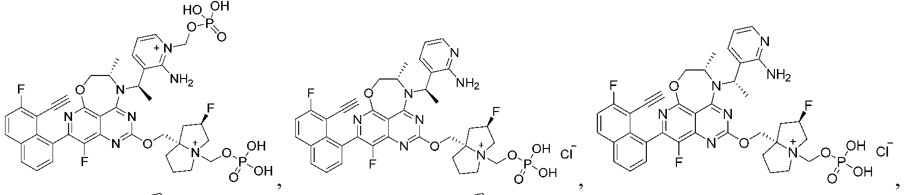 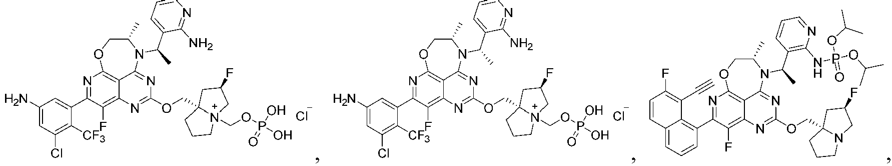 and

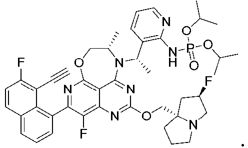

14. A pharmaceutical composition, comprising the compound of formula (I) or the pharmaceutically acceptable salt thereof according to any one of claims 1-13, and optionally further comprising a pharmaceutically acceptable excipient.

15. Use of the compound of formula (I) or the pharmaceutically acceptable salt thereof according to any one of claims 1-13, or the pharmaceutical composition according to claim 14 for preparing a medicament for treating a disease.
